# EUROPEAN PATENT APPLICATION

(11) **EP 3 508 480 A1**
(43) Date of publication of application: **10.07.2019**
(21) Application number: 18150628.8
(22) Date of filing: 08.01.2018
(51) Int. Cl.: C07D 257/06, C07D 249/14, C07D 271/08, C07D 271/113, A01N 43/647, A01N 43/713, A01N 43/82

(54) **BENZAMIDE COMPOUNDS AND THEIR USE AS HERBICIDES**

(71) Applicant: BASF SE, 67056 Ludwigshafen am Rhein (DE)
(72) Inventor: KORDES, Markus, 67056 Ludwigshafen (DE); ZIERKE, Thomas, 67056 Ludwigshafen (DE); SEITZ, Thomas, 67056 Ludwigshafen (DE); NIELSON, Ryan Louis, 67117 Limburgerhof (DE)
(74) Representative: Reitstötter Kinzebach

(57) **Abstract**

The present invention relates to benzamide compounds of formual (I), the N-oxides and the salts thereof and to compositions comprising the same. The invention also relates to the use of the benzamide compounds, the N-oxides or the salts thereof and to the use of the compositions comprising such compounds for controlling unwanted vegetation. 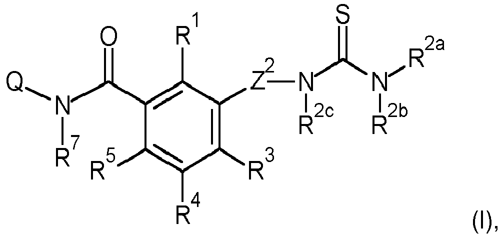 In formula I,
Q
is selected from the group consisting of radicals of the formulae Q¹, Q², Q³ and Q⁴; 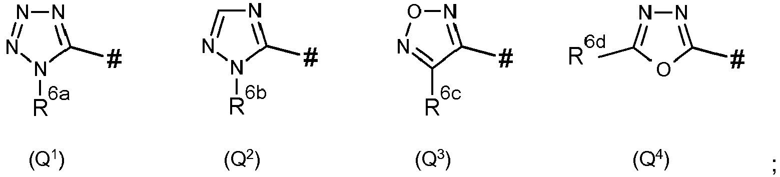 where # in formulae Q¹, Q², Q³ and Q⁴ indicates the point of attachment to the nitrogen atom;
Z²
is a covalent bond or a bivalent radical selected from C₁-C₄-alkanediyl and C₁-C₄-alkanediyl-O-C₁-C₄-alkanediyl, where in the last two mentioned radicals the C₁-C₄-alkanediyl groups are linear and where C₁-C₄-alkanediyl is unsubstituted, or partly or completely fluorinated or substituted by 1, 2, 3 or 4 groups R^{z};
and R¹, R^{2a}, R^{2b}, R^{2c}, R³, R⁴, R⁵, R^{6a}, R^{6b}, R^{6c}, R^{6d}, R⁷ and R^{z} are as defined in the claims.

## Description

The present invention relates to benzamide compounds, the N-oxides and the salts thereof and to compositions comprising the same. The invention also relates to the use of the benzamide compounds, the N-oxides or the salts thereof and to the use of the compositions comprising such compounds for controlling unwanted vegetation. Furthermore, the invention relates to methods of applying such compounds.

For the purposes of controlling unwanted vegetation, especially in crops, there is an ongoing need for new herbicides which have high activities and selectivities, preferably together with a substantial lack of toxicity for humans and animals.

WO 2012/028579 describes N-(tetrazol-4-yl)- and N-(triazol-3-yl)arylcarboxylic acid amide compounds, which carry 3 substituents in the 2-, 3- and 4-positions of the aryl ring, and their use as herbicides.

WO2013/017559 describes N-(tetrazol-5-yl)- and N-(triazol-5-yl)arylcarboxylic acid amides, which carry 3 substituents in the 2-, 3- and 4-positions of the aryl ring, and their use as herbicides.

WO2015/052153 describes N-(tetrazol-5-yl)- and N-(triazol-5-yl)arylcarboxylic acid amides, which carry at least 2 substituents in the 2- and 6-positions of the aryl ring and a further substituent on the amide nitrogen, and their use as herbicides.

WO2017/102275 describes N-(tetrazol-5-yl)- and N-(triazol-5-yl)benzamides, which carry a urea group in 3-position and two further substituents in the 2- and 6-positions of the aryl ring, and their use as herbicides.

The compounds of the prior art often suffer from insufficient herbicidal activity in particular at low application rates and/or unsatisfactory selectivity resulting in a low compatibility with crop plants.

Accordingly, it is an object of the present invention to provide further benzamide compounds having a strong herbicidal activity, in particular even at low application rates, a sufficiently low toxicity for humans and animals and/or a high compatibility with crop plants. The benzamide compounds should also show a broad activity spectrum against a large number of different unwanted plants.

These and further objectives are achieved by the compounds of formula I defined below, their N-oxides and their agriculturally suitable salts.

Therefore, in a first aspect the present invention relates to compounds of formula I 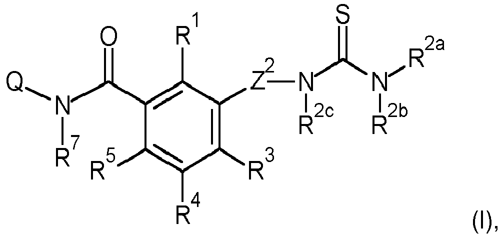 wherein
Q is selected from the group consisting of radicals of the formulae Q¹, Q², Q³ and Q⁴; 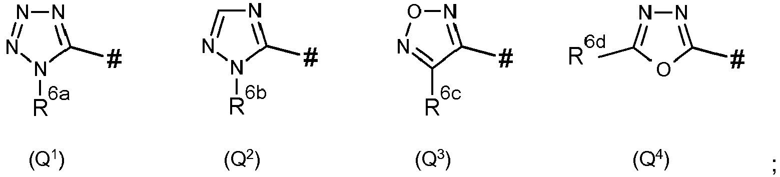 where # in formulae Q¹, Q², Q³ and Q⁴ indicates the point of attachment to the nitrogen atom;
   - R¹: is selected from the group consisting of cyano, halogen, nitro, C₁-C₈-alkyl, C₂-C₈-alkenyl, C₂-C₈-alkynyl, C₁-C₈-haloalkyl, C₁-C₄-alkoxy-C₁-C₄-alkyl, C₁-C₄-haloalkoxy-C₁-C₄-alkyl, C₁-C₈-alkoxy, C₁-C₄-alkoxy-C₁-C₄-alkoxy-Z¹-, C₁-C₆-haloalkoxy, R^{1b}-S(O)ₖ-Z¹-;
   - R^{2a}: is selected from the group consisting of hydrogen, C₁-C₆-alkyl, C₁-C₆-alkoxy, C₂-C₆-alkenyl, C₂-C₆-alkynyl, C₁-C₆-alkylamino, di-C₁-C₆-alkylamino, C₃-C₁₀-cycloalkyl and C₃-C₁₀-cycloalkyl-C₁-C₄-alkyl, where the alkyl, alkoxy, alkenyl, alkynyl and cycloalkyl parts of the eight aforementioned radicals are unsubstituted, partially or completely halogenated, or substituted by 1 or 2 radicals R^{2f} and where the cycloalkyl parts may also be fused to a benzene ring, which is unsubstituted or substituted by 1, 2, 3 or 4 radicals R²¹, which are identical or different,
   - R^{2a}: may also be selected from phenyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups R²¹, which are identical or different;
   - R^{2b}: is selected from the group consisting of hydrogen, C₁-C₆-alkyl, C₂-C₆-alkenyl, C₂-C₆-alkynyl, C₃-C₇-cycloalkyl, C₃-C₇-cycloalkyl-C₁-C₄-alkyl, where the alkyl, alkenyl, alkynyl and cycloalkyl parts of the five aforementioned radicals are unsubstituted, partially or completely halogenated, or substituted by 1 or 2 radicals R^{2f};
   - R^{2b}: may also be selected from phenyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups R²¹, which are identical or different; or
   - R^{2a}, R^{2b}: together with the nitrogen atom, to which they are bound may form a 4-, 5-, 6-, 7- or 8-membered, saturated or partially unsaturated heterocyclic radical, which may carry as a ring member a further heteroatom selected from O, S and N and where the heterocyclic radical is unsubstituted or carries 1, 2, 3 or 4 groups R^{2d} or carries 1 group R^{2e} and 0, 1, 2 or 3 groups R^{2d};
   - R^{2c}: is selected from the group consisting of hydrogen, C₁-C₆-alkyl, C₂-C₆-alkenyl, C₂-C₆-alkynyl, C₁-C₆-alkylamino, di-C₁-C₆-alkylamino, C₃-C₁₀-cycloalkyl and C₃-C₁₀-cycloalkyl-C₁-C₄-alkyl, where the alkyl, alkenyl, alkynyl and cycloalkyl parts of the seven aforementioned radicals are unsubstituted, partially or completely halogenated, or substituted by 1 or 2 radicals R^{2f} and where the cycloalkyl parts may also be fused to a benzene ring, which is unsubstituted or substituted by 1, 2, 3 or 4 radicals R²¹, which are identical or different, R^{2c} may also be selected from phenyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups R²¹, which are identical or different;
   - R^{2d}: are identical or different and selected from the group consisting of halogen, CN, C₁-C₄-alkyl, C₁-C₄-haloalkyl, C₁-C₄-alkoxy and C₁-C₄-haloalkoxy, or two groups R^{2d}, which are obound to adjacent carbon atoms may together form a fused benzene ring, which is unsubstituted or substituted by 1, 2, 3 or 4 groups R²¹, which are identical or different;
   - R^{2e}: is selected from the group consisting of =O, OH, R^{4b}-S(O)ₖ-Z⁴-, R^{4c}-C(O)-Z⁴-, R^{4d}O-C(O)-Z⁴-, R^{4e}R^{4f}N-C(O)-Z⁴-, R^{4g}R^{4h}N-Z⁴- and R^{4e}R^{4f}NS(O)₂-Z⁴-;
   - R^{2f}: is selected from the group consisting of OH, CN, NH₂, SH, SCN, nitro, tri(C₁-C₄-alkyl)silyl, C₁-C₄-alkoxy, C₁-C₄-haloalkoxy, R^{3e}R^{3f}N-, R^{3c}-C(O)-, HO-C(O)-R^{3d}O-C(O)-, R^{3d}S-C(O)-, R^{3e}R^{3f}N-C(O)-, R^{3e}R^{3f}N-NR^{3h}-C(O)-, R^{3c}-C(S)-, R^{3d}O-C(S)-, R^{3d}S-C(S)-, R^{3e}R^{3f}N-C(S), R^{3c}-S(O)ₙ-, R^{3c}-S(O)₂-O-, R^{3d}O-S(O)₂-, R^{3e}R^{3f}NS(O)₂-, R^{3c}-C(O)-NR^{3g}-, R^{3d}O-C(O)-NR^{3g}-, R^{3e}R^{3f}N-C(O)-NR^{3g}-, R^{3e}R^{3f}N-NR^{3h-}C(O)-NR^{3g}-, R^{3d}O-S(O)₂-NR^{3g}-, R^{3e}R^{3f}NS(O)₂-NR^{3g}-, R^{3c}-C(O)-NR^{3g}-C(O)-, R^{3d}O-C(O)-NR^{3g}-C(O)-, R^{3e}R^{3f}N-C(O)-NR^{3g}-C(O)-, R^{3d}O-S(O)₂-NR^{3g}-C(O)-, R^{3e}R^{3f}NS(O)₂-NR^{3g}-C(O)-, R^{3c}-C(O)-NR^{3g}-S(O)₂-, R^{3d}O-C(O)-NR^{3g}-S(O)₂-, R^{3e}R^{3f}N-C(O)-NR^{3g}-S(O)₂-, (OH)₂P(O)-, (C₁-C₄-alkoxy)₂P(O)-, R^{3d}O-N=CH-, phenyl-X and heterocyclyl-X, where heterocyclyl in the last two mentioned radicals is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups R¹¹, which are identical or different;
   - R³: is selected from the group consisting of hydrogen, cyano, thiocyanato, halogen, nitro, hydroxy-Z³-, C₁-C₆-alkyl, C₂-C₈-alkenyl, C₂-C₈-alkynyl, C₃-C₁₀-cycloalkyl-Z³-, C₃-C₆-cycloalkenyl-Z³-, C₃-C₁₀-cycloalkoxy-Z³-, C₃-C₁₀-cycloalkyl-C₁-C₂-alkoxy, where the cyclic groups of the four aforementioned radicals are unsubstituted or partially or completely halogenated, C₁-C₄-cyanoalkyl, C₁-C₈-haloalkyl, C₂-C₈-haloalkenyl, C₃-C₈-haloalkynyl, C₁-C₈-alkoxy-Z³-, C₁-C₈-haloalkoxy-Z³-, C₁-C₄-alkoxy-C₁-C₄-alkoxy-Z³-, C₁-C₄-haloalkoxy-C₁-C₄-alkoxy-Z³-, C₂-C₈-alkenyloxy-Z³-, C₂-C₈-alkynyloxy-Z³-, C₂-C₈-haloalkenyloxy-Z³-, C₃-C₈-haloalkynyloxy-Z³-, R^{3b}-S(O)ₖ-Z³-, R^{3c}-C(O)-Z³-, R^{3d}O-C(O)-Z³-, R^{3d}O-N=CH-Z³-, R^{3e}R^{3f}N-C(O)Z³-, R^{3g}R^{3h}N-Z³-, R²²C(O)O-Z³-, R²⁵OC(O)O-Z³-, (R²²)₂NC(O)O-Z³-, R²⁵S(O)₂O-Z³-, R²²OS(O)₂-Z³-, (R²²)₂NS(O)₂-Z³-, R²⁵OC(O)N(R²²)-Z³-, (R²²)₂NC(O)N(R²²)-Z³-, (R²²)₂NS(O)₂N(R²²)-Z³-, (OH)₂P(O)-Z³-, (C₁-C₄-alkoxy)₂P(O)-Z³-, phenyl-Z^{3a}-, heterocyclyl-Z^{3a}-, where heterocyclyl is a 3-, 4-, 5- or 6-membered monocyclic or 8-, 9- or 10-membered bicyclic saturated, partially unsaturated or aromatic heterocycle, which contains 1, 2, 3 or 4 heteroatoms as ring members, which are selected from the group consisting of O, N and S, where cyclic groups in phenyl-Z^{3a}-and heterocyclyl-Z^{3a}- are unsubstituted or substituted by 1, 2, 3 or 4 groups R²¹, which are identical or different;
   - R⁴: is selected from the group consisting of hydrogen, halogen, cyano-Z¹, C₁-C₈-alkyl, nitro, C₃-C₇-CyCloalkyl, C₃-C₇-cycloalkyl-C₁-C₄-alkyl, where the C₃-C₇-cycloalkyl groups in the two aforementioned radicals are unsubstituted or partially or completely halogenated, C₂-C₈-alkenyl, C₂-C₈-alkynyl, C₁-C₈-haloalkyl, C₁-C₃-alkylamino, C₁-C₃-dialkylamino, C₁-C₃-alkylamino-S(O)ₖ, C₁-C₃-alkylcarbonyl, C₁-C₈-alkoxy, C₁-C₄-alkoxy-C₁-C₄-alkyl, C₁-C₄-alkoxy-C₁-C₄-alkoxy-Z¹-, C₁-C₄-alkylthio-C₁-C₄-alkylthio-Z¹-, C₂-C₆-alkenyloxy, C₂-C₆-alkynyloxy, C₁-C₆-haloalkoxy, C₁-C₄-haloalkoxy-C₁-C₄-alkyl, C₁-C₄-haloalkoxy-C₁-C₄-alkoxy-Z¹-, R^{1b}-S(O)ₖ-Z¹-, phenoxy-Z¹- and heterocyclyloxy-Z¹-, where heterocyclyloxy is an oxygen bound 5- or 6- membered monocyclic or 8-, 9- or 10-membered bicyclic saturated, partially unsaturated or aromatic heterocycle, which contains 1, 2, 3 or 4 heteroatoms as ring members, which are selected from the group consisting of O, N and S, where the cyclic groups in phenoxy and heterocyclyloxy are unsubstituted or substituted by 1, 2, 3 or 4 groups R¹¹, which are identical or different;
   - R⁵: is hydrogen, halogen, cyano-Z¹-, nitro, C₁-C₈-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₂-C₈-alkenyl, C₂-C₈-alkynyl, C₁-C₈-haloalkyl, C₁-C₃-alkylamino, C₁-C₃-dialkylamino, C₁-C₃-alkylamino-S(O)ₖ, C₁-C₃-alkylcarbonyl, C₁-C₈-alkoxy, C₁-C₄-alkoxy-C₁-C₄-alkyl, C₁-C₄-alkoxy-C₁-C₄-alkoxy-Z¹-, C₁-C₄-alkylthio-C₁-C₄-alkyl, C₁-C₄-alkylthio-C₁-C₄-alkylthio-Z¹-, C₂-C₆-alkenyloxy, C₂-C₆-alkynyloxy, C₁-C₆-haloalkoxy, C₁-C₄-haloalkoxy-C₁-C₄-alkyl, C₁-C₄-haloalkoxy-C₁-C₄-alkoxy-Z¹-, R^{1b}-S(O)ₖ-Z¹-, phenoxy-Z¹- and heterocyclyloxy-Z¹-, where heterocyclyloxy is an 5- or 6-membered monocyclic or 8-, 9- or 10-membered bicyclic saturated, partially unsaturated or aromatic heterocycle which is bound via an oxygen atom and which contains 1, 2, 3 or 4 heteroatoms as ring members, which are selected from the group consisting of O, N and S, where the cyclic groups in phenoxy and heterocyclyloxy are unsubstituted or substituted by 1, 2, 3 or 4 groups R¹¹, which are identical or different;
   - R^{6a}, R^{6b}, R^{6c}, R^{6d}: are, independently of each other, selected from the group consisting of C₁-C₆-alkyl, C₃-C₇-CyCloalkyl, C₃-C₇-cycloalkyl-C₁-C₄-alkyl, where the C₃-C₇-cycloalkyl groups of the two aforementioned radicals are unsubstituted or partially or completely halogenated, C₁-C₆-haloalkyl, C₂-C₆-alkenyl, C₂-C₆-alkynyl, C₂-C₆-haloalkenyl, C₃-C₆-haloalkynyl, C₁-C₄-alkoxy-C₁-C₄-alkyl, C₁-C₄-haloalkoxy-C₁-C₄-alkyl, R^{b}-S(O)ₙ-C₁-C₃-alkyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
   - R⁷: is selected from the group consisting of hydrogen, CN, NH₂, C₁-C₆-alkyl, C₃-C₇-cycloalkyl, C₃-C₇-cycloalkyl-C₁-C₄-alkyl, where the C₃-C₇-cycloalkyl groups of the two aforementioned radicals are unsubstituted or partially or completely halogenated, C₁-C₆-haloalkyl, C₂-C₆-alkenyl, C₂-C₆-alkynyl, C₂-C₆-haloalkenyl, C₃-C₆-haloalkynyl, C₁-C₄-alkoxy-C₁-C₄-alkyl, C₁-C₄-haloalkoxy-C₁-C₄-alkyl, C₁-C₄-cyanoalkyl, R^{3b}-S(O)ₖ-Z^{3b}-, R^{3c}-C(O)-Z^{3b}-, R^{3d}O-C(O)-Z^{3b}-, R^{3d}O-N=CH-Z^{3b}-, R^{3e}R^{3f}N-C(O)-Z^{3b}-, R^{3g}R^{3h}N-Z^{3b}-, R²²C(O)O-Z^{3b}- R²⁵OC(O)O-Z^{3b}-, (R²²)₂NC(O)O-Z^{3b}-, R²⁵S(O)₂O-Z^{3b}-, R²²OS(O)₂-Z^{3b}-, (R²²)₂NS(O)₂-Z^{3b}-, R²⁵OC(O)N(R²²)-Z^{3b}-, (R²²)₂NC(O)N(R²²)-Z^{3b}-, (R²²)₂NS(O)₂N(R²²)-Z^{3b}-, (OH)₂P(O)-Z^{3b}-, (C₁-C₄-alkoxy)₂P(O)-Z^{3b}-, phenyl-Z^{3a}-, heterocyclyl-Z^{3a}-, where heterocyclyl is a 3-, 4-, 5- or 6-membered monocyclic or 8-, 9- or 10-membered bicyclic saturated, partially unsaturated or aromatic heterocycle, which contains 1, 2, 3 or 4 heteroatoms as ring members, which are selected from the group consisting of O, N and S, where cyclic groups in phenyl-Z^{3a}- and heterocyclyl-Z^{3a}- are unsubstituted or substituted by 1, 2, 3 or 4 groups R²¹, which are identical or different;
   - R¹¹, R²¹: independently of each other are selected from the group consisting of cyano, OH, halogen, nitro, C₁-C₆-alkyl, C₃-C₇-cycloalkyl, C₃-C₇-halocycloalkyl, C₁-C₆-haloalkyl, C₂-C₆-alkenyl, C₂-C₆-haloalkenyl, C₂-C₆-alkynyl, C₃-C₆-haloalkynyl, C₁-C₆-alkoxy, C₁-C₄-alkoxy-C₁-C₄-alkyl, C₁-C₄-alkylthio-C₁-C₄-alkyl, C₁-C₄-haloalkoxy-C₁-C₄-alkyl, C₁-C₄-alkoxy-C₁-C₄-alkoxy, C₃-C₇-CyCloalkoxy and C₁-C₆-haloalkoxy, or two radicals R¹¹ or R²¹, resepctively, which are bound to the same carbon atom, together may form a group =O or two radicals R¹¹ or R²¹, resepctively, which are bound to adjacent carbon atoms, together may form a group selected from C₃-C₅-alkylene, O-C₂-C₄-alkylene and O-(C₁-C₃-alkylene)-O-;
   - Z¹, Z³: independently of each other are selected from the group consisting of a covalent bond and C₁-C₄-alkanediyl, which is unsubstituted, partly or completely fluorinated;
   - Z²: is a covalent bond or a bivalent radical selected from C₁-C₄-alkanediyl and C₁-C₄-alkanediyl-O-C₁-C₄-alkanediyl, where in the last two mentioned radicals the C₁-C₄-alkanediyl groups are linear and where C₁-C₄-alkanediyl is unsubstituted, or partly or completely fluorinated or substituted by 1, 2, 3 or 4 groups R^{z};
   - Z^{3a}: is selected from the group consisting of a covalent bond, C₁-C₄-alkanediyl, O-C₁-C₄-alkanediyl, C₁-C₄-alkanediyl-O and C₁-C₄-alkanediyl-O-C₁-C₄-alkanediyl;
   - Z^{3b}: is selected from the group consisting of C₁-C₄-alkanediyl, C₁-C₄-alkanediyl-O and C₁-C₄-alkanediyl-O-C₁-C₄-alkanediyl;
   - R^{z}: are identical or different and selected from the group consisting of C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₁-C₆-alkoxy, C₂-C₆-alkenyl, C₁-C₄-alkyl-C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkyl-S(O)ₙ-C₁-C₄-alkyl, C₁-C₄-alkylamino-C₁-C₄-alkyl, C₁-C₄-dialkylamino-C₁-C₄-alkyl, C₁-C₆-cyanoalkyl, phenyl, benzyl, heterocyclyl and heterocyclylmethyl, where heterocyclyl in the last two mentioned radicals is a 5- or 6-membered monocyclic saturated, partially unsaturated or aromatic heterocycle, which contains 1, 2, 3 or 4 heteroatoms as ring members, which are selected from the group consisting of O, N and S, where phenyl, benzyl, heterocyclyl and heterocyclylmethyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
   - R^{b}, R^{1b}, R^{3b}, R^{4b}: independently of each other are selected from the group consisting of C₁-C₆-alkyl, C₃-C₇-CyCloalkyl, C₁-C₆-haloalkyl, C₂-C₆-alkenyl, C₂-C₆-haloalkenyl, C₂-C₆-alkynyl, C₃-C₆-haloalkynyl, phenyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
   - R^{3c}, R^{4c}: independently of each other are selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₁-C₆-alkoxy, C₂-C₆-alkenyl, C₁-C₄-alkyl-C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkyl-S(O)ₙ-C₁-C₄-alkyl, C₁-C₄-alkylamino-C₁-C₄-alkyl, C₁-C₄-dialkylamino-C₁-C₄-alkyl, C₁-C₆-cyanoalkyl, phenyl, benzyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, benzyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
   - R^{3d}, R^{4d}: independently of each other are selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₁-C₆-alkoxy, C₂-C₆-alkenyl, C₁-C₄-alkyl-C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkyl-S(O)ₙ-C₁-C₄-alkyl, C₁-C₄-alkylamino-C₁-C₄-alkyl, C₁-C₄-dialkylamino-C₁-C₄-alkyl, C₁-C₆-cyanoalkyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
   - R^{3e}, R^{3f}, R^{4e}, R^{4f}: independently of each other are selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy; or
   - R^{3e} and R^{3f} or R^{4e} and R^{4f}: together with the nitrogen atom, to which they are bound may form a 4-, 5-, 6- or 7-membered, saturated or unsaturated heterocyclic radical, which may carry as a ring member a further heteroatom selected from O, S and N and which is unsubstituted or carries 1, 2, 3 or 4 groups, which are identical or different and selected from the group consisting of =O, halogen, C₁-C₄-alkyl, C₁-C₄-haloalkyl, C₁-C₄-alkoxy and C₁-C₄-haloalkoxy;
   - R^{3g}, R^{4g}: independently of each other are selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkylsulfonyl, C₁-C₄-alkylcarbonyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
   - R^{3h}, R^{4h}: independently of each other are selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkylsulfonyl, C₁-C₄-alkylcarbonyl, a radical C(O)R^{k}, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy; or
   - R^{3g} and R^{3h} or R^{4g} and R^{4h}: together with the nitrogen atom, to which they are bound may form a 4-, 5-, 6- or 7-membered, saturated or partially unsaturated heterocyclic radical, which may carry as a ring member a further heteroatom selected from O, S and N and which is unsubstituted or carries 1, 2, 3 or 4 groups, which are identical or different and selected from the group consisting of =O, halogen, C₁-C₄-alkyl, C₁-C₄-haloalkyl, C₁-C₄-alkoxy and C₁-C₄-haloalkoxy;
   - R²²: is selected from the group consisting of hydrogen, C₁-C₆-alkyl, C₁-C₆-haloalkyl, C₂-C₆-alkenyl, C₂-C₆-haloalkenyl, C₂-C₆-alkynyl, C₃-C₆-haloalkynyl, C₃-C₆-cycloalkyl, C₃-C₆-cycloalkenyl, C₃-C₆-halocycloalkyl, C₃-C₆-cycloalkyl-C₁-C₆-alkyl, C₁-C₆-alkoxy-C₁-C₆-alkyl, C₃-C₆-cycloalkyl-C₁-C₆-alkoxy-C₁-C₆-alkyl, phenyl-Z¹, phenyl-O-C₁-C₆-alkyl, phenyl-N(R²³)-C₁-C₆-alkyl, phenyl-S(O)ₙ-C₁-C₆-alkyl, heterocyclyl-Z¹, heterocyclyl-N(R²³)-C₁-C₆-alkyl, heterocyclyl-O-C₁-C₆-alkyl, heterocyclyl-S(O)ₙ-C₁-C₆-alkyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, heterocyclyl are unsubstituted or substituted by 1, 2, 3 or 4 groups , which are identical or different and selected from the group consisting of cyano, halogen, nitro, thiocyanato, C₁-C₆-alkyl, C₁-C₆-haloalkyl, C₃-C₆-cycloalkyl, C(O)OR²³, C(O)N(R²³)₂, OR²³, N(R²³)₂, S(O)ₙR²⁴, S(O)₂OR²³, S(O)₂N(R²³)₂, and R²³O-C₁-C₆-alkyl, and where heterocyclyl bears 0, 1 or 2 oxo groups;
   - R²³: is selected from the group consisting of hydrogen, C₁-C₆-alkyl, C₁-C₆-haloalkyl, C₂-C₆-alkenyl, C₂-C₆-alkynyl, C₃-C₆-cycloalkyl, C₃-C₆-cycloalkyl-C₁-C₆-alkyl, and phenyl;
   - R²⁴: is selected from the group consisting of C₁-C₆-alkyl, C₁-C₆-haloalkyl, C₂-C₆-alkenyl, C₂-C₆-alkynyl, C₃-C₆-cycloalkyl, C₃-C₆-cycloalkyl-C₁-C₆-alkyl, and phenyl;
   - R²⁵: is selected from the group consisting of C₁-C₆-alkyl, C₁-C₆-haloalkyl, C₂-C₆-alkenyl, C₂-C₆-haloalkenyl, C₂-C₆-alkynyl, C₃-C₆-haloalkynyl, C₃-C₆-cycloalkyl, C₃-C₆-cycloalkenyl, C₃-C₆-halocycloalkyl, C₃-C₆-cycloalkyl-C₁-C₆-alkyl, C₁-C₆-alkoxy-C₁-C₆-alkyl, C₃-C₆-cycloalkyl-C₁-C₆-alkoxy-C₁-C₆-alkyl, phenyl-Z¹, phenyl-O-C₁-C₆-alkyl, phenyl-N(R²³)-C₁-C₆-alkyl, phenyl-S(O)ₙ-C₁-C₆-alkyl, heterocyclyl-Z¹, heterocyclyl-N(R²³)-C₁-C₆-alkyl, heterocyclyl-O-C₁-C₆-alkyl, heterocyclyl-S(O)ₙ-C₁-C₆-alkyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, heterocyclyl are unsubstituted or substituted by 1, 2, 3 or 4 groups , which are identical or different and selected from the group consisting of cyano, halogen, nitro, thiocyanato, C₁-C₆-alkyl, C₁-C₆-haloalkyl, C₃-C₆-cycloalkyl, C(O)OR²³, C(O)N(R²³)₂, OR²³, N(R²³)₂, S(O)ₙR²⁴, S(O)₂OR²³, S(O)₂N(R²³)₂, and R²³O-C₁-C₆-alkyl, and where heterocyclyl bears 0, 1 or 2 oxo groups;
   - X: is a covalent bond, O, S(O)ₙ or NR, where R is hydrogen or C₁-C₄-alkyl;
   - k: is 0, 1 or 2;
   - n: is 0, 1 or 2;
   - p: is 0, 1 or 2;
   - R^{k}: has the meanings of R^{3c}.

The present invention also relates to the N-oxides of the compounds of formula I, to the salts, in particular to the agriculturally suitable salts, of the compounds of formula I and to the salts of said N-oxides.

The compounds of the present invention, i.e. the compounds of formula I, their N-oxides and their agriculturally suitable salts, are particularly useful for controlling unwanted vegetation. Therefore, the invention also relates to the use of a compound of formula I of an N-oxide thereof or an agriculturally suitable salt thereof or of a composition comprising at least one compound of formula I, an N-oxide thereof or an agriculturally suitable salt thereof for combating or controlling unwanted vegetation.

The invention also relates to a composition comprising at least one compound of formula I, an N-oxide or a salt thereof, and at least one auxiliary. In particular, the invention relates to an agricultural composition comprising at least one compound of formula I or an agriculturally suitable salt thereof, and at least one auxiliary customary for crop protection formulations.

The present invention also relates to a method for combating or controlling unwanted vegetation, which method comprises allowing a herbicidally effective amount of at least one compound of formula I or a salt thereof, to act on unwanted plants, their seed and/or their habitat.

Depending on the kind of substitutents, the compounds of formula I may have one or more centers of chirality, in which case they may be present as mixtures of enantiomers or diastereomers but also in the form of the pure enantiomers or pure diastereomers. The invention provides both the pure enantiomers or pure diastereomers of the compounds of formula I, and their mixtures and the use according to the invention of the pure enantiomers or pure diastereomers of the compound of formula I or its mixtures. Suitable compounds of formula I also include all possible geometrical stereoisomers (cis/trans isomers) and mixtures thereof. Cis/trans isomers may be present with respect to an alkene, carbon-nitrogen double-bond, nitrogen-sulfur double bond or amide group. The term "stereoisomer(s)" encompasses both optical isomers, such as enantiomers or diastereomers, the latter existing due to more than one center of chirality in the molecule, as well as geometrical isomers (cis/trans isomers).

The present invention moreover relates to compounds as defined herein, wherein one or more of the atoms depicted in formula I have been replaced by its stable, preferably nonradioactive isotope (e.g., hydrogen by deuterium, ¹²C by ¹³C, ¹⁴N by ¹⁵N, ¹⁶O by ¹⁸O) and in particular wherein at least one hydrogen atom has been replaced by a deuterium atom. Of course, the compounds according to the invention contain more of the respective isotope than this naturally occurs and thus is anyway present in the compounds of formula I.

The compounds of the present invention may be amorphous or may exist in one ore more different crystalline states (polymorphs) which may have different macroscopic properties such as stability or show different biological properties such as activities. The present invention includes both amorphous and crystalline compounds of formula I, their enantiomers or diastereomers, mixtures of different crystalline states of the respective compound of formula I, its enantiomers or diastereomers, as well as amorphous or crystalline salts thereof.

Salts of the compounds of the present invention are preferably agriculturally suitable salts. They can be formed in a customary method, e.g. by reacting the compound with an acid if the compound of the present invention has a basic functionality or by reacting the compound with a suitable base if the compound of the present invention has an acidic functionality.

Useful agriculturally suitable salts are especially the salts of those cations or the acid addition salts of those acids whose cations and anions, respectively, do not have any adverse effect on the herbicidal action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH₄⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethylammonium, bis(2-hydroxyethyl)ammonium, benzyltrimethylammonium and benzl-triethylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting compounds of the present invention with an acid of the corresponding anion, preferably with hydrochloric acid, hydrobromic acid, sulfuric acid, phosphoric acid or nitric acid.

The term " N-oxides" relates to the derivatives of the compounds of formula I, which bear at least one oxygen atom at a nitrogen atom. For example, the term " N-oxide" may relate to a derivative of a compound of formula I, wherein one of the imino nitrogens in the radicals Q¹, Q², Q³ or Q⁴ bears an oxygen atom.

The term "undesired vegetation" ("weeds") is understood to include any vegetation growing in non-crop-areas or at a crop plant site or locus of seeded and otherwise desired crop, where the vegetation is any plant species, including their germinant seeds, emerging seedlings and established vegetation, other than the seeded or desired crop (if any). Weeds, in the broadest sense, are plants considered undesirable in a particular location.

The organic moieties mentioned in the above definitions of the variables are - like the term halogen - collective terms for individual listings of the individual group members. The prefix Cₙ-Cₘ indicates in each case the possible number of carbon atoms in the group.

The term "halogen" denotes in each case fluorine, bromine, chlorine or iodine, in particular fluorine, chlorine or bromine.

The term "partially or completely halogenated" will be taken to mean that 1 or more, e.g. 1, 2, 3, 4 or 5 or all of the hydrogen atoms of a given radical have been replaced by a halogen atom, in particular by fluorine or chlorine. A partially or completely halogenated radical is termed below also "halo-radical". For example, partially or completely halogenated alkyl is also termed haloalkyl.

The term "alkyl" as used herein (and in the alkyl moieties of other groups comprising an alkyl group, e.g. alkoxy, alkylamino, dialkylamino, alkylcarbonyl, alkoxycarbonyl, alkylthio, alkylsulfonyl and alkoxyalkyl) denotes in each case a straight-chain or branched alkyl group having usually from 1 to 10 carbon atoms (= C₁-C₁₀-alkyl) or 1 to 8 carbon atoms (= C₁-C₈-alkyl), frequently from 1 to 6 carbon atoms (= C₁-C₆-alkyl), in particular 1 to 4 carbon atoms (= C₁-C₄-alkyl) and especially from 1 to 3 carbon atoms (= C₁-C₃-alkyl). Examples of C₁-C₄-alkyl are methyl, ethyl, n-propyl, iso-propyl, n-butyl, 2-butyl (= sec-butyl), isobutyl and tert-butyl. Examples for C₁-C₆-alkyl are, apart those mentioned for C₁-C₄-alkyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Examples for C₁-C₁₀-alkyl are, apart those mentioned for C₁-C₆-alkyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, n-octyl, 1-methyloctyl, 2-methylheptyl, 1-ethylhexyl, 2-ethylhexyl, 1,2-dimethylhexyl, 1-propylpentyl, 2-propylpentyl, nonyl, decyl, 2-propylheptyl and 3-propylheptyl.

The term "alkylene" (or alkanediyl) as used herein in each case denotes an alkyl radical as defined above, wherein one hydrogen atom at any position of the carbon backbone is replaced by one further binding site, thus forming a bivalent moiety.

The term "haloalkyl" as used herein (and in the haloalkyl moieties of other groups comprising a haloalkyl group, e.g. haloalkoxy, haloalkylthio, haloalkylcarbonyl, haloalkylsulfonyl and haloalkylsulfinyl) denotes in each case a straight-chain or branched alkyl group having usually from 1 to 8 carbon atoms (= C₁-C₈-haloalkyl), frequently from 1 to 6 carbon atoms (= C₁-C₆-haloalkyl), more frequently 1 to 4 carbon atoms (= C₁-C₄-haloalkyl), wherein the hydrogen atoms of this group are partially or totally replaced with halogen atoms. Preferred haloalkyl moieties are selected from C₁-C₄-haloalkyl, more preferably from C₁-C₂-haloalkyl, more preferably from halomethyl, in particular from C₁-C₂-fluoroalkyl. Halomethyl is methyl in which 1, 2 or 3 of the hydrogen atoms are replaced by halogen atoms. Examples are bromomethyl, chloromethyl, dichloromethyl, trichloromethyl, fluoromethyl, difluoromethyl, trifluoromethyl, chlorofluoromethyl, dichlorofluoromethyl, chlorodifluoromethyl and the like. Examples for C₁-C₂-fluoroalkyl are fluoromethyl, difluoromethyl, trifluoromethyl, 1-fluoroethyl, 2-fluoroethyl, 2,2-difluoroethyl, 2,2,2-trifluoroethyl, pentafluoroethyl, and the like. Examples for C₁-C₂-haloalkyl are, apart those mentioned for C₁-C₂-fluoroalkyl, chloromethyl, dichloromethyl, trichloromethyl, bromomethyl, chlorofluoromethyl, dichlorofluoromethyl, chlorodifluoromethyl, 1-chloroethyl, 2-chloroethyl, 2,2,-dichloroethyl, 2,2,2-trichloroethyl, 2-chloro-2-fluoroethyl, 2-chloro-2,2-difluoroethyl, 2,2-dichloro-2-fluoroethyl, 1-bromoethyl, and the like. Examples for C₁-C₄-haloalkyl are, apart those mentioned for C₁-C₂-haloalkyl, 1-fluoropropyl, 2-fluoropropyl, 3-fluoropropyl, 3,3-difluoropropyl, 3,3,3-trifluoropropyl, heptafluoropropyl, 1,1,1-trifluoroprop-2-yl, 3-chloropropyl, 4-chlorobutyl and the like.

The term "cycloalkyl" as used herein (and in the cycloalkyl moieties of other groups comprising a cycloalkyl group, e.g. cycloalkoxy and cycloalkylalkyl) denotes in each case a mono- or bicyclic, saturated cycloaliphatic radical having usually from 3 to 10 carbon atoms (= C₃-C₁₀-cycloalkyl), preferably 3 to 7 carbon atoms (= C₃-C₇-CyCloalkyl) or in particular 3 to 6 carbon atoms (= C₃-C₆-cycloalkyl). Examples of monocyclic saturated cycloaliphatic radicals having 3 to 6 carbon atoms comprise cyclopropyl, cyclobutyl, cyclopentyl and cyclohexyl. Examples of monocyclic saturated cycloaliphatic radicals having 3 to 7 carbon atoms comprise cyclopropyl, cyclobutyl, cyclopentyl, cyclohexyl and cycloheptyl. Examples of bicyclic radicals having 6 to 10 carbon atoms comprise bicyclo[2.1.1]hexyl, bicyclo[2.2.1]heptyl, bicyclo[3.1.1]heptyl, bicyclo[2.2.1]heptyl, bicyclo[2.2.2]octyl and bicyclo[3.2.1]octyl.

The term "halocycloalkyl" as used herein (and in the halocycloalkyl moieties of other groups comprising an halocycloalkyl group, e.g. halocycloalkylmethyl) denotes in each case a mono- or bicyclic cycloaliphatic radical having usually from 3 to 10 carbon atoms, preferably 3 to 7 carbon atoms or in particular 3 to 6 carbon atoms, wherein at least one, e.g. 1, 2, 3, 4 or 5 of the hydrogen atoms are replaced by halogen, in particular by fluorine or chlorine. Examples are 1- and 2- fluorocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, 2,5-dichlorocyclopentyl and the like.

The term "cycloalkyl-alkyl" used herein denotes a cycloalkyl group, as defined above, which is bound to the remainder of the molecule via an alkylene group. The term "C₃-C₇-cycloalkyl-C₁-C₄-alkyl" refers to a C₃-C₇-Cycloalkyl group as defined above which is bound to the remainder of the molecule via a C₁-C₄-alkyl group, as defined above. Examples are cyclopropylmethyl, cyclopropylethyl, cyclopropylpropyl, cyclobutylmethyl, cyclobutylethyl, cyclobutylpropyl, cyclopentylmethyl, cyclopentylethyl, cyclopentylpropyl, cyclohexylmethyl, cyclohexylethyl, cyclohexylpropyl, and the like.

The term "alkenyl" as used herein denotes in each case a monounsaturated straight-chain or branched hydrocarbon radical having usually 2 to 8 (= C₂-C₈-alkenyl), preferably 2 to 6 carbon atoms (= C₂-C₆-alkenyl), in particular 2 to 4 carbon atoms (= C₂-C₄-alkenyl), and a double bond in any position, for example C₂-C₄-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl or 2-methyl-2-propenyl; C₂-C₆-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl and the like, or C₂-C₈-alkenyl, such as the radicals mentioned for C₂-C₆-alkenyl and additionally 1-heptenyl, 2-heptenyl, 3-heptenyl, 1-octenyl, 2-octenyl, 3-octenyl, 4-octenyl and the positional isomers thereof.

The term "haloalkenyl" as used herein, which may also be expressed as "alkenyl which is substituted by halogen", and the haloalkenyl moieties in haloalkenyloxy and the like refers to unsaturated straight-chain or branched hydrocarbon radicals having 2 to 8 (= C₂-C₈-haloalkenyl) or 2 to 6 (= C₂-C₆-haloalkenyl) or 2 to 4 (= C₂-C₄-haloalkenyl) carbon atoms and a double bond in any position, where some or all of the hydrogen atoms in these groups are replaced by halogen atoms as mentioned above, in particular fluorine, chlorine and bromine, for example chlorovinyl, chloroallyl and the like.

The term "alkynyl" as used herein denotes unsaturated straight-chain or branched hydrocarbon radicals having usually 2 to 8 (= C₂-C₈-alkynyl), frequently 2 to 6 (= C₂-C₆-alkynyl), preferably 2 to 4 carbon atoms (= C₂-C₄-alkynyl) and a triple bond in any position, for example C₂-C₄-alkynyl, such as ethynyl, 1-propynyl, 2-propynyl, 1-butynyl, 2-butynyl, 3-butynyl, 1-methyl-2-propynyl and the like, C₂-C₆-alkynyl, such as ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl, 1-ethyl-1-methyl-2-propynyl and the like.

The term "haloalkynyl" as used herein, which is also expressed as "alkynyl which is substituted by halogen", refers to unsaturated straight-chain or branched hydrocarbon radicals having usually 3 to 8 carbon atoms (= C₃-C₈-haloalkynyl), frequently 3 to 6 (= C₃-C₆-haloalkynyl), preferabyl 3 to 4 carbon atoms (= C₃-C₄-haloalkynyl), and a triple bond in any position (as mentioned above), where some or all of the hydrogen atoms in these groups are replaced by halogen atoms as mentioned above, in particular fluorine, chlorine and bromine.

The term "alkoxy" as used herein denotes in each case a straight-chain or branched alkyl group usually having from 1 to 8 carbon atoms (= C₁-C₈-alkoxy), frequently from 1 to 6 carbon atoms (= C₁-C₆-alkoxy), preferably 1 to 4 carbon atoms (= C₁-C₄-alkoxy), which is bound to the remainder of the molecule via an oxygen atom. C₁-C₂-Alkoxy is methoxy or ethoxy. C₁-C₄-Alkoxy is additionally, for example, n-propoxy, 1-methylethoxy (isopropoxy), butoxy, 1-methylpropoxy (sec-butoxy), 2-methylpropoxy (isobutoxy) or 1,1-dimethylethoxy (tert-butoxy). C₁-C₆-Alkoxy is additionally, for example, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy. C₁-C₈-Alkoxy is additionally, for example, heptyloxy, octyloxy, 2-ethylhexyloxy and positional isomers thereof.

The term "haloalkoxy" as used herein denotes in each case a straight-chain or branched alkoxy group, as defined above, having from 1 to 8 carbon atoms (= C₁-C₈-haloalkoxy), frequently from 1 to 6 carbon atoms (= C₁-C₆-haloalkoxy), preferably 1 to 4 carbon atoms (= C₁-C₄-haloalkoxy), more preferably 1 to 3 carbon atoms (= C₁-C₃-haloalkoxy), wherein the hydrogen atoms of this group are partially or totally replaced with halogen atoms, in particular fluorine atoms. C₁-C₂-Haloalkoxy is, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy or OC₂F₅. C₁-C₄-Haloalkoxy is additionally, for example, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy or nonafluorobutoxy. C₁-C₆-Haloalkoxy is additionally, for example, 5-fluoropentoxy, 5-chloropentoxy, 5-brompentoxy, 5-iodopentoxy, undecafluoropentoxy, 6-fluorohexoxy, 6-chlorohexoxy, 6-bromohexoxy, 6-iodohexoxy or dodecafluorohexoxy.

The term "alkoxyalkyl" as used herein denotes in each case alkyl usually comprising 1 to 6 carbon atoms, preferably 1 to 4 carbon atoms, wherein 1 carbon atom carries an alkoxy radical usually comprising 1 to 8, frequently 1 to 6, in particular 1 to 4, carbon atoms as defined above. The term "C₁-C₆-alkoxy-C₁-C₆-alkyl" relates to a C₁-C₆-alkyl group, as defined above, in which one hydrogen atom is replaced by a C₁-C₆-alkoxy group, as defined above. Examples are CH₂OCH₃, CH₂-OC₂H₅, n-propoxymethyl, CH₂-OCH(CH₃)₂, n-butoxymethyl, (1-methylpropoxy)-methyl, (2-methylpropoxy)methyl, CH₂-OC(CH₃)₃, 2-(methoxy)ethyl, 2-(ethoxy)ethyl, 2-(n-propoxy)-ethyl, 2-(1-methylethoxy)-ethyl, 2-(n-butoxy)ethyl, 2-(1-methylpropoxy)-ethyl, 2-(2-methylpropoxy)-ethyl, 2-(1,1-dimethylethoxy)-ethyl, 2-(methoxy)-propyl, 2-(ethoxy)-propyl, 2-(n-propoxy)-propyl, 2-(1-methylethoxy)-propyl, 2-(n-butoxy)-propyl, 2-(1-methylpropoxy)-propyl, 2-(2-methylpropoxy)-propyl, 2-(1,1-dimethylethoxy)-propyl, 3-(methoxy)-propyl, 3-(ethoxy)-propyl, 3-(n-propoxy)-propyl, 3-(1-methylethoxy)-propyl, 3-(n-butoxy)-propyl, 3-(1-methylpropoxy)-propyl, 3-(2-methylpropoxy)-propyl, 3-(1,1-dimethylethoxy)-propyl, 2-(methoxy)-butyl, 2-(ethoxy)-butyl, 2-(n-propoxy)-butyl, 2-(1-methylethoxy)-butyl, 2-(n-butoxy)-butyl, 2-(1-methylpropoxy)-butyl, 2-(2-methyl-propoxy)-butyl, 2-(1,1-dimethylethoxy)-butyl, 3-(methoxy)-butyl, 3-(ethoxy)-butyl, 3-(n-propoxy)-butyl, 3-(1-methylethoxy)-butyl, 3-(n-butoxy)-butyl, 3-(1-methylpropoxy)-butyl, 3-(2-methylpropoxy)-butyl, 3-(1,1-dimethylethoxy)-butyl, 4-(methoxy)-butyl, 4-(ethoxy)-butyl, 4-(n-propoxy)-butyl, 4-(1-methylethoxy)-butyl, 4-(n-butoxy)-butyl, 4-(1-methylpropoxy)-butyl, 4-(2-methylpropoxy)-butyl, 4-(1,1-dimethylethoxy)-butyl and the like.

The term "haloalkoxy-alkyl" as used herein denotes in each case alkyl as defined above, usually comprising 1 to 6 carbon atoms, preferably 1 to 4 carbon atoms, wherein 1 carbon atom carries an haloalkoxy radical as defined above, usually comprising 1 to 8, frequently 1 to 6, in particular 1 to 4, carbon atoms as defined above. Examples are fluoromethoxymethyl, difluoromethoxymethyl, trifluoromethoxymethyl, 1-fluoroethoxymethyl, 2-fluoroethoxymethyl, 1,1-difluoroethoxymethyl, 1,2-difluoroethoxymethyl, 2,2-difluoroethoxymethyl, 1,1,2-trifluoroethoxymethyl, 1,2,2-trifluoroethoxymethyl, 2,2,2-trifluoroethoxymethyl, pentafluoroethoxymethyl, 1-fluoroethoxy-1-ethyl, 2-fluoroethoxy-1-ethyl, 1,1-difluoroethoxy-1-ethyl, 1,2-difluoroethoxy-1-ethyl, 2,2-difluoroethoxy-1-ethyl, 1,1,2-trifluoroethoxy-1-ethyl, 1,2,2-trifluoroethoxy-1-ethyl, 2,2,2-trifluoroethoxy-1 -ethyl, pentafluoroethoxy-1-ethyl, 1-fluoroethoxy-2-ethyl, 2-fluoroethoxy-2-ethyl, 1,1-difluoroethoxy-2-ethyl, 1,2-difluoroethoxy-2-ethyl, 2,2-difluoroethoxy-2-ethyl, 1,1,2-trifluoroethoxy-2-ethyl, 1,2,2-trifluoroethoxy-2-ethyl, 2,2,2-trifluoroethoxy-2-ethyl, pentafluoroethoxy-2-ethyl, and the like.

The term "alkylthio" (also alkylsulfanyl, "alkyl-S" or "alkyl-S(O)ₖ" (wherein k is 0) as used herein denotes in each case a straight-chain or branched saturated alkyl group as defined above, usually comprising 1 to 8 carbon atoms (= C₁-C₈-alkylthio), frequently comprising 1 to 6 carbon atoms (= C₁-C₆-alkylthio), preferably 1 to 4 carbon atoms (= C₁-C₄-alkylthio), which is attached via a sulfur atom at any position in the alkyl group. C₁-C₂-Alkylthio is methylthio or ethylthio. C₁-C₄-Alkylthio is additionally, for example, n-propylthio, 1-methylethylthio (iso-propylthio), butylthio, 1-methylpropylthio (sec-butylthio), 2-methylpropylthio (isobutylthio) or 1,1-dimethylethylthio (tert-butylthio). C₁-C₆-Alkylthio is additionally, for example, pentylthio, 1-methylbutylthio, 2-methylbutylthio, 3-methylbutylthio, 1,1-dimethylpropylthio, 1,2-dimethylpropylthio, 2,2-dimethylpropylthio, 1-ethylpropylthio, hexylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio or 1-ethyl-2-methylpropylthio. C₁-C₈-Alkylthio is additionally, for example, heptylthio, octylthio, 2-ethylhexylthio and positional isomers thereof.

The term "haloalkylthio" as used herein refers to an alkylthio group as defined above wherein the hydrogen atoms are partially or completely substituted by fluorine, chlorine, bromine and/or iodine. C₁-C₂-Haloalkylthio is, for example, SCH₂F, SCHF₂, SCF₃, SCH₂Cl, SCHCl₂, SCCl₃, chlorofluoromethylthio, dichlorofluoromethylthio, chlorodifluoromethylthio, 2-fluoroethylthio, 2-chloroethylthio, 2-bromoethylthio, 2-iodoethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio or SC₂F₅. C₁-C₄-Haloalkylthio is additionally, for example, 2-fluoropropylthio, 3-fluoropropylthio, 2,2-difluoropropylthio, 2,3-difluoropropylthio, 2-chloropropylthio, 3-chloropropylthio, 2,3-dichloropropylthio, 2-bromopropylthio, 3-bromopropylthio, 3,3,3-trifluoropropylthio, 3,3,3-trichloropropylthio, SCH₂-C₂F₅, SCF₂-C₂F₅, 1-(CH₂F)-2-fluoroethylthio, 1-(CH₂Cl)-2-chloroethylthio, 1-(CH₂Br)-2-bromoethylthio, 4-fluorobutylthio, 4-chlorobutylthio, 4-bromobutylthio or nonafluorobutylthio. C₁-C₆-Haloalkylthio is additionally, for example, 5-fluoropentylthio, 5-chloropentylthio, 5-brompentylthio, 5-iodopentylthio, undecafluoropentylthio, 6-fluorohexylthio, 6-chlorohexylthio, 6-bromohexylthio, 6-iodohexylthio or dodecafluorohexylthio.

The terms "alkylsulfinyl" and "alkyl-S(O)ₖ" (wherein k is 1) are equivalent and, as used herein, denote an alkyl group, as defined above, attached via a sulfinyl [S(O)] group. For example, the term "C₁-C₂-alkylsulfinyl" refers to a C₁-C₂-alkyl group, as defined above, attached via a sulfinyl [S(O)] group. The term "C₁-C₄-alkylsulfinyl" refers to a C₁-C₄-alkyl group, as defined above, attached via a sulfinyl [S(O)] group. The term "C₁-C₆-alkylsulfinyl" refers to a C₁-C₆-alkyl group, as defined above, attached via a sulfinyl [S(O)] group. C₁-C₂-alkylsulfinyl is methylsulfinyl or ethylsulfinyl. C₁-C₄-alkylsulfinyl is additionally, for example, n-propylsulfinyl, 1-methylethylsulfinyl (isopropylsulfinyl), butylsulfinyl, 1-methylpropylsulfinyl (sec-butylsulfinyl), 2-methylpropylsulfinyl (isobutylsulfinyl) or 1,1-dimethylethylsulfinyl (tert-butylsulfinyl). C₁-C₆-alkylsulfinyl is additionally, for example, pentylsulfinyl, 1-methylbutylsulfinyl, 2-methylbutylsulfinyl, 3-methylbutylsulfinyl, 1,1-dimethylpropylsulfinyl, 1,2-dimethylpropylsulfinyl, 2,2-dimethylpropylsulfinyl, 1-ethylpropylsulfinyl, hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl or 1-ethyl-2-methylpropylsulfinyl.

The terms "alkylsulfonyl" and "alkyl-S(O)ₖ" (wherein k is 2) are equivalent and, as used herein, denote an alkyl group, as defined above, attached via a sulfonyl [S(O)₂] group. The term "C₁-C₂-alkylsulfonyl" refers to a C₁-C₂-alkyl group, as defined above, attached via a sulfonyl [S(O)₂] group. The term "C₁-C₄-alkylsulfonyl" refers to a C₁-C₄-alkyl group, as defined above, attached via a sulfonyl [S(O)₂] group. The term "C₁-C₆-alkylsulfonyl" refers to a C₁-C₆-alkyl group, as defined above, attached via a sulfonyl [S(O)₂] group. C₁-C₂-alkylsulfonyl is methylsulfonyl or ethylsulfonyl. C₁-C₄-alkylsulfonyl is additionally, for example, n-propylsulfonyl, 1-methylethylsulfonyl (isopropylsulfonyl), butylsulfonyl, 1-methylpropylsulfonyl (sec-butylsulfonyl), 2-methylpropylsulfonyl (isobutylsulfonyl) or 1,1-dimethylethylsulfonyl (tert-butylsulfonyl). C₁-C₆-alkylsulfonyl is additionally, for example, pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl or 1-ethyl-2-methylpropylsulfonyl.

The term "alkylamino" as used herein denotes in each case a group R*HN-, wherein R* is a straight-chain or branched alkyl group usually having from 1 to 6 carbon atoms (= C₁-C₆-alkylamino), preferably 1 to 4 carbon atoms(= C₁-C₄-alkylamino). Examples of C₁-C₆-alkylamino are methylamino, ethylamino, n-propylamino, isopropylamino, n-butylamino, 2-butylamino, iso-butylamino, tert-butylamino, and the like.

The term "dialkylamino" as used herein denotes in each case a group R*R°N-, wherein R* and R°, independently of each other, are a straight-chain or branched alkyl group each usually having from 1 to 6 carbon atoms (= di-(C₁-C₆-alkyl)-amino), preferably 1 to 4 carbon atoms (= di-(C₁-C₄-alkyl)-amino). Examples of a di-(C₁-C₆-alkyl)-amino group are dimethylamino, diethylamino, dipropylamino, dibutylamino, methyl-ethyl-amino, methyl-propyl-amino, methyl-isopropylamino, methyl-butyl-amino, methyl-isobutyl-amino, ethyl-propyl-amino, ethyl-isopropylamino, ethyl-butyl-amino, ethyl-isobutyl-amino, and the like.

The suffix "-carbonyl" in a group denotes in each case that the group is bound to the remainder of the molecule via a carbonyl C=O group. This is the case e.g. in alkylcarbonyl, haloalkylcarbonyl, aminocarbonyl, alkylaminocarbonyl, dialkylaminocarbonyl, alkoxycarbonyl, haloalkoxycarbonyl.

The term "aryl" as used herein refers to a mono-, bi- or tricyclic aromatic hydrocarbon radical such as phenyl or naphthyl, in particular phenyl.

The term "het(ero)aryl" as used herein refers to a mono-, bi- or tricyclic heteroaromatic hydrocarbon radical, preferably to a monocyclic heteroaromatic radical, such as pyridyl, pyrimidyl and the like.

The term "3-, 4-, 5- or 6-membered monocyclic or 8-, 9- or 10-membered bicyclic saturated, unsaturated or aromatic heterocycle containing 1, 2, 3 or 4 heteroatoms as ring members selected from the groups consisting of N, O and S" as used herein denotes monocyclic or bicyclic radicals, the monocyclic or bicyclic radicals being saturated, unsaturated or aromatic where N can optionally be oxidized, i.e. in the form of an N-oxide, and S can also optionally be oxidized to various oxidation states, i.e. as SO or SO₂. An unsaturated heterocycle contains at least one C-C and/or C-N and/or N-N double bond(s). A fully unsaturated heterocycle contains as many conjugated C-C and/or C-N and/or N-N double bonds as allowed by the size(s) of the ring(s). An aromatic monocyclic heterocycle is a fully unsaturated 5- or 6-membered monocyclic heterocycle. An aromatic bicyclic heterocycle is an 8-, 9- or 10-membered bicyclic heterocycle consisting of a 5- or 6-membered heteroaromatic ring which is fused to a phenyl ring or to another 5- or 6-membered heteroaromatic ring. The heterocycle may be attached to the remainder of the molecule via a carbon ring member or via a nitrogen ring member. As a matter of course, the heterocyclic ring contains at least one carbon ring atom. If the ring contains more than one O ring atom, these are not adjacent.

Examples of a 3-, 4-, 5- or 6-membered monocyclic saturated heterocycle include: oxirane-2-yl, aziridine-1-yl, aziridine-2-yl, oxetan-2-yl, azetidine-1-yl, azetidine-2-yl, azetidine-3-yl, thietane-1-yl, thietan-2-yl, thietane-3-yl, tetrahydrofuran-2-yl, tetrahydrofuran-3-yl, tetrahydrothien-2-yl, tetrahydrothien-3-yl, pyrrolidin-1-yl, pyrrolidin-2-yl, pyrrolidin-3-yl, pyrazolidin-1-yl, pyrazolidin-3-yl, pyrazolidin-4-yl, pyrazolidin-5-yl, imidazolidin-1-yl, imidazolidin-2-yl, imidazolidin-4-yl, oxazolidin-2-yl, oxazolidin-3-yl, oxazolidin-4-yl, oxazolidin-5-yl, isoxazolidin-2-yl, isoxazolidin-3-yl, isoxazolidin-4-yl, isoxazolidin-5-yl, thiazolidin-2-yl, thiazolidin-3-yl, thiazolidin-4-yl, thiazolidin-5-yl, isothiazolidin-2-yl, isothiazolidin-3-yl, isothiazolidin-4-yl, isothiazolidin-5-yl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-1-yl, 1,3,4-triazolidin-2-yl, 2-tetrahydropyranyl, 4-tetrahydropyranyl, 1,3-dioxan-5-yl, 1,4-dioxan-2-yl, piperidin-1-yl, piperidin-2-yl, piperidin-3-yl, piperidin-4-yl, hexahydropyridazin-3-yl, hexahydropyridazin-4-yl, hexahydropyrimidin-2-yl, hexahydropyrimidin-4-yl, hexahydropyrimidin-5-yl, piperazin-1-yl, piperazin-2-yl, 1,3,5-hexahydrotriazin-1-yl, 1,3,5-hexahydrotriazin-2-yl and 1,2,4-hexahydrotriazin-3-yl, morpholin-2-yl, morpholin-3-yl, morpholin-4-yl, thiomorpholin-2-yl, thiomorpholin-3-yl, thiomorpholin-4-yl, 1-oxothiomorpholin-2-yl, 1-oxothiomorpholin-3-yl, 1-oxothiomorpholin-4-yl, 1,1-dioxothiomorpholin-2-yl, 1,1-dioxothiomorpholin-3-yl, 1,1-dioxothiomorpholin-4-yl and the like.

Examples of a 5- or 6-membered monocyclic partially unsaturated heterocycle include: 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1 -yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-, 3-, 4-, 5- or 6-di- or tetrahydropyridinyl, 3-di- or tetrahydropyridazinyl, 4-di- or tetrahydropyridazinyl, 2-di- or tetrahydropyrimidinyl, 4-di- or tetrahydropyrimidinyl, 5-di- or tetrahydropyrimidinyl, di- or tetrahydropyrazinyl, 1,3,5-di- or tetrahydrotriazin-2-yl and 1,2,4-di- or tetrahydrotriazin-3-yl.

A 5- or 6-membered monocyclic fully unsaturated (including aromatic) heterocyclic ring is e.g. a 5- or 6-membered monocyclic fully unsaturated (including aromatic) heterocyclic ring. Examples are: 2-furyl, 3-furyl, 2-thienyl, 3-thienyl, 1-pyrrolyl, 2-pyrrolyl, 3-pyrrolyl, 1-pyrazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 1-imidazolyl, 2-imidazolyl, 4-imidazolyl, 1,3,4-triazol-1-yl, 1,3,4-triazol-2-yl, 2-pyridinyl, 3-pyridinyl, 4-pyridinyl, 1-oxopyridin-2-yl, 1-oxopyridin-3-yl, 1-oxopyridin-4-yl,3-pyridazinyl, 4-pyridazinyl, 2-pyrimidinyl, 4-pyrimidinyl, 5-pyrimidinyl and 2-pyrazinyl.

Examples of a 5- or 6-membered heteroaromatic ring fused to a phenyl ring or to a 5- or 6-membered heteroaromatic radical include benzofuranyl, benzothienyl, indolyl, indazolyl, benzimidazolyl, benzoxathiazolyl, benzoxadiazolyl, benzothiadiazolyl, benzoxazinyl, chinolinyl, isochinolinyl, purinyl, 1,8-naphthyridyl, pteridyl, pyrido[3,2-d]pyrimidyl or pyridoimidazolyl and the like.

If two radicals bound on the same nitrogen atom (for example R^{2a} and R^{2b} or R^{3c} and R^{3d} or R^{3e} and R^{3f} or R^{3g} and R^{3h} or R^{4e} and R^{4f} or R^{4g} and R^{4h}) together with the nitrogen atom, to which they are bound, form a 5-, 6 or 7-membered, saturated or unsaturated N-bound heterocyclic radical, which may carry as a ring member a further heteroatom selected from O, S and N, this is for example pyrrolidine-1-yl, pyrazolidin-1-yl, imidazolidin-1-yl, oxazolidin-3-yl, thiazolidin-3-yl, isoxazolidin-2-yl, isothiazolin-2-yl, [1,2,3]-triazolidin-1-yl, [1,2,3]-triazolidin-2-yl, [1,2,4]-triazolidin-1-yl, [1,2,4]-triazolidin-4-yl, [1,2,3]-oxadiazolidin-2-yl, [1,2,3]-oxadiazolidin-3-yl, [1,2,5]-oxadiazolidin-2-yl, [1,2,4]-oxadiazolidin-2-yl, [1,2,4]-oxadiazolidin-4-yl, [1,3,4]-oxadiazolidin-3-yl, [1,2,3]-thiadiazolidin-2-yl, [1,2,3]- thiadiazolidin-3-yl, [1,2,5]-thiadiazolidin-2-yl, [1,2,4]-thiadiazolidin-2-yl, [1,2,4]-thiadiazolidin-4-yl, [1,3,4]-thiadiazolidin-3-yl, piperdin-1-yl, piperazine-1-yl, morpholin-1-yl, thiomorpholin-1-yl, 1-oxothiomorpholin-1-yl, 1,1-dioxothiomorpholin-1-yl, azepan-1-yl, 1,4-diazepan-1-yl, pyrrolin-1-yl, pyrazolin-1-yl, imidazolin-1-yl, oxazolin-3-yl, isoxazolin-2-yl, thiazolin-3-yl, isothiazolin-1-yl, 1,2-dihydropyridin-1-yl, 1,2,3,4-tetrahydropyridin-1-yl, 1,2,5,6-tetrahydropyridin-1-yl, 1,2-dihydropyridazin, 1,6-dihydropyridazin, 1,2,3,4-tetrahydropyridazin-1-yl, 1,2,5,6-tetrahydropyridazin-1-yl, 1,2-dihydropyrimidin, 1,6-dihydropyrimidin, 1,2,3,4-tetrahydropyrimidin-1-yl, 1,2,5,6-tetrahydropyrimidin-1-yl, 1,2-dihydropyrazin-1-yl, 1,2,3,4-tetrahydropyrazin-1-yl, 1,2,5,6-tetrahydropyrazin-1-yl, pyrrol-1-yl, pyrazol-1-yl, imidazol-1-yl, [1,2,3]-1H-triazol-1-yl, [1,2,3]-2H-triazol-2-yl, [1,2,4]-1H-triazol-1-yl and [1,2,4]-4H-triazol-4-yl.

The remarks made below as to preferred embodiments of the variables (substituents) of the compounds of formula I are valid on their own as well as preferably in combination with each other, as well as in combination with the stereoisomers, salts, or tautomers thereof.

The remarks made below concerning preferred embodiments of the variables further are valid on their own as well as preferably in combination with each other concerning the compounds of formulae I, where applicable, as well as concerning the uses and methods according to the invention and the composition according to the invention.

Preferred compounds according to the invention are compounds of formula I, including its stereoisomers, or an N-oxide or salt thereof, wherein the salt is an agriculturally suitable salt.

According to one group 1 of embodiments of the invention the variable Q in the compounds of formula I is Q¹: 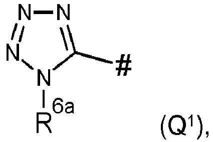 where # in formula Q¹ indicates the point of attachment to the nitrogen atom. Compounds of formula I wherein Q is Q¹ have the following formula I.A, where the variables R¹, Z², R^{2a}, R^{2b}, R^{2c}, R³, R⁴, R⁵, R^{6a} and R⁷ are as defined herein:

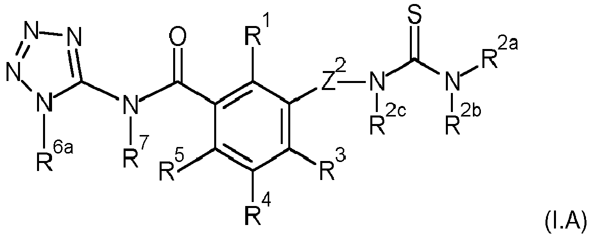

According to another group 2 of embodiments of the invention the variable Q in the compounds of formula I is Q²: 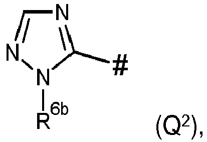 where # in formula Q² indicates the point of attachment to the nitrogen atom. Compounds of formula I wherein Q is Q² have the following formula I.B, where the variables R¹, Z², R^{2a}, R^{2b}, R^{2c}, R³, R⁴, R⁵, R^{6b} and R⁷ are as defined herein:

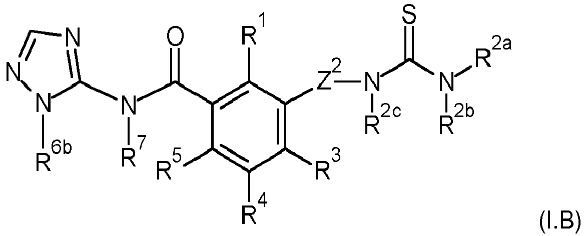

According to a further group 3 of embodiments of the invention the variable Q in the compounds of formula I is Q³: 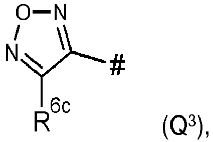 where # in formula Q³ indicates the point of attachment to the nitrogen atom. Compounds of formula I wherein Q is Q³ have the following formula I.C, where the variables R¹, Z², R^{2a}, R^{2b}, R^{2c}, R³, R⁴, R⁵, R^{6c} and R⁷ are as defined herein:

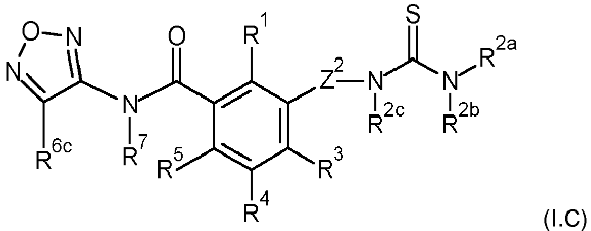

According to yet a further group 4 of embodiments of the invention the variable Q in the compounds of formula I is Q⁴: 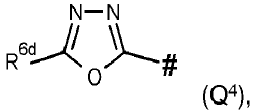 where # in formula Q³ indicates the point of attachment to the nitrogen atom. Compounds of formula I wherein Q is Q⁴ have the following formula I.D, where the variables R¹, Z², R^{2a}, R^{2b}, R^{2c}, R³, R⁴, R⁵, R^{6d} and R⁷ are as defined herein:

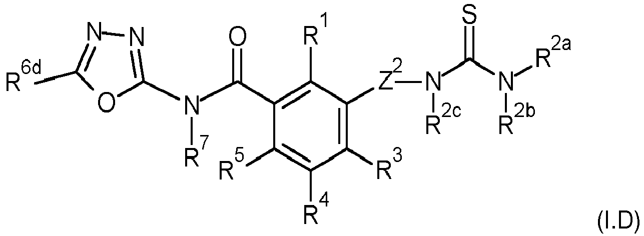

Amongst the compounds of formula I, their N-oxides and their agriculturally suitable salts, particular preference is given to the compounds of group 1 of embodiments.

Amongst the compounds of formula I, their N-oxides and their agriculturally suitable salts, particular preference is also given to the compounds of group 2 of embodiments.

The compounds of formula I of the present invention are preferably selected from compounds of the formulae I.A, I.B, I.C and I.D, their N-oxides and their agriculturally suitable salts, where the variables R¹, Z², R^{2a}, R^{2b}, R^{2c}, R³, R⁴, R⁵, R⁷ and R^{6a}, R^{6b}, R^{6c} or R^{6d} are as defined herein and in particular have the preferred meanings given herein below. The following preferred meanings apply to each of groups 1, 2, 3 and 4 of embodiments in the same manner.

A particular group of compounds according to the invention are compounds of formulae I, I.A, I.B, I.C and I.D, their N-oxides and their salts, wherein Z² is a covalent bond, i.e. a covalent single bond. These compounds are hereinafter termed compounds of the formulae I' , I.A' , I.B' , I.C' and I.D' , respectively.

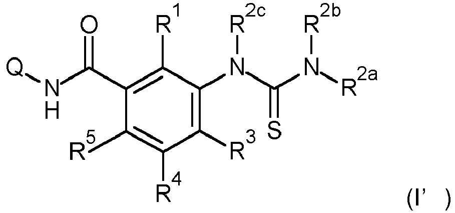

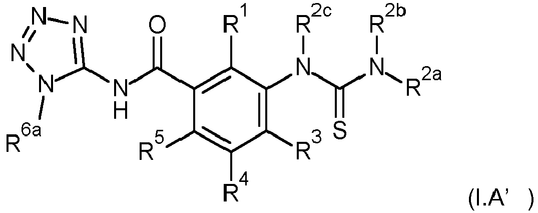

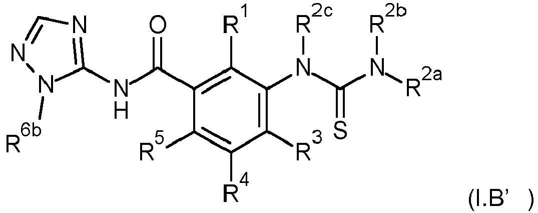

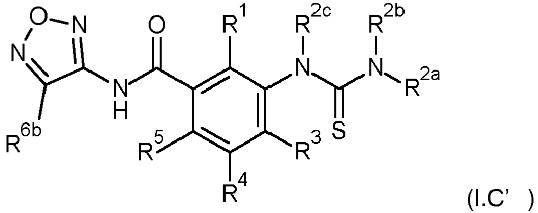

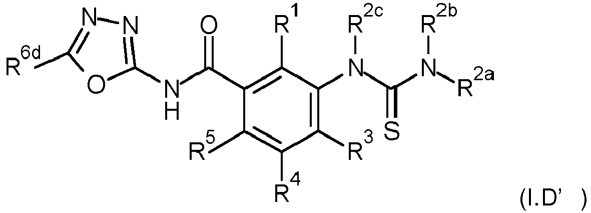

Another particular group of compounds according to the invention are compounds of formulae I, I.A, I.B, I.C and I.D, their N-oxides and their salts, wherein Z² is a bivalent radical, which is in particular linear C₁-C₄-alkanediyl and especially -CH₂- or -CH₂CH₂-.

Preferred compounds according to the invention are compounds of formulae I, I.A, I.B, I.C and I.D, and likewise compounds of the formulae I' , I.A' , I.B' , I.C' and I.D, their N-oxides and their salts, wherein R¹ is selected from the group consisting of cyano, halogen, nitro, C₁-C₆-alkyl, C₁-C₆-haloalkyl, C₁-C₄-alkoxy-C₁-C₄-alkyl, C₁-C₄-haloalkoxy-C₁-C₄-alkyl, C₁-C₆-alkoxy, C₁-C₄-alkoxy-C₁-C₄-alkoxy-Z¹, R^{1b}-S(O)ₖ-Z¹, where k and Z¹ are as defined herein and where R^{1b} is as defined above and in particular selected from the group consisting of C₁-C₄-alkyl and C₁-C₄-haloalkyl. In this context Z¹ is in particular a covalent bond.

More preferably, R¹ in formulae I, I.A, I.B, I.C, I.D, I' , I.A' , I.B' , I.C' and I.D is selected from halogen, nitro, cyano, C₁-C₄-alkyl, C₁-C₄-haloalkyl, C₁-C₄-alkoxy-C₁-C₄-alkoxy-Z¹ and R^{1b}-S(O)ₖ-Z¹, where k and Z¹ are as defined herein and where R^{1b} is as defined above and in particular selected from the group consisting of C₁-C₄-alkyl and C₁-C₄-haloalkyl. In this context Z¹ is in particular a covalent bond.

In particular, R¹ in formulae I, I.A, I.B, I.C, I.D, I' , I.A' , I.B' , I.C' and I.D is selected from the group consisting of halogen, nitro, C₁-C₄-alkyl, C₁-C₄-haloalkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl. Specifically R¹ in formulae I, I.A, I.B, I.C, I.D, I' , I.A' , I.B' , I.C' and I.D is selected from the group consisting of F, Cl, Br, I, nitro, CH₃, CF₃, SCH₃, SCF₃, SO₂CH₃ and CH₂OCH₂CH₂OCH₃. More specifically R¹ in formulae I, I.A, I.B, I.C, I.D, I' , I.A' , I.B' , I.C' and I.D is selected from the group consisting of F, Cl, Br, I, nitro, CH₃, CF₃, SCH₃ and SO₂CH₃, even more specifically R¹ in formulae I, I.A, I.B, I.C, I.D, I' , I.A' , I.B' , I.C' and I.D is selected from the group consisting of Cl, Br, CH₃ and CF₃, and especially R¹ in formulae I, I.A, I.B, I.C, I.D, I' , I.A' , I.B' , I.C' and I.D is Cl or CH₃.

Preferred compounds according to the invention are compounds of formulae I, I.A, I.B, I.C and I.D, and likewise compounds of the formulae I' , I.A' , I.B' , I.C' and I.D' , their N-oxides and their salts, wherein R^{2a} is selected from the group consisting of hydrogen, C₁-C₆-alkyl, C₁-C₆-alkoxy, C₃-C₇-CyCloalkyl, C₃-C₇-cycloalkyl-C₁-C₄-alkyl, C₁-C₆-haloalkyl, phenyl, benzyl, and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, benzyl and heterocyclyl are unsubstituted or substituted by 1, 2, 3 or 4 groups R²¹, which are identical or different and where R²¹ is preferably selected from the group consisting of halogen, CN, C₁-C₄-alkyl, C₁-C₄-haloalkyl, C₁-C₄-alkoxy and C₁-C₄-haloalkoxy and where R²¹ is in particular selected from the group consisting of halogen, C₁-C₄-alkyl, C₁-C₂-fluoroalkyl, C₁-C₄-alkoxy and C₁-C₄-fluoroalkoxy and especially from the group consisting of halogen, methyl and methoxy.

Preference is given to compounds of the invention, where the radical R^{2a} in formulae I, I.A, I.B, I.C, I.D, I' , I.A' , I.B' , I.C' and I.D' is different from hydrogen.

In the context of R^{2a}, heterocyclyl is in particular a 5- or 6-membered monocyclic saturated or aromatic heterocycle, which contains 1, 2 or 3 heteroatoms as ring members, which are selected from the group consisting of O, N and S. In this context, heterocyclyl is in particular a 5- or 6-membered monocyclic aromatic heterocycle, which contains 1 or 2 heteroatoms as ring members, which are selected from the group consisting of O, N and S;

In formulae I, I.A, I.B, I.C, I.D, I' , I.A' , I.B' , I.C' and I.D' , R^{2a} is in particular selected from the group consisting of C₁-C₄-alkyl, C₁-C₄-alkoxy, C₃-C₆-cycloalkyl, C₃-C₆-cycloalkylmethyl, C₁-C₄-fluoroalkyl, phenyl and benzyl, where phenyl and benzyl are unsubstituted or substituted by 1, 2, 3 or 4 radicals R²¹, which are identical or different and where R²¹ is preferably selected from the group consisting of halogen, CN, C₁-C₄-alkyl, C₁-C₄-haloalkyl, C₁-C₄-alkoxy and C₁-C₄-haloalkoxy and where R²¹ is in particular selected from the group consisting of halogen, C₁-C₄-alkyl, C₁-C₂-fluoroalkyl, C₁-C₄-alkoxy and C₁-C₄-fluoroalkoxy and especially from the group consisting of halogen, methyl and methoxy.

In formulae I, I.A, I.B, I.C, I.D, I' , I.A' , I.B' , I.C' and I.D' , R^{2a} is more particularly selected from the group consisting of C₁-C₄-alkyl, C₃-C₇-CyCloalkyl, C₁-C₄-fluoroalkyl, C₁-C₄-alkoxy and phenyl, where phenyl is unsubstituted or substituted by 1 or 2 groups R²¹, which are identical or different and which are preferably selected from the group consisting of halogen, C₁-C₄-alkyl, C₁-C₂-fluoroalkyl, C₁-C₄-alkoxy and C₁-C₂-fluoroalkoxy and especially from the group consisting of halogen, methyl and methoxy.

In formulae I, I.A, I.B, I.C, I.D, I', I.A', I.B', I.C' and I.D', R^{2a} is even more particularly selected from the group consisting of C₁-C₄-alkyl, C₁-C₂-fluoroalkyl, C₃-C₄-cycloalkyl, and phenyl, which is unsubstituted or substituted by 1, 2 or 3 radicals R²¹ selected from the group consisting of halogen, C₁-C₄-alkyl, C₁-C₂-fluoroalkyl, C₁-C₄-alkoxy and C₁-C₂-fluoroalkoxy and where phenyl is in particular unsubstituted or substituted by 1, 2 or 3 radicals selected from the group consisting of halogen, methyl and methoxy.

In formulae I, I.A, I.B, I.C, I.D, I', I.A', I.B', I.C' and I.D', R^{2a} is especially selected from the group consisting of methyl, ethyl, isopropyl (iPr), cyclopropyl (cPr), CHF₂CH₂-, CF₃CH₂-, CH₃O-, 4-Cl-phenyl, 4-methoxyphenyl, 4-methylphenyl and 2,6-dimethylphenyl.

Preferred compounds according to the invention are compounds of formulae I, I.A, I.B, I.C and I.D, and likewise compounds of the formulae I', I.A', I.B', I.C' and I.D', their N-oxides and their salts, wherein R^{2b} is selected from the group consisting of hydrogen, C₁-C₆-alkyl, C₁-C₆-alkoxy, C₃-C₇-cycloalkyl, C₃-C₇-cycloalkyl-C₁-C₄-alkyl, C₁-C₆-haloalkyl, phenyl, benzyl, and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, benzyl and heterocyclyl are unsubstituted or substituted by 1, 2, 3 or 4 groups R²¹, which are identical or different and where R²¹ is preferably selected from the group consisting of halogen, CN, C₁-C₄-alkyl, C₁-C₄-haloalkyl, C₁-C₄-alkoxy and C₁-C₄-haloalkoxy and where R²¹ is in particular selected from the group consisting of halogen, C₁-C₄-alkyl, C₁-C₂-fluoroalkyl, C₁-C₄-alkoxy and C₁-C₂-fluoroalkoxy and especially from the group consisting of halogen, methyl and methoxy.

Preference is given to compounds of the invention, where the radical R^{2b} in formulae I, I.A, I.B, I.C, I.D, I', I.A', I.B', I.C' and I.D' is different from hydrogen.

In the context of R^{2b}, heterocyclyl is in particular a 5- or 6-membered monocyclic saturated or aromatic heterocycle, which contains 1, 2 or 3 heteroatoms as ring members, which are selected from the group consisting of O, N and S. In this context, heterocyclyl is in particular a 5- or 6-membered monocyclic aromatic heterocycle, which contains 1 or 2 heteroatoms as ring members, which are selected from the group consisting of O, N and S.

In formulae I, I.A, I.B, I.C, I.D, I', I.A', I.B', I.C' and I.D', R^{2b} is in particular selected from the group consisting of hydrogen, C₁-C₄-alkyl, C₃-C₆-cycloalkyl, C₃-C₆-cycloalkyl-methyl and C₁-C₄-fluoroalkyl.

In formulae I, I.A, I.B, I.C, I.D, I', I.A', I.B', I.C' and I.D', R^{2b} is more particularly selected from the group consisting of hydrogen, C₁-C₄-alkyl, C₃-C₇-cycloalkyl and C₁-C₄-fluoroalkyl.

In formulae I, I.A, I.B, I.C, I.D, I', I.A', I.B', I.C' and I.D', R^{2b} is even more particularly selected from the group consisting of hydrogen, C₁-C₄-alkyl, C₁-C₂-fluoroalkyl and C₃-C₄-cycloalkyl.

In formulae I, I.A, I.B, I.C, I.D, I', I.A', I.B' , I.C' and I.D', R^{2b} is especially selected from the group consisting of hydrogen, methyl, ethyl, isopropyl (iPr), cyclopropyl (cPr), CHF₂CH₂- and CF₃CH₂-.

Likewise, preference is given to the compounds of formulae I, I.A, I.B, I.C, I.D, I', I.A', I.B', I.C' and I.D', their N-oxides and their salts, wherein R^{2a} and R^{2b} together with the nitrogen atom, to which they are bound form a 4-, 5-, 6- or 7-membered, saturated or unsaturated heterocyclic radical, which may carry as a ring member a further heteroatom selected from O, S and N and which is unsubstituted or may carry 1, 2, 3 or 4 groups R^{2d}, which are identical or different and in particular selected from the group consisting of =O, OH, halogen, C₁-C₄-alkyl, C₁-C₄-haloalkyl, C₁-C₄-alkoxy and C₁-C₄-haloalkoxy. Amongtst these, particular preference is given to those compounds, where in formulae I, I.A, I.B, I.C, I.D, I', I.A', I.B', I.C' and I.D' the variables R^{2a} and R^{2b} together with the nitrogen atom, to which they are bound may form a 5- or 6- membered, saturated or unsaturated heterocyclic radical, which may carry as a ring member a further heteroatom selected from O, S and N and which is unsubstituted or may carry 1, 2, 3 or 4 groups R^{2d}, which are identical or different and in particular selected from the group consisting of =O, halogen, C₁-C₄-alkyl, C₁-C₄-haloalkyl, C₁-C₄-alkoxy and C₁-C₄-haloalkoxy. Amongtst these, special preference is given to those compounds, where in formulae I, I.A, I.B, I.C, I.D, I', I.A', I.B', I.C' and I.D' the variables, R^{2a} and R^{2b} together with the nitrogen atom, to which they are bound form 4-morpholinyl, 4-thiomorpholinyl, 1-piperidinyl and 1-pyrrolidinyl, where 4-morpholinyl, 4-thiomorpholinyl, 1-piperidinyl and 1-pyrrolidinyl are unsubstituted or carry 1, 2, 3 or 4 groups R^{2d} which are selected from the group consisting of C₁-C₄-alkyl and C₁-C₄-fluoroalkyl, preferably from methyl, ethyl and trifluoromethyl.

Preferred compounds according to the invention are compounds of formulae I, I.A, I.B, I.C and I.D, and likewise compounds of the formulae I', I.A', I.B', I.C' and I.D', their N-oxides and their salts, wherein R^{2c} is hydrogen or C₁-C₄-alkyl and wherein R^{2c} is especially hydrogen.

In formulae I, I.A, I.B, I.C, I.D, I', I.A', I.B', I.C' and I.D', the combination of R^{2a}, R^{2b} and R^{2c} is in particular as follows:
- R^{2a}: is seleced from the group consisting of C₁-C₄-alkyl, C₁-C₄-alkoxy, C₃-C₆-cycloalkyl, C₃-C₆-cycloalkyl-methyl, C₁-C₄-fluoroalkyl, phenyl and benzyl, where phenyl and benzyl are unsubstituted or substituted by 1, 2, 3 or 4 radicals R²¹, which are identical or different and where R²¹ is preferably selected from the group consisting of halogen, CN, C₁-C₄-alkyl, C₁-C₄-haloalkyl, C₁-C₄-alkoxy and C₁-C₄-haloalkoxy and where R²¹ is in particular selected from the group consisting of halogen, C₁-C₄-alkyl, C₁-C₂-fluoroalkyl, C₁-C₄-alkoxy and C₁-C₂-fluoroalkoxy and especially from the group consisting of halogen, methyl and methoxy; hydrogen, C₁-C₄-alkyl, C₃-C₆-cycloalkyl, C₃-C₆-cycloalkyl-methyl and C₁-C₄-fluoroalkyl, R^{2a} is more particularly selected from the group consisting of C₁-C₄-alkyl, C₃-C₇-cycloalkyl, C₁-C₄-fluoroalkyl, C₁-C₄-alkoxy and phenyl, where phenyl is unsubstituted or substituted by 1 or 2 groups R²¹, which are identical or different and which are preferably selected from the group consisting of halogen, C₁-C₄-alkyl, C₁-C₂-fluoroalkyl, C₁-C₄-alkoxy and C₁-C₂-fluoroalkoxy and especially from the group consisting of halogen, methyl and methoxy, R^{2a} is even more particularly selected from the group consisting of C₁-C₄-alkyl, C₁-C₂-fluoroalkyl, C₃-C₄-cycloalkyl, and phenyl, which is unsubstituted or substituted by 1, 2 or 3 radicals R²¹ selected from the group consisting of halogen, C₁-C₄-alkyl, C₁-C₂-fluoroalkyl, C₁-C₄-alkoxy and C₁-C₂-fluoroalkoxy and where phenyl is in particular unsubstituted or substituted by 1, 2 or 3 radicals selected from the group consisting of halogen, methyl and methoxy,
- R^{2a}: is especially selected from the group consisting of methyl, ethyl, isopropyl (iPr), cyclo-propyl (cPr), CHF₂CH₂-, CF₃CH₂-, CH₃O-, 4-Cl-phenyl, 4-methoxyphenyl, 4-methylphenyl and 2,6-dimethylphenyl;
- R^{2b}: is selected from the group consisting of hydrogen, C₁-C₄-alkyl, C₃-C₇-cycloalkyl and C₁-C₄-fluoroalkyl, in particular selected from the group consisting of hydrogen, C₁-C₄-alkyl, C₁-C₂-fluoroalkyl and C₃-C₄-cycloalkyl and especially selected from the group consisting of hydrogen, methyl, ethyl, isopropyl (iPr), cyclopropyl (cPr), CHF₂CH₂- and CF₃CH₂-;
- or R^{2a} and R^{2b}: together with the nitrogen atom, to which they are bound may form a 5- or 6- membered, saturated or unsaturated heterocyclic radical, which may carry as a ring member a further heteroatom selected from O, S and N and which is unsubstituted or may carry 1, 2, 3 or 4 groups R^{2d}, which are identical or different and in particular selected from the group consisting of =O, halogen, C₁-C₄-alkyl, C₁-C₄-haloalkyl, C₁-C₄-alkoxy and C₁-C₄-haloalkoxy, and where R^{2a} and R^{2b} together with the nitrogen atom, to which they are bound in particular form 4-morpholinyl, 4-thiomorpholinyl, 1-piperidinyl and 1-pyrrolidinyl, where 4-morpholinyl, 4-thiomorpholinyl, 1-piperidinyl and 1-pyrrolidinyl are unsubstituted or carry 1, 2, 3 or 4 groups R^{2d} which are selected from the group consisting of C₁-C₄-alkyl and C₁-C₄-fluoroalkyl, preferably from methyl, ethyl and trifluoromethyl.
- R^{2c}: is hydrogen or C₁-C₄-alkyl and especially hydrogen.

Preferred compounds according to the invention are compounds of formulae I, I.A, I.B, I.C and I.D, and likewise compounds of the formulae I', I.A', I.B', I.C' and I.D', their N-oxides and their salts, wherein R³ is selected from the group consisting of hydrogen, cyano, halogen, nitro, C₁-C₆-alkyl, C₁-C₈-haloalkyl, C₁-C₄-alkoxy, C₁-C₈-haloalkoxy-Z³, C₂-C₄-alkenyl, C2-C4-alkynyl, C2-C4-alkenyloxy, C2-C4-alkynyloxy and R^{3b}-S(O)ₖ-Z³, where the variables k, R^{3b} and Z³ have one of the herein defined meanings; more preferably, R³ is selected from the group consisting of halogen, nitro, C₁-C₆-alkyl, C₁-C₈-haloalkyl, C₁-C₈-haloalkoxy-Z³, and R^{3b}-S(O)ₖ-Z³, where the variables k, R^{3b} and Z³ have one of the herein defined meanings; even more preferably, R³ is selected from the group consisting of halogen, nitro, C₁-C₄-alkyl, C₁-C₄-haloalkyl, C₁-C₄-haloalkoxy, and R^{3b}-S(O)ₖ, where the variables k and R^{3b} have one of the herein defined meanings.

In the context of R³, R^{3b} is preferably selected from the group consisting of C₁-C₆-alkyl, C₃-C₇-cycloalkyl, C₃-C₇-cycloalkyl-C₁-C₄-alkyl, C₁-C₆-haloalkyl, phenyl, and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy. More preferably, R^{3b} is selected from the group consisting of C₁-C₄-alkyl, C₃-C₇-cycloalkyl, C₁-C₄-haloalkyl, and phenyl, where phenyl is unsubstituted or substituted by 1 or 2 groups, which are identical or different and selected from the group consisting of halogen and C₁-C₄-alkoxy; Herein, particularly preferably, R^{3b} is methyl, ethyl, cyclopropyl (cPr), CF₃, CHF₂CH₂-, CF₃CH₂- or CF₃FF₂-.

In the context of R³, R^{3c} is preferably selected from the group consisting of C₁-C₆-alkyl, C₃-C₇-CyCloalkyl, C₃-C₇-cycloalkyl-C₁-C₄-alkyl, C₁-C₆-haloalkyl, C₁-C₄-alkoxy, C₁-C₆-haloalkoxy, phenyl, and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy. More preferably, R^{3c} is selected from the group consisting of C₁-C₄-alkyl, C₁-C₄-alkoxy, C₃-C₇-cycloalkyl, C₁-C₄-haloalkyl, and phenyl, where phenyl is unsubstituted or substituted by 1 or 2 groups, which are identical or different and selected from the group consisting of halogen and C₁-C₄-alkoxy.

In the context of R³, R^{3d} is preferably selected from the group consisting of C₁-C₆-alkyl, C₃-C₇-cycloalkyl, C₃-C₇-cycloalkyl-C₁-C₄-alkyl, C₁-C₆-haloalkyl and phenyl where phenyl is unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy; more preferably, R^{3d} is selected from the group consisting of C₁-C₄-alkyl, C₃-C₇-cycloalkyl, and C₁-C₄-haloalkyl; particularly preferably, R^{3d} is methyl, ethyl, isopropyl (iPr), cyclopropyl (cPr), CHF₂CH₂- or CF₃CH₂-.

In the context of R³, Z³ is in particular a covaltent bond or linear C₁-C₄-alkanediyl; preferably, Z³ is a covalent bond, -CH₂- or -CH₂CH₂-.

In particular, R³ in formulae I, I.A, I.B, I.C, I.D, I', I.A', I.B', I.C' and I.D' is selected from the group consisting of halogen, C₁-C₄-alkyl, C₁-C₄-haloalkyl, C₁-C₄-haloalkoxy, C₁-C₄-alkylthio, C₁-C₄-haloalkylthio, C₁-C₄-alkyl-S(O), C₁-C₄-haloalkyl-S(O), C₁-C₄-alkyl-S(O)₂, and C₁-C₄-haloalkyl-S(O)₂.

More particular, R³ in formulae I, I.A, I.B, I.C, I.D, I', I.A', I.B', I.C' and I.D' is selected from the group consisting of halogen, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂, especially from the group consisting of CI, F, Br, I, CH₃, CF₃, CHF₂, OCF₃, OCHF₂, SCH₃, SCF₃, SCHF₂, S(O)CH₃, S(O)CH₂CH₃, S(O)₂CH₃ and S(O)₂CH₂CH₃, and more specifically R³ is Cl, Br, CF₃, SCH₃, S(O)CH₃ or S(O)₂CH₃.

Preferred compounds according to the invention are compounds of formulae I, I.A, I.B, I.C and I.D, and likewise compounds of the formulae I', I.A', I.B', I.C' and I.D', their N-oxides and their salts, wherein R⁴ is selected from the group consisting of hydrogen, cyano, halogen, nitro, CH₃, CHF₂, and CF₃, in particular from hydrogen, cyano, chlorine, fluorine and CH₃.

Even more preferred compounds according to the invention are compounds of formulae I, I.A, I.B, I.C, I.D, I', I.A', I.B', I.C' and I.D', their N-oxides and their salts, wherein R⁴ is hydrogen, chlorine or fluorine.

Most preferred compounds according to the invention are compounds of formulae I, I.A, I.B, I.C, I.D, I', I.A', I.B', I.C' and I.D', their N-oxides and their salts, wherein R⁴ is hydrogen.

Preferred compounds according to the invention are compounds of formulae I, I.A, I.B, I.C, I.D, I', I.A', I.B', I.C' and I.D', their N-oxides and their salts, wherein R⁵ is selected from the group consisting of CHF₂, CF₃ and halogen. More preferably, R⁵ is halogen, in particular chlorine or fluorine, especially fluorine.

Preferred compounds according to the invention are compounds of formulae I, I.A, I.B, I.C, I.D, I', I.A', I.B', I.C' and I.D', their N-oxides and their salts, wherein R^{6a}, R^{6b}, R^{6c}, R^{6d} are, independently of each other, selected from the group consisting of C₁-C₆-alkyl, C₁-C₄-alkoxy-C₁-C₄-alkyl, and phenyl. In particular, R^{6a}, R^{6b}, R^{6c}, R^{6d} are, independently of each other, selected from the group consisting of C₁-C₂-alkyl, C₁-C₂-alkoxy-C₁-C₂-alkyl, specifically from CH₃, CH₃CH₂, CH₃OCH₂CH₂ and CH₃OCH₂, and in particular are, independently of each other, CH₃.

In the context of the present invention, the variables R¹¹, R²¹, R²², R²³, R²⁴, R²⁵, Z¹, Z³, Z^{3a}, R^{b}, R^{1b}, R^{3b}, R^{3c}, R^{3d}, R^{3e}, R^{3f}, R^{3g}, R^{3h}, R^{4b}, R^{4c}, R^{4d}, R^{4e}, R^{4f}, R^{4g}, R^{4h}, R^{k}, R^{z}, n and k, independently of each other, preferably have one of the following meanings:
In particular, R¹¹, R²¹ independently of each other are selected from the group consisting of halogen, C₁-C₄-alkyl, C₃-C₆-cycloalkyl, C₃-C₆-halocycloalkyl, C₁-C₄-haloalkyl, C₁-C₄-alkoxy, C₁-C₄-alkoxy-C₁-C₄-alkyl, C₁-C₄-alkylthio-C₁-C₄-alkyl, C₁-C₄-alkoxy-C₁-C₄-alkoxy and C₁-C₆-haloalkyloxy, more preferably from halogen, C₁-C₄-alkyl, C₃-C₆-cycloalkyl, C₁-C₄-haloalkyl and C₁-C₄-alkoxy.

More preferably R¹¹, R²¹ independently of each other are selected from the group consisting of halogen, C₁-C₄-alkyl, C₃-C₆-cycloalkyl, C₁-C₄-haloalkyl, C₁-C₄-alkoxy, C₁-C₄-alkoxy-C₁-C₄-alkyl, C₁-C₄-alkylthio-C₁-C₄-alkyl and C₁-C₄-alkoxy-C₁-C₄-alkoxy; in particular from halogen, C₁-C₄-alkyl, C₁-C₄-alkoxy, C₁-C₄-haloalkyl, C₁-C₄-alkoxy-C₁-C₄-alkyl and C₁-C₄-alkoxy-C₁-C₄-alkoxy; and specifically from Cl, F, Br, methyl, ethyl, methoxy and trifluoromethyl.

In particular, R²² is selected from the group consisting of hydrogen, C₁-C₆-alkyl, C₃-C₆-cycloalkyl, C₃-C₆-halocycloalkyl, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl, benzyl and heterocyclyl, where heterocyclyl is a 5- or 6-membered monocyclic saturated, partially unsaturated or aromatic heterocycle, which contains 1, 2 or 3 heteroatoms as ring members, which are selected from the group consisting of O, N and S, where the rings of phenyl, benzyl and heterocyclyl are unsubstituted or substituted by 1, 2 or 3 groups, which are identical or different and selected from the group consisting of halogen, C₁-C₄-alkyl, C₁-C₄-haloalkyl and C₁-C₄-alkoxy.

More preferably R²² is selected from hydrogen, C₁-C₄-alkyl, C₁-C₄-haloalkyl, C₂-C₄-alkenyl, C₂-C₄-haloalkenyl, C₂-C₄-alkynyl, C₃-C₆-cycloalkyl, phenyl and heterocyclyl, where heterocyclyl is a 5- or 6-membered monocyclic saturated, partially unsaturated or aromatic heterocycle, which contains 1, 2 or 3 heteroatoms as ring members, which are selected from the group consisting of O, N and S.

Especially, R²² is selected from the group consisting of hydrogen, C₁-C₄-alkyl, C₁-C₄-haloalkyl, C₂-C₄-alkenyl, C₂-C₄-haloalkenyl, C₃-C₆-cycloalkyl, phenyl and heterocyclyl, where heterocyclyl is a 5- or 6-membered aromatic heterocyclic radical having 1 or 2 nitrogen atoms as ring members.

In particular, R²³ is selected from the group consisting of hydrogen, C₁-C₆-alkyl, C₁-C₆-haloalkyl, C₂-C₆-alkenyl, C₃-C₆-cycloalkyl and phenyl. More preferably R²³ is selected from the group consisting of hydrogen, C₁-C₄-alkyl, C₁-C₄-haloalkyl and C₃-C₆-cycloalkyl; and in particular, R²³ is selected from hydrogen, C₁-C₃-alkyl and C₁-C₃-haloalkyl.

R²⁴ is in particular selected from the group consisting of C₁-C₆-alkyl, C₁-C₆-haloalkyl, C₂-C₆-alkenyl, C₃-C₆-cycloalkyl and phenyl.

More preferably R²⁴ is in particular selected from the group consisting of C₁-C₄-alkyl, C₁-C₄-haloalkyl and C₃-C₆-cycloalkyl; and in particular R²³ is selected from the group consisting of C₁-C₃-alkyl and C₁-C₃-haloalkyl.

R²⁵ is in particular selected from the group consisting of C₁-C₆-alkyl, C₃-C₆-cycloalkyl, C₃-C₆-halocycloalkyl, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl, benzyl and heterocyclyl, where heterocyclyl is a 5- or 6-membered monocyclic saturated, partially unsaturated or aromatic heterocycle, which contains 1, 2 or 3 heteroatoms as ring members, which are selected from the group consisting of O, N and S, where the rings of phenyl, benzyl and heterocyclyl are unsubstituted or substituted by 1, 2 or 3 groups, which are identical or different and selected from the group consisting of halogen, C₁-C₄-alkyl, C₁-C₄-haloalkyl and C₁-C₄-alkoxy.

More preferably R²⁵ is selected from the group consisting of C₁-C₄-alkyl, C₁-C₄-haloalkyl, C₂-C₄-alkenyl, C₂-C₄-haloalkenyl, C₂-C₄-alkynyl, C₃-C₆-cycloalkyl, phenyl and heterocyclyl, where heterocyclyl is a 5- or 6-membered monocyclic saturated, partially unsaturated or aromatic heterocycle, which contains 1, 2 or 3 heteroatoms as ring members, which are selected from the group consisting of O, N and S.

In particular, R²⁵ is selected from C₁-C₄-alkyl, C₁-C₄-haloalkyl, C₂-C₄-alkenyl, C₂-C₄-haloalkenyl, C₃-C₆-cycloalkyl, phenyl and heterocyclyl, where heterocyclyl is a 5- or 6-membered aromatic heterocyclic radical having 1 or 2 nitrogen atoms as ring members.

Z¹, Z³ independently of each other are in particular selected from the group consisting of a covalent bond, methanediyl and ethanediyl, and in particular are a covalent bond.

Z^{3a} is in particular selected from the group consisting of a covalent bond, C₁-C₂-alkanediyl, O-C₁-C₂-alkanediyl, C₁-C₂-alkanediyl-O and C₁-C₂-alkanediyl-O-C₁-C₂-alkanediyl; more preferably from a covalent bond, methanediyl, ethanediyl, O-methanediyl, O-ethanediyl, methanediyl-O, and ethanediyl-O; and in particular from a covalent bond, methanediyl and ethanediyl.

R^{b}, R^{1b}, R^{3b}, R^{4b} independently of each other are in particular selected from the group consisting of C₁-C₆-alkyl, C₃-C₇-cycloalkyl, C₁-C₆-haloalkyl, C₂-C₆-alkenyl, C₂-C₆-haloalkenyl, C₂-C₆-alkynyl, C₂-C₆-haloalkynyl, phenyl and heterocyclyl, where heterocyclyl is a 5- or 6-membered monocyclic saturated, partially unsaturated or aromatic heterocycle, which contains 1, 2 or 3 heteroatoms as ring members, which are selected from the group consisting of O, N and S, where phenyl and heterocyclyl are unsubstituted or substituted by 1, 2 or 3 groups, which are identical or different and selected from the group consisting of halogen, C₁-C₄-alkyl, C₁-C₂-haloalkyl and C₁-C₂-alkoxy.

More preferably R^{b}, R^{1b}, R^{3b}, R^{4b} independently of each other are selected from the group consisting of C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₃-C₆-cycloalkyl, phenyl and heterocyclyl, where heterocyclyl is a 5- or 6-membered monocyclic saturated, partially unsaturated or aromatic heterocycle, which contains 1, 2 or 3 heteroatoms as ring members, which are selected from the group consisting of O, N and S.

In particular, R^{b}, R^{1b}, R^{3b}, R^{4b} independently of each other are selected from the group consisting of C₁-C₄-alkyl, C₁-C₄-haloalkyl, C₂-C₄-alkenyl, C₂-C₄-haloalkenyl, C₂-C₄-alkynyl, C₃-C₆-cycloalkyl, phenyl and heterocyclyl, where heterocyclyl is a 5- or 6-membered aromatic heterocyclic radical having 1 or 2 nitrogen atoms as ring members.

R^{3c}, R^{4c}, R^{k} independently of each other are in particular selected from the group consisting of hydrogen, C₁-C₆-alkyl, C₃-C₇-cycloalkyl, which is unsubstituted or partly or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl, benzyl and heterocyclyl, where heterocyclyl is a 5- or 6-membered monocyclic saturated, partially unsaturated or aromatic heterocycle, which contains 1, 2 or 3 heteroatoms as ring members, which are selected from the group consisting of O, N and S, where phenyl, benzyl and heterocyclyl are unsubstituted or substituted by 1, 2 or 3 groups, which are identical or different and selected from the group consisting of halogen, C₁-C₄-alkyl, C₁-C₄-haloalkyl and C₁-C₄-alkoxy.

More preferably R^{3c}, R^{4c}, R^{k} independently of each other are selected from the group consisting of hydrogen, C₁-C₄-alkyl, C₁-C₄-haloalkyl, C₂-C₄-alkenyl, C₂-C₄-haloalkenyl, C₂-C₄-alkynyl, C₃-C₆-cycloalkyl, phenyl and heterocyclyl, where heterocyclyl is a 5- or 6-membered monocyclic saturated, partially unsaturated or aromatic heterocycle, which contains 1, 2 or 3 heteroatoms as ring members, which are selected from the group consisting of O, N and S.

In particular, R^{3c}, R^{4c}, R^{k} independently of each other are selected from the group consisting of hydrogen, C₁-C₄-alkyl, C₁-C₄-haloalkyl, C₂-C₄-alkenyl, C₂-C₄-haloalkenyl, C₃-C₆-cycloalkyl, phenyl and heterocyclyl, where heterocyclyl is a 5- or 6-membered aromatic heterocyclic radical having 1 or 2 nitrogen atoms as ring members.

R^{3d}, R^{4d} independently of each other are in particular selected from the group consisting of C₁-C₆-alkyl, C₃-C₇-cycloalkyl, which is unsubstituted or partly or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl and benzyl.

More preferably R^{3d}, R^{4d} independently of each other are selected from the group consisting of C₁-C₆-alkyl, C₁-C₆-haloalkyl, C₂-C₆-alkenyl, C₂-C₆-haloalkenyl, C₂-C₆-alkynyl, C₁-C₄-alkoxy-C₁-C₄-alkyl and C₃-C₇-cycloalkyl, which is unsubstituted or partly or completely halogenated, and in particular selected from the group consisting of C₁-C₄-alkyl, C₁-C₄-haloalkyl, C₂-C₄-alkenyl, C₂-C₄-haloalkenyl, C₂-C₄-alkynyl and C₃-C₆-cycloalkyl.

R^{3e}, R^{3f}, R^{4e}, R^{4f}, independently of each other are in particular selected from the group consisting of hydrogen, C₁-C₆-alkyl, C₃-C₇-cycloalkyl, which is unsubstituted or partially or completely halogenated, C₁-C₆-haloalkyl, C₂-C₆-alkenyl, C₂-C₆-haloalkenyl, C₁-C₄-alkoxy-C₁-C₄-alkyl, phenyl and benzyl, where phenyl and benzyl are unsubstituted or substituted by 1, 2 or 3 groups, which are identical or different and selected from the group consisting of halogen, C₁-C₄-alkyl, C₁-C₄-haloalkyl and C₁-C₄-alkoxy, or R^{3e} and R^{3f} together with the nitrogen atom, to which they are bound may form a 5-, 6 or 7-membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of halogen, C₁-C₄-alkyl, C₁-C₄-haloalkyl and C₁-C₄-alkoxy.

More preferably R^{3e}, R^{3f}, R^{4e}, R^{4f}, independently of each other are selected from the group consisting of hydrogen, C₁-C₆-alkyl, C₁-C₆-haloalkyl and benzyl, or R^{3e} and R^{3f} together with the nitrogen atom, to which they are bound may form a 5- or 6-membered, saturated or unsaturated heterocyclic radical, which may carry as a ring member a further heteroatom selected from O, S and N and which is unsubstituted or may carry 1, 2 or 3 groups, which are identical or different and selected from the group consisting of halogen, C₁-C₄-alkyl and C₁-C₄-haloalkyl.

In particular, R^{3e}, R^{3f}, R^{4e}, R^{4f}, independently of each other are selected from the group consisting of hydrogen and C₁-C₄-alkyl, or R^{3e} and R^{3f} together with the nitrogen atom, to which they are bound may form a 5- or 6-membered, saturated heterocyclic radical, which may carry as a ring member a further heteroatom selected from O, S and N and which is unsubstituted or may carry 1, 2 or 3 methyl groups.

R^{3g}, R^{4g} independently of each other are in particular selected from the group consisting of hydrogen, C₁-C₆-alkyl, C₃-C₇-cycloalkyl, which is unsubstituted or partly or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl and benzyl.

More preferably R^{3g}, R^{4g} independently of each other are in particular selected from the group consisting of hydrogen, C₁-C₆-alkyl, C₁-C₆-haloalkyl, C₂-C₆-alkenyl, C₂-C₆-haloalkenyl, benzyl, C₁-C₄-alkoxy-C₁-C₄-alkyl and C₃-C₇-cycloalkyl, which is unsubstituted or partly or completely halogenated, and in particular selected from the group consisting of hydrogen, C₁-C₄-alkyl, C₁-C₄-haloalkyl, C₂-C₄-alkenyl, C₂-C₄-haloalkenyl, benzyl and C₃-C₆-cycloalkyl.

R^{3h}, R^{4h}, independently of each other are in particular selected from the group consisting of hydrogen, C₁-C₆-alkyl, C₃-C₇-cycloalkyl, which is unsubstituted or partly or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl, benzyl and a radical C(=O)-R^{k}, where R^{k} is H, C₁-C₄-alkyl, C₁-C₄-haloalkyl or phenyl.

More preferably R^{3h}, R^{4h}, independently of each other are selected from the group consisting of hydrogen, C₁-C₆-alkyl, C₁-C₆-haloalkyl, C₂-C₆-alkenyl, C₂-C₆-haloalkenyl, benzyl, C₁-C₄-alkoxy-C₁-C₄-alkyl and C₃-C₇-cycloalkyl, which is unsubstituted or partly or completely halogenated, and in particular selected from hydrogen, C₁-C₄-alkyl, C₁-C₄-haloalkyl, C₂-C₄-alkenyl, C₂-C₄-haloalkenyl, benzyl and C₃-C₆-cycloalkyl; or

R^{3g} and R^{3h} or R^{4g} and R^{4h}, together with the nitrogen atom, to which they are bound may in particular also form a 5-, 6 or 7-membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of =O, halogen, C₁-C₄-alkyl and C₁-C₄-haloalkyl and C₁-C₄-alkoxy; more preferably R^{3g} and R^{3h} or R^{4g} and R^{4h}, together with the nitrogen atom, to which they are bound may form a 5- or 6-membered, saturated or unsaturated heterocyclic radical, which may carry as a ring member a further heteroatom selected from O, S and N and which is unsubstituted or may carry 1, 2 or 3 groups, which are identical or different and selected from the group consisting of halogen, C₁-C₄-alkyl and C₁-C₄-haloalkyl; and in particular, R^{3g} and R^{3h} or R^{4g} and R^{4h}, together with the nitrogen atom, to which they are bound may form a 5- or 6-membered, saturated heterocyclic radical, which may carry as a ring member a further heteroatom selected from O, S and N and which is unsubstituted or may carry 1, 2 or 3 methyl groups.

R^{z} is selected from the group consisting of C₁-C₆-alkyl, C₁-C₆-haloalkyl, C₃-C₇-cycloalkyl, C₁-C₆-alkoxy, C₂-C₆-alkenyl, phenyl, benzyl, heterocyclyl and heterocyclylmethyl, where heterocyclyl in the last two mentioned radicals is a 5- or 6-membered monocyclic saturated, partially unsaturated or aromatic heterocycle, which contains 1, 2 or 3 heteroatoms as ring members, which are selected from the group consisting of O, N and S, where phenyl, benzyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy.

Preferably, R^{z} is selected from the group consisting of C₁-C₆-alkyl, C₁-C₆-haloalkyl, C₁-C₆-alkoxy, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy.

More preferably, R^{z} is selected from the group consisting of C₁-C₄-alkyl, C₁-C₃-haloalkyl, C₁-C₄-alkoxy, phenyl and benzyl, where phenyl and benzyl are unsubstituted or substituted by 1 or 2 groups, which are identical or different and selected from halogen, C₁-C₄-alkyl and C₁-C₄-haloalkyl; and in particular R^{z} is C₁-C₃-alkyl, C₁-C₃-haloalkyl or C₁-C₄-alkoxy.

Irrespectively of its occurrence, the variable n is in particular 0 or 2.

Irrespectively of its occurrence, the variable k is in particular 0 or 2, and especially 2.

Particularly preferred are compounds of formulae I, I.A, I.B, I.C, I.D, I' , I.A' , I.B' , I.C' and I.D' , their N-oxides and their salts, wherein the combination of variables R¹ and R³ have the following meanings:
- R¹: is selected from the group consisting of halogen, nitro, C₁-C₄-alkyl, C₁-C₄-haloalkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl, in particular from the group consisting of F, Cl, Br, I, nitro, CH₃, CF₃, SCH₃, SCF₃, SO₂CH₃ or CH₂OCH₂CH₂OCH₃, more specifically from the group consisting of F, Cl, Br, I, nitro, CH₃, CF₃, SCH₃ or SO₂CH₃, even more specifically from the group consisting of Cl, Br, CH₃ or CF₃, and especially R¹ is Cl or CH₃; and
- R³: is selected from the group consisting of halogen, C₁-C₄-alkyl, C₁-C₄-haloalkyl, C₁-C₄-haloalkoxy, C₁-C₄-alkylthio, C₁-C₄-haloalkylthio, C₁-C₄-alkyl-S(O), C₁-C₄-haloalkyl-S(O), C₁-C₄-alkyl-S(O)₂, and C₁-C₄-haloalkyl-S(O)₂, more particularly from the group consisting of halogen, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂, especially from the group consisting of CI, F, Br, I, CH₃, CF₃, CHF₂, OCF₃, OCHF₂, SCH₃, SCF₃, SCHF₂, S(O)CH₃, S(O)CH₂CH₃, S(O)₂CH₃ and S(O)₂CH₂CH₃, and more specifically R³ is Cl, Br, CF₃, SCH₃, S(O)CH₃ or S(O)₂CH₃.

A particular preferred group 1 of compounds of the invention are compounds of formulae I, I.A, I.B, I.C, I.D, I', I.A', I.B', I.C' and I.D', their N-oxides and their salts, wherein the combination of variables R⁴ and R⁵ have the following meanings:
R⁴ is hydrogen, chlorine or fluorine; and in particular is hydrogen;
R⁵ is different from hydrogen and in particular selected from the group consisting of halogen, CHF₂ and CF₃; and in particular from chlorine and fluorine.

A further particular preferred group 2 of compounds of the invention are compounds of formulae I, I.A, I.B, I.C, I.D, I', I.A', I.B', I.C' and I.D', their N-oxides and their salts, wherein the combination of variables R⁴ and R⁵ have the following meanings:
R⁴ is chlorine or fluorine;
R⁵ is hydrogen.

A further particular preferred group 3 of compounds of the invention are compounds of formulae I, I.A, I.B, I.C, I.D, I', I.A', I.B', I.C' and I.D', their N-oxides and their salts, wherein both variables R⁴ and R⁵ are hydrogen.

Amongst the compounds of groups 1, 2 and 3, preference is given to those, where Q is Q¹ or Q².

Amongst the compounds of groups 1, 2 and 3, preference is given to those, where Z² is a covalent bond.

Amongst the compounds of groups 1, 2 and 3, particular preference is given to those, where Q is Q¹ or Q² and Z² is a covalent bond.

Amongst the compounds of groups 1, 2 and 3, preference is given to those, where Q is Q¹ or Q², Z² is a covalent bond and where the combination of R¹ and R³ is as follows:
- R¹: is selected from the group consisting of halogen, nitro, C₁-C₄-alkyl, C₁-C₄-haloalkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl, in particular from the group consisting of F, Cl, Br, I, nitro, CH₃, CF₃, SCH₃, SCF₃, SO₂CH₃ or CH₂OCH₂CH₂OCH₃, more specifically from the group consisting of F, Cl, Br, I, nitro, CH₃, CF₃, SCH₃ or SO₂CH₃, even more specifically from the group consisting of Cl, Br, CH₃ or CF₃, and especially R¹ is Cl or CH₃; and
- R³: is selected from the group consisting of halogen, C₁-C₄-alkyl, C₁-C₄-haloalkyl, C₁-C₄-haloalkoxy, C₁-C₄-alkylthio, C₁-C₄-haloalkylthio, C₁-C₄-alkyl-S(O), C₁-C₄-haloalkyl-S(O), C₁-C₄-alkyl-S(O)₂, and C₁-C₄-haloalkyl-S(O)₂, more particularly from the group consisting of halogen, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂, especially from the group consisting of CI, F, Br, I, CH₃, CF₃, CHF₂, OCF₃, OCHF₂, SCH₃, SCF₃, SCHF₂, S(O)CH₃, S(O)CH₂CH₃, S(O)₂CH₃ and S(O)₂CH₂CH₃, and more specifically R³ is Cl, Br, CF₃, SCH₃, S(O)CH₃ or S(O)₂CH₃.

A particular preferred group 1a of compounds of the invention are compounds of formulae I, I.A, I.B, I.C, I.D, I', I.A', I.B', I.C' and I.D', their N-oxides and their salts, wherein wherein the variables R¹, R³, R⁴, R^{6a}, R^{6b}, R^{6c}, R^{6d} and R⁷ have the following meanings:
- R¹: is selected from the group consisting of halogen, nitro, C₁-C₄-alkyl, C₁-C₄-haloalkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl, in particular from the group consisting of F, Cl, Br, I, nitro, CH₃, CF₃, SCH₃, SCF₃, SO₂CH₃ or CH₂OCH₂CH₂OCH₃, more specifically from the group consisting of F, Cl, Br, I, nitro, CH₃, CF₃, SCH₃ or SO₂CH₃, even more specifically from the group consisting of Cl, Br, CH₃ or CF₃, and especially R¹ is Cl or CH₃;
- R³: is selected from halogen, nitro, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂ and where R³ is in particular selected from the group consisting of Cl, Br, CF₃, SCH₃, S(O)CH₃ and S(O)₂CH₃;
- R⁴: is hydrogen, chlorine or fluorine; and in particular is hydrogen;
- R⁵: is selected from the group consisting of halogen, CHF₂ and CF₃; and in particular from chlorine and fluorine;
- R^{6a}, R^{6b}, R^{6c}, R^{6d}: are, independently of each other, selected from CH₃, CH₃CH₂, CH₃OCH₂CH₂ and CH₃OCH₂; and in particular from CH₃ and CH₃CH₂; and
- R⁷: is hydrogen.

Another particular preferred group 2a of compounds of the invention are compounds of formulae I, I.A, I.B, I.C, I.D, I' , I.A' , I.B' , I.C' and I.D' , their N-oxides and their salts, wherein wherein Q is Q¹, Q², Q³ or Q⁴ and the variables R¹, R³, R⁴, R^{6a}, R^{6b}, R^{6c}, R^{6d} and R⁷ have the following meanings:
- R¹: is selected from the group consisting of halogen, nitro, C₁-C₄-alkyl, C₁-C₄-haloalkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl, in particular from the group consisting of F, Cl, Br, I, nitro, CH₃, CF₃, SCH₃, SCF₃, SO₂CH₃ or CH₂OCH₂CH₂OCH₃, more specifically from the group consisting of F, Cl, Br, I, nitro, CH₃, CF₃, SCH₃ or SO₂CH₃, even more specifically from the group consisting of Cl, Br, CH₃ or CF₃, and especially R¹ is Cl or CH₃;
- R³: is selected from halogen, nitro, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂ and where R³ is in particular selected from the group consisting of Cl, Br, CF₃, SCH₃, S(O)CH₃ and S(O)₂CH₃;
- R⁴: is chlorine or fluorine;
- R⁵: is hydrogen;
- R^{6a}, R^{6b}, R^{6c}, R^{6d}: are, independently of each other, selected from CH₃, CH₃CH₂, CH₃OCH₂CH₂ and CH₃OCH₂; and in particular from CH₃ and CH₃CH₂; and
- R⁷: is hydrogen.

A further particular preferred group 3a of compounds of the invention are compounds of formulae I, I.A, I.B, I.C, I.D, I', I.A', I.B', I.C' and I.D' , their N-oxides and their salts, wherein wherein Q is Q¹, Q², Q³ or Q⁴ and the variables R¹, R³, R⁴, R^{6a}, R^{6b}, R^{6c}, R^{6d} and R⁷ have the following meanings:
- R¹: is selected from the group consisting of halogen, nitro, C₁-C₄-alkyl, C₁-C₄-haloalkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl, in particular from the group consisting of F, Cl, Br, I, nitro, CH₃, CF₃, SCH₃, SCF₃, SO₂CH₃ or CH₂OCH₂CH₂OCH₃, more specifically from the group consisting of F, Cl, Br, I, nitro, CH₃, CF₃, SCH₃ or SO₂CH₃, even more specifically from the group consisting of Cl, Br, CH₃ or CF₃, and especially R¹ is Cl or CH₃;
- R³: is selected from halogen, nitro, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂ and where R³ is in particular selected from the group consisting of Cl, Br, CF₃, SCH₃, S(O)CH₃ and S(O)₂CH₃;
- R⁴: is hydrogen;
- R⁵: is hydrogen;
- R^{6a}, R^{6b}, R^{6c}, R^{6d}: are, independently of each other, selected from CH₃, CH₃CH₂, CH₃OCH₂CH₂ and CH₃OCH₂; and in particular from CH₃ and CH₃CH₂; and
- R⁷: is hydrogen.

Amongst the compounds of groups 1a, 2a and 3a, preference is given to those, where Q is Q¹ or Q².

Amongst the compounds of groups 1a, 2a and 3a, preference is given to those, where Z² is a covalent bond.

Amongst the compounds of groups 1a, 2a and 3a, particular preference is given to those, where Q is Q¹ or Q² and Z² is a covalent bond.

A an even more preferred group 1b of compounds of the invention are compounds of formulae I, I.A, I.B, I.C, I.D, I', I.A', I.B', I.C' and I.D' , their N-oxides and their salts, wherein wherein the variables R¹, R^{2a}, R^{2b}, R^{2c}, R³, R⁴, R^{6a}, R^{6b}, R^{6c}, R^{6d} and R⁷ have the following meanings:
- R¹: is selected from the group consisting of halogen, nitro, C₁-C₄-alkyl, C₁-C₄-haloalkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl, in particular from the group consisting of F, Cl, Br, I, nitro, CH₃, CF₃, SCH₃, SCF₃, SO₂CH₃ or CH₂OCH₂CH₂OCH₃, more specifically from the group consisting of F, Cl, Br, I, nitro, CH₃, CF₃, SCH₃ or SO₂CH₃, even more specifically from the group consisting of Cl, Br, CH₃ or CF₃, and especially R¹ is Cl or CH₃;
- R^{2a}: is seleced from the group consisting of C₁-C₄-alkyl, C₁-C₄-alkoxy, C₃-C₆-cycloalkyl, C₃-C₆-cycloalkyl-methyl, C₁-C₄-fluoroalkyl, phenyl and benzyl, where phenyl and benzyl are unsubstituted or substituted by 1, 2, 3 or 4 radicals R²¹, which are identical or different and where R²¹ is preferably selected from the group consisting of halogen, CN, C₁-C₄-alkyl, C₁-C₄-haloalkyl, C₁-C₄-alkoxy and C₁-C₄-haloalkoxy and where R²¹ is in particular selected from the group consisting of halogen, C₁-C₄-alkyl, C₁-C₂-fluoroalkyl, C₁-C₄-alkoxy and C₁-C₂-fluoroalkoxy and especially from the group consisting of halogen, methyl and methoxy; hydrogen, C₁-C₄-alkyl, C₃-C₆-cycloalkyl, C₃-C₆-cycloalkyl-methyl and C₁-C₄-fluoroalkyl, R^{2a} is more particularly selected from the group consisting of C₁-C₄-alkyl, C₃-C₇-cycloalkyl, C₁-C₄-fluoroalkyl, C₁-C₄-alkoxy and phenyl, where phenyl is unsubstituted or substituted by 1 or 2 groups R²¹, which are identical or different and which are preferably selected from the group consisting of halogen, C₁-C₄-alkyl, C₁-C₂-fluoroalkyl, C₁-C₄-alkoxy and C₁-C₂-fluoroalkoxy and especially from the group consisting of halogen, methyl and methoxy, R^{2a} is even more particularly selected from the group consisting of C₁-C₄-alkyl, C₁-C₂-fluoroalkyl, C₃-C₄-cycloalkyl, and phenyl, which is unsubstituted or substituted by 1, 2 or 3 radicals R²¹ selected from the group consisting of halogen, C₁-C₄-alkyl, C₁-C₂-fluoroalkyl, C₁-C₄-alkoxy and C₁-C₂-fluoroalkoxy and where phenyl is in particular unsubstituted or substituted by 1, 2 or 3 radicals selected from the group consisting of halogen, methyl and methoxy,
R^{2a} is especially selected from the group consisting of methyl, ethyl, isopropyl (iPr), cyclopropyl (cPr), CHF₂CH₂-, CF₃CH₂-, CH₃O-, 4-Cl-phenyl, 4-methoxyphenyl, 4-methylphenyl and 2,6-dimethylphenyl;
- R^{2b}: is selected from the group consisting of hydrogen, C₁-C₄-alkyl, C₃-C₇-cycloalkyl and C₁-C₄-fluoroalkyl, in particular selected from the group consisting of hydrogen, C₁-C₄-alkyl, C₁-C₂-fluoroalkyl and C₃-C₄-cycloalkyl and especially selected from the group consisting of hydrogen, methyl, ethyl, isopropyl (iPr), cyclopropyl (cPr), CHF₂CH₂- and CF₃CH₂-;
or
- R^{2a} and R^{2b}: together with the nitrogen atom, to which they are bound may form a 5- or 6- membered, saturated or unsaturated heterocyclic radical, which may carry as a ring member a further heteroatom selected from O, S and N and which is unsubstituted or may carry 1, 2, 3 or 4 groups R^{2d}, which are identical or different and in particular selected from the group consisting of =O, halogen, C₁-C₄-alkyl, C₁-C₄-haloalkyl, C₁-C₄-alkoxy and C₁-C₄-haloalkoxy, and where R^{2a} and R^{2b} together with the nitrogen atom, to which they are bound in particular form 4-morpholinyl, 4-thiomorpholinyl, 1-piperidinyl and 1-pyrrolidinyl, where 4-morpholinyl, 4-thiomorpholinyl, 1-piperidinyl and 1-pyrrolidinyl are unsubstituted or carry 1, 2, 3 or 4 groups R^{2d} which are selected from the group consisting of C₁-C₄-alkyl and C₁-C₄-fluoroalkyl, preferably from methyl, ethyl and trifluoromethyl;
- R^{2c}: is hydrogen or C₁-C₄-alkyl and especially hydrogen;
- R³: is selected from halogen, nitro, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂ and where R³ is in particular selected from the group consisting of Cl, Br, CF₃, SCH₃, S(O)CH₃ and S(O)₂CH₃;
- R⁴: is hydrogen, chlorine or fluorine; and in particular is hydrogen;
- R⁵: is selected from the group consisting of halogen, CHF₂ and CF₃; and in particular from chlorine and fluorine;
- R^{6a}, R^{6b}, R^{6c}, R^{6d}: are, independently of each other, selected from CH₃, CH₃CH₂, CH₃OCH₂CH₂ and CH₃OCH₂; and in particular from CH₃ and CH₃CH₂; and
- R⁷: is hydrogen.

A likewise more preferred group 2b of compounds of the invention are compounds of formulae I, I.A, I.B, I.C, I.D, I' , I.A' , I.B' , I.C' and I.D' , their N-oxides and their salts, wherein wherein the variables R¹, R^{2a}, R^{2b}, R^{2c}, R³, R⁴, R^{6a}, R^{6b}, R^{6c}, R^{6d} and R⁷ have the following meanings:
- R¹: is selected from the group consisting of halogen, nitro, C₁-C₄-alkyl, C₁-C₄-haloalkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl, in particular from the group consisting of F, Cl, Br, I, nitro, CH₃, CF₃, SCH₃, SCF₃, SO₂CH₃ or CH₂OCH₂CH₂OCH₃, more specifically from the group consisting of F, Cl, Br, I, nitro, CH₃, CF₃, SCH₃ or SO₂CH₃, even more specifically from the group consisting of Cl, Br, CH₃ or CF₃, and especially R¹ is Cl or CH₃;
- R^{2a}: is seleced from the group consisting of C₁-C₄-alkyl, C₁-C₄-alkoxy, C₃-C₆-cycloalkyl, C₃-C₆-cycloalkyl-methyl, C₁-C₄-fluoroalkyl, phenyl and benzyl, where phenyl and benzyl are unsubstituted or substituted by 1, 2, 3 or 4 radicals R²¹, which are identical or different and where R²¹ is preferably selected from the group consisting of halogen, CN, C₁-C₄-alkyl, C₁-C₄-haloalkyl, C₁-C₄-alkoxy and C₁-C₄-haloalkoxy and where R²¹ is in particular selected from the group consisting of halogen, C₁-C₄-alkyl, C₁-C₂-fluoroalkyl, C₁-C₄-alkoxy and C₁-C₂-fluoroalkoxy and especially from the group consisting of halogen, methyl and methoxy; hydrogen, C₁-C₄-alkyl, C₃-C₆-cycloalkyl, C₃-C₆-cycloalkyl-methyl and C₁-C₄-fluoroalkyl, R^{2a} is more particularly selected from the group consisting of C₁-C₄-alkyl, C₃-C₇-cycloalkyl, C₁-C₄-fluoroalkyl, C₁-C₄-alkoxy and phenyl, where phenyl is unsubstituted or substituted by 1 or 2 groups R²¹, which are identical or different and which are preferably selected from the group consisting of halogen, C₁-C₄-alkyl, C₁-C₂-fluoroalkyl, C₁-C₄-alkoxy and C₁-C₂-fluoroalkoxy and especially from the group consisting of halogen, methyl and methoxy, R^{2a} is even more particularly selected from the group consisting of C₁-C₄-alkyl, C₁-C₂-fluoroalkyl, C₃-C₄-cycloalkyl, and phenyl, which is unsubstituted or substituted by 1, 2 or 3 radicals R²¹ selected from the group consisting of halogen, C₁-C₄-alkyl, C₁-C₂-fluoroalkyl, C₁-C₄-alkoxy and C₁-C₂-fluoroalkoxy and where phenyl is in particular unsubstituted or substituted by 1, 2 or 3 radicals selected from the group consisting of halogen, methyl and methoxy,
R^{2a} is especially selected from the group consisting of methyl, ethyl, isopropyl (iPr), cyclopropyl (cPr), CHF₂CH₂-, CF₃CH₂-, CH₃O-, 4-Cl-phenyl, 4-methoxyphenyl, 4-methylphenyl and 2,6-dimethylphenyl;
- R^{2b}: is selected from the group consisting of hydrogen, C₁-C₄-alkyl, C₃-C₇-cycloalkyl and C₁-C₄-fluoroalkyl, in particular selected from the group consisting of hydrogen, C₁-C₄-alkyl, C₁-C₂-fluoroalkyl and C₃-C₄-cycloalkyl and especially selected from the group consisting of hydrogen, methyl, ethyl, isopropyl (iPr), cyclopropyl (cPr), CHF₂CH₂- and CF₃CH₂-;
or
- R^{2a} and R^{2b}: together with the nitrogen atom, to which they are bound may form a 5- or 6- membered, saturated or unsaturated heterocyclic radical, which may carry as a ring member a further heteroatom selected from O, S and N and which is unsubstituted or may carry 1, 2, 3 or 4 groups R^{2d}, which are identical or different and in particular selected from the group consisting of =O, halogen, C₁-C₄-alkyl, C₁-C₄-haloalkyl, C₁-C₄-alkoxy and C₁-C₄-haloalkoxy, and where R^{2a} and R^{2b} together with the nitrogen atom, to which they are bound in particular form 4-morpholinyl, 4-thiomorpholinyl, 1-piperidinyl and 1-pyrrolidinyl, where 4-morpholinyl, 4-thiomorpholinyl, 1-piperidinyl and 1-pyrrolidinyl are unsubstituted or carry 1, 2, 3 or 4 groups R^{2d} which are selected from the group consisting of C₁-C₄-alkyl and C₁-C₄-fluoroalkyl, preferably from methyl, ethyl and trifluoromethyl;
- R^{2c}: is hydrogen or C₁-C₄-alkyl and especially hydrogen;
- R³: is selected from halogen, nitro, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂ and where R³ is in particular selected from the group consisting of Cl, Br, CF₃, SCH₃, S(O)CH₃ and S(O)₂CH₃;
- R⁴: is chlorine or fluorine;
- R⁵: is hydrogen;
- R^{6a}, R^{6b}, R^{6c}, R^{6d}: are, independently of each other, selected from CH₃, CH₃CH₂, CH₃OCH₂CH₂ and CH₃OCH₂; and in particular from CH₃ and CH₃CH₂; and
- R⁷: is hydrogen.

A likewise more preferred group 3b of compounds of the invention are compounds of formulae I, I.A, I.B, I.C, I.D, I' , I.A' , I.B' , I.C' and I.D' , their N-oxides and their salts, wherein wherein the variables R¹, R^{2a}, R^{2b}, R^{2c}, R³, R⁴, R^{6a}, R^{6b}, R^{6c}, R^{6d} and R⁷ have the following meanings:
- R¹: is selected from the group consisting of halogen, nitro, C₁-C₄-alkyl, C₁-C₄-haloalkyl, C₁-C₄-alkoxy-C₁-C₄-alkoxy-C₁-C₄-alkyl, C₁-C₄-alkylthio, C₁-C₄-haloalkylthio and C₁-C₄-alkylsulfonyl, in particular from the group consisting of F, Cl, Br, I, nitro, CH₃, CF₃, SCH₃, SCF₃, SO₂CH₃ or CH₂OCH₂CH₂OCH₃, more specifically from the group consisting of F, Cl, Br, I, nitro, CH₃, CF₃, SCH₃ or SO₂CH₃, even more specifically from the group consisting of Cl, Br, CH₃ or CF₃, and especially R¹ is Cl or CH₃;
- R^{2a}: is seleced from the group consisting of C₁-C₄-alkyl, C₁-C₄-alkoxy, C₃-C₆-cycloalkyl, C₃-C₆-cycloalkyl-methyl, C₁-C₄-fluoroalkyl, phenyl and benzyl, where phenyl and benzyl are unsubstituted or substituted by 1, 2, 3 or 4 radicals R²¹, which are identical or different and where R²¹ is preferably selected from the group consisting of halogen, CN, C₁-C₄-alkyl, C₁-C₄-haloalkyl, C₁-C₄-alkoxy and C₁-C₄-haloalkoxy and where R²¹ is in particular selected from the group consisting of halogen, C₁-C₄-alkyl, C₁-C₂-fluoroalkyl, C₁-C₄-alkoxy and C₁-C₂-fluoroalkoxy and especially from the group consisting of halogen, methyl and methoxy; hydrogen, C₁-C₄-alkyl, C₃-C₆-cycloalkyl, C₃-C₆-cycloalkyl-methyl and C₁-C₄-fluoroalkyl, R^{2a} is more particularly selected from the group consisting of C₁-C₄-alkyl, C₃-C₇-cycloalkyl, C₁-C₄-fluoroalkyl, C₁-C₄-alkoxy and phenyl, where phenyl is unsubstituted or substituted by 1 or 2 groups R²¹, which are identical or different and which are preferably selected from the group consisting of halogen, C₁-C₄-alkyl, C₁-C₂-fluoroalkyl, C₁-C₄-alkoxy and C₁-C₂-fluoroalkoxy and especially from the group consisting of halogen, methyl and methoxy, R^{2a} is even more particularly selected from the group consisting of C₁-C₄-alkyl, C₁-C₂-fluoroalkyl, C₃-C₄-cycloalkyl, and phenyl, which is unsubstituted or substituted by 1, 2 or 3 radicals R²¹ selected from the group consisting of halogen, C₁-C₄-alkyl, C₁-C₂-fluoroalkyl, C₁-C₄-alkoxy and C₁-C₂-fluoroalkoxy and where phenyl is in particular unsubstituted or substituted by 1, 2 or 3 radicals selected from the group consisting of halogen, methyl and methoxy,
R^{2a} is especially selected from the group consisting of methyl, ethyl, isopropyl (iPr), cyclopropyl (cPr), CHF₂CH₂-, CF₃CH₂-, CH₃O-, 4-Cl-phenyl, 4-methoxyphenyl, 4-methylphenyl and 2,6-dimethylphenyl;
- R^{2b}: is selected from the group consisting of hydrogen, C₁-C₄-alkyl, C₃-C₇-cycloalkyl and C₁-C₄-fluoroalkyl, in particular selected from the group consisting of hydrogen, C₁-C₄-alkyl, C₁-C₂-fluoroalkyl and C₃-C₄-cycloalkyl and especially selected from the group consisting of hydrogen, methyl, ethyl, isopropyl (iPr), cyclopropyl (cPr), CHF₂CH₂- and CF₃CH₂-;
or
- R^{2a} and R^{2b}: together with the nitrogen atom, to which they are bound may form a 5- or 6- membered, saturated or unsaturated heterocyclic radical, which may carry as a ring member a further heteroatom selected from O, S and N and which is unsubstituted or may carry 1, 2, 3 or 4 groups R^{2d}, which are identical or different and in particular selected from the group consisting of =O, halogen, C₁-C₄-alkyl, C₁-C₄-haloalkyl, C₁-C₄-alkoxy and C₁-C₄-haloalkoxy, and where R^{2a} and R^{2b} together with the nitrogen atom, to which they are bound in particular form 4-morpholinyl, 4-thiomorpholinyl, 1-piperidinyl and 1-pyrrolidinyl, where 4-morpholinyl, 4-thiomorpholinyl, 1-piperidinyl and 1-pyrrolidinyl are unsubstituted or carry 1, 2, 3 or 4 groups R^{2d} which are selected from the group consisting of C₁-C₄-alkyl and C₁-C₄-fluoroalkyl, preferably from methyl, ethyl and trifluoromethyl;
- R^{2c}: is hydrogen or C₁-C₄-alkyl and especially hydrogen.
- R³: is selected from halogen, nitro, C₁-C₂-alkyl, C₁-C₂-haloalkyl, C₁-C₂-haloalkoxy, C₁-C₂-alkylthio, C₁-C₂-haloalkylthio, C₁-C₂-alkyl-S(O), C₁-C₂-haloalkyl-S(O), C₁-C₂-alkyl-S(O)₂ and C₁-C₂-haloalkyl-S(O)₂ and where R³ is in particular selected from the group consisting of Cl, Br, CF₃, SCH₃, S(O)CH₃ and S(O)₂CH₃;
- R⁴: is hydrogen;
- R⁵: is hydrogen;
- R^{6a}, R^{6b}, R^{6c}, R^{6d}: are, independently of each other, selected from CH₃, CH₃CH₂, CH₃OCH₂CH₂ and CH₃OCH₂; and in particular from CH₃ and CH₃CH₂; and
- R⁷: is hydrogen.

Amongst the compounds of groups 1b, 2b and 3b, preference is given to those, where Q is Q¹ or Q².

Amongst the compounds of groups 1b, 2b and 3b, preference is given to those, where Z² is a covalent bond.

Amongst the compounds of groups 1b, 2b and 3b, particular preference is given to those, where Q is Q¹ or Q² and Z² is a covalent bond.

Amongst the compounds of groups 1b, 2b and 3b, particular preference is given to those, where Q is Q¹ or Q² and Z² is a covalent bond.

A very preferred group 1 c of compounds of the invention are compounds of formulae I.A' , and I.B' , their N-oxides and their salts, wherein wherein the variables R¹, R^{2a}, R^{2b}, R^{2c}, R³, R⁴, R^{6a}, R^{6b}, R^{6c}, R^{6d} and R⁷ have the following meanings:
- R¹: is halogen or C₁-C₂-alkyl, in particular Cl or CH₃;
- R^{2a}: is selected from the group consisting of hydrogen, C₁-C₄-alkyl, C₁-C₄-alkoxy, C₃-C₆-cycloalkyl, C₃-C₆-cycloalkyl-methyl, C₁-C₄-fluoroalkyl, phenyl and benzyl, where phenyl and benzyl are unsubstituted or substituted by 1, 2, 3 or 4 radicals selected from the group consisting of halogen, methyl and methoxy, R^{2a} is in particular selected from the group consisting of C₁-C₄-alkyl, , C₁-C₄-alkoxy C₃-C₇-cycloalkyl, C₁-C₄-fluoroalkyl and phenyl, where phenyl is unsubstituted or substituted by 1, 2 or 3 radicals selected from the group consisting of halogen, methyl and methoxy, R^{2a} is even more particularly selected from the group consisting of C₁-C₄-alkyl, C₁-C₂-fluoroalkyl, C₃-C₄-cycloalkyl, and phenyl, which is unsubstituted or substituted by 1, 2 or 3 radicals selected from the group consisting of halogen, methyl and methoxy, R^{2a} is especially selected from the group consisting of methyl, ethyl, isopropyl (iPr), cyclopropyl (cPr), CHF₂CH₂-, CF₃CH₂-, CH₃O-, 4-Cl-phenyl, 4-methoxyphenyl, 4-methylphenyl and 2,6-dimethylphenyl;
- R^{2b}: is selected from the group consisting of C₁-C₄-alkyl, C₃-C₆-cycloalkyl, C₃-C₆-cycloalkyl-methyl and C₁-C₄-fluoroalkyl; in particular selected from the group consisting of hydrogen, C₁-C₄-alkyl, C₁-C₂-fluoroalkyl and C₃-C₄-cycloalkyl and especially selected from the group consisting of hydrogen, methyl, ethyl, isopropyl (iPr), cyclopropyl (cPr), CHF₂CH₂- and CF₃CH₂-;
or
- R^{2a}, R^{2b}: together with the nitrogen atom, to which they are bound form a 5- or 6-membered, saturated heterocyclic radical, which may carry as a ring member a further heteroatom selected from O, S and N and where the heterocyclic radical is selected from the group consisting of 4-morpholinyl, 4-thiomorpholinyl, 1-piperidinyl and 1-pyrrolidinyl, where 4-morpholinyl, 4-thiomorpholinyl, 1-piperidinyl and 1-pyrrolidinyl are unsubstituted or carry 1, 2, 3 or 4 groups R^{2d} which are selected from the group consisting of C₁-C₄-alkyl and C₁-C₄-fluoroalkyl, where R^{2d} is especially selected from the group consisting of methyl and trifluoromethyl;
- R^{2c}: is selected from the group consisting of hydrogen and C₁-C₄-alkyl, especially hydrogen;
- R³: is selected from the group consisting of halogen, C₁-C₂-alkyl, C₁-C₂-fluoroalkyl, S-C₁-C₂-alkyl, S(O)-C₁-C₂-alkyl, S(O)₂-C₁-C₂-alkyl, S-C₁-C₂-fluoroalkyl, S(O)-C₁-C₂-fluoroalkyl and S(O)₂-C₁-C₂-fluoroalkyl and where R³ is in particular selected from the group consisting of Cl, Br, CF₃, SCH₃, S(O)CH₃ and S(O)₂CH₃;
- R⁴: is hydrogen;
- R⁵: is fluorine;
- R^{6a}, R^{6b},: independently of each othe rare selected from the group consisting of C₁-C₄-alkyl and C₁-C₄-alkoxy-C₁-C₄-alkyl ;
- R⁷: is as defined above and in particular hydrogen.

Examples of preferred compounds are the individual compounds compiled in the following tables 1 to 48 below. Moreover, the meanings mentioned below for the individual variables in the Tables are per se, independently of the combination in which they are mentioned, a particularly preferred embodiment of the substituents in question.

Compounds I.A' .I, wherein Q is Q¹, R⁴ is hydrogen, R⁵ is hydrogen, R^{6a} is methyl, R⁷ is hydrogen, Z² in R² is a covalent bond and R^{2c} in R² is H: 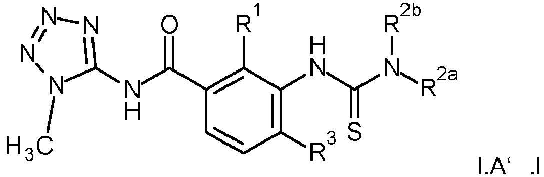
Table 1 Compounds of formula I.A' .I (I.A' .1.1 - I.A' .1.1104) in which the combination of R¹, R^{2a}, R^{2b}, and R³ for a compound corresponds in each case to one row of Table A;
   Compounds I.A' .II, wherein Q is Q¹, R⁴ is hydrogen, R⁵ is hydrogen, R^{6a} is ethyl, R⁷ is hydrogen, Z² in R² is a covalent bond and R^{2c} in R² is H: 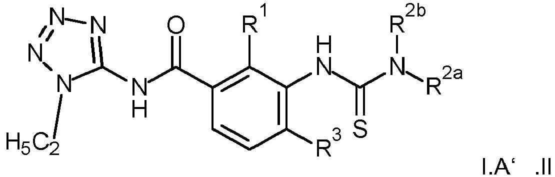
Table 2 Compounds of formula I.A' .II (I.A' .II.1 - I.A' .11.1104) in which the combination of R¹, R^{2a}, R^{2b}, and R³ for a compound corresponds in each case to one row of Table A;
   Compounds I.A' .III, wherein Q is Q¹, R⁴ is hydrogen, R⁵ is hydrogen, R^{6a} is methyl, R⁷ is hydrogen, Z² in R² is a covalent bond and R^{2c} in R² is methyl: 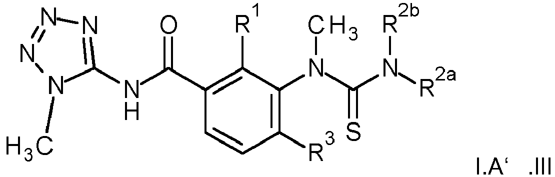
Table 3 Compounds of formula I.A' .III (I.A' .III.1 - I.A' .III.1104) in which the combination of R¹, R^{2a}, R^{2b}, and R³ for a compound corresponds in each case to one row of Table A;
   Compounds I.A' .IV, wherein Q is Q¹, R⁴ is hydrogen, R⁵ is hydrogen, R^{6a} is ethyl, R⁷ is hydrogen, Z² in R² is a covalent bond and R^{2c} in R² is methyl: 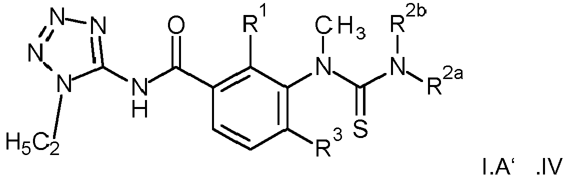
Table 4 Compounds of formula I.A' .IV (I.A' .IV.1 - I.A' .IV.1104) in which the combination of R¹, R^{2a}, R^{2b}, and R³ for a compound corresponds in each case to one row of Table A;
   Compounds I.A' .V, wherein Q is Q¹, R⁴ is hydrogen, R⁵ is hydrogen, R^{6a} is methyl, R⁷ is hydrogen, Z² in R² is a covalent bond and R^{2c} in R² is ethyl: 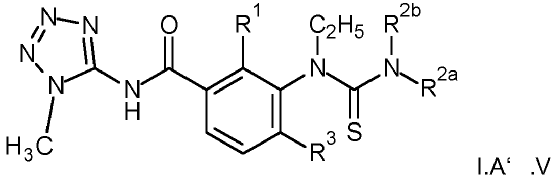
Table 5 Compounds of formula I.A' .V (I.A' .V.1 - I.A' .V.1104) in which the combination of R¹, R^{2a}, R^{2b}, and R³ for a compound corresponds in each case to one row of Table A;
   Compounds I.A' .VI, wherein Q is Q¹, R⁴ is hydrogen, R⁵ is hydrogen, R^{6a} is ethyl, R⁷ is hydrogen, Z² in R² is a covalent bond and R^{2c} in R² is ethyl: 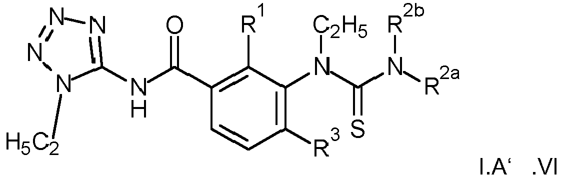
Table 6 Compounds of formula I.A' .VI (I.A' .VI.1 - I.A' .VI.1104) in which the combination of R¹, R^{2a}, R^{2b}, and R³ for a compound corresponds in each case to one row of Table A;
   Compounds I.A' .VII, wherein Q is Q¹, R⁴ is hydrogen, R⁵ is fluorine, R^{6a} is methyl, R⁷ is hydrogen, Z² in R² is a covalent bond and R^{2c} in R² is H: 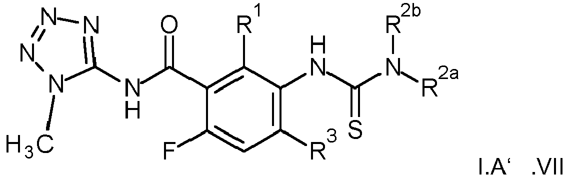
Table 7 Compounds of formula I.A' .VII (I.A' .VII.1 - I.A' .VII.1104) in which the combination of R¹, R^{2a}, R^{2b}, and R³ for a compound corresponds in each case to one row of Table A;
   Compounds I.A' .VIII, wherein Q is Q¹, R⁴ is hydrogen, R⁵ is fluorine, R^{6a} is ethyl, R⁷ is hydrogen, Z² in R² is a covalent bond and R^{2c} in R² is H: 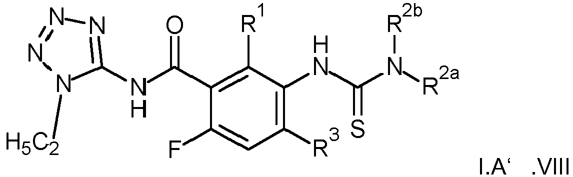
Table 8 Compounds of formula I.A' .VIII (I.A' .VIII.1 - I.A' .VIII.1104) in which the combination of R¹, R^{2a}, R^{2b}, and R³ for a compound corresponds in each case to one row of Table A;
   Compounds I.A' .IX, wherein Q is Q¹, R⁴ is hydrogen, R⁵ is fluorine, R^{6a} is methyl, R⁷ is hydrogen, Z² in R² is a covalent bond and R^{2c} in R² is methyl: 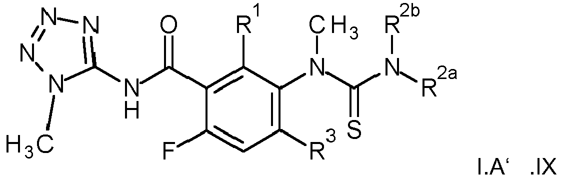
Table 9 Compounds of formula IA' IX (I.A' .IX.1 - I.A' .IX.1104) in which the combination of R¹, R^{2a}, R^{2b}, and R³ for a compound corresponds in each case to one row of Table A;
   Compounds I.A' .X, wherein Q is Q¹, R⁴ is hydrogen, R⁵ is fluorine, R^{6a} is ethyl, R⁷ is hydrogen, Z² in R² is a covalent bond and R^{2c} in R² is methyl: 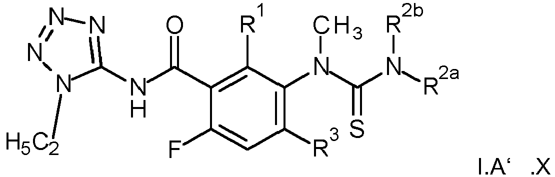
Table 10 Compounds of formula I.A' .X (I.A' .X.1 - I.A' .X.1104) in which the combination of R¹, R^{2a}, R^{2b}, and R³ for a compound corresponds in each case to one row of Table A;
   Compounds I.A' .XI, wherein Q is Q¹, R⁴ is hydrogen, R⁵ is fluorine, R^{6a} is methyl, R⁷ is hydrogen, Z² in R² is a covalent bond and R^{2c} in R² is ethyl: 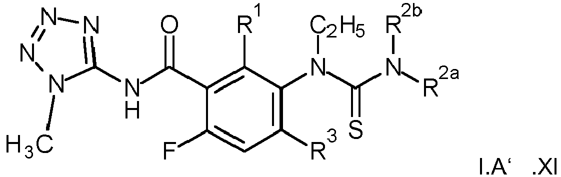
Table 11 Compounds of formula I.A' .XI (I.A' .XI.1 - I.A' .XI.1104) in which the combination of R¹, R^{2a}, R^{2b}, and R³ for a compound corresponds in each case to one row of Table A;
   Compounds I.A' .XII, wherein Q is Q¹, R⁴ is hydrogen, R⁵ is fluorine, R^{6a} is ethyl, R⁷ is hydrogen, Z² in R² is a covalent bond and R^{2c} in R² is ethyl: 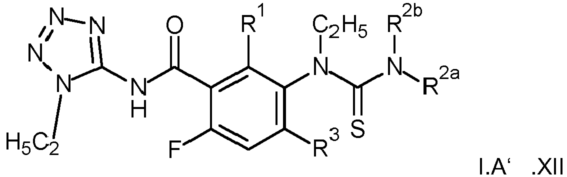
Table 12 Compounds of formula I.A' .XII (I.A' .XII.1 - I.A' .XII.11 04) in which the combination of R¹, R^{2a}, R^{2b}, and R³ for a compound corresponds in each case to one row of Table A;
   Compounds I.B' .I, wherein Q is Q², R⁴ is hydrogen, R⁵ is hydrogen, R^{6a} is methyl, R⁷ is hydrogen, Z² in R² is a covalent bond and R^{2c} in R² is H: 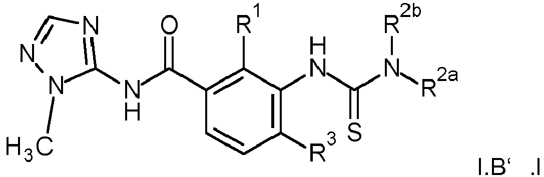
Table 13 Compounds of formula I.B' .I (I.B' .1.1 - I.B' .1.1104) in which the combination of R¹, R^{2a}, R^{2b}, and R³ for a compound corresponds in each case to one row of Table A;
   Compounds I.B' .II, wherein Q is Q², R⁴ is hydrogen, R⁵ is hydrogen, R^{6a} is ethyl, R⁷ is hydrogen, Z² in R² is a covalent bond and R^{2c} in R² is H: 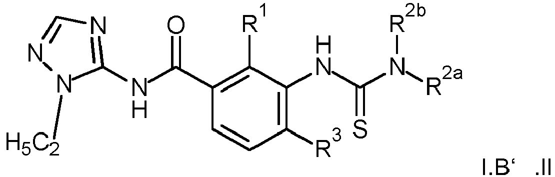
Table 14 Compounds of formula I.B' .II (I.B' .11.1 - I.B' .11.1104) in which the combination of R¹, R^{2a}, R^{2b}, and R³ for a compound corresponds in each case to one row of Table A;
   Compounds I.B' .III, wherein Q is Q², R⁴ is hydrogen, R⁵ is hydrogen, R^{6a} is methyl, R⁷ is hydrogen, Z² in R² is a covalent bond and R^{2c} in R² is methyl: 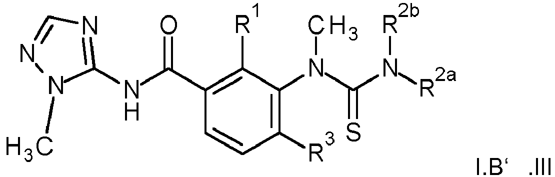
Table 15 Compounds of formula I.B' .III (I.B' .III.1 - I.B' .111.1104) in which the combination of R¹, R^{2a}, R^{2b}, and R³ for a compound corresponds in each case to one row of Table A;
   Compounds I.B' .IV, wherein Q is Q², R⁴ is hydrogen, R⁵ is hydrogen, R^{6a} is ethyl, R⁷ is hydrogen, Z² in R² is a covalent bond and R^{2c} in R² is methyl: 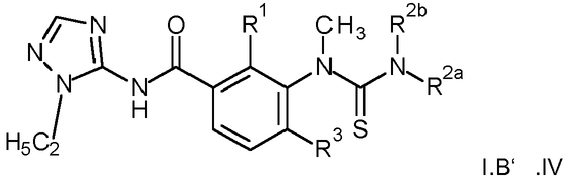
Table 16 Compounds of formula I.B' .IV (I.B' .IV.1 - I.B' .IV.1104) in which the combination of R¹, R^{2a}, R^{2b}, and R³ for a compound corresponds in each case to one row of Table A;
   Compounds I.B' .V, wherein Q is Q², R⁴ is hydrogen, R⁵ is hydrogen, R^{6a} is methyl, R⁷ is hydrogen, Z² in R² is a covalent bond and R^{2c} in R² is ethyl: 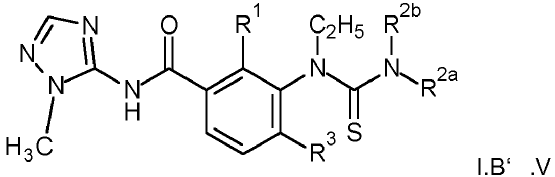
Table 17 Compounds of formula I.B' .V (I.B' .V.1 - I.B' .V.1104) in which the combination of R¹, R^{2a}, R^{2b}, and R³ for a compound corresponds in each case to one row of Table A;
   Compounds I.B' .VI, wherein Q is Q², R⁴ is hydrogen, R⁵ is hydrogen, R^{6a} is ethyl, R⁷ is hydrogen, Z² in R² is a covalent bond and R^{2c} in R² is ethyl: 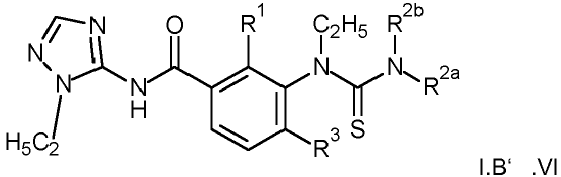
Table 18 Compounds of formula I.B' .VI (I.B' .VI.1 - I.B' .VI.1104) in which the combination of R¹, R^{2a}, R^{2b}, and R³ for a compound corresponds in each case to one row of Table A;
   Compounds I.B' .VII, wherein Q is Q², R⁴ is hydrogen, R⁵ is fluorine, R^{6a} is methyl, R⁷ is hydrogen, Z² in R² is a covalent bond and R^{2c} in R² is H: 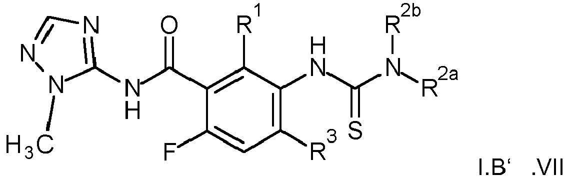
Table 19 Compounds of formula I.B' .VII (I.B' .VII.1 - I.B' .VII.1104) in which the combination of R¹, R^{2a}, R^{2b}, and R³ for a compound corresponds in each case to one row of Table A;
   Compounds I.B' .VIII, wherein Q is Q², R⁴ is hydrogen, R⁵ is fluorine, R^{6a} is ethyl, R⁷ is hydrogen, Z² in R² is a covalent bond and R^{2c} in R² is H: 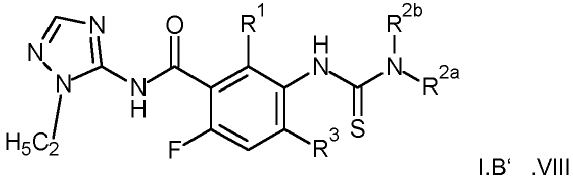
Table 20 Compounds of formula I.B' .VIII (I.B' .VIII.1 - I.B' VIII.1104) in which the combination of R¹, R^{2a}, R^{2b}, and R³ for a compound corresponds in each case to one row of Table A;
   Compounds I.B' .IX, wherein Q is Q², R⁴ is hydrogen, R⁵ is fluorine, R^{6a} is methyl, R⁷ is hydrogen, Z² in R² is a covalent bond and R^{2c} in R² is methyl: 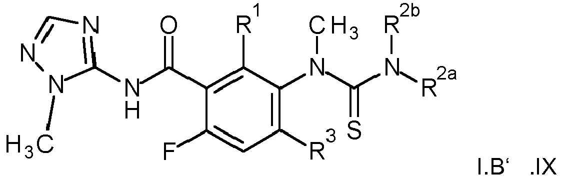
Table 21 Compounds of formula I.B' .IX (I.B' .IX.1 - I.B' .IX.1104) in which the combination of R¹, R^{2a}, R^{2b}, and R³ for a compound corresponds in each case to one row of Table A;
   Compounds I.B' .X, wherein Q is Q², R⁴ is hydrogen, R⁵ is fluorine, R^{6a} is ethyl, R⁷ is hydrogen, Z² in R² is a covalent bond and R^{2c} in R² is methyl: 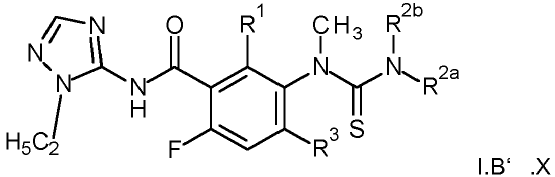
Table 22 Compounds of formula I.B' .X (I.B' .X.1 - I.B' .X.1104) in which the combination of R¹, R^{2a}, R^{2b}, and R³ for a compound corresponds in each case to one row of Table A;
   Compounds I.B' .XI, wherein Q is Q², R⁴ is hydrogen, R⁵ is fluorine, R^{6a} is methyl, R⁷ is hydrogen, Z² in R² is a covalent bond and R^{2c} in R² is ethyl: 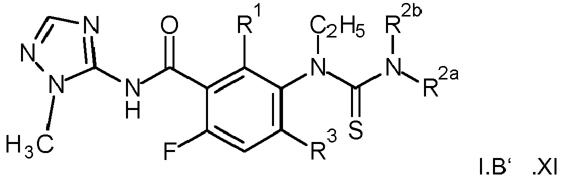
Table 23 Compounds of formula I.B' .XI (I.B' .XI.1 - I.B' .XI.1104) in which the combination of R¹, R^{2a}, R^{2b}, and R³ for a compound corresponds in each case to one row of Table A;
   Compounds I.B' .XII, wherein Q is Q², R⁴ is hydrogen, R⁵ is fluorine, R^{6a} is ethyl, R⁷ is hydrogen, Z² in R² is a covalent bond and R^{2c} in R² is ethyl: 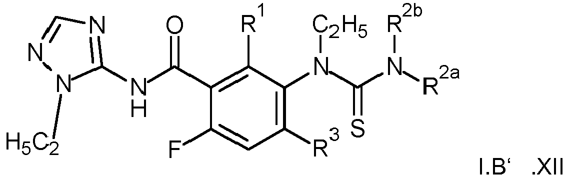
Table 24 Compounds of formula I.B' .XII (I.B' .XII.1 - I.B' .XII.1104) in which the combination of R¹, R^{2a}, R^{2b}, and R³ for a compound corresponds in each case to one row of Table A;
   Compounds I.C' .I, wherein Q is Q³, R⁴ is hydrogen, R⁵ is hydrogen, R^{6a} is methyl, R⁷ is hydrogen, Z² in R² is a covalent bond and R^{2c} in R² is H: 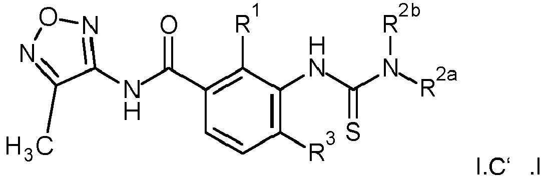
Table 25 Compounds of formula I.C' .I (I.B' .I.1 -I.C' .I.1104) in which the combination of R¹, R^{2a}, R^{2b}, and R³ for a compound corresponds in each case to one row of Table A;
   Compounds I.C' .II, wherein Q is Q³, R⁴ is hydrogen, R⁵ is hydrogen, R^{6a} is ethyl, R⁷ is hydrogen, Z² in R² is a covalent bond and R^{2c} in R² is H: 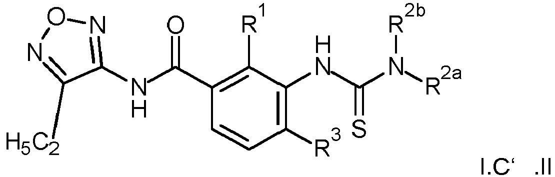
Table 26 Compounds of formula I.C' .II (I.C' .II.1 - I.C' .II.1104) in which the combination of R¹, R^{2a}, R^{2b}, and R³ for a compound corresponds in each case to one row of Table A;
   Compounds I.C' .III, wherein Q is Q³, R⁴ is hydrogen, R⁵ is hydrogen, R^{6a} is methyl, R⁷ is hydrogen, Z² in R² is a covalent bond and R^{2c} in R² is methyl: 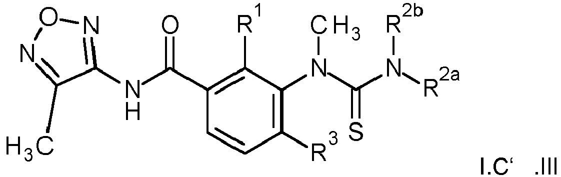
Table 27 Compounds of formula I.C' .III (I.C' .III.1 - I.C' .III.1104) in which the combination of R¹, R^{2a}, R^{2b}, and R³ for a compound corresponds in each case to one row of Table A;
   Compounds I.C' .IV, wherein Q is Q³, R⁴ is hydrogen, R⁵ is hydrogen, R^{6a} is ethyl, R⁷ is hydrogen, Z² in R² is a covalent bond and R^{2c} in R² is methyl: 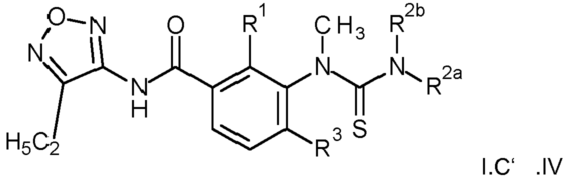
Table 28 Compounds of formula I.C' .IV (I.C' .IV.1 - I.C' .IV.1104) in which the combination of R¹, R^{2a}, R^{2b}, and R³ for a compound corresponds in each case to one row of Table A;
   Compounds I.C' .V, wherein Q is Q³, R⁴ is hydrogen, R⁵ is hydrogen, R^{6a} is methyl, R⁷ is hydrogen, Z² in R² is a covalent bond and R^{2c} in R² is ethyl: 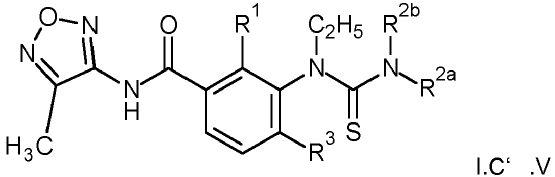
Table 29 Compounds of formula I.C' .V (I.C' .V.1 - I.C' .V.1104) in which the combination of R¹, R^{2a}, R^{2b}, and R³ for a compound corresponds in each case to one row of Table A;
   Compounds I.C' .VI, wherein Q is Q³, R⁴ is hydrogen, R⁵ is hydrogen, R^{6a} is ethyl, R⁷ is hydrogen, Z² in R² is a covalent bond and R^{2c} in R² is ethyl: 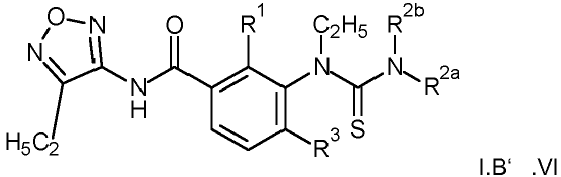
Table 30 Compounds of formula I.C' .VI (I.C' .VI.1 - I.C' .VI. 1104) in which the combination of R¹, R^{2a}, R^{2b}, and R³ for a compound corresponds in each case to one row of Table A;
   Compounds I.C' .VII, wherein Q is Q³, R⁴ is hydrogen, R⁵ is fluorine, R^{6a} is methyl, R⁷ is hydrogen, Z² in R² is a covalent bond and R^{2c} in R² is H: 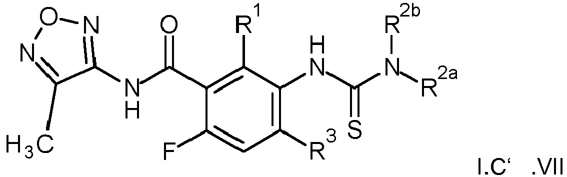
Table 31 Compounds of formula I.B' .VII (I.B' .VII.1 - I.B' .VII.1104) in which the combination of R¹, R^{2a}, R^{2b}, and R³ for a compound corresponds in each case to one row of Table A;
   Compounds I.C' .VIII, wherein Q is Q³, R⁴ is hydrogen, R⁵ is fluorine, R^{6a} is ethyl, R⁷ is hydrogen, Z² in R² is a covalent bond and R^{2c} in R² is H: 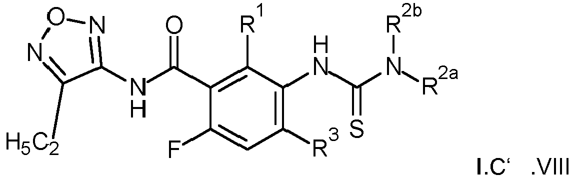
Table 32 Compounds of formula I.C' .VIII (I.C' .VIII.1 - I.C' .VIII.1104) in which the combination of R¹, R^{2a}, R^{2b}, and R³ for a compound corresponds in each case to one row of Table A;
   Compounds I.C' .IX, wherein Q is Q³, R⁴ is hydrogen, R⁵ is fluorine, R^{6a} is methyl, R⁷ is hydrogen, Z² in R² is a covalent bond and R^{2c} in R² is methyl: 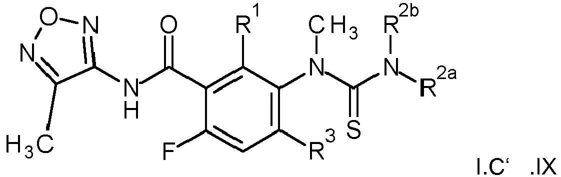
Table 33 Compounds of formula I.C' .IX (I.C' .IX.1 - I.C' .IX.1104) in which the combination of R¹, R^{2a}, R^{2b}, and R³ for a compound corresponds in each case to one row of Table A;
   Compounds I.C' .X, wherein Q is Q³, R⁴ is hydrogen, R⁵ is fluorine, R^{6a} is ethyl, R⁷ is hydrogen, Z² in R² is a covalent bond and R^{2c} in R² is methyl: 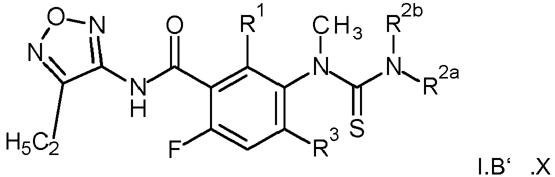
Table 34 Compounds of formula I.C' .X (I.C' .X.1 - I.C' .X.1104) in which the combination of R¹, R^{2a}, R^{2b}, and R³ for a compound corresponds in each case to one row of Table A;
   Compounds I.C' .XI, wherein Q is Q³, R⁴ is hydrogen, R⁵ is fluorine, R^{6a} is methyl, R⁷ is hydrogen, Z² in R² is a covalent bond and R^{2c} in R² is ethyl: 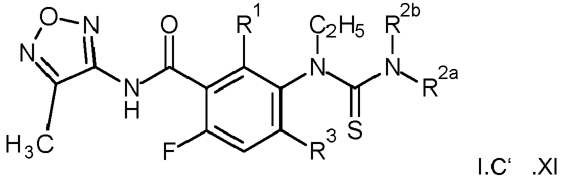
Table 35 Compounds of formula I.C' .XI (I.C' .XI.1 - I.C' .XI.1104) in which the combination of R¹, R^{2a}, R^{2b}, and R³ for a compound corresponds in each case to one row of Table A;
   Compounds I.C' .XII, wherein Q is Q³, R⁴ is hydrogen, R⁵ is fluorine, R^{6a} is ethyl, R⁷ is hydrogen, Z² in R² is a covalent bond and R^{2c} in R² is ethyl: 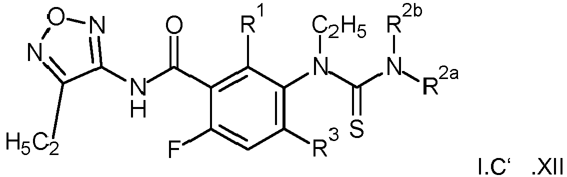
Table 36 Compounds of formula I.C' .XII (I.C' .XII.1 - I.C' .XII.1104) in which the combination of R¹, R^{2a}, R^{2b}, and R³ for a compound corresponds in each case to one row of Table A;
   Compounds I.D' .I, wherein Q is Q⁴, R⁴ is hydrogen, R⁵ is hydrogen, R^{6a} is methyl, R⁷ is hydrogen, Z² in R² is a covalent bond and R^{2c} in R² is H: 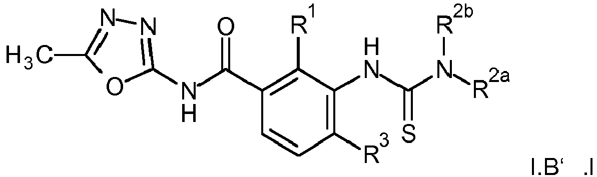
Table 37 Compounds of formula I.D' .I (I.D' .1.1 - I.D' .1.1104) in which the combination of R¹, R^{2a}, R^{2b}, and R³ for a compound corresponds in each case to one row of Table A;
   Compounds I.D' .II, wherein Q is Q⁴, R⁴ is hydrogen, R⁵ is hydrogen, R^{6a} is ethyl, R⁷ is hydrogen, Z² in R² is a covalent bond and R^{2c} in R² is H: 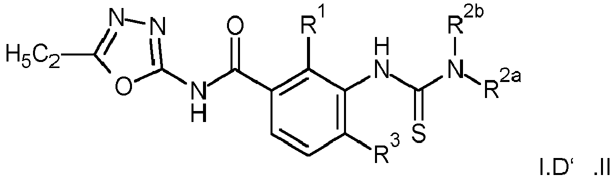
Table 38 Compounds of formula I.D' .II (I.D' .II.1 - I.D' .11.1104) in which the combination of R¹, R^{2a}, R^{2b}, and R³ for a compound corresponds in each case to one row of Table A;
   Compounds I.D' .III, wherein Q is Q⁴, R⁴ is hydrogen, R⁵ is hydrogen, R^{6a} is methyl, R⁷ is hydrogen, Z² in R² is a covalent bond and R^{2c} in R² is methyl: 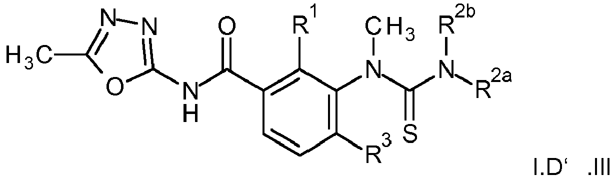
Table 39 Compounds of formula I.D' .III (I.D' .III.1 - I.D' .III.1104) in which the combination of R¹, R^{2a}, R^{2b}, and R³ for a compound corresponds in each case to one row of Table A;
   Compounds I.D' .IV, wherein Q is Q⁴, R⁴ is hydrogen, R⁵ is hydrogen, R^{6a} is ethyl, R⁷ is hydrogen, Z² in R² is a covalent bond and R^{2c} in R² is methyl: 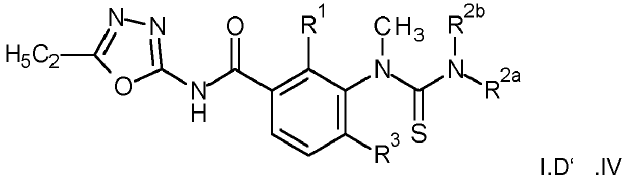
Table 40 Compounds of formula I.D' .IV (I.D' .IV.1 - I.D' .IV.1104) in which the combination of R¹, R^{2a}, R^{2b}, and R³ for a compound corresponds in each case to one row of Table A;
   Compounds I.D' .V, wherein Q is Q⁴, R⁴ is hydrogen, R⁵ is hydrogen, R^{6a} is methyl, R⁷ is hydrogen, Z² in R² is a covalent bond and R^{2c} in R² is ethyl: 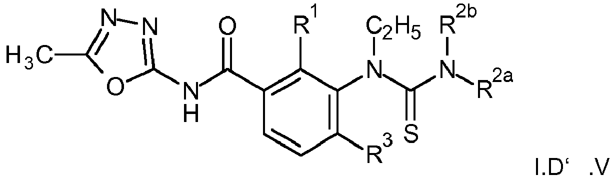
Table 41 Compounds of formula I.D' .V (I.D' .V.1 - I.D' .V.1104) in which the combination of R¹, R^{2a}, R^{2b}, and R³ for a compound corresponds in each case to one row of Table A;
   Compounds I.D' .VI, wherein Q is Q⁴, R⁴ is hydrogen, R⁵ is hydrogen, R^{6a} is ethyl, R⁷ is hydrogen, Z² in R² is a covalent bond and R^{2c} in R² is ethyl: 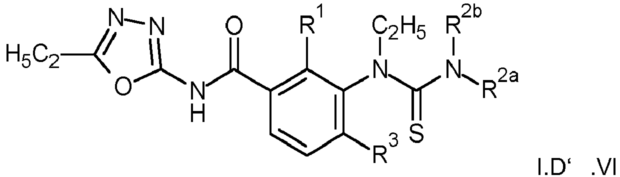
Table 42 Compounds of formula I.D' .VI (I.D' .VI.1 - I.D' .VI.1104) in which the combination of R¹, R^{2a}, R^{2b}, and R³ for a compound corresponds in each case to one row of Table A;
   Compounds I.D' .VII, wherein Q is Q⁴, R⁴ is hydrogen, R⁵ is fluorine, R^{6a} is methyl, R⁷ is hydrogen, Z² in R² is a covalent bond and R^{2c} in R² is H: 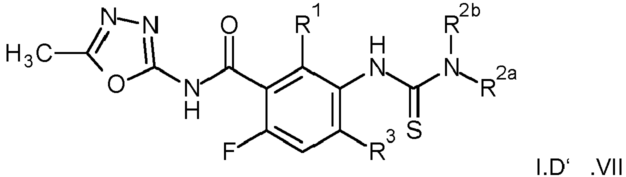
Table 43 Compounds of formula I.D' .VII (I.D' .VII.1 - I.D' .VII.1104) in which the combination of R¹, R^{2a}, R^{2b}, and R³ for a compound corresponds in each case to one row of Table A;
   Compounds I.D' .VIII, wherein Q is Q⁴, R⁴ is hydrogen, R⁵ is fluorine, R^{6a} is ethyl, R⁷ is hydrogen, Z² in R² is a covalent bond and R^{2c} in R² is H: 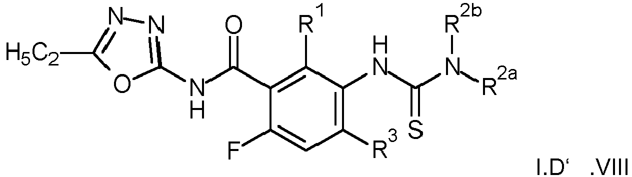
Table 44 Compounds of formula I.D' .VIII (I.D' .VIII.1 - I.D' .VIII.1104) in which the combination of R¹, R^{2a}, R^{2b}, and R³ for a compound corresponds in each case to one row of Table A;
   Compounds I.D' .IX, wherein Q is Q⁴, R⁴ is hydrogen, R⁵ is fluorine, R^{6a} is methyl, R⁷ is hydrogen, Z² in R² is a covalent bond and R^{2c} in R² is methyl: 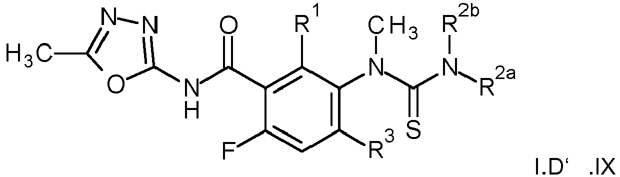
Table 45 Compounds of formula I.D' .IX (I.D' .IX.1 - I.D' .IX.1104) in which the combination of R¹, R^{2a}, R^{2b}, and R³ for a compound corresponds in each case to one row of Table A;
   Compounds I.D' .X, wherein Q is Q⁴, R⁴ is hydrogen, R⁵ is fluorine, R^{6a} is ethyl, R⁷ is hydrogen, Z² in R² is a covalent bond and R^{2c} in R² is methyl: 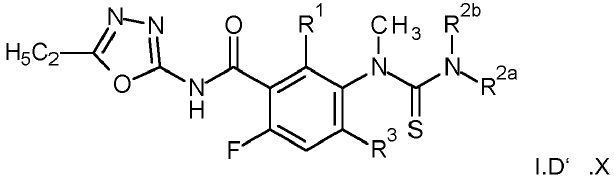
Table 46 Compounds of formula I.D' .X (I.D' .X.1 - I.D' .X.1104) in which the combination of R¹, R^{2a}, R^{2b}, and R³ for a compound corresponds in each case to one row of Table A;
   Compounds I.D' .XI, wherein Q is Q⁴, R⁴ is hydrogen, R⁵ is fluorine, R^{6a} is methyl, R⁷ is hydrogen, Z² in R² is a covalent bond and R^{2c} in R² is ethyl: 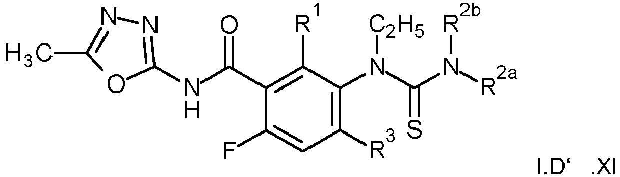
Table 47 Compounds of formula I.D' .XI (I.D' .XI.1 - I.D' .XI.1104) in which the combination of R¹, R^{2a}, R^{2b}, and R³ for a compound corresponds in each case to one row of Table A;
   Compounds I.D' .XII, wherein Q is Q⁴, R⁴ is hydrogen, R⁵ is fluorine, R^{6a} is ethyl, R⁷ is hydrogen, Z² in R² is a covalent bond and R^{2c} in R² is ethyl: 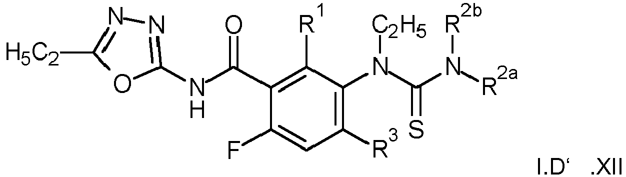
Table 48 Compounds of formula I.D' .XII (I.D' .XII.1 - I.D' .XII.1104) in which the combination of R¹, R^{2a}, R^{2b}, and R³ for a compound corresponds in each case to one row of Table A;

**Table A:**

| | R¹ | R^{2a} | R^{2b} | R³ |
|---|---|---|---|---|
| 1 | Cl | CH₃ | H | Cl |
| 2 | Cl | CH₃ | H | Br |
| 3 | Cl | CH₃ | H | I |
| 4 | Cl | CH₃ | H | CF₃ |
| 5 | Cl | CH₃ | H | CH₃S(O) |
| 6 | Cl | CH₃ | H | CH₃S(O)₂ |
| 7 | Cl | C₂H₅ | H | Cl |
| 8 | Cl | C₂H₅ | H | Br |
| 9 | Cl | C₂H₅ | H | I |
| 10 | Cl | C₂H₅ | H | CF₃ |
| 11 | Cl | C₂H₅ | H | CH₃S(O) |
| 12 | Cl | C₂H₅ | H | CH₃S(O)₂ |
| 13 | Cl | CH₃CH₂CH₂ | H | Cl |
| 14 | Cl | CH₃CH₂CH₂ | H | Br |
| 15 | Cl | CH₃CH₂CH₂ | H | I |
| 16 | Cl | CH₃CH₂CH₂ | H | CF₃ |
| 17 | Cl | CH₃CH₂CH₂ | H | CH₃S(O) |
| 18 | Cl | CH₃CH₂CH₂ | H | CH₃S(O)₂ |
| 19 | Cl | (CH₃)₂CH | H | Cl |
| 20 | Cl | (CH₃)₂CH | H | Br |
| 21 | Cl | (CH₃)₂CH | H | I |
| 22 | Cl | (CH₃)₂CH | H | CF₃ |
| 23 | Cl | (CH₃)₂CH | H | CH₃S(O) |
| 24 | Cl | (CH₃)₂CH | H | CH₃S(O)₂ |
| 25 | Cl | (CH₃)₃C | H | Cl |
| 26 | Cl | (CH₃)₃C | H | Br |
| 27 | Cl | (CH₃)₃C | H | I |
| 28 | Cl | (CH₃)₃C | H | CF₃ |
| 29 | Cl | (CH₃)₃C | H | CH₃S(O) |
| 30 | Cl | (CH₃)₃C | H | CH₃S(O)₂ |
| 31 | Cl | c-C₃H₅ | H | Cl |
| 32 | Cl | c-C₃H₅ | H | Br |
| 33 | Cl | c-C₃H₅ | H | I |
| 34 | Cl | c-C₃H₅ | H | CF₃ |
| 35 | Cl | c-C₃H₅ | H | CH₃S(O) |
| 36 | Cl | c-C₃H₅ | H | CH₃S(O)₂ |
| 37 | Cl | CHF₂CH₂ | H | Cl |
| 38 | Cl | CHF₂CH₂ | H | Br |
| 39 | Cl | CHF₂CH₂ | H | I |
| 40 | Cl | CHF₂CH₂ | H | CF₃ |
| 41 | Cl | CHF₂CH₂ | H | CH₃S(O) |
| 42 | Cl | CHF₂CH₂ | H | CH₃S(O)₂ |
| 43 | Cl | CF₃CH₂ | H | Cl |
| 44 | Cl | CF₃CH₂ | H | Br |
| 45 | Cl | CF₃CH₂ | H | I |
| 46 | Cl | CF₃CH₂ | H | CF₃ |
| 47 | Cl | CF₃CH₂ | H | CH₃S(O) |
| 48 | Cl | CF₃CH₂ | H | CH₃S(O)₂ |
| 49 | Cl | CH₃O | H | Cl |
| 50 | Cl | CH₃O | H | Br |
| 51 | Cl | CH₃O | H | I |
| 52 | Cl | CH₃O | H | CF₃ |
| 53 | Cl | CH₃O | H | CH₃S(O) |
| 54 | Cl | CH₃O | H | CH₃S(O)₂ |
| 55 | Cl | C₆H₅ | H | Cl |
| 56 | Cl | C₆H₅ | H | Br |
| 57 | Cl | C₆H₅ | H | I |
| 58 | Cl | C₆H₅ | H | CF₃ |
| 59 | Cl | C₆H₅ | H | CH₃S(O) |
| 60 | Cl | C₆H₅ | H | CH₃S(O)₂ |
| 61 | Cl | 4-Cl-C₆H₄ | H | Cl |
| 62 | Cl | 4-Cl-C₆H₄ | H | Br |
| 63 | Cl | 4-Cl-C₆H₄ | H | I |
| 64 | Cl | 4-Cl-C₆H₄ | H | CF₃ |
| 65 | Cl | 4-Cl-C₆H₄ | H | CH₃S(O) |
| 66 | Cl | 4-Cl-C₆H₄ | H | CH₃S(O)₂ |
| 67 | Cl | 4-CH₃-C₆H₄ | H | Cl |
| 68 | Cl | 4-CH₃-C₆H₄ | H | Br |
| 69 | Cl | 4-CH₃-C₆H₄ | H | I |
| 70 | Cl | 4-CH₃-C₆H₄ | H | CF₃ |
| 71 | Cl | 4-CH₃-C₆H₄ | H | CH₃S(O) |
| 72 | Cl | 4-CH₃-C₆H₄ | H | CH₃S(O)₂ |
| 73 | Cl | 4-CH₃O-C₆H₄ | H | Cl |
| 74 | Cl | 4-CH₃O-C₆H₄ | H | Br |
| 75 | Cl | 4-CH₃O-C₆H₄ | H | I |
| 76 | Cl | 4-CH₃O-C₆H₄ | H | CF₃ |
| 77 | Cl | 4-CH₃O-C₆H₄ | H | CH₃S(O) |
| 78 | Cl | 4-CH₃O-C₆H₄ | H | CH₃S(O)₂ |
| 79 | Cl | 2,6-(CH₃)₂-C₆H₃ | H | Cl |
| 80 | Cl | 2,6-(CH₃)₂-C₆H₃ | H | Br |
| 81 | Cl | 2,6-(CH₃)₂-C₆H₃ | H | I |
| 82 | Cl | 2,6-(CH₃)₂-C₆H₃ | H | CF₃ |
| 83 | Cl | 2,6-(CH₃)₂-C₆H₃ | H | CH₃S(O) |
| 84 | Cl | 2,6-(CH₃)₂-C₆H₃ | H | CH₃S(O)₂ |
| 85 | Cl | CH₃ | CH₃ | Cl |
| 86 | Cl | CH₃ | CH₃ | Br |
| 87 | Cl | CH₃ | CH₃ | I |
| 88 | Cl | CH₃ | CH₃ | CF₃ |
| 89 | Cl | CH₃ | CH₃ | CH₃S(O) |
| 90 | Cl | CH₃ | CH₃ | CH₃S(O)₂ |
| 91 | Cl | C₂H₅ | CH₃ | Cl |
| 92 | Cl | C₂H₅ | CH₃ | Br |
| 93 | Cl | C₂H₅ | CH₃ | I |
| 94 | Cl | C₂H₅ | CH₃ | CF₃ |
| 95 | Cl | C₂H₅ | CH₃ | CH₃S(O) |
| 96 | Cl | C₂H₅ | CH₃ | CH₃S(O)₂ |
| 97 | Cl | CH₃CH₂CH₂ | CH₃ | Cl |
| 98 | Cl | CH₃CH₂CH₂ | CH₃ | Br |
| 99 | Cl | CH₃CH₂CH₂ | CH₃ | I |
| 100 | Cl | CH₃CH₂CH₂ | CH₃ | CF₃ |
| 101 | Cl | CH₃CH₂CH₂ | CH₃ | CH₃S(O) |
| 102 | Cl | CH₃CH₂CH₂ | CH₃ | CH₃S(O)₂ |
| 103 | Cl | (CH₃)₂CH | CH₃ | Cl |
| 104 | Cl | (CH₃)₂CH | CH₃ | Br |
| 105 | Cl | (CH₃)₂CH | CH₃ | I |
| 106 | Cl | (CH₃)₂CH | CH₃ | CF₃ |
| 107 | Cl | (CH₃)₂CH | CH₃ | CH₃S(O) |
| 108 | Cl | (CH₃)₂CH | CH₃ | CH₃S(O)₂ |
| 109 | Cl | (CH₃)₃C | CH₃ | Cl |
| 110 | Cl | (CH₃)₃C | CH₃ | Br |
| 111 | Cl | (CH₃)₃C | CH₃ | I |
| 112 | Cl | (CH₃)₃C | CH₃ | CF₃ |
| 113 | Cl | (CH₃)₃C | CH₃ | CH₃S(O) |
| 114 | Cl | (CH₃)₃C | CH₃ | CH₃S(O)₂ |
| 115 | Cl | c-C₃H₅ | CH₃ | Cl |
| 116 | Cl | c-C₃H₅ | CH₃ | Br |
| 117 | Cl | c-C₃H₅ | CH₃ | I |
| 118 | Cl | c-C₃H₅ | CH₃ | CF₃ |
| 119 | Cl | c-C₃H₅ | CH₃ | CH₃S(O) |
| 120 | Cl | c-C₃H₅ | CH₃ | CH₃S(O)₂ |
| 121 | Cl | CHF₂CH₂ | CH₃ | Cl |
| 122 | Cl | CHF₂CH₂ | CH₃ | Br |
| 123 | Cl | CHF₂CH₂ | CH₃ | I |
| 124 | Cl | CHF₂CH₂ | CH₃ | CF₃ |
| 125 | Cl | CHF₂CH₂ | CH₃ | CH₃S(O) |
| 126 | Cl | CHF₂CH₂ | CH₃ | CH₃S(O)₂ |
| 127 | Cl | CF₃CH₂ | CH₃ | Cl |
| 128 | Cl | CF₃CH₂ | CH₃ | Br |
| 129 | Cl | CF₃CH₂ | CH₃ | I |
| 130 | Cl | CF₃CH₂ | CH₃ | CF₃ |
| 131 | Cl | CF₃CH₂ | CH₃ | CH₃S(O) |
| 132 | Cl | CF₃CH₂ | CH₃ | CH₃S(O)₂ |
| 133 | Cl | CH₃O | CH₃ | Cl |
| 134 | Cl | CH₃O | CH₃ | Br |
| 135 | Cl | CH₃O | CH₃ | I |
| 136 | Cl | CH₃O | CH₃ | CF₃ |
| 137 | Cl | CH₃O | CH₃ | CH₃S(O) |
| 138 | Cl | CH₃O | CH₃ | CH₃S(O)₂ |
| 139 | Cl | C₆H₅ | CH₃ | Cl |
| 140 | Cl | C₆H₅ | CH₃ | Br |
| 141 | Cl | C₆H₅ | CH₃ | I |
| 142 | Cl | C₆H₅ | CH₃ | CF₃ |
| 143 | Cl | C₆H₅ | CH₃ | CH₃S(O) |
| 144 | Cl | C₆H₅ | CH₃ | CH₃S(O)₂ |
| 145 | Cl | 4-Cl-C₆H₄ | CH₃ | Cl |
| 146 | Cl | 4-Cl-C₆H₄ | CH₃ | Br |
| 147 | Cl | 4-Cl-C₆H₄ | CH₃ | I |
| 148 | Cl | 4-Cl-C₆H₄ | CH₃ | CF₃ |
| 149 | Cl | 4-Cl-C₆H₄ | CH₃ | CH₃S(O) |
| 150 | Cl | 4-Cl-C₆H₄ | CH₃ | CH₃S(O)₂ |
| 151 | Cl | 4-CH₃-C₆H₄ | CH₃ | Cl |
| 152 | Cl | 4-CH₃-C₆H₄ | CH₃ | Br |
| 153 | Cl | 4-CH₃-C₆H₄ | CH₃ | I |
| 154 | Cl | 4-CH₃-C₆H₄ | CH₃ | CF₃ |
| 155 | Cl | 4-CH₃-C₆H₄ | CH₃ | CH₃S(O) |
| 156 | Cl | 4-CH₃-C₆H₄ | CH₃ | CH₃S(O)₂ |
| 157 | Cl | 4-CH₃O-C₆H₄ | CH₃ | Cl |
| 158 | Cl | 4-CH₃O-C₆H₄ | CH₃ | Br |
| 159 | Cl | 4-CH₃O-C₆H₄ | CH₃ | I |
| 160 | Cl | 4-CH₃O-C₆H₄ | CH₃ | CF₃ |
| 161 | Cl | 4-CH₃O-C₆H₄ | CH₃ | CH₃S(O) |
| 162 | Cl | 4-CH₃O-C₆H₄ | CH₃ | CH₃S(O)₂ |
| 163 | Cl | 2,6-(CH₃)₂-C₆H₃ | CH₃ | Cl |
| 164 | Cl | 2,6-(CH₃)₂-C₆H₃ | CH₃ | Br |
| 165 | Cl | 2,6-(CH₃)₂-C₆H₃ | CH₃ | I |
| 166 | Cl | 2,6-(CH₃)₂-C₆H₃ | CH₃ | CF₃ |
| 167 | Cl | 2,6-(CH₃)₂-C₆H₃ | CH₃ | CH₃S(O) |
| 168 | Cl | 2,6-(CH₃)₂-C₆H₃ | CH₃ | CH₃S(O)₂ |
| 169 | Cl | C₂H₅ | C₂H₅ | Cl |
| 170 | Cl | C₂H₅ | C₂H₅ | Br |
| 171 | Cl | C₂H₅ | C₂H₅ | I |
| 172 | Cl | C₂H₅ | C₂H₅ | CF₃ |
| 173 | Cl | C₂H₅ | C₂H₅ | CH₃S(O) |
| 174 | Cl | C₂H₅ | C₂H₅ | CH₃S(O)₂ |
| 175 | Cl | CH₃CH₂CH₂ | C₂H₅ | Cl |
| 176 | Cl | CH₃CH₂CH₂ | C₂H₅ | Br |
| 177 | Cl | CH₃CH₂CH₂ | C₂H₅ | I |
| 178 | Cl | CH₃CH₂CH₂ | C₂H₅ | CF₃ |
| 179 | Cl | CH₃CH₂CH₂ | C₂H₅ | CH₃S(O) |
| 180 | Cl | CH₃CH₂CH₂ | C₂H₅ | CH₃S(O)₂ |
| 181 | Cl | (CH₃)₂CH | C₂H₅ | Cl |
| 182 | Cl | (CH₃)₂CH | C₂H₅ | Br |
| 183 | Cl | (CH₃)₂CH | C₂H₅ | I |
| 184 | Cl | (CH₃)₂CH | C₂H₅ | CF₃ |
| 185 | Cl | (CH₃)₂CH | C₂H₅ | CH₃S(O) |
| 186 | Cl | (CH₃)₂CH | C₂H₅ | CH₃S(O)₂ |
| 187 | Cl | (CH₃)₃C | C₂H₅ | Cl |
| 188 | Cl | (CH₃)₃C | C₂H₅ | Br |
| 189 | Cl | (CH₃)₃C | C₂H₅ | I |
| 190 | Cl | (CH₃)₃C | C₂H₅ | CF₃ |
| 191 | Cl | (CH₃)₃C | C₂H₅ | CH₃S(O) |
| 192 | Cl | (CH₃)₃C | C₂H₅ | CH₃S(O)₂ |
| 193 | Cl | c-C₃H₅ | C₂H₅ | Cl |
| 194 | Cl | c-C₃H₅ | C₂H₅ | Br |
| 195 | Cl | c-C₃H₅ | C₂H₅ | I |
| 196 | Cl | c-C₃H₅ | C₂H₅ | CF₃ |
| 197 | Cl | c-C₃H₅ | C₂H₅ | CH₃S(O) |
| 198 | Cl | c-C₃H₅ | C₂H₅ | CH₃S(O)₂ |
| 199 | Cl | CHF₂CH₂ | C₂H₅ | Cl |
| 200 | Cl | CHF₂CH₂ | C₂H₅ | Br |
| 201 | Cl | CHF₂CH₂ | C₂H₅ | I |
| 202 | Cl | CHF₂CH₂ | C₂H₅ | CF₃ |
| 203 | Cl | CHF₂CH₂ | C₂H₅ | CH₃S(O) |
| 204 | Cl | CHF₂CH₂ | C₂H₅ | CH₃S(O)₂ |
| 205 | Cl | CF₃CH₂ | C₂H₅ | Cl |
| 206 | Cl | CF₃CH₂ | C₂H₅ | Br |
| 207 | Cl | CF₃CH₂ | C₂H₅ | I |
| 208 | Cl | CF₃CH₂ | C₂H₅ | CF₃ |
| 209 | Cl | CF₃CH₂ | C₂H₅ | CH₃S(O) |
| 210 | Cl | CF₃CH₂ | C₂H₅ | CH₃S(O)₂ |
| 211 | Cl | CH₃O | C₂H₅ | Cl |
| 212 | Cl | CH₃O | C₂H₅ | Br |
| 213 | Cl | CH₃O | C₂H₅ | I |
| 214 | Cl | CH₃O | C₂H₅ | CF₃ |
| 215 | Cl | CH₃O | C₂H₅ | CH₃S(O) |
| 216 | Cl | CH₃O | C₂H₅ | CH₃S(O)₂ |
| 217 | Cl | C₆H₅ | C₂H₅ | Cl |
| 218 | Cl | C₆H₅ | C₂H₅ | Br |
| 219 | Cl | C₆H₅ | C₂H₅ | I |
| 220 | Cl | C₆H₅ | C₂H₅ | CF₃ |
| 221 | Cl | C₆H₅ | C₂H₅ | CH₃S(O) |
| 222 | Cl | C₆H₅ | C₂H₅ | CH₃S(O)₂ |
| 223 | Cl | 4-Cl-C₆H₄ | C₂H₅ | Cl |
| 224 | Cl | 4-Cl-C₆H₄ | C₂H₅ | Br |
| 225 | Cl | 4-Cl-C₆H₄ | C₂H₅ | I |
| 226 | Cl | 4-Cl-C₆H₄ | C₂H₅ | CF₃ |
| 227 | Cl | 4-Cl-C₆H₄ | C₂H₅ | CH₃S(O) |
| 228 | Cl | 4-Cl-C₆H₄ | C₂H₅ | CH₃S(O)₂ |
| 229 | Cl | 4-CH₃-C₆H₄ | C₂H₅ | Cl |
| 230 | Cl | 4-CH₃-C₆H₄ | C₂H₅ | Br |
| 231 | Cl | 4-CH₃-C₆H₄ | C₂H₅ | I |
| 232 | Cl | 4-CH₃-C₆H₄ | C₂H₅ | CF₃ |
| 233 | Cl | 4-CH₃-C₆H₄ | C₂H₅ | CH₃S(O) |
| 234 | Cl | 4-CH₃-C₆H₄ | C₂H₅ | CH₃S(O)₂ |
| 235 | Cl | 4-CH₃O-C₆H₄ | C₂H₅ | Cl |
| 236 | Cl | 4-CH₃O-C₆H₄ | C₂H₅ | Br |
| 237 | Cl | 4-CH₃O-C₆H₄ | C₂H₅ | I |
| 238 | Cl | 4-CH₃O-C₆H₄ | C₂H₅ | CF₃ |
| 239 | Cl | 4-CH₃O-C₆H₄ | C₂H₅ | CH₃S(O) |
| 240 | Cl | 4-CH₃O-C₆H₄ | C₂H₅ | CH₃S(O)₂ |
| 241 | Cl | 2,6-(CH₃)₂-C₆H₃ | C₂H₅ | Cl |
| 242 | Cl | 2,6-(CH₃)₂-C₆H₃ | C₂H₅ | Br |
| 243 | Cl | 2,6-(CH₃)₂-C₆H₃ | C₂H₅ | I |
| 244 | Cl | 2,6-(CH₃)₂-C₆H₃ | C₂H₅ | CF₃ |
| 245 | Cl | 2,6-(CH₃)₂-C₆H₃ | C₂H₅ | CH₃S(O) |
| 246 | Cl | 2,6-(CH₃)₂-C₆H₃ | C₂H₅ | CH₃S(O)₂ |
| 247 | Cl | CH₃CH₂CH₂ | CH₃CH₂CH₂ | Cl |
| 248 | Cl | CH₃CH₂CH₂ | CH₃CH₂CH₂ | Br |
| 249 | Cl | CH₃CH₂CH₂ | CH₃CH₂CH₂ | I |
| 250 | Cl | CH₃CH₂CH₂ | CH₃CH₂CH₂ | CF₃ |
| 251 | Cl | CH₃CH₂CH₂ | CH₃CH₂CH₂ | CH₃S(O) |
| 252 | Cl | CH₃CH₂CH₂ | CH₃CH₂CH₂ | CH₃S(O)₂ |
| 253 | Cl | (CH₃)₂CH | CH₃CH₂CH₂ | Cl |
| 254 | Cl | (CH₃)₂CH | CH₃CH₂CH₂ | Br |
| 255 | Cl | (CH₃)₂CH | CH₃CH₂CH₂ | I |
| 256 | Cl | (CH₃)₂CH | CH₃CH₂CH₂ | CF₃ |
| 257 | Cl | (CH₃)₂CH | CH₃CH₂CH₂ | CH₃S(O) |
| 258 | Cl | (CH₃)₂CH | CH₃CH₂CH₂ | CH₃S(O)₂ |
| 259 | Cl | (CH₃)₃C | CH₃CH₂CH₂ | Cl |
| 260 | Cl | (CH₃)₃C | CH₃CH₂CH₂ | Br |
| 261 | Cl | (CH₃)₃C | CH₃CH₂CH₂ | I |
| 262 | Cl | (CH₃)₃C | CH₃CH₂CH₂ | CF₃ |
| 263 | Cl | (CH₃)₃C | CH₃CH₂CH₂ | CH₃S(O) |
| 264 | Cl | (CH₃)₃C | CH₃CH₂CH₂ | CH₃S(O)₂ |
| 265 | Cl | c-C₃H₅ | CH₃CH₂CH₂ | Cl |
| 266 | Cl | c-C₃H₅ | CH₃CH₂CH₂ | Br |
| 267 | Cl | c-C₃H₅ | CH₃CH₂CH₂ | I |
| 268 | Cl | c-C₃H₅ | CH₃CH₂CH₂ | CF₃ |
| 269 | Cl | c-C₃H₅ | CH₃CH₂CH₂ | CH₃S(O) |
| 270 | Cl | c-C₃H₅ | CH₃CH₂CH₂ | CH₃S(O)₂ |
| 271 | Cl | CHF₂CH₂ | CH₃CH₂CH₂ | Cl |
| 272 | Cl | CHF₂CH₂ | CH₃CH₂CH₂ | Br |
| 273 | Cl | CHF₂CH₂ | CH₃CH₂CH₂ | I |
| 274 | Cl | CHF₂CH₂ | CH₃CH₂CH₂ | CF₃ |
| 275 | Cl | CHF₂CH₂ | CH₃CH₂CH₂ | CH₃S(O) |
| 276 | Cl | CHF₂CH₂ | CH₃CH₂CH₂ | CH₃S(O)₂ |
| 277 | Cl | CF₃CH₂ | CH₃CH₂CH₂ | Cl |
| 278 | Cl | CF₃CH₂ | CH₃CH₂CH₂ | Br |
| 279 | Cl | CF₃CH₂ | CH₃CH₂CH₂ | I |
| 280 | Cl | CF₃CH₂ | CH₃CH₂CH₂ | CF₃ |
| 281 | Cl | CF₃CH₂ | CH₃CH₂CH₂ | CH₃S(O) |
| 282 | Cl | CF₃CH₂ | CH₃CH₂CH₂ | CH₃S(O)₂ |
| 283 | Cl | CH₃O | CH₃CH₂CH₂ | Cl |
| 284 | Cl | CH₃O | CH₃CH₂CH₂ | Br |
| 285 | Cl | CH₃O | CH₃CH₂CH₂ | I |
| 286 | Cl | CH₃O | CH₃CH₂CH₂ | CF₃ |
| 287 | Cl | CH₃O | CH₃CH₂CH₂ | CH₃S(O) |
| 288 | Cl | CH₃O | CH₃CH₂CH₂ | CH₃S(O)₂ |
| 289 | Cl | C₆H₅ | CH₃CH₂CH₂ | Cl |
| 290 | Cl | C₆H₅ | CH₃CH₂CH₂ | Br |
| 291 | Cl | C₆H₅ | CH₃CH₂CH₂ | I |
| 292 | Cl | C₆H₅ | CH₃CH₂CH₂ | CF₃ |
| 293 | Cl | C₆H₅ | CH₃CH₂CH₂ | CH₃S(O) |
| 294 | Cl | C₆H₅ | CH₃CH₂CH₂ | CH₃S(O)₂ |
| 295 | Cl | 4-Cl-C₆H₄ | CH₃CH₂CH₂ | Cl |
| 296 | Cl | 4-Cl-C₆H₄ | CH₃CH₂CH₂ | Br |
| 297 | Cl | 4-Cl-C₆H₄ | CH₃CH₂CH₂ | I |
| 298 | Cl | 4-Cl-C₆H₄ | CH₃CH₂CH₂ | CF₃ |
| 299 | Cl | 4-Cl-C₆H₄ | CH₃CH₂CH₂ | CH₃S(O) |
| 300 | Cl | 4-Cl-C₆H₄ | CH₃CH₂CH₂ | CH₃S(O)₂ |
| 301 | Cl | 4-CH₃-C₆H₄ | CH₃CH₂CH₂ | Cl |
| 302 | Cl | 4-CH₃-C₆H₄ | CH₃CH₂CH₂ | Br |
| 303 | Cl | 4-CH₃-C₆H₄ | CH₃CH₂CH₂ | I |
| 304 | Cl | 4-CH₃-C₆H₄ | CH₃CH₂CH₂ | CF₃ |
| 305 | Cl | 4-CH₃-C₆H₄ | CH₃CH₂CH₂ | CH₃S(O) |
| 306 | Cl | 4-CH₃-C₆H₄ | CH₃CH₂CH₂ | CH₃S(O)₂ |
| 307 | Cl | 4-CH₃O-C₆H₄ | CH₃CH₂CH₂ | Cl |
| 308 | Cl | 4-CH₃O-C₆H₄ | CH₃CH₂CH₂ | Br |
| 309 | Cl | 4-CH₃O-C₆H₄ | CH₃CH₂CH₂ | I |
| 310 | Cl | 4-CH₃O-C₆H₄ | CH₃CH₂CH₂ | CF₃ |
| 311 | Cl | 4-CH₃O-C₆H₄ | CH₃CH₂CH₂ | CH₃S(O) |
| 312 | Cl | 4-CH₃O-C₆H₄ | CH₃CH₂CH₂ | CH₃S(O)₂ |
| 313 | Cl | 2,6-(CH₃)₂-C₆H₃ | CH₃CH₂CH₂ | Cl |
| 314 | Cl | 2,6-(CH₃)₂-C₆H₃ | CH₃CH₂CH₂ | Br |
| 315 | Cl | 2,6-(CH₃)₂-C₆H₃ | CH₃CH₂CH₂ | I |
| 316 | Cl | 2,6-(CH₃)₂-C₆H₃ | CH₃CH₂CH₂ | CF₃ |
| 317 | Cl | 2,6-(CH₃)₂-C₆H₃ | CH₃CH₂CH₂ | CH₃S(O) |
| 318 | Cl | 2,6-(CH₃)₂-C₆H₃ | CH₃CH₂CH₂ | CH₃S(O)₂ |
| 319 | Cl | (CH₃)₂CH | (CH₃)₂CH | Cl |
| 320 | Cl | (CH₃)₂CH | (CH₃)₂CH | Br |
| 321 | Cl | (CH₃)₂CH | (CH₃)₂CH | I |
| 322 | Cl | (CH₃)₂CH | (CH₃)₂CH | CF₃ |
| 323 | Cl | (CH₃)₂CH | (CH₃)₂CH | CH₃S(O) |
| 324 | Cl | (CH₃)₂CH | (CH₃)₂CH | CH₃S(O)₂ |
| 325 | Cl | c-C₃H₅ | (CH₃)₂CH | Cl |
| 326 | Cl | c-C₃H₅ | (CH₃)₂CH | Br |
| 327 | Cl | c-C₃H₅ | (CH₃)₂CH | I |
| 328 | Cl | c-C₃H₅ | (CH₃)₂CH | CF₃ |
| 329 | Cl | c-C₃H₅ | (CH₃)₂CH | CH₃S(O) |
| 330 | Cl | c-C₃H₅ | (CH₃)₂CH | CH₃S(O)₂ |
| 331 | Cl | CHF₂CH₂ | (CH₃)₂CH | Cl |
| 332 | Cl | CHF₂CH₂ | (CH₃)₂CH | Br |
| 333 | Cl | CHF₂CH₂ | (CH₃)₂CH | I |
| 334 | Cl | CHF₂CH₂ | (CH₃)₂CH | CF₃ |
| 335 | Cl | CHF₂CH₂ | (CH₃)₂CH | CH₃S(O) |
| 336 | Cl | CHF₂CH₂ | (CH₃)₂CH | CH₃S(O)₂ |
| 337 | Cl | CF₃CH₂ | (CH₃)₂CH | Cl |
| 338 | Cl | CF₃CH₂ | (CH₃)₂CH | Br |
| 339 | Cl | CF₃CH₂ | (CH₃)₂CH | I |
| 340 | Cl | CF₃CH₂ | (CH₃)₂CH | CF₃ |
| 341 | Cl | CF₃CH₂ | (CH₃)₂CH | CH₃S(O) |
| 342 | Cl | CF₃CH₂ | (CH₃)₂CH | CH₃S(O)₂ |
| 343 | Cl | CH₃O | (CH₃)₂CH | Cl |
| 344 | Cl | CH₃O | (CH₃)₂CH | Br |
| 345 | Cl | CH₃O | (CH₃)₂CH | I |
| 346 | Cl | CH₃O | (CH₃)₂CH | CF₃ |
| 347 | Cl | CH₃O | (CH₃)₂CH | CH₃S(O) |
| 348 | Cl | CH₃O | (CH₃)₂CH | CH₃S(O)₂ |
| 349 | Cl | C₆H₅ | (CH₃)₂CH | Cl |
| 350 | Cl | C₆H₅ | (CH₃)₂CH | Br |
| 351 | Cl | C₆H₅ | (CH₃)₂CH | I |
| 352 | Cl | C₆H₅ | (CH₃)₂CH | CF₃ |
| 353 | Cl | C₆H₅ | (CH₃)₂CH | CH₃S(O) |
| 354 | Cl | C₆H₅ | (CH₃)₂CH | CH₃S(O)₂ |
| 355 | Cl | 4-Cl-C₆H₄ | (CH₃)₂CH | Cl |
| 356 | Cl | 4-Cl-C₆H₄ | (CH₃)₂CH | Br |
| 357 | Cl | 4-Cl-C₆H₄ | (CH₃)₂CH | I |
| 358 | Cl | 4-Cl-C₆H₄ | (CH₃)₂CH | CF₃ |
| 359 | Cl | 4-Cl-C₆H₄ | (CH₃)₂CH | CH₃S(O) |
| 360 | Cl | 4-Cl-C₆H₄ | (CH₃)₂CH | CH₃S(O)₂ |
| 361 | Cl | 4-CH₃-C₆H₄ | (CH₃)₂CH | Cl |
| 362 | Cl | 4-CH₃-C₆H₄ | (CH₃)₂CH | Br |
| 363 | Cl | 4-CH₃-C₆H₄ | (CH₃)₂CH | I |
| 364 | Cl | 4-CH₃-C₆H₄ | (CH₃)₂CH | CF₃ |
| 365 | Cl | 4-CH₃-C₆H₄ | (CH₃)₂CH | CH₃S(O) |
| 366 | Cl | 4-CH₃-C₆H₄ | (CH₃)₂CH | CH₃S(O)₂ |
| 367 | Cl | 4-CH₃O-C₆H₄ | (CH₃)₂CH | Cl |
| 368 | Cl | 4-CH₃O-C₆H₄ | (CH₃)₂CH | Br |
| 369 | Cl | 4-CH₃O-C₆H₄ | (CH₃)₂CH | I |
| 370 | Cl | 4-CH₃O-C₆H₄ | (CH₃)₂CH | CF₃ |
| 371 | Cl | 4-CH₃O-C₆H₄ | (CH₃)₂CH | CH₃S(O) |
| 372 | Cl | 4-CH₃O-C₆H₄ | (CH₃)₂CH | CH₃S(O)₂ |
| 373 | Cl | 2,6-(CH₃)₂-C₆H₃ | (CH₃)₂CH | Cl |
| 374 | Cl | 2,6-(CH₃)₂-C₆H₃ | (CH₃)₂CH | Br |
| 375 | Cl | 2,6-(CH₃)₂-C₆H₃ | (CH₃)₂CH | I |
| 376 | Cl | 2,6-(CH₃)₂-C₆H₃ | (CH₃)₂CH | CF₃ |
| 377 | Cl | 2,6-(CH₃)₂-C₆H₃ | (CH₃)₂CH | CH₃S(O) |
| 378 | Cl | 2,6-(CH₃)₂-C₆H₃ | (CH₃)₂CH | CH₃S(O)₂ |
| 379 | Cl | c-C₃H₅ | c-C₃H₅ | Cl |
| 380 | Cl | c-C₃H₅ | c-C₃H₅ | Br |
| 381 | Cl | c-C₃H₅ | c-C₃H₅ | I |
| 382 | Cl | c-C₃H₅ | c-C₃H₅ | CF₃ |
| 383 | Cl | c-C₃H₅ | c-C₃H₅ | CH₃S(O) |
| 384 | Cl | c-C₃H₅ | c-C₃H₅ | CH₃S(O)₂ |
| 385 | Cl | CHF₂CH₂ | c-C₃H₅ | Cl |
| 386 | Cl | CHF₂CH₂ | c-C₃H₅ | Br |
| 387 | Cl | CHF₂CH₂ | c-C₃H₅ | I |
| 388 | Cl | CHF₂CH₂ | c-C₃H₅ | CF₃ |
| 389 | Cl | CHF₂CH₂ | c-C₃H₅ | CH₃S(O) |
| 390 | Cl | CHF₂CH₂ | c-C₃H₅ | CH₃S(O)₂ |
| 391 | Cl | CF₃CH₂ | c-C₃H₅ | Cl |
| 392 | Cl | CF₃CH₂ | c-C₃H₅ | Br |
| 393 | Cl | CF₃CH₂ | c-C₃H₅ | I |
| 394 | Cl | CF₃CH₂ | c-C₃H₅ | CF₃ |
| 395 | Cl | CF₃CH₂ | c-C₃H₅ | CH₃S(O) |
| 396 | Cl | CF₃CH₂ | c-C₃H₅ | CH₃S(O)₂ |
| 397 | Cl | CH₃O | c-C₃H₅ | Cl |
| 398 | Cl | CH₃O | c-C₃H₅ | Br |
| 399 | Cl | CH₃O | c-C₃H₅ | I |
| 400 | Cl | CH₃O | c-C₃H₅ | CF₃ |
| 401 | Cl | CH₃O | c-C₃H₅ | CH₃S(O) |
| 402 | Cl | CH₃O | c-C₃H₅ | CH₃S(O)₂ |
| 403 | Cl | C₆H₅ | c-C₃H₅ | Cl |
| 404 | Cl | C₆H₅ | c-C₃H₅ | Br |
| 405 | Cl | C₆H₅ | c-C₃H₅ | I |
| 406 | Cl | C₆H₅ | c-C₃H₅ | CF₃ |
| 407 | Cl | C₆H₅ | c-C₃H₅ | CH₃S(O) |
| 408 | Cl | C₆H₅ | c-C₃H₅ | CH₃S(O)₂ |
| 409 | Cl | 4-Cl-C₆H₄ | c-C₃H₅ | Cl |
| 410 | Cl | 4-Cl-C₆H₄ | c-C₃H₅ | Br |
| 411 | Cl | 4-Cl-C₆H₄ | c-C₃H₅ | I |
| 412 | Cl | 4-Cl-C₆H₄ | c-C₃H₅ | CF₃ |
| 413 | Cl | 4-Cl-C₆H₄ | c-C₃H₅ | CH₃S(O) |
| 414 | Cl | 4-Cl-C₆H₄ | c-C₃H₅ | CH₃S(O)₂ |
| 415 | Cl | 4-CH₃-C₆H₄ | c-C₃H₅ | Cl |
| 416 | Cl | 4-CH₃-C₆H₄ | c-C₃H₅ | Br |
| 417 | Cl | 4-CH₃-C₆H₄ | c-C₃H₅ | I |
| 418 | Cl | 4-CH₃-C₆H₄ | c-C₃H₅ | CF₃ |
| 419 | Cl | 4-CH₃-C₆H₄ | c-C₃H₅ | CH₃S(O) |
| 420 | Cl | 4-CH₃-C₆H₄ | c-C₃H₅ | CH₃S(O)₂ |
| 421 | Cl | 4-CH₃O-C₆H₄ | c-C₃H₅ | Cl |
| 422 | Cl | 4-CH₃O-C₆H₄ | c-C₃H₅ | Br |
| 423 | Cl | 4-CH₃O-C₆H₄ | c-C₃H₅ | I |
| 424 | Cl | 4-CH₃O-C₆H₄ | c-C₃H₅ | CF₃ |
| 425 | Cl | 4-CH₃O-C₆H₄ | c-C₃H₅ | CH₃S(O) |
| 426 | Cl | 4-CH₃O-C₆H₄ | c-C₃H₅ | CH₃S(O)₂ |
| 427 | Cl | 2,6-(CH₃)₂-C₆H₃ | c-C₃H₅ | Cl |
| 428 | Cl | 2,6-(CH₃)₂-C₆H₃ | c-C₃H₅ | Br |
| 429 | Cl | 2,6-(CH₃)₂-C₆H₃ | c-C₃H₅ | I |
| 430 | Cl | 2,6-(CH₃)₂-C₆H₃ | c-C₃H₅ | CF₃ |
| 431 | Cl | 2,6-(CH₃)₂-C₆H₃ | c-C₃H₅ | CH₃S(O) |
| 432 | Cl | 2,6-(CH₃)₂-C₆H₃ | c-C₃H₅ | CH₃S(O)₂ |
| 433 | Cl | (CH₃)₃C | CHF₂CH₂ | Cl |
| 434 | Cl | (CH₃)₃C | CHF₂CH₂ | Br |
| 435 | Cl | (CH₃)₃C | CHF₂CH₂ | I |
| 436 | Cl | (CH₃)₃C | CHF₂CH₂ | CF₃ |
| 437 | Cl | (CH₃)₃C | CHF₂CH₂ | CH₃S(O) |
| 438 | Cl | (CH₃)₃C | CHF₂CH₂ | CH₃S(O)₂ |
| 439 | Cl | CHF₂CH₂ | CHF₂CH₂ | Cl |
| 440 | Cl | CHF₂CH₂ | CHF₂CH₂ | Br |
| 441 | Cl | CHF₂CH₂ | CHF₂CH₂ | I |
| 442 | Cl | CHF₂CH₂ | CHF₂CH₂ | CF₃ |
| 443 | Cl | CHF₂CH₂ | CHF₂CH₂ | CH₃S(O) |
| 444 | Cl | CHF₂CH₂ | CHF₂CH₂ | CH₃S(O)₂ |
| 445 | Cl | CF₃CH₂ | CHF₂CH₂ | Cl |
| 446 | Cl | CF₃CH₂ | CHF₂CH₂ | Br |
| 447 | Cl | CF₃CH₂ | CHF₂CH₂ | I |
| 448 | Cl | CF₃CH₂ | CHF₂CH₂ | CF₃ |
| 449 | Cl | CF₃CH₂ | CHF₂CH₂ | CH₃S(O) |
| 450 | Cl | CF₃CH₂ | CHF₂CH₂ | CH₃S(O)₂ |
| 451 | Cl | CH₃O | CHF₂CH₂ | Cl |
| 452 | Cl | CH₃O | CHF₂CH₂ | Br |
| 453 | Cl | CH₃O | CHF₂CH₂ | I |
| 454 | Cl | CH₃O | CHF₂CH₂ | CF₃ |
| 455 | Cl | CH₃O | CHF₂CH₂ | CH₃S(O) |
| 456 | Cl | CH₃O | CHF₂CH₂ | CH₃S(O)₂ |
| 457 | Cl | C₆H₅ | CHF₂CH₂ | Cl |
| 458 | Cl | C₆H₅ | CHF₂CH₂ | Br |
| 459 | Cl | C₆H₅ | CHF₂CH₂ | I |
| 460 | Cl | C₆H₅ | CHF₂CH₂ | CF₃ |
| 461 | Cl | C₆H₅ | CHF₂CH₂ | CH₃S(O) |
| 462 | Cl | C₆H₅ | CHF₂CH₂ | CH₃S(O)₂ |
| 463 | Cl | 4-Cl-C₆H₄ | CHF₂CH₂ | Cl |
| 464 | Cl | 4-Cl-C₆H₄ | CHF₂CH₂ | Br |
| 465 | Cl | 4-Cl-C₆H₄ | CHF₂CH₂ | I |
| 466 | Cl | 4-Cl-C₆H₄ | CHF₂CH₂ | CF₃ |
| 467 | Cl | 4-Cl-C₆H₄ | CHF₂CH₂ | CH₃S(O) |
| 468 | Cl | 4-Cl-C₆H₄ | CHF₂CH₂ | CH₃S(O)₂ |
| 469 | Cl | 4-CH₃-C₆H₄ | CHF₂CH₂ | Cl |
| 470 | Cl | 4-CH₃-C₆H₄ | CHF₂CH₂ | Br |
| 471 | Cl | 4-CH₃-C₆H₄ | CHF₂CH₂ | I |
| 472 | Cl | 4-CH₃-C₆H₄ | CHF₂CH₂ | CF₃ |
| 473 | Cl | 4-CH₃-C₆H₄ | CHF₂CH₂ | CH₃S(O) |
| 474 | Cl | 4-CH₃-C₆H₄ | CHF₂CH₂ | CH₃S(O)₂ |
| 475 | Cl | 4-CH₃O-C₆H₄ | CHF₂CH₂ | Cl |
| 476 | Cl | 4-CH₃O-C₆H₄ | CHF₂CH₂ | Br |
| 477 | Cl | 4-CH₃O-C₆H₄ | CHF₂CH₂ | I |
| 478 | Cl | 4-CH₃O-C₆H₄ | CHF₂CH₂ | CF₃ |
| 479 | Cl | 4-CH₃O-C₆H₄ | CHF₂CH₂ | CH₃S(O) |
| 480 | Cl | 4-CH₃O-C₆H₄ | CHF₂CH₂ | CH₃S(O)₂ |
| 481 | Cl | 2,6-(CH₃)₂-C₆H₃ | CHF₂CH₂ | Cl |
| 482 | Cl | 2,6-(CH₃)₂-C₆H₃ | CHF₂CH₂ | Br |
| 483 | Cl | 2,6-(CH₃)₂-C₆H₃ | CHF₂CH₂ | I |
| 484 | Cl | 2,6-(CH₃)₂-C₆H₃ | CHF₂CH₂ | CF₃ |
| 485 | Cl | 2,6-(CH₃)₂-C₆H₃ | CHF₂CH₂ | CH₃S(O) |
| 486 | Cl | 2,6-(CH₃)₂-C₆H₃ | CHF₂CH₂ | CH₃S(O)₂ |
| 487 | Cl | (CH₃)₃C | CF₃CH₂ | Cl |
| 488 | Cl | (CH₃)₃C | CF₃CH₂ | Br |
| 489 | Cl | (CH₃)₃C | CF₃CH₂ | I |
| 490 | Cl | (CH₃)₃C | CF₃CH₂ | CF₃ |
| 491 | Cl | (CH₃)₃C | CF₃CH₂ | CH₃S(O) |
| 492 | Cl | (CH₃)₃C | CF₃CH₂ | CH₃S(O)₂ |
| 493 | Cl | CF₃CH₂ | CF₃CH₂ | Cl |
| 494 | Cl | CF₃CH₂ | CF₃CH₂ | Br |
| 495 | Cl | CF₃CH₂ | CF₃CH₂ | I |
| 496 | Cl | CF₃CH₂ | CF₃CH₂ | CF₃ |
| 497 | Cl | CF₃CH₂ | CF₃CH₂ | CH₃S(O) |
| 498 | Cl | CF₃CH₂ | CF₃CH₂ | CH₃S(O)₂ |
| 499 | Cl | CH₃O | CF₃CH₂ | Cl |
| 500 | Cl | CH₃O | CF₃CH₂ | Br |
| 501 | Cl | CH₃O | CF₃CH₂ | I |
| 502 | Cl | CH₃O | CF₃CH₂ | CF₃ |
| 503 | Cl | CH₃O | CF₃CH₂ | CH₃S(O) |
| 504 | Cl | CH₃O | CF₃CH₂ | CH₃S(O)₂ |
| 505 | Cl | C₆H₅ | CF₃CH₂ | Cl |
| 506 | Cl | C₆H₅ | CF₃CH₂ | Br |
| 507 | Cl | C₆H₅ | CF₃CH₂ | I |
| 508 | Cl | C₆H₅ | CF₃CH₂ | CF₃ |
| 509 | Cl | C₆H₅ | CF₃CH₂ | CH₃S(O) |
| 510 | Cl | C₆H₅ | CF₃CH₂ | CH₃S(O)₂ |
| 511 | Cl | 4-Cl-C₆H₄ | CF₃CH₂ | Cl |
| 512 | Cl | 4-Cl-C₆H₄ | CF₃CH₂ | Br |
| 513 | Cl | 4-Cl-C₆H₄ | CF₃CH₂ | I |
| 514 | Cl | 4-Cl-C₆H₄ | CF₃CH₂ | CF₃ |
| 515 | Cl | 4-Cl-C₆H₄ | CF₃CH₂ | CH₃S(O) |
| 516 | Cl | 4-Cl-C₆H₄ | CF₃CH₂ | CH₃S(O)₂ |
| 517 | Cl | 4-CH₃-C₆H₄ | CF₃CH₂ | Cl |
| 518 | Cl | 4-CH₃-C₆H₄ | CF₃CH₂ | Br |
| 519 | Cl | 4-CH₃-C₆H₄ | CF₃CH₂ | I |
| 520 | Cl | 4-CH₃-C₆H₄ | CF₃CH₂ | CF₃ |
| 521 | Cl | 4-CH₃-C₆H₄ | CF₃CH₂ | CH₃S(O) |
| 522 | Cl | 4-CH₃-C₆H₄ | CF₃CH₂ | CH₃S(O)₂ |
| 523 | Cl | 4-CH₃O-C₆H₄ | CF₃CH₂ | Cl |
| 524 | Cl | 4-CH₃O-C₆H₄ | CF₃CH₂ | Br |
| 525 | Cl | 4-CH₃O-C₆H₄ | CF₃CH₂ | I |
| 526 | Cl | 4-CH₃O-C₆H₄ | CF₃CH₂ | CF₃ |
| 527 | Cl | 4-CH₃O-C₆H₄ | CF₃CH₂ | CH₃S(O) |
| 528 | Cl | 4-CH₃O-C₆H₄ | CF₃CH₂ | CH₃S(O)₂ |
| 529 | Cl | 2,6-(CH₃)₂-C₆H₃ | CF₃CH₂ | Cl |
| 530 | Cl | 2,6-(CH₃)₂-C₆H₃ | CF₃CH₂ | Br |
| 531 | Cl | 2,6-(CH₃)₂-C₆H₃ | CF₃CH₂ | I |
| 532 | Cl | 2,6-(CH₃)₂-C₆H₃ | CF₃CH₂ | CF₃ |
| 533 | Cl | 2,6-(CH₃)₂-C₆H₃ | CF₃CH₂ | CH₃S(O) |
| 534 | Cl | 2,6-(CH₃)₂-C₆H₃ | CF₃CH₂ | CH₃S(O)₂ |
| 535 | Cl | NR^{2a}R^{2b} = | 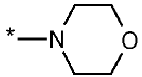 | Cl |
| 536 | Cl | NR^{2a}R^{2b} = | 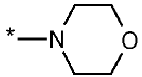 | Br |
| 537 | Cl | NR^{2a}R^{2b} = | 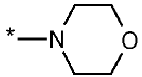 | I |
| 538 | Cl | NR^{2a}R^{2b} = | 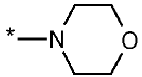 | CF₃ |
| 539 | Cl | NR^{2a}R^{2b} = | 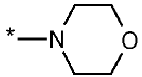 | CH₃S(O) |
| 540 | Cl | NR^{2a}R^{2b} = | 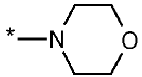 | CH₃S(O)₂ |
| 541 | Cl | NR^{2a}R^{2b} = | 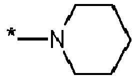 | Cl |
| 542 | Cl | NR^{2a}R^{2b} = | 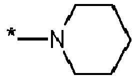 | Br |
| 543 | Cl | NR^{2a}R^{2b} = | 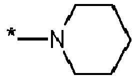 | I |
| 544 | Cl | NR^{2a}R^{2b} = | 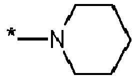 | CF₃ |
| 545 | Cl | NR^{2a}R^{2b} = | 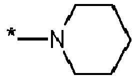 | CH₃S(O) |
| 546 | Cl | NR^{2a}R^{2b} = | 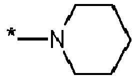 | CH₃S(O)₂ |
| 547 | Cl | NR^{2a}R^{2b} = | 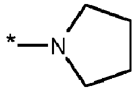 | Cl |
| 548 | Cl | NR^{2a}R^{2b} = | 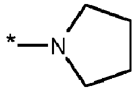 | Br |
| 549 | Cl | NR^{2a}R^{2b} = | 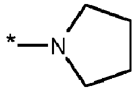 | I |
| 550 | Cl | NR^{2a}R^{2b} = | 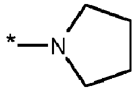 | CF₃ |
| 551 | Cl | NR^{2a}R^{2b} = | 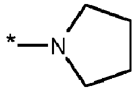 | CH₃S(O) |
| 552 | Cl | NR^{2a}R^{2b} = | 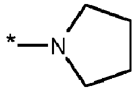 | CH₃S(O)₂ |
| 553 | CH₃ | CH₃ | H | Cl |
| 554 | CH₃ | CH₃ | H | Br |
| 555 | CH₃ | CH₃ | H | I |
| 556 | CH₃ | CH₃ | H | CF₃ |
| 557 | CH₃ | CH₃ | H | CH₃S(O) |
| 558 | CH₃ | CH₃ | H | CH₃S(O)₂ |
| 559 | CH₃ | C₂H₅ | H | Cl |
| 560 | CH₃ | C₂H₅ | H | Br |
| 561 | CH₃ | C₂H₅ | H | I |
| 562 | CH₃ | C₂H₅ | H | CF₃ |
| 563 | CH₃ | C₂H₅ | H | CH₃S(O) |
| 564 | CH₃ | C₂H₅ | H | CH₃S(O)₂ |
| 565 | CH₃ | CH₃CH₂CH₂ | H | Cl |
| 566 | CH₃ | CH₃CH₂CH₂ | H | Br |
| 567 | CH₃ | CH₃CH₂CH₂ | H | I |
| 568 | CH₃ | CH₃CH₂CH₂ | H | CF₃ |
| 569 | CH₃ | CH₃CH₂CH₂ | H | CH₃S(O) |
| 570 | CH₃ | CH₃CH₂CH₂ | H | CH₃S(O)₂ |
| 571 | CH₃ | (CH₃)₂CH | H | Cl |
| 572 | CH₃ | (CH₃)₂CH | H | Br |
| 573 | CH₃ | (CH₃)₂CH | H | I |
| 574 | CH₃ | (CH₃)₂CH | H | CF₃ |
| 575 | CH₃ | (CH₃)₂CH | H | CH₃S(O) |
| 576 | CH₃ | (CH₃)₂CH | H | CH₃S(O)₂ |
| 577 | CH₃ | (CH₃)₃C | H | Cl |
| 578 | CH₃ | (CH₃)₃C | H | Br |
| 579 | CH₃ | (CH₃)₃C | H | I |
| 580 | CH₃ | (CH₃)₃C | H | CF₃ |
| 581 | CH₃ | (CH₃)₃C | H | CH₃S(O) |
| 582 | CH₃ | (CH₃)₃C | H | CH₃S(O)₂ |
| 583 | CH₃ | c-C₃H₅ | H | Cl |
| 584 | CH₃ | c-C₃H₅ | H | Br |
| 585 | CH₃ | c-C₃H₅ | H | I |
| 586 | CH₃ | c-C₃H₅ | H | CF₃ |
| 587 | CH₃ | c-C₃H₅ | H | CH₃S(O) |
| 588 | CH₃ | c-C₃H₅ | H | CH₃S(O)₂ |
| 589 | CH₃ | CHF₂CH₂ | H | Cl |
| 590 | CH₃ | CHF₂CH₂ | H | Br |
| 591 | CH₃ | CHF₂CH₂ | H | I |
| 592 | CH₃ | CHF₂CH₂ | H | CF₃ |
| 593 | CH₃ | CHF₂CH₂ | H | CH₃S(O) |
| 594 | CH₃ | CHF₂CH₂ | H | CH₃S(O)₂ |
| 595 | CH₃ | CF₃CH₂ | H | Cl |
| 596 | CH₃ | CF₃CH₂ | H | Br |
| 597 | CH₃ | CF₃CH₂ | H | I |
| 598 | CH₃ | CF₃CH₂ | H | CF₃ |
| 599 | CH₃ | CF₃CH₂ | H | CH₃S(O) |
| 600 | CH₃ | CF₃CH₂ | H | CH₃S(O)₂ |
| 601 | CH₃ | CH₃O | H | Cl |
| 602 | CH₃ | CH₃O | H | Br |
| 603 | CH₃ | CH₃O | H | I |
| 604 | CH₃ | CH₃O | H | CF₃ |
| 605 | CH₃ | CH₃O | H | CH₃S(O) |
| 606 | CH₃ | CH₃O | H | CH₃S(O)₂ |
| 607 | CH₃ | C₆H₅ | H | Cl |
| 608 | CH₃ | C₆H₅ | H | Br |
| 609 | CH₃ | C₆H₅ | H | I |
| 610 | CH₃ | C₆H₅ | H | CF₃ |
| 611 | CH₃ | C₆H₅ | H | CH₃S(O) |
| 612 | CH₃ | C₆H₅ | H | CH₃S(O)₂ |
| 613 | CH₃ | 4-Cl-C₆H₄ | H | Cl |
| 614 | CH₃ | 4-Cl-C₆H₄ | H | Br |
| 615 | CH₃ | 4-Cl-C₆H₄ | H | I |
| 616 | CH₃ | 4-Cl-C₆H₄ | H | CF₃ |
| 617 | CH₃ | 4-Cl-C₆H₄ | H | CH₃S(O) |
| 618 | CH₃ | 4-Cl-C₆H₄ | H | CH₃S(O)₂ |
| 619 | CH₃ | 4-CH₃-C₆H₄ | H | Cl |
| 620 | CH₃ | 4-CH₃-C₆H₄ | H | Br |
| 621 | CH₃ | 4-CH₃-C₆H₄ | H | I |
| 622 | CH₃ | 4-CH₃-C₆H₄ | H | CF₃ |
| 623 | CH₃ | 4-CH₃-C₆H₄ | H | CH₃S(O) |
| 624 | CH₃ | 4-CH₃-C₆H₄ | H | CH₃S(O)₂ |
| 625 | CH₃ | 4-CH₃O-C₆H₄ | H | Cl |
| 626 | CH₃ | 4-CH₃O-C₆H₄ | H | Br |
| 627 | CH₃ | 4-CH₃O-C₆H₄ | H | I |
| 628 | CH₃ | 4-CH₃O-C₆H₄ | H | CF₃ |
| 629 | CH₃ | 4-CH₃O-C₆H₄ | H | CH₃S(O) |
| 630 | CH₃ | 4-CH₃O-C₆H₄ | H | CH₃S(O)₂ |
| 631 | CH₃ | 2,6-(CH₃)₂-C₆H₃ | H | Cl |
| 632 | CH₃ | 2,6-(CH₃)₂-C₆H₃ | H | Br |
| 633 | CH₃ | 2,6-(CH₃)₂-C₆H₃ | H | I |
| 634 | CH₃ | 2,6-(CH₃)₂-C₆H₃ | H | CF₃ |
| 635 | CH₃ | 2,6-(CH₃)₂-C₆H₃ | H | CH₃S(O) |
| 636 | CH₃ | 2,6-(CH₃)₂-C₆H₃ | H | CH₃S(O)₂ |
| 637 | CH₃ | CH₃ | CH₃ | Cl |
| 638 | CH₃ | CH₃ | CH₃ | Br |
| 639 | CH₃ | CH₃ | CH₃ | I |
| 640 | CH₃ | CH₃ | CH₃ | CF₃ |
| 641 | CH₃ | CH₃ | CH₃ | CH₃S(O) |
| 642 | CH₃ | CH₃ | CH₃ | CH₃S(O)₂ |
| 643 | CH₃ | C₂H₅ | CH₃ | Cl |
| 644 | CH₃ | C₂H₅ | CH₃ | Br |
| 645 | CH₃ | C₂H₅ | CH₃ | I |
| 646 | CH₃ | C₂H₅ | CH₃ | CF₃ |
| 647 | CH₃ | C₂H₅ | CH₃ | CH₃S(O) |
| 648 | CH₃ | C₂H₅ | CH₃ | CH₃S(O)₂ |
| 649 | CH₃ | CH₃CH₂CH₂ | CH₃ | Cl |
| 650 | CH₃ | CH₃CH₂CH₂ | CH₃ | Br |
| 651 | CH₃ | CH₃CH₂CH₂ | CH₃ | I |
| 652 | CH₃ | CH₃CH₂CH₂ | CH₃ | CF₃ |
| 653 | CH₃ | CH₃CH₂CH₂ | CH₃ | CH₃S(O) |
| 654 | CH₃ | CH₃CH₂CH₂ | CH₃ | CH₃S(O)₂ |
| 655 | CH₃ | (CH₃)₂CH | CH₃ | Cl |
| 656 | CH₃ | (CH₃)₂CH | CH₃ | Br |
| 657 | CH₃ | (CH₃)₂CH | CH₃ | I |
| 658 | CH₃ | (CH₃)₂CH | CH₃ | CF₃ |
| 659 | CH₃ | (CH₃)₂CH | CH₃ | CH₃S(O) |
| 660 | CH₃ | (CH₃)₂CH | CH₃ | CH₃S(O)₂ |
| 661 | CH₃ | (CH₃)₃C | CH₃ | Cl |
| 662 | CH₃ | (CH₃)₃C | CH₃ | Br |
| 663 | CH₃ | (CH₃)₃C | CH₃ | I |
| 664 | CH₃ | (CH₃)₃C | CH₃ | CF₃ |
| 665 | CH₃ | (CH₃)₃C | CH₃ | CH₃S(O) |
| 666 | CH₃ | (CH₃)₃C | CH₃ | CH₃S(O)₂ |
| 667 | CH₃ | c-C₃H₅ | CH₃ | Cl |
| 668 | CH₃ | c-C₃H₅ | CH₃ | Br |
| 669 | CH₃ | c-C₃H₅ | CH₃ | I |
| 670 | CH₃ | c-C₃H₅ | CH₃ | CF₃ |
| 671 | CH₃ | c-C₃H₅ | CH₃ | CH₃S(O) |
| 672 | CH₃ | c-C₃H₅ | CH₃ | CH₃S(O)₂ |
| 673 | CH₃ | CHF₂CH₂ | CH₃ | Cl |
| 674 | CH₃ | CHF₂CH₂ | CH₃ | Br |
| 675 | CH₃ | CHF₂CH₂ | CH₃ | I |
| 676 | CH₃ | CHF₂CH₂ | CH₃ | CF₃ |
| 677 | CH₃ | CHF₂CH₂ | CH₃ | CH₃S(O) |
| 678 | CH₃ | CHF₂CH₂ | CH₃ | CH₃S(O)₂ |
| 679 | CH₃ | CF₃CH₂ | CH₃ | Cl |
| 680 | CH₃ | CF₃CH₂ | CH₃ | Br |
| 681 | CH₃ | CF₃CH₂ | CH₃ | I |
| 682 | CH₃ | CF₃CH₂ | CH₃ | CF₃ |
| 683 | CH₃ | CF₃CH₂ | CH₃ | CH₃S(O) |
| 684 | CH₃ | CF₃CH₂ | CH₃ | CH₃S(O)₂ |
| 685 | CH₃ | CH₃O | CH₃ | Cl |
| 686 | CH₃ | CH₃O | CH₃ | Br |
| 687 | CH₃ | CH₃O | CH₃ | I |
| 688 | CH₃ | CH₃O | CH₃ | CF₃ |
| 689 | CH₃ | CH₃O | CH₃ | CH₃S(O) |
| 690 | CH₃ | CH₃O | CH₃ | CH₃S(O)₂ |
| 691 | CH₃ | C₆H₅ | CH₃ | Cl |
| 692 | CH₃ | C₆H₅ | CH₃ | Br |
| 693 | CH₃ | C₆H₅ | CH₃ | I |
| 694 | CH₃ | C₆H₅ | CH₃ | CF₃ |
| 695 | CH₃ | C₆H₅ | CH₃ | CH₃S(O) |
| 696 | CH₃ | C₆H₅ | CH₃ | CH₃S(O)₂ |
| 697 | CH₃ | 4-Cl-C₆H₄ | CH₃ | Cl |
| 698 | CH₃ | 4-Cl-C₆H₄ | CH₃ | Br |
| 699 | CH₃ | 4-Cl-C₆H₄ | CH₃ | I |
| 700 | CH₃ | 4-Cl-C₆H₄ | CH₃ | CF₃ |
| 701 | CH₃ | 4-Cl-C₆H₄ | CH₃ | CH₃S(O) |
| 702 | CH₃ | 4-Cl-C₆H₄ | CH₃ | CH₃S(O)₂ |
| 703 | CH₃ | 4-CH₃-C₆H₄ | CH₃ | Cl |
| 704 | CH₃ | 4-CH₃-C₆H₄ | CH₃ | Br |
| 705 | CH₃ | 4-CH₃-C₆H₄ | CH₃ | I |
| 706 | CH₃ | 4-CH₃-C₆H₄ | CH₃ | CF₃ |
| 707 | CH₃ | 4-CH₃-C₆H₄ | CH₃ | CH₃S(O) |
| 708 | CH₃ | 4-CH₃-C₆H₄ | CH₃ | CH₃S(O)₂ |
| 709 | CH₃ | 4-CH₃O-C₆H₄ | CH₃ | Cl |
| 710 | CH₃ | 4-CH₃O-C₆H₄ | CH₃ | Br |
| 711 | CH₃ | 4-CH₃O-C₆H₄ | CH₃ | I |
| 712 | CH₃ | 4-CH₃O-C₆H₄ | CH₃ | CF₃ |
| 713 | CH₃ | 4-CH₃O-C₆H₄ | CH₃ | CH₃S(O) |
| 714 | CH₃ | 4-CH₃O-C₆H₄ | CH₃ | CH₃S(O)₂ |
| 715 | CH₃ | 2,6-(CH₃)₂-C₆H₃ | CH₃ | Cl |
| 716 | CH₃ | 2,6-(CH₃)₂-C₆H₃ | CH₃ | Br |
| 717 | CH₃ | 2,6-(CH₃)₂-C₆H₃ | CH₃ | I |
| 718 | CH₃ | 2,6-(CH₃)₂-C₆H₃ | CH₃ | CF₃ |
| 719 | CH₃ | 2,6-(CH₃)₂-C₆H₃ | CH₃ | CH₃S(O) |
| 720 | CH₃ | 2,6-(CH₃)₂-C₆H₃ | CH₃ | CH₃S(O)₂ |
| 721 | CH₃ | C₂H₅ | C₂H₅ | Cl |
| 722 | CH₃ | C₂H₅ | C₂H₅ | Br |
| 723 | CH₃ | C₂H₅ | C₂H₅ | I |
| 724 | CH₃ | C₂H₅ | C₂H₅ | CF₃ |
| 725 | CH₃ | C₂H₅ | C₂H₅ | CH₃S(O) |
| 726 | CH₃ | C₂H₅ | C₂H₅ | CH₃S(O)₂ |
| 727 | CH₃ | CH₃CH₂CH₂ | C₂H₅ | Cl |
| 728 | CH₃ | CH₃CH₂CH₂ | C₂H₅ | Br |
| 729 | CH₃ | CH₃CH₂CH₂ | C₂H₅ | I |
| 730 | CH₃ | CH₃CH₂CH₂ | C₂H₅ | CF₃ |
| 731 | CH₃ | CH₃CH₂CH₂ | C₂H₅ | CH₃S(O) |
| 732 | CH₃ | CH₃CH₂CH₂ | C₂H₅ | CH₃S(O)₂ |
| 733 | CH₃ | (CH₃)₂CH | C₂H₅ | Cl |
| 734 | CH₃ | (CH₃)₂CH | C₂H₅ | Br |
| 735 | CH₃ | (CH₃)₂CH | C₂H₅ | I |
| 736 | CH₃ | (CH₃)₂CH | C₂H₅ | CF₃ |
| 737 | CH₃ | (CH₃)₂CH | C₂H₅ | CH₃S(O) |
| 738 | CH₃ | (CH₃)₂CH | C₂H₅ | CH₃S(O)₂ |
| 739 | CH₃ | (CH₃)₃C | C₂H₅ | Cl |
| 740 | CH₃ | (CH₃)₃C | C₂H₅ | Br |
| 741 | CH₃ | (CH₃)₃C | C₂H₅ | I |
| 742 | CH₃ | (CH₃)₃C | C₂H₅ | CF₃ |
| 743 | CH₃ | (CH₃)₃C | C₂H₅ | CH₃S(O) |
| 744 | CH₃ | (CH₃)₃C | C₂H₅ | CH₃S(O)₂ |
| 745 | CH₃ | c-C₃H₅ | C₂H₅ | Cl |
| 746 | CH₃ | c-C₃H₅ | C₂H₅ | Br |
| 747 | CH₃ | c-C₃H₅ | C₂H₅ | I |
| 748 | CH₃ | c-C₃H₅ | C₂H₅ | CF₃ |
| 749 | CH₃ | c-C₃H₅ | C₂H₅ | CH₃S(O) |
| 750 | CH₃ | c-C₃H₅ | C₂H₅ | CH₃S(O)₂ |
| 751 | CH₃ | CHF₂CH₂ | C₂H₅ | Cl |
| 752 | CH₃ | CHF₂CH₂ | C₂H₅ | Br |
| 753 | CH₃ | CHF₂CH₂ | C₂H₅ | I |
| 754 | CH₃ | CHF₂CH₂ | C₂H₅ | CF₃ |
| 755 | CH₃ | CHF₂CH₂ | C₂H₅ | CH₃S(O) |
| 756 | CH₃ | CHF₂CH₂ | C₂H₅ | CH₃S(O)₂ |
| 757 | CH₃ | CF₃CH₂ | C₂H₅ | Cl |
| 758 | CH₃ | CF₃CH₂ | C₂H₅ | Br |
| 759 | CH₃ | CF₃CH₂ | C₂H₅ | I |
| 760 | CH₃ | CF₃CH₂ | C₂H₅ | CF₃ |
| 761 | CH₃ | CF₃CH₂ | C₂H₅ | CH₃S(O) |
| 762 | CH₃ | CF₃CH₂ | C₂H₅ | CH₃S(O)₂ |
| 763 | CH₃ | CH₃O | C₂H₅ | Cl |
| 764 | CH₃ | CH₃O | C₂H₅ | Br |
| 765 | CH₃ | CH₃O | C₂H₅ | I |
| 766 | CH₃ | CH₃O | C₂H₅ | CF₃ |
| 767 | CH₃ | CH₃O | C₂H₅ | CH₃S(O) |
| 768 | CH₃ | CH₃O | C₂H₅ | CH₃S(O)₂ |
| 769 | CH₃ | C₆H₅ | C₂H₅ | Cl |
| 770 | CH₃ | C₆H₅ | C₂H₅ | Br |
| 771 | CH₃ | C₆H₅ | C₂H₅ | I |
| 772 | CH₃ | C₆H₅ | C₂H₅ | CF₃ |
| 773 | CH₃ | C₆H₅ | C₂H₅ | CH₃S(O) |
| 774 | CH₃ | C₆H₅ | C₂H₅ | CH₃S(O)₂ |
| 775 | CH₃ | 4-Cl-C₆H₄ | C₂H₅ | Cl |
| 776 | CH₃ | 4-Cl-C₆H₄ | C₂H₅ | Br |
| 777 | CH₃ | 4-Cl-C₆H₄ | C₂H₅ | I |
| 778 | CH₃ | 4-Cl-C₆H₄ | C₂H₅ | CF₃ |
| 779 | CH₃ | 4-Cl-C₆H₄ | C₂H₅ | CH₃S(O) |
| 780 | CH₃ | 4-Cl-C₆H₄ | C₂H₅ | CH₃S(O)₂ |
| 781 | CH₃ | 4-CH₃-C₆H₄ | C₂H₅ | Cl |
| 782 | CH₃ | 4-CH₃-C₆H₄ | C₂H₅ | Br |
| 783 | CH₃ | 4-CH₃-C₆H₄ | C₂H₅ | I |
| 784 | CH₃ | 4-CH₃-C₆H₄ | C₂H₅ | CF₃ |
| 785 | CH₃ | 4-CH₃-C₆H₄ | C₂H₅ | CH₃S(O) |
| 786 | CH₃ | 4-CH₃-C₆H₄ | C₂H₅ | CH₃S(O)₂ |
| 787 | CH₃ | 4-CH₃O-C₆H₄ | C₂H₅ | Cl |
| 788 | CH₃ | 4-CH₃O-C₆H₄ | C₂H₅ | Br |
| 789 | CH₃ | 4-CH₃O-C₆H₄ | C₂H₅ | I |
| 790 | CH₃ | 4-CH₃O-C₆H₄ | C₂H₅ | CF₃ |
| 791 | CH₃ | 4-CH₃O-C₆H₄ | C₂H₅ | CH₃S(O) |
| 792 | CH₃ | 4-CH₃O-C₆H₄ | C₂H₅ | CH₃S(O)₂ |
| 793 | CH₃ | 2,6-(CH₃)₂-C₆H₃ | C₂H₅ | Cl |
| 794 | CH₃ | 2,6-(CH₃)₂-C₆H₃ | C₂H₅ | Br |
| 795 | CH₃ | 2,6-(CH₃)₂-C₆H₃ | C₂H₅ | I |
| 796 | CH₃ | 2,6-(CH₃)₂-C₆H₃ | C₂H₅ | CF₃ |
| 797 | CH₃ | 2,6-(CH₃)₂-C₆H₃ | C₂H₅ | CH₃S(O) |
| 798 | CH₃ | 2,6-(CH₃)₂-C₆H₃ | C₂H₅ | CH₃S(O)₂ |
| 799 | CH₃ | CH₃CH₂CH₂ | CH₃CH₂CH₂ | Cl |
| 800 | CH₃ | CH₃CH₂CH₂ | CH₃CH₂CH₂ | Br |
| 801 | CH₃ | CH₃CH₂CH₂ | CH₃CH₂CH₂ | I |
| 802 | CH₃ | CH₃CH₂CH₂ | CH₃CH₂CH₂ | CF₃ |
| 803 | CH₃ | CH₃CH₂CH₂ | CH₃CH₂CH₂ | CH₃S(O) |
| 804 | CH₃ | CH₃CH₂CH₂ | CH₃CH₂CH₂ | CH₃S(O)₂ |
| 805 | CH₃ | (CH₃)₂CH | CH₃CH₂CH₂ | Cl |
| 806 | CH₃ | (CH₃)₂CH | CH₃CH₂CH₂ | Br |
| 807 | CH₃ | (CH₃)₂CH | CH₃CH₂CH₂ | I |
| 808 | CH₃ | (CH₃)₂CH | CH₃CH₂CH₂ | CF₃ |
| 809 | CH₃ | (CH₃)₂CH | CH₃CH₂CH₂ | CH₃S(O) |
| 810 | CH₃ | (CH₃)₂CH | CH₃CH₂CH₂ | CH₃S(O)₂ |
| 811 | CH₃ | (CH₃)₃C | CH₃CH₂CH₂ | Cl |
| 812 | CH₃ | (CH₃)₃C | CH₃CH₂CH₂ | Br |
| 813 | CH₃ | (CH₃)₃C | CH₃CH₂CH₂ | I |
| 814 | CH₃ | (CH₃)₃C | CH₃CH₂CH₂ | CF₃ |
| 815 | CH₃ | (CH₃)₃C | CH₃CH₂CH₂ | CH₃S(O) |
| 816 | CH₃ | (CH₃)₃C | CH₃CH₂CH₂ | CH₃S(O)₂ |
| 817 | CH₃ | c-C₃H₅ | CH₃CH₂CH₂ | Cl |
| 818 | CH₃ | c-C₃H₅ | CH₃CH₂CH₂ | Br |
| 819 | CH₃ | c-C₃H₅ | CH₃CH₂CH₂ | I |
| 820 | CH₃ | c-C₃H₅ | CH₃CH₂CH₂ | CF₃ |
| 821 | CH₃ | c-C₃H₅ | CH₃CH₂CH₂ | CH₃S(O) |
| 822 | CH₃ | c-C₃H₅ | CH₃CH₂CH₂ | CH₃S(O)₂ |
| 823 | CH₃ | CHF₂CH₂ | CH₃CH₂CH₂ | Cl |
| 824 | CH₃ | CHF₂CH₂ | CH₃CH₂CH₂ | Br |
| 825 | CH₃ | CHF₂CH₂ | CH₃CH₂CH₂ | I |
| 826 | CH₃ | CHF₂CH₂ | CH₃CH₂CH₂ | CF₃ |
| 827 | CH₃ | CHF₂CH₂ | CH₃CH₂CH₂ | CH₃S(O) |
| 828 | CH₃ | CHF₂CH₂ | CH₃CH₂CH₂ | CH₃S(O)₂ |
| 829 | CH₃ | CF₃CH₂ | CH₃CH₂CH₂ | Cl |
| 830 | CH₃ | CF₃CH₂ | CH₃CH₂CH₂ | Br |
| 831 | CH₃ | CF₃CH₂ | CH₃CH₂CH₂ | I |
| 832 | CH₃ | CF₃CH₂ | CH₃CH₂CH₂ | CF₃ |
| 833 | CH₃ | CF₃CH₂ | CH₃CH₂CH₂ | CH₃S(O) |
| 834 | CH₃ | CF₃CH₂ | CH₃CH₂CH₂ | CH₃S(O)₂ |
| 835 | CH₃ | CH₃O | CH₃CH₂CH₂ | Cl |
| 836 | CH₃ | CH₃O | CH₃CH₂CH₂ | Br |
| 837 | CH₃ | CH₃O | CH₃CH₂CH₂ | I |
| 838 | CH₃ | CH₃O | CH₃CH₂CH₂ | CF₃ |
| 839 | CH₃ | CH₃O | CH₃CH₂CH₂ | CH₃S(O) |
| 840 | CH₃ | CH₃O | CH₃CH₂CH₂ | CH₃S(O)₂ |
| 841 | CH₃ | C₆H₅ | CH₃CH₂CH₂ | Cl |
| 842 | CH₃ | C₆H₅ | CH₃CH₂CH₂ | Br |
| 843 | CH₃ | C₆H₅ | CH₃CH₂CH₂ | I |
| 844 | CH₃ | C₆H₅ | CH₃CH₂CH₂ | CF₃ |
| 845 | CH₃ | C₆H₅ | CH₃CH₂CH₂ | CH₃S(O) |
| 846 | CH₃ | C₆H₅ | CH₃CH₂CH₂ | CH₃S(O)₂ |
| 847 | CH₃ | 4-Cl-C₆H₄ | CH₃CH₂CH₂ | Cl |
| 848 | CH₃ | 4-Cl-C₆H₄ | CH₃CH₂CH₂ | Br |
| 849 | CH₃ | 4-Cl-C₆H₄ | CH₃CH₂CH₂ | I |
| 850 | CH₃ | 4-Cl-C₆H₄ | CH₃CH₂CH₂ | CF₃ |
| 851 | CH₃ | 4-Cl-C₆H₄ | CH₃CH₂CH₂ | CH₃S(O) |
| 852 | CH₃ | 4-Cl-C₆H₄ | CH₃CH₂CH₂ | CH₃S(O)₂ |
| 853 | CH₃ | 4-CH₃-C₆H₄ | CH₃CH₂CH₂ | Cl |
| 854 | CH₃ | 4-CH₃-C₆H₄ | CH₃CH₂CH₂ | Br |
| 855 | CH₃ | 4-CH₃-C₆H₄ | CH₃CH₂CH₂ | I |
| 856 | CH₃ | 4-CH₃-C₆H₄ | CH₃CH₂CH₂ | CF₃ |
| 857 | CH₃ | 4-CH₃-C₆H₄ | CH₃CH₂CH₂ | CH₃S(O) |
| 858 | CH₃ | 4-CH₃-C₆H₄ | CH₃CH₂CH₂ | CH₃S(O)₂ |
| 859 | CH₃ | 4-CH₃O-C₆H₄ | CH₃CH₂CH₂ | Cl |
| 860 | CH₃ | 4-CH₃O-C₆H₄ | CH₃CH₂CH₂ | Br |
| 861 | CH₃ | 4-CH₃O-C₆H₄ | CH₃CH₂CH₂ | I |
| 862 | CH₃ | 4-CH₃O-C₆H₄ | CH₃CH₂CH₂ | CF₃ |
| 863 | CH₃ | 4-CH₃O-C₆H₄ | CH₃CH₂CH₂ | CH₃S(O) |
| 864 | CH₃ | 4-CH₃O-C₆H₄ | CH₃CH₂CH₂ | CH₃S(O)₂ |
| 865 | CH₃ | 2,6-(CH₃)₂-C₆H₃ | CH₃CH₂CH₂ | Cl |
| 866 | CH₃ | 2,6-(CH₃)₂-C₆H₃ | CH₃CH₂CH₂ | Br |
| 867 | CH₃ | 2,6-(CH₃)₂-C₆H₃ | CH₃CH₂CH₂ | I |
| 868 | CH₃ | 2,6-(CH₃)₂-C₆H₃ | CH₃CH₂CH₂ | CF₃ |
| 869 | CH₃ | 2,6-(CH₃)₂-C₆H₃ | CH₃CH₂CH₂ | CH₃S(O) |
| 870 | CH₃ | 2,6-(CH₃)₂-C₆H₃ | CH₃CH₂CH₂ | CH₃S(O)₂ |
| 871 | CH₃ | (CH₃)₂CH | (CH₃)₂CH | Cl |
| 872 | CH₃ | (CH₃)₂CH | (CH₃)₂CH | Br |
| 873 | CH₃ | (CH₃)₂CH | (CH₃)₂CH | I |
| 874 | CH₃ | (CH₃)₂CH | (CH₃)₂CH | CF₃ |
| 875 | CH₃ | (CH₃)₂CH | (CH₃)₂CH | CH₃S(O) |
| 876 | CH₃ | (CH₃)₂CH | (CH₃)₂CH | CH₃S(O)₂ |
| 877 | CH₃ | c-C₃H₅ | (CH₃)₂CH | Cl |
| 878 | CH₃ | c-C₃H₅ | (CH₃)₂CH | Br |
| 879 | CH₃ | c-C₃H₅ | (CH₃)₂CH | I |
| 880 | CH₃ | c-C₃H₅ | (CH₃)₂CH | CF₃ |
| 881 | CH₃ | c-C₃H₅ | (CH₃)₂CH | CH₃S(O) |
| 882 | CH₃ | c-C₃H₅ | (CH₃)₂CH | CH₃S(O)₂ |
| 883 | CH₃ | CHF₂CH₂ | (CH₃)₂CH | Cl |
| 884 | CH₃ | CHF₂CH₂ | (CH₃)₂CH | Br |
| 885 | CH₃ | CHF₂CH₂ | (CH₃)₂CH | I |
| 886 | CH₃ | CHF₂CH₂ | (CH₃)₂CH | CF₃ |
| 887 | CH₃ | CHF₂CH₂ | (CH₃)₂CH | CH₃S(O) |
| 888 | CH₃ | CHF₂CH₂ | (CH₃)₂CH | CH₃S(O)₂ |
| 889 | CH₃ | CF₃CH₂ | (CH₃)₂CH | Cl |
| 890 | CH₃ | CF₃CH₂ | (CH₃)₂CH | Br |
| 891 | CH₃ | CF₃CH₂ | (CH₃)₂CH | I |
| 892 | CH₃ | CF₃CH₂ | (CH₃)₂CH | CF₃ |
| 893 | CH₃ | CF₃CH₂ | (CH₃)₂CH | CH₃S(O) |
| 894 | CH₃ | CF₃CH₂ | (CH₃)₂CH | CH₃S(O)₂ |
| 895 | CH₃ | CH₃O | (CH₃)₂CH | Cl |
| 896 | CH₃ | CH₃O | (CH₃)₂CH | Br |
| 897 | CH₃ | CH₃O | (CH₃)₂CH | I |
| 898 | CH₃ | CH₃O | (CH₃)₂CH | CF₃ |
| 899 | CH₃ | CH₃O | (CH₃)₂CH | CH₃S(O) |
| 900 | CH₃ | CH₃O | (CH₃)₂CH | CH₃S(O)₂ |
| 901 | CH₃ | C₆H₅ | (CH₃)₂CH | Cl |
| 902 | CH₃ | C₆H₅ | (CH₃)₂CH | Br |
| 903 | CH₃ | C₆H₅ | (CH₃)₂CH | I |
| 904 | CH₃ | C₆H₅ | (CH₃)₂CH | CF₃ |
| 905 | CH₃ | C₆H₅ | (CH₃)₂CH | CH₃S(O) |
| 906 | CH₃ | C₆H₅ | (CH₃)₂CH | CH₃S(O)₂ |
| 907 | CH₃ | 4-Cl-C₆H₄ | (CH₃)₂CH | Cl |
| 908 | CH₃ | 4-Cl-C₆H₄ | (CH₃)₂CH | Br |
| 909 | CH₃ | 4-Cl-C₆H₄ | (CH₃)₂CH | I |
| 910 | CH₃ | 4-Cl-C₆H₄ | (CH₃)₂CH | CF₃ |
| 911 | CH₃ | 4-Cl-C₆H₄ | (CH₃)₂CH | CH₃S(O) |
| 912 | CH₃ | 4-Cl-C₆H₄ | (CH₃)₂CH | CH₃S(O)₂ |
| 913 | CH₃ | 4-CH₃-C₆H₄ | (CH₃)₂CH | Cl |
| 914 | CH₃ | 4-CH₃-C₆H₄ | (CH₃)₂CH | Br |
| 915 | CH₃ | 4-CH₃-C₆H₄ | (CH₃)₂CH | I |
| 916 | CH₃ | 4-CH₃-C₆H₄ | (CH₃)₂CH | CF₃ |
| 917 | CH₃ | 4-CH₃-C₆H₄ | (CH₃)₂CH | CH₃S(O) |
| 918 | CH₃ | 4-CH₃-C₆H₄ | (CH₃)₂CH | CH₃S(O)₂ |
| 919 | CH₃ | 4-CH₃O-C₆H₄ | (CH₃)₂CH | Cl |
| 920 | CH₃ | 4-CH₃O-C₆H₄ | (CH₃)₂CH | Br |
| 921 | CH₃ | 4-CH₃O-C₆H₄ | (CH₃)₂CH | I |
| 922 | CH₃ | 4-CH₃O-C₆H₄ | (CH₃)₂CH | CF₃ |
| 923 | CH₃ | 4-CH₃O-C₆H₄ | (CH₃)₂CH | CH₃S(O) |
| 924 | CH₃ | 4-CH₃O-C₆H₄ | (CH₃)₂CH | CH₃S(O)₂ |
| 925 | CH₃ | 2,6-(CH₃)₂-C₆H₃ | (CH₃)₂CH | Cl |
| 926 | CH₃ | 2,6-(CH₃)₂-C₆H₃ | (CH₃)₂CH | Br |
| 927 | CH₃ | 2,6-(CH₃)₂-C₆H₃ | (CH₃)₂CH | I |
| 928 | CH₃ | 2,6-(CH₃)₂-C₆H₃ | (CH₃)₂CH | CF₃ |
| 929 | CH₃ | 2,6-(CH₃)₂-C₆H₃ | (CH₃)₂CH | CH₃S(O) |
| 930 | CH₃ | 2,6-(CH₃)₂-C₆H₃ | (CH₃)₂CH | CH₃S(O)₂ |
| 931 | CH₃ | c-C₃H₅ | c-C₃H₅ | Cl |
| 932 | CH₃ | c-C₃H₅ | c-C₃H₅ | Br |
| 933 | CH₃ | c-C₃H₅ | c-C₃H₅ | I |
| 934 | CH₃ | c-C₃H₅ | c-C₃H₅ | CF₃ |
| 935 | CH₃ | c-C₃H₅ | c-C₃H₅ | CH₃S(O) |
| 936 | CH₃ | c-C₃H₅ | c-C₃H₅ | CH₃S(O)₂ |
| 937 | CH₃ | CHF₂CH₂ | c-C₃H₅ | Cl |
| 938 | CH₃ | CHF₂CH₂ | c-C₃H₅ | Br |
| 939 | CH₃ | CHF₂CH₂ | c-C₃H₅ | I |
| 940 | CH₃ | CHF₂CH₂ | c-C₃H₅ | CF₃ |
| 941 | CH₃ | CHF₂CH₂ | c-C₃H₅ | CH₃S(O) |
| 942 | CH₃ | CHF₂CH₂ | c-C₃H₅ | CH₃S(O)₂ |
| 943 | CH₃ | CF₃CH₂ | c-C₃H₅ | Cl |
| 944 | CH₃ | CF₃CH₂ | c-C₃H₅ | Br |
| 945 | CH₃ | CF₃CH₂ | c-C₃H₅ | I |
| 946 | CH₃ | CF₃CH₂ | c-C₃H₅ | CF₃ |
| 947 | CH₃ | CF₃CH₂ | c-C₃H₅ | CH₃S(O) |
| 948 | CH₃ | CF₃CH₂ | c-C₃H₅ | CH₃S(O)₂ |
| 949 | CH₃ | CH₃O | c-C₃H₅ | Cl |
| 950 | CH₃ | CH₃O | c-C₃H₅ | Br |
| 951 | CH₃ | CH₃O | c-C₃H₅ | I |
| 952 | CH₃ | CH₃O | c-C₃H₅ | CF₃ |
| 953 | CH₃ | CH₃O | c-C₃H₅ | CH₃S(O) |
| 954 | CH₃ | CH₃O | c-C₃H₅ | CH₃S(O)₂ |
| 955 | CH₃ | C₆H₅ | c-C₃H₅ | Cl |
| 956 | CH₃ | C₆H₅ | c-C₃H₅ | Br |
| 957 | CH₃ | C₆H₅ | c-C₃H₅ | I |
| 958 | CH₃ | C₆H₅ | c-C₃H₅ | CF₃ |
| 959 | CH₃ | C₆H₅ | c-C₃H₅ | CH₃S(O) |
| 960 | CH₃ | C₆H₅ | c-C₃H₅ | CH₃S(O)₂ |
| 961 | CH₃ | 4-Cl-C₆H₄ | c-C₃H₅ | Cl |
| 962 | CH₃ | 4-Cl-C₆H₄ | c-C₃H₅ | Br |
| 963 | CH₃ | 4-Cl-C₆H₄ | c-C₃H₅ | I |
| 964 | CH₃ | 4-Cl-C₆H₄ | c-C₃H₅ | CF₃ |
| 965 | CH₃ | 4-Cl-C₆H₄ | c-C₃H₅ | CH₃S(O) |
| 966 | CH₃ | 4-Cl-C₆H₄ | c-C₃H₅ | CH₃S(O)₂ |
| 967 | CH₃ | 4-CH₃-C₆H₄ | c-C₃H₅ | Cl |
| 968 | CH₃ | 4-CH₃-C₆H₄ | c-C₃H₅ | Br |
| 969 | CH₃ | 4-CH₃-C₆H₄ | c-C₃H₅ | I |
| 970 | CH₃ | 4-CH₃-C₆H₄ | c-C₃H₅ | CF₃ |
| 971 | CH₃ | 4-CH₃-C₆H₄ | c-C₃H₅ | CH₃S(O) |
| 972 | CH₃ | 4-CH₃-C₆H₄ | c-C₃H₅ | CH₃S(O)₂ |
| 973 | CH₃ | 4-CH₃O-C₆H₄ | c-C₃H₅ | Cl |
| 974 | CH₃ | 4-CH₃O-C₆H₄ | c-C₃H₅ | Br |
| 975 | CH₃ | 4-CH₃O-C₆H₄ | c-C₃H₅ | I |
| 976 | CH₃ | 4-CH₃O-C₆H₄ | c-C₃H₅ | CF₃ |
| 977 | CH₃ | 4-CH₃O-C₆H₄ | c-C₃H₅ | CH₃S(O) |
| 978 | CH₃ | 4-CH₃O-C₆H₄ | c-C₃H₅ | CH₃S(O)₂ |
| 979 | CH₃ | 2,6-(CH₃)₂-C₆H₃ | c-C₃H₅ | Cl |
| 980 | CH₃ | 2,6-(CH₃)₂-C₆H₃ | c-C₃H₅ | Br |
| 981 | CH₃ | 2,6-(CH₃)₂-C₆H₃ | c-C₃H₅ | I |
| 982 | CH₃ | 2,6-(CH₃)₂-C₆H₃ | c-C₃H₅ | CF₃ |
| 983 | CH₃ | 2,6-(CH₃)₂-C₆H₃ | c-C₃H₅ | CH₃S(O) |
| 984 | CH₃ | 2,6-(CH₃)₂-C₆H₃ | c-C₃H₅ | CH₃S(O)₂ |
| 985 | CH₃ | (CH₃)₃C | CHF₂CH₂ | Cl |
| 986 | CH₃ | (CH₃)₃C | CHF₂CH₂ | Br |
| 987 | CH₃ | (CH₃)₃C | CHF₂CH₂ | I |
| 988 | CH₃ | (CH₃)₃C | CHF₂CH₂ | CF₃ |
| 989 | CH₃ | (CH₃)₃C | CHF₂CH₂ | CH₃S(O) |
| 990 | CH₃ | (CH₃)₃C | CHF₂CH₂ | CH₃S(O)₂ |
| 991 | CH₃ | CHF₂CH₂ | CHF₂CH₂ | Cl |
| 992 | CH₃ | CHF₂CH₂ | CHF₂CH₂ | Br |
| 993 | CH₃ | CHF₂CH₂ | CHF₂CH₂ | I |
| 994 | CH₃ | CHF₂CH₂ | CHF₂CH₂ | CF₃ |
| 995 | CH₃ | CHF₂CH₂ | CHF₂CH₂ | CH₃S(O) |
| 996 | CH₃ | CHF₂CH₂ | CHF₂CH₂ | CH₃S(O)₂ |
| 997 | CH₃ | CF₃CH₂ | CHF₂CH₂ | Cl |
| 998 | CH₃ | CF₃CH₂ | CHF₂CH₂ | Br |
| 999 | CH₃ | CF₃CH₂ | CHF₂CH₂ | I |
| 1000 | CH₃ | CF₃CH₂ | CHF₂CH₂ | CF₃ |
| 1001 | CH₃ | CF₃CH₂ | CHF₂CH₂ | CH₃S(O) |
| 1002 | CH₃ | CF₃CH₂ | CHF₂CH₂ | CH₃S(O)₂ |
| 1003 | CH₃ | CH₃O | CHF₂CH₂ | Cl |
| 1004 | CH₃ | CH₃O | CHF₂CH₂ | Br |
| 1005 | CH₃ | CH₃O | CHF₂CH₂ | I |
| 1006 | CH₃ | CH₃O | CHF₂CH₂ | CF₃ |
| 1007 | CH₃ | CH₃O | CHF₂CH₂ | CH₃S(O) |
| 1008 | CH₃ | CH₃O | CHF₂CH₂ | CH₃S(O)₂ |
| 1009 | CH₃ | C₆H₅ | CHF₂CH₂ | Cl |
| 1010 | CH₃ | C₆H₅ | CHF₂CH₂ | Br |
| 1011 | CH₃ | C₆H₅ | CHF₂CH₂ | I |
| 1012 | CH₃ | C₆H₅ | CHF₂CH₂ | CF₃ |
| 1013 | CH₃ | C₆H₅ | CHF₂CH₂ | CH₃S(O) |
| 1014 | CH₃ | C₆H₅ | CHF₂CH₂ | CH₃S(O)₂ |
| 1015 | CH₃ | 4-Cl-C₆H₄ | CHF₂CH₂ | Cl |
| 1016 | CH₃ | 4-Cl-C₆H₄ | CHF₂CH₂ | Br |
| 1017 | CH₃ | 4-Cl-C₆H₄ | CHF₂CH₂ | I |
| 1018 | CH₃ | 4-Cl-C₆H₄ | CHF₂CH₂ | CF₃ |
| 1019 | CH₃ | 4-Cl-C₆H₄ | CHF₂CH₂ | CH₃S(O) |
| 1020 | CH₃ | 4-Cl-C₆H₄ | CHF₂CH₂ | CH₃S(O)₂ |
| 1021 | CH₃ | 4-CH₃-C₆H₄ | CHF₂CH₂ | Cl |
| 1022 | CH₃ | 4-CH₃-C₆H₄ | CHF₂CH₂ | Br |
| 1023 | CH₃ | 4-CH₃-C₆H₄ | CHF₂CH₂ | I |
| 1024 | CH₃ | 4-CH₃-C₆H₄ | CHF₂CH₂ | CF₃ |
| 1025 | CH₃ | 4-CH₃-C₆H₄ | CHF₂CH₂ | CH₃S(O) |
| 1026 | CH₃ | 4-CH₃-C₆H₄ | CHF₂CH₂ | CH₃S(O)₂ |
| 1027 | CH₃ | 4-CH₃O-C₆H₄ | CHF₂CH₂ | Cl |
| 1028 | CH₃ | 4-CH₃O-C₆H₄ | CHF₂CH₂ | Br |
| 1029 | CH₃ | 4-CH₃O-C₆H₄ | CHF₂CH₂ | I |
| 1030 | CH₃ | 4-CH₃O-C₆H₄ | CHF₂CH₂ | CF₃ |
| 1031 | CH₃ | 4-CH₃O-C₆H₄ | CHF₂CH₂ | CH₃S(O) |
| 1032 | CH₃ | 4-CH₃O-C₆H₄ | CHF₂CH₂ | CH₃S(O)₂ |
| 1033 | CH₃ | 2,6-(CH₃)₂-C₆H₃ | CHF₂CH₂ | Cl |
| 1034 | CH₃ | 2,6-(CH₃)₂-C₆H₃ | CHF₂CH₂ | Br |
| 1035 | CH₃ | 2,6-(CH₃)₂-C₆H₃ | CHF₂CH₂ | I |
| 1036 | CH₃ | 2,6-(CH₃)₂-C₆H₃ | CHF₂CH₂ | CF₃ |
| 1037 | CH₃ | 2,6-(CH₃)₂-C₆H₃ | CHF₂CH₂ | CH₃S(O) |
| 1038 | CH₃ | 2,6-(CH₃)₂-C₆H₃ | CHF₂CH₂ | CH₃S(O)₂ |
| 1039 | CH₃ | (CH₃)₃C | CF₃CH₂ | Cl |
| 1040 | CH₃ | (CH₃)₃C | CF₃CH₂ | Br |
| 1041 | CH₃ | (CH₃)₃C | CF₃CH₂ | I |
| 1042 | CH₃ | (CH₃)₃C | CF₃CH₂ | CF₃ |
| 1043 | CH₃ | (CH₃)₃C | CF₃CH₂ | CH₃S(O) |
| 1044 | CH₃ | (CH₃)₃C | CF₃CH₂ | CH₃S(O)₂ |
| 1045 | CH₃ | CF₃CH₂ | CF₃CH₂ | Cl |
| 1046 | CH₃ | CF₃CH₂ | CF₃CH₂ | Br |
| 1047 | CH₃ | CF₃CH₂ | CF₃CH₂ | I |
| 1048 | CH₃ | CF₃CH₂ | CF₃CH₂ | CF₃ |
| 1049 | CH₃ | CF₃CH₂ | CF₃CH₂ | CH₃S(O) |
| 1050 | CH₃ | CF₃CH₂ | CF₃CH₂ | CH₃S(O)₂ |
| 1051 | CH₃ | CH₃O | CF₃CH₂ | Cl |
| 1052 | CH₃ | CH₃O | CF₃CH₂ | Br |
| 1053 | CH₃ | CH₃O | CF₃CH₂ | I |
| 1054 | CH₃ | CH₃O | CF₃CH₂ | CF₃ |
| 1055 | CH₃ | CH₃O | CF₃CH₂ | CH₃S(O) |
| 1056 | CH₃ | CH₃O | CF₃CH₂ | CH₃S(O)₂ |
| 1057 | CH₃ | C₆H₅ | CF₃CH₂ | Cl |
| 1058 | CH₃ | C₆H₅ | CF₃CH₂ | Br |
| 1059 | CH₃ | C₆H₅ | CF₃CH₂ | I |
| 1060 | CH₃ | C₆H₅ | CF₃CH₂ | CF₃ |
| 1061 | CH₃ | C₆H₅ | CF₃CH₂ | CH₃S(O) |
| 1062 | CH₃ | C₆H₅ | CF₃CH₂ | CH₃S(O)₂ |
| 1063 | CH₃ | 4-Cl-C₆H₄ | CF₃CH₂ | Cl |
| 1064 | CH₃ | 4-Cl-C₆H₄ | CF₃CH₂ | Br |
| 1065 | CH₃ | 4-Cl-C₆H₄ | CF₃CH₂ | I |
| 1066 | CH₃ | 4-Cl-C₆H₄ | CF₃CH₂ | CF₃ |
| 1067 | CH₃ | 4-Cl-C₆H₄ | CF₃CH₂ | CH₃S(O) |
| 1068 | CH₃ | 4-Cl-C₆H₄ | CF₃CH₂ | CH₃S(O)₂ |
| 1069 | CH₃ | 4-CH₃-C₆H₄ | CF₃CH₂ | Cl |
| 1070 | CH₃ | 4-CH₃-C₆H₄ | CF₃CH₂ | Br |
| 1071 | CH₃ | 4-CH₃-C₆H₄ | CF₃CH₂ | I |
| 1072 | CH₃ | 4-CH₃-C₆H₄ | CF₃CH₂ | CF₃ |
| 1073 | CH₃ | 4-CH₃-C₆H₄ | CF₃CH₂ | CH₃S(O) |
| 1074 | CH₃ | 4-CH₃-C₆H₄ | CF₃CH₂ | CH₃S(O)₂ |
| 1075 | CH₃ | 4-CH₃O-C₆H₄ | CF₃CH₂ | Cl |
| 1076 | CH₃ | 4-CH₃O-C₆H₄ | CF₃CH₂ | Br |
| 1077 | CH₃ | 4-CH₃O-C₆H₄ | CF₃CH₂ | I |
| 1078 | CH₃ | 4-CH₃O-C₆H₄ | CF₃CH₂ | CF₃ |
| 1079 | CH₃ | 4-CH₃O-C₆H₄ | CF₃CH₂ | CH₃S(O) |
| 1080 | CH₃ | 4-CH₃O-C₆H₄ | CF₃CH₂ | CH₃S(O)₂ |
| 1081 | CH₃ | 2,6-(CH₃)₂-C₆H₃ | CF₃CH₂ | Cl |
| 1082 | CH₃ | 2,6-(CH₃)₂-C₆H₃ | CF₃CH₂ | Br |
| 1083 | CH₃ | 2,6-(CH₃)₂-C₆H₃ | CF₃CH₂ | I |
| 1084 | CH₃ | 2,6-(CH₃)₂-C₆H₃ | CF₃CH₂ | CF₃ |
| 1085 | CH₃ | 2,6-(CH₃)₂-C₆H₃ | CF₃CH₂ | CH₃S(O) |
| 1086 | CH₃ | 2,6-(CH₃)₂-C₆H₃ | CF₃CH₂ | CH₃S(O)₂ |
| 1087 | CH₃ | NR^{2a}R^{2b} = | 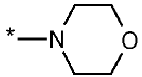 | Cl |
| 1088 | CH₃ | NR^{2a}R^{2b} = | 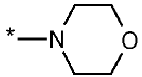 | Br |
| 1089 | CH₃ | NR^{2a}R^{2b} = | 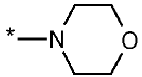 | I |
| 1090 | CH₃ | NR^{2a}R^{2b} = | 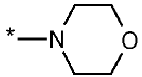 | CF₃ |
| 1091 | CH₃ | NR^{2a}R^{2b} = | 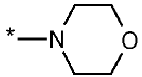 | CH₃S(O) |
| 1092 | CH₃ | NR^{2a}R^{2b} = | 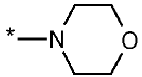 | CH₃S(O)₂ |
| 1093 | CH₃ | NR^{2a}R^{2b} = | 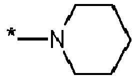 | Cl |
| 1094 | CH₃ | NR^{2a}R^{2b} = | 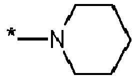 | Br |
| 1095 | CH₃ | NR^{2a}R^{2b} = | 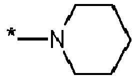 | I |
| 1096 | CH₃ | NR^{2a}R^{2b} = | 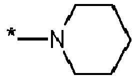 | CF₃ |
| 1097 | CH₃ | NR^{2a}R^{2b} = | 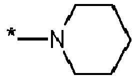 | CH₃S(O) |
| 1098 | CH₃ | NR^{2a}R^{2b} = | 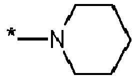 | CH₃S(O)₂ |
| 1099 | CH₃ | NR^{2a}R^{2b} = | 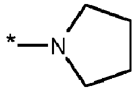 | Cl |
| 1100 | CH₃ | NR^{2a}R^{2b} = | 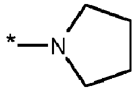 | Br |
| 1101 | CH₃ | NR^{2a}R^{2b} = | 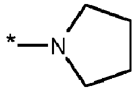 | I |
| 1102 | CH₃ | NR^{2a}R^{2b} = | 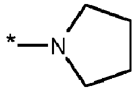 | CF₃ |
| 1103 | CH₃ | NR^{2a}R^{2b} = | 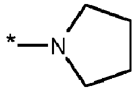 | CH₃S(O) |
| 1104 | CH₃ | NR^{2a}R^{2b} = | 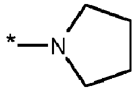 | CH₃S(O)₂ |

In table A, the following abbreviations are used:
c-C₃H₅ cyclopropyl;
C₆H₅ phenyl
4-Cl-C₆H₄ 4-chlorophenyl
4-CH₃-C₆H₄ 4-methylphenyl
4-CH₃O-C₆H₄ 4-methoxyphenyl
2,6-(CH₃)₂-C₆H₃ 2,6-dimethylphenyl

The compounds of formula I can be prepared by standard methods of organic chemistry, e.g. by the methods described in the schemes below. The substituents, variables and indices used in the schemes are as defined above for the compounds of formula I, if not specified otherwise.

The compounds of the formula I, where R^{2c} is H can be prepared from the corresponding benzamides of the formula II, which comprises reacting the compound of formula II with thiophosgene (III) to obtain an isothiocyanate of the formula IV. Subsequent reaction of (IV) with ammonia or an amine of the formula V yields the compound of formual (I), where R^{2c} is H. The method is depicted in the following scheme 1.

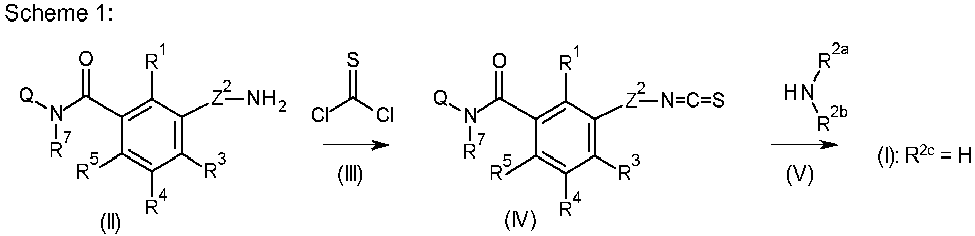

The reaction of the compound of the formula II with thiophosgene III or thiophosgene equivalent and the secondary amine of formula V can be performed by analogy to the preparation of mixed thioureas by reaction of two different amine with thiiophosgene or thiophosgene equivalent. Usually, the compound of the formula II is firstly reacted with thiophosgene to obtain the isothiocyanate (IV) as an intermediate compound, which is subsequently reacted with the secondary amine of the formula IV. The isothiocyanate of formula IV may be isolated from the reaction mixture. For economical reasons, the isothiocyanate (IV) is usually not isolated but the reaction mixture obtained from the reaction of the compound (II) with the thiophosgene or thiophosgene equivalent is subjected to the reaction with ammonia or the amine of formula V.

Further details can be taken from the preparation examples contained herein. Apart from that, a skilled person will easily find suitable reaction conditions for the synthesis depicted in scheme 1 by routine.

In a similar manner a compound of formula (II') can be reacted with an isothiocyanate compound (IV'), which has been prepared from primary amine (V), where R^{2b} is H and R^{2a} is different from H, to obtain a compound of the formula I, where R^{2b} is H and R^{2a} is different from H. The reaction is depicted in scheme 2:

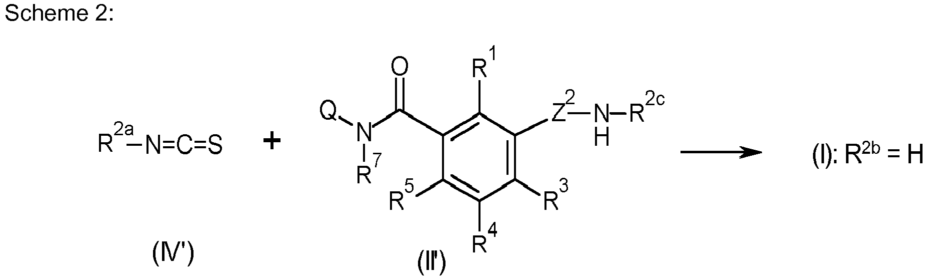

The compounds of the formulae (II) and (II') are known, e.g. from WO 2017/102275 or can be easily prepared by analogy to the methods described in WO 2017/102275 or from the corresponding 3-nitrobenzamide compounds or 3-cyanobenzamide compounds by reduction of the 3-nitro group or 3-cyanao group, respectively, according to standard procedures.

The compounds of formula I, where R⁷ is in particular H, can also be prepared by the reaction depicted in scheme 3 below.

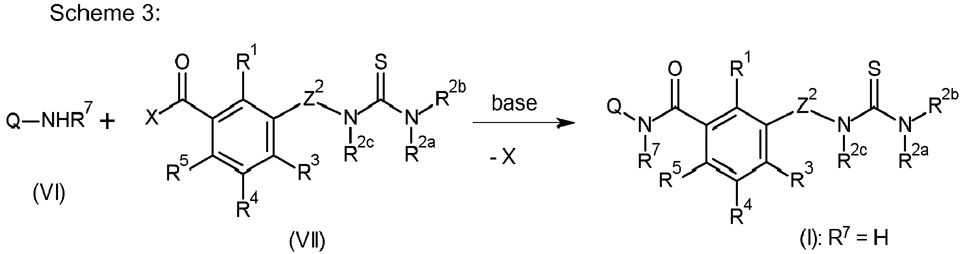

Compounds of formula VI can be reacted with benzoyl compounds of formula VII to afford compounds of formula I, wherein R⁷ is hydrogen. Herein, X is a leaving group, such as halogen, in particular Cl, an anhydride residue or an active ester residue. Especially in case of X being halogen the reaction is suitably carried out in the presence of a base. Suitable bases are for example carbonates, such as lithium, sodium, potassium or cesium carbonates, amines, such as trimethylamine or triethylamine, and basic N-heterocycles, such as pyridine, 2,6-dimethylpyridine or 2,4,6-trimethylpyridine. Suitable solvents are in particular aprotic solvents such as pentane, hexane, heptane, octane, cyclohexane, dichloromethane, chloroform, 1,2-dichlorethane, benzene, chlorobenzene, toluene, the xylenes, dichlorobenzene, trimethylbenzene, pyridine, 2,6-dimethylpyridine, 2,4,6-trimethylpyridine, acetonitrile, diethyl ether, tetrahydrofuran, 2-methyl tetrahydrofuran, methyl tert-butylether, 1,4-dioxane, N,N-dimethyl formamide, N-methyl pyrrolidinone or mixtures thereof. The starting materials are generally reacted with one another in equimolar or nearly equimolar amounts at a reaction temperature usually in the range of -20°C to 100°C and preferably in the range of -5°C to 50°C.

Alternatively, compounds of formula I can also be prepared as shown in scheme 4 below. Reaction of the amino compound of formula VI with a benzoic acid compound of formula VIII in the presence of an activating compound yields the compound of formula I, wherein R⁷ is in particular H. The reaction of compound (VI) with compound (VIII) is preferably carried out in the presence of a suitable activating agent, which converts the carboxyl group of compound (VIII) into an activated ester or amide. For this purpose activating agents known in the art, such as 1,1',carbonyldiimidazole (CDI), dicyclohexyl carbodiimide (DCC), 1-ethyl-3-(3-dimethylaminopropyl)carbodiimide (EDC) or 2,4,6-tripropyl-1,3,5,2,4,6-trioxatriphosphorinane-2,4,6-trioxide (T3P) can be used. The activated ester or amide can be formed, depending in particular on the specific activating agent used, either in situ by contacting benzoic acid compound of formula VIII with the activating agent in the presence of the amine compound of the formula VI, or in a separate step prior to the reaction with amine compound of formula VIII. It may be advantageous, especially in cases where DCC or EDC are used as activating agent, to include further additives in the activating reaction, such as hydroxybenzotriazole (HOBt), nitrophenol, pentafluorophenol, 2,4,5-trichlorophenol or N-hydroxysuccinimide. It may further be advantageous to prepare the activated ester or amide in the presence of a base, for example a tertiary amine. The activated ester or amide is either in situ or subsequently reacted with the amine of formula III or IV to afford the amide of formula I. The reaction normally takes place in anhydrous inert solvents, such as chlorinated hydrocarbons, e.g. dichloromethane or dichloroethane, ethers, e.g. tetrahydrofuran or 1,4-dioxane or carboxamides, e.g. N,N-dimethylformamide, N,N-dimethylacetamide or N-methylpyrrolidone. The reaction is frequently carried out at temperatures in the range from -20°C to +50°C, depending on the reactivity of the compounds (VI) and (VIII), respectively, and the activating agent used.

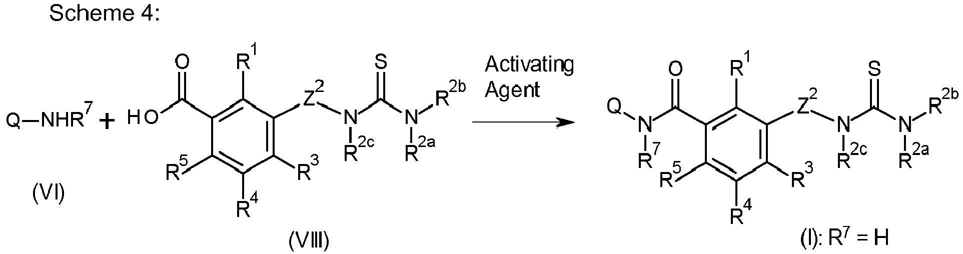

The compounds of formula VI are either commercially available or can be obtained according to standard methods of organic chemistry. For example, the compound of formula VI, where Q is Q¹ and R^{6a} is alkyl or a similar radical, can be prepared from 5-aminotetrazole according to the method described in the Journal of the American Chemical Society, 1954, 76, 923-924. Alternatively, compounds of formula VI, where Q is Q¹, can be prepared according to the method described in the Journal of the American Chemical Society, 1954, 76, 88-89 starting from 3-(R^{6a})-substituted 1-aminoguandidines.

The compounds of formula VI, where Q is Q² and R^{6b} is alkyl or a similar radical, can be prepared from 3-amino-1,2,4-triazole according to the method described in Zeitschrift für Chemie, 1990, 30, 12, 436-437 by reacting 1-(R^{6b})-substituted 1-aminoguandidines and formic acid.

The compounds of formula VI, where Q is Q³ are either commercially available or can be prepared by analogy to standard methods for preparing 4-amino-1,2,5-oxadiazole compounds known from the literature. For example, 3-(R^{6c})-subsituted-4-amino-1,2,5-oxadiazoles can be prepared from β-ketoesters pursuant to a procedure described in Russian Chemical Bulletin, Int. Ed., 54(4), 1032-1037 (2005). Compounds of formula VI, where Q is Q³ and where R^{6c} is halogen, can be prepared from commercially available 3,4-diamino-1,2,5-oxadiazole according to procedures described in the literature, e.g. by the Sandmeyer-type reaction described in Heteroatom Chemistry, 15(3), 199-207 (2004). Compounds of formula VI, where Q is Q³ and where R^{6c} is halogen, can be used as starting materials for introducing further substituents R6c by nucleophilic replacement in accordance to the methods described in Journal of Chemical Research, Synopses (6), 190 (1985), in Izvestiya Akademii Nauk SSSR, Seriya Khimicheskaya (9), 2086-8 (1986) or in Russian Chemical Bulletin (Translation of Izvestiya Akademii Nauk, Seriya Khimicheskaya), 53(3), 596-614 (2004).

The compounds of formula VI, where Q is Q⁴ are either known or can be prepared by analogy to standard methods for preparing 2-amino-1,3,4-oxadiazole compounds known from the literature. For example, 5-(R^{6d})-subsituted-2-amino-1,3,4-oxadiazoles can be prepared from aldehydes and semicarbazide pursuant to a procedure described in J. Org. Chem., 2015, 80, 1018-1024. 5-(R^{6d})-subsituted-2-amino-1,3,4-oxadiazoles can also be prepared by intramolecular cyclization of semicarbanzones by the protocol described in Synlett, 2015, 26, 1201-1206. Further methods for preparing 5-(R^{6d})-subsituted-2-amino-1,2,5-oxadiazoles are described in Org. Lett., 2014, 16, 2342-2345, J. Org. Chem., 2006, Synlett, 2012, 23, 1970-1972 and J. Org. Chem., 2013, 78, 438-444.

The compounds of formulae VII and VIII can be prepared from the corresponding 3-aminobenzoic acid compounds according to standard procedures for preparing thio ureas, e.g. by analogy to the method depicted in schemes 1 and 2.

Apart from that, compounds of formula VI and the benzoic acid precursors of formulae VII and VIII are known or can also be prepared by processes known in the art.

Compounds of formula I, where R^{2b} or R^{2c} are different from hydrogen, can be prepared from of formula I, where R^{2b} or R^{2c} are hydrogen by analogy to standard methods of N-substitution of ureas and thioureas respectively. Compounds of formula I, where R⁷ is different from hydrogen, can be prepared from of formula I, where R⁷ is hydrogen by analogy to standard methods of N-substitution of carboxamides.

As a rule, the compounds of formula I including their stereoisomers, salts, and tautomers, and their precursors in the synthesis process, can be prepared by the methods described above. If individual compounds can not be prepared via the above-described routes, they can be prepared by derivatization of other compounds I or the respective precursor or by customary modifications of the synthesis routes described. For example, in individual cases, certain compounds of formula I can advantageously be prepared from other compounds of formula I by derivatization, e.g. by ester hydrolysis, amidation, esterification, ether cleavage, olefination, reduction, oxidation and the like, or by customary modifications of the synthesis routes described.

The reaction mixtures are worked up in the customary manner, for example by mixing with water, separating the phases, and, if appropriate, purifying the crude products by chromatography, for example on alumina or on silica gel. Some of the intermediates and end products may be obtained in the form of colorless or pale brown viscous oils which are freed or purified from volatile components under reduced pressure and at moderately elevated temperature. If the intermediates and end products are obtained as solids, they may be purified by recrystallization or trituration.

The compounds of formula I and their agriculturally suitable salts are useful as herbicides. They are useful as such or as an appropriately formulated composition. The herbicidal compositions comprising the compound of formula I, an N-oxide or a salt thereof, in particular the preferred aspects thereof, control vegetation on non-crop areas very efficiently, especially at high rates of application. They act against broad-leaved weeds and weed grasses in crops such as wheat, rice, corn, soybeans and cotton without causing any significant damage to the crop plants. This effect is mainly observed at low rates of application.

Depending on the application method in question, the compounds of formula I, in particular the preferred aspects thereof, or compositions comprising them can additionally be employed in a further number of crop plants for eliminating unwanted plants. Examples of suitable crops are the following:
Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera, Zea mays.

The compounds of the present invention are particularly suitable for use in crops from the family poaceae, in particular crops of the tribum triticeae, e.g. crops of the generae hordeum, sorghum, triticium and secale, and crops of the generae zea, e.g. zea mays and oryza, e.g. oryza sativa.

The term "crop plants" also includes plants which have been modified by breeding, mutagenesis or genetic engineering. Genetically modified plants are plants whose genetic material has been modified in a manner which does not occur under natural conditions by crossing, mutations or natural recombination (i.e. reassembly of the genetic information). Here, in general, one or more genes are integrated into the genetic material of the plant to improve the properties of the plant.

Accordingly, the term "crop plants" also includes plants which, by breeding and genetic engineering, have acquired tolerance to certain classes of herbicides, such as hydroxy-phenylpyruvate dioxygenase (HPPD) inhibitors, acetolactate synthase (ALS) inhibitors, such as, for example, sulfonylureas (EP-A-0257993, US 5,013,659) or imidazolinones (see, for example, US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073), enolpyruvylshikimate 3-phosphate synthase (EPSPS) inhibitors, such as, for example, glyphosate (see, for example, WO 92/00377), glutamine synthetase (GS) inhibitors, such as, for example, glufosinate (see, for example, EP-A-0242236, EP-A-242246), or oxynil herbicides (see, for example, US 5,559,024).

In a preferred embodiment, the term "crop plants" refers to plants that comprise in their genomes a gene encoding a herbicide-tolerant wild-type or mutated HPPD protein. Such a gene may be an endogenous gene or a transgene, as described hereinafter.

By a "herbicide-tolerant" or "herbicide-resistant" plant, it is intended that a plant that is tolerant or resistant to at least one herbicide at a level that would normally kill, or inhibit the growth of, a normal or wild-type plant. By "herbicide-tolerant wild-type or mutated HPPD protein" or "herbicide -resistant wild-type or mutated HPPD protein", it is intended that such a HPPD protein displays higher HPPD activity, relative to the HPPD activity of a wild-type or reference HPPD protein, when in the presence of at least one herbicide that is known to interfere with HPPD activity and at a concentration or level of the herbicide that is known to inhibit the HPPD activity of the reference wild-type HPPD protein. Furthermore, the HPPD activity of such a herbicide-tolerant or herbicide-resistant HPPD protein may be referred to herein as "herbicide-tolerant" or "herbicide-resistant" HPPD activity.

The term "mutated HPPD nucleic acid" refers to an HPPD nucleic acid having a sequence that is mutated from a wild-type HPPD nucleic acid and that confers increased " HPPD-inhibiting herbicide" tolerance to a plant in which it is expressed. Furthermore, the term " mutated hydroxyphenyl pyruvate dioxygenase (mutated HPPD)" refers to the replacement of an amino acid of the wild-type primary sequences SEQ ID NO: 2, 5, 8, 11, 14, 17, 20, 22, 24, 26, 28, 30, 32, 34, 36, 38, 40, 42, 44, 46, 48, 50, 53, 55, 57, 58, 59, 60, 61, 62, 63, 64, 65, 66, 67, a variant, a derivative, a homologue, an orthologue, or paralogue thereof, with another amino acid. The expression "mutated amino acid" will be used below to designate the amino acid which is replaced by another amino acid, thereby designating the site of the mutation in the primary sequence of the protein.

Several HPPDs and their primary sequences have been described in the state of the art, in particular the HPPDs of bacteria such as Pseudomonas (Ruetschi etal., Eur.J.Biochem., 205, 459-466, 1992, WO96/38567), of plants such as Arabidopsis (WO96/38567, Genebank AF047834) or of carrot (WO96/38567, Genebank 87257), of Coccicoides (Genebank COITRP), HPPDs of Brassica, cotton, Synechocystis, and tomato (US 7,297,541), of mammals such as the mouse or the pig. Furthermore, artificial HPPD sequences have been described, for example in US6,768,044; US6,268,549;

In a preferred embodiment, the nucleotide sequence of (i) comprises the sequence of SEQ ID NO: 1, 51, 3, 4, 6, 7, 9, 10, 12, 13, 15, 16, 18, 19, 21, 23, 25, 27, 29, 31, 33, 35, 37, 39, 41, 43, 45, 47, 49, 52, 54, 56, 68, 69 or a variant or derivative thereof.

In a particularly preferred embodiment, the mutated HPPD nucleic acid useful for the present invention comprises a mutated nucleic acid sequence of SEQ ID NO: 1 or SEQ ID NO: 52, or a variant or derivative thereof.

Furthermore, it will be understood by the person skilled in the art that the nucleotide sequences of (i) or (ii) encompass homologues, paralogues and orthologues of SEQ ID NO: 1, 51, 3, 4, 6, 7, 9, 10, 12, 13, 15, 16, 18, 19, 21, 23, 25, 27, 29, 31, 33, 35, 37, 39, 41, 43, 45, 47, 49, 52, 54, 56, 68, 69, as defined hereinafter.

The term "variant" with respect to a sequence (e.g., a polypeptide or nucleic acid sequence such as - for example - a transcription regulating nucleotide sequence of the invention) is intended to mean substantially similar sequences. For nucleotide sequences comprising an open reading frame, variants include those sequences that, because of the degeneracy of the genetic code, encode the identical amino acid sequence of the native protein. Naturally occurring allelic variants such as these can be identified with the use of well-known molecular biology techniques, as, for example, with polymerase chain reaction (PCR) and hybridization techniques. Variant nucleotide sequences also include synthetically derived nucleotide sequences, such as those generated, for example, by using site-directed mutagenesis and for open reading frames, encode the native protein, as well as those that encode a polypeptide having amino acid substitutions relative to the native protein. Generally, nucleotide sequence variants of the invention will have at least 30, 40, 50, 60, to 70%, e.g., preferably 71%, 72%, 73%, 74%, 75%, 76%, 77%, 78%, to 79%, generally at least 80%, e.g., 81%-84%, at least 85%, e.g., 86%, 87%, 88%, 89%, 90%, 91%, 92%, 93%, 94%, 95%, 96%, 97%, to 98% and 99% nucleotide" sequence identity" to the nucleotide sequence of SEQ ID NO:1, 51, 3, 4, 6, 7, 9, 10, 12, 13, 15, 16, 18, 19, 21, 23, 25, 27, 29, 31, 33, 35, 37, 39, 41, 43, 45, 47, 49, 52, 54, 56, 68, 69, 47, or 49. By "variant" polypeptide is intended a polypeptide derived from the protein of SEQ ID NO: 2, 5, 8, 11, 14, 17, 20, 22, 24, 26, 28, 30, 32, 34, 36, 38, 40, 42, 44, 46, 48, 50, 53, 55, 57, 58, 59, 60, 61, 62, 63, 64, 65, 66, 67, by deletion (so-called truncation) or addition of one or more amino acids to the N-terminal and/or C-terminal end of the native protein; deletion or addition of one or more amino acids at one or more sites in the native protein; or substitution of one or more amino acids at one or more sites in the native protein. Such variants may result from, for example, genetic polymorphism or from human manipulation. Methods for such manipulations are generally known in the art.

In a preferred embodiment, variants of the polynucleotides useful for the present invention will have at least 30, 40, 50, 60, to 70%, e.g., preferably 71%, 72%, 73%, 74%, 75%, 76%, 77%, 78%, to 79%, generally at least 80%, e.g., 81%-84%, at least 85%, e.g., 86%, 87%, 88%, 89%, 90%, 91%, 92%, 93%, 94%, 95%, 96%, 97%, to 98% and 99% nucleotide " sequence identity" to the nucleotide sequence of SEQ ID NO:1, 47, 49, or SEQ ID NO: 52.

It is recognized that the polynucleotide molecules and polypeptides of the invention encompass polynucleotide molecules and polypeptides comprising a nucleotide or an amino acid sequence that is sufficiently identical to nucleotide sequences set forth in SEQ ID NOs: 1, 51, 3, 4, 6, 7, 9, 10, 12, 13, 15, 16, 18, 19, 21, 23, 25, 27, 29, 31, 33, 35, 37, 39, 41, 43, 45, 47, 49, 52, 54, 56, 68, 69, 47, or 49, or to the amino acid sequences set forth in SEQ ID NOs: 2, 5, 8, 11, 14, 17, 20, 22, 24, 26, 28, 30, 32, 34, 36, 38, 40, 42, 44, 46, 48, 50, 53, 55, 57, 58, 59, 60, 61, 62, 63, 64, 65, 66, 67, 48, or 50 . The term "sufficiently identical" is used herein to refer to a first amino acid or nucleotide sequence that contains a sufficient or minimum number of identical or equivalent (e.g., with a similar side chain) amino acid residues or nucleotides to a second amino acid or nucleotide sequence such that the first and second amino acid or nucleotide sequences have a common structural domain and/or common functional activity.

"Sequence identity" refers to the extent to which two optimally aligned DNA or amino acid sequences are invariant throughout a window of alignment of components, e.g., nucleotides or amino acids. An "identity fraction" for aligned segments of a test sequence and a reference sequence is the number of identical components that are shared by the two aligned sequences divided by the total number of components in reference sequence segment, i.e., the entire reference sequence or a smaller defined part of the reference sequence. "Percent identity" is the identity fraction times 100. Optimal alignment of sequences for aligning a comparison window are well known to those skilled in the art and may be conducted by tools such as the local homology algorithm of Smith and Waterman, the homology alignment algorithm of Needleman and Wunsch, the search for similarity method of Pearson and Lipman, and preferably by computerized implementations of these algorithms such as GAP, BESTFIT, FASTA, and TFASTA available as part of the GCG. Wisconsin Package. (Accelrys Inc. Burlington, Mass.)

The terms "polynucleotide(s)", "nucleic acid sequence(s)", "nucleotide sequence(s)", " nucleic acid(s)" , " nucleic acid molecule" are used interchangeably herein and refer to nucleotides, either ribonucleotides or deoxyribonucleotides or a combination of both, in a polymeric unbranched form of any length.

"Derivatives" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag• 100 epitope, c-myc epitope, FLAG®-epitope, lacZ, CMP (cal-modulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α -helical structures or β -sheet structures). Amino acid substitutions are typically of single residues, but may be clustered depending upon functional constraints placed upon the polypeptide and may range from 1 to 10 amino acids; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds).

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuikChange Site Directed mutagenesis (Stratagene, San Diego, CA), PCR-mediated site-directed mutagenesis or other site-directed mutagenesis protocols.

"Derivatives" further include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

"Orthologues" and "paralogues" encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

It is well-known in the art that paralogues and orthologues may share distinct domains harboring suitable amino acid residues at given sites, such as binding pockets for particular substrates or binding motifs for interaction with other proteins.

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

The term "motif" or "consensus sequence" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

By substituting one or more of the key amino acid residues, the herbicide tolerance or resistance of a plant to the herbicide as described herein could be remarkably increased as compared to the activity of the wild type HPPD enzymes with SEQ ID NO: 2, 5, 8, 11, 14, 17, 20, 22, 24, 26, 28, 30, 32, 34, 36, 38, 40, 42, 44, 46, 48, 50, 53, 55, 57, 58, 59, 60, 61, 62, 63, 64, 65, 66, 67. Preferred substitutions of mutated HPPD are those that increase the herbicide tolerance of the plant, but leave the biological activitiy of the dioxygenase activity substantially unaffected.

It will be understood by the person skilled in the art that amino acids located in a close proximity to the positions of amino acids mentioned below may also be substituted. Thus, in another embodiment the mutated HPPD useful for the present invention comprises a sequence of SEQ ID NO: 2, 5, 8, 11, 14, 17, 20, 22, 24, 26, 28, 30, 32, 34, 36, 38, 40, 42, 44, 46, 48, 50, 53, 55, 57, 58, 59, 60, 61, 62, 63, 64, 65, 66, 67, or a variant, derivative, orthologue, paralogue or homologue thereof, wherein an amino acid ±3, ±2 or ±1 amino acid positions from a key amino acid is substituted by any other amino acid.

Based on techniques well-known in the art, a highly characteristic sequence pattern can be developed, by means of which further of mutated HPPD candidates with the desired activity may be searched.

Searching for further mutated HPPD candidates by applying a suitable sequence pattern would also be encompassed by the present invention. It will be understood by a skilled reader that the present sequence pattern is not limited by the exact distances between two adjacent amino acid residues of said pattern. Each of the distances between two neighbours in the above patterns may, for example, vary independently of each other by up to ±10, ± 5, ±3, ±2 or ±1 amino acid positions without substantially affecting the desired activity.

In line with said above functional and spatial analysis of individual amino acid residues based on the crystallographic data as obtained according to the present invention, unique partial amino acid sequences characteristic of potentially useful mutated HPPD candidates of the invention may be identified.

In a particularly preferred embodiment, the mutated HPPD refers to a variant or derivative of SEQ ID NO: 2 wherein the substitutions are selected from the following Table 49a.

**Table 49a: (Sequence ID No: 2): single amino acid substitutions**

| Key amino acid position | Substituents |
|---|---|
| Val212 | Ile, Leu |
| Val213 | Thr, Ala |
| Asn215 | Ala, His |
| Ala236 | Leu, Ser, Arg |
| Phe238 | Val, Ala |
| Leu250 | Val, Met |
| Ser252 | Thr |
| Pro265 | Ala |
| Asn267 | Tyr, Gln |
| Gln278 | His, Asn, Ser |
| Ile279 | Thr |
| Arg309 | Lys, Ala |
| Leu320 | Asn, Gln, His, Tyr, |
| Pro321 | Ala, Arg, Gly, Asn |
| Leu334 | Glu, Cys |
| Leu353 | Met, Tyr, Ala, Ser |
| Phe366 | Ile, Leu, Tyr |
| Gly371 | Ile, Phe |
| Thr375 | Pro |
| Phe377 | Ala, Leu, Ser |
| Gly403 | Arg |
| Phe404 | Leu, Pro |
| Lys406 | Thr |
| Gly407 | Cys, His |
| Phe409 | Ile, His |
| Glu411 | Thr |
| Leu412 | Met, Phe, Trp, Ala, Ser |
| Ile416 | Val, Phe |
| Ser410 | Gly |
| Val254 | Ala |

Furthermore, by substituting at least two of the key amino acid residues of SEQ ID NO: 2 with specific residues, the herbicide tolerance or resistance could be remarkably increased as compared to the activity of the wild type HPPD enzymes or HPPD enzymes in which only one amino acid residue had been substituted. Therefore, in another preferred embodiment, the variant or derivative of the mutated HPPD refers to a polypeptide of SEQ ID NO: 2, wherein two, three, four or five key amino acids are substituted by another amino acid residue. Particularly preferred double, triple, quadruple, or quintuple mutations are described in Table 49b.

**Table 49b: (with reference to Sequence ID No: 2): combined amino acid substitutions**

| Combination No | Key amino acid position and and its substitutents |
|---|---|
| 1 | A236L, E411T |
| 2 | L320H, P321A |
| 3 | L320H, P321R |
| 4 | L320N, P321A |
| 5 | L320N, P321R |
| 6 | L320Q, P321A |
| 7 | L320Q, P321R |
| 8 | L320Y, P321A |
| 9 | L320Y, P321R |
| 10 | L353M, P321R |
| 11 | L353M, P321R, A236L |
| 12 | L353M, P321R, A236L, E411T |
| 13 | L353M, P321R, E411T |
| 14 | L353M, P321R, L320H |
| 15 | L353M, P321R, L320N |
| 16 | L353M, P321R, L320Q |
| 17 | L353M, P321R, L320Y |
| 18 | L353M, P321R, V212I |
| 19 | L353M, P321R, V212I, L334E |
| 20 | L353M, P321R, V212L, L334E |
| 21 | L353M, P321R, V212L, L334E, A236L |
| 22 | L353M, P321R, V212L, L334E, A236L, E411T |
| 23 | L353M, P321R, V212L, L334E, E411T |
| 24 | L353M, P321R, V212L, L334E, L320H |
| 25 | L353M, P321R, V212L, L334E, L320N |
| 26 | L353M, P321R, V212L, L334E, L320Q |
| 27 | L353M, P321R, V212L, L334E, L320Y |
| 28 | L353M, V212I |

In a particularly preferred embodiment, the mutated HPPD enzyme comprising a polypeptide of SEQ ID NO: 2, a variant, derivative, homologue, paralogue or orthologue thereof, useful for the present invention comprises one or more of the following: the amino acid corresponding to or at position 320 is histidine, asparagine or glutamine; the amino acid position 334 is glutamic acid; the amino acid position 353 is methionine; the amino acid corresponding to or at position 321 alanine or arginine; the amino acid corresponding to or at position 212 is isoleucine.

In an especially particularly preferred embodiment, the mutated HPPD refers to a polypeptide comprising SEQ ID NO: 2, wherein the leucine corresponding to or at position 320 is substituted by a histidine, and the proline corresponding to or at position 321 is substituted by an alanine.

In another especially particularly preferred embodiment, the mutated HPPD refers to a polypeptide comprising SEQ ID NO: 2, wherein Leucine corresponding to or at position 353 is substituted by a Methionine, the Proline corresponding to or at position 321 is substituted by an Arginine, and the Leucine corresponding to or at position 320 is substituted by an Asparagine.

In another especially particularly preferred embodiment, the mutated HPPD refers to a polypeptide comprising SEQ ID NO: 2, wherein the Leucine corresponding to or at position 353 is substituted by a Methionine, the Proline corresponding to or at position 321 is substituted by an Arginine, and the Leucine corresponding to or at position 320 is substituted by a glutamine.

In another preferred embodiment, the mutated HPPD refers to a variant or derivative of SEQ ID NO: 53 wherein the substitutions are selected from the following Table 49c.

**Table 49c: (Sequence ID No: 53): single amino acid substitutions**

| | | |
|---|---|---|
| Key amino acid position | Substituents | Preferred substituents |
| Val228 | Thr, Ala | Thr, Ala |
| Asn230 | Ala, His | Ala, His |
| Ala251 | Ser, Arg | Ser, Arg |
| Phe253 | Val, Ala | Val, Ala |
| Leu265 | Val, Met | Val, Met |
| Ser267 | Thr | Thr |
| Pro280 | Ala | Ala |
| Asn282 | Tyr, Gln | Tyr, Gln |
| Lys291 | Arg, Ala | Arg |
| Gln293 | Ala, Leu, Ile, Val, His, Asn, Ser | His, Asn, Ser |
| Ile294 | Thr | Thr |
| Arg324 | Lys, Ala | Lys, Ala |
| Met335 | Ala, Trp, Phe, Leu, Ile, Val, Asn, Gln, His, Tyr, Ser, Thr, Cys | Gln, Asn, His, Tyr |
| Pro336 | Ala, Arg, Gly, Asn | Ala, Gly |
| Ser337 | Ala, Pro, Thr | Pro, Thr |
| Pro339 | Deletion | Deletion |
| Pro340 | Gly | Gly |
| Glu363 | Gln | Gln |
| Leu368 | Met, Tyr, | Met |
| Phe381 | Ile, Leu, Tyr | Ile, Leu |
| Leu385 | Ala, Val, Gln, Asp | Val, Asp |
| Gly386 | Ile, Phe | Ile, Phe |
| Thr390 | Pro | Pro |
| Phe392 | Ala, Leu, Ser | Ala |
| Ile393 | Ala, Leu, Phe, Val | Leu |
| Phe419 | Leu, Pro | Leu, Pro |
| Lys421 | Thr | Thr |
| Gly422 | His, Met, Phe, Cys | His, Cys |
| Phe424 | Ile, His | Ile, His |
| Leu427 | Phe, Trp, Ala, Ser, Met | Phe |
| Ile431 | Val, Phe | Val, Phe |
| Ser425 | Gly | Gly |
| Val269 | Ala | Ala |

In another preferred embodiment, the variant or derivative of the mutated HPPD useful for the present invention refers to a polypeptide of SEQ ID NO: 53, a homologue, orthologue, or paralogue thereof, wherein two, three, four or five key amino acids are substituted by another amino acid residue. Particularly preferred double, triple, quadruple, or quintuple mutations are described in Table 49d.

**Table 49d: (reference to Sequence ID No: 53): combined amino acid substitutions**

| Combination No | Key amino acid position | Substituents | Preferred substituents |
|---|---|---|---|
| 1 | Pro336 | Ala, Arg | Ala |
| | Glu363 | Gln | Gln |
| 2 | Pro336 | Ala, Arg | Ala |
| | Glu363 | Gln | Gln |
| | Leu385 | Ala, Val | Val |
| 3 | Pro336 | Ala, Arg | Ala |
| | Glu363 | Gln | Gln |
| | Leu385 | Ala, Val | Val |
| | Ile393 | Ala, Leu | Leu |
| 4 | Leu385 | Ala, Val | Val |
| | Ile393 | Ala, Leu | Leu |
| 5 | Met335 | Ala, Trp, Phe, Leu, Ile, Val, Asn, Gln, His, Tyr, Ser, Thr, Cys | Gln, Asn, His, Tyr |
| | Pro336 | Ala, Arg, Gly | Ala, Gly |
| 6 | Met335 | Ala, Trp, Phe, Leu, Ile, Val, Asn, Gln, | Gln, Asn, His, Tyr |
| | | His, Tyr, Ser, Thr, Cys | |
| | Pro336 | Ala, Arg, Gly | Ala, Gly |
| | Glu363 | Gln | Gln |
| 7 | Met335 | Ala, Trp, Phe, Leu, Ile, Val, Asn, Gln, His, Tyr, Ser, Thr, Cys | Gln, Asn, His, Tyr, Leu |
| | Pro336 | Ala, Arg, Gly | Ala, Arg, Gly |
| | Ser337 | Ala, Pro, Thr | Pro, Thr |
| | Pro339 | Deletion | Deletion |
| | Pro340 | Gly | Gly |

Furthermore, by substituting the amino acids at some positions in the HPPD polypeptide sequences of Scenedesmus obliquus, the tolerance of crop plants as described herein towards the herbicides as described herein could be remarkably increased.

Thus, in a preferred embodiment,-the mutated HPPD of the present invention comprises a variant of the sequence of SEQ ID NO: 50, or a homologue or functional equivalent thereof, which comprises one or more of the following:
the amino acid corresponding to or at position 30 is other than proline, the amino acid corresponding to or at position 39 is other than Phe, the amino acid corresponding to or at position 54 is other than Gly, the amino acid corresponding to or at position 57 is other than Met, the amino acid corresponding to or at position 84 is other than Phe, the amino acid corresponding to or at position 210 is other than Val, the amino acid corresponding to or at position 212 is other than Asn, the amino acid corresponding to or at position 223 is other than Val, the amino acid corresponding to or at position 243 is other than Val, the amino acid corresponding to or at position 247 is other than Leu, the amino acid corresponding to or at position 249 is other than Ser, the amino acid corresponding to or at position 251 is other than Val, the amino acid corresponding to or at position 264 is other than Asn, the amino acid corresponding to or at position 291 is other than Leu, the amino acid corresponding to or at position 306 is other than His, the amino acid corresponding to or at position 317 is other than Gin, the amino acid corresponding to or at position 318 is other than Ala, the amino acid corresponding to or at position 319 is other than Ala, the amino acid corresponding to or at position 321 is other than Gly, the amino acid corresponding to or at position 326 is other than Lys, the amino acid corresponding to or at position 327 is other than Arg, the amino acid corresponding to or at position 331 is other than Lys, the amino acid corresponding to or at position 341 is other than Trp, the amino acid corresponding to or at position 342 is other than Ala, the amino acid corresponding to or at position 345 is other than Glu, the amino acid corresponding to or at position 350 is other than Leu, the amino acid corresponding to or at position 363 is other than Phe, the amino acid corresponding to or at position 367 is other than Leu, the amino acid corresponding to or at position 373 is other than Ile, the amino acid corresponding to or at position 374 is other than Phe, the amino acid corresponding to or at position 375 is other than Ile, the amino acid corresponding to or at position 379 is other than Glu, the amino acid corresponding to or at position 405 is other than Gly, the amino acid corresponding to or at position 407 is other than Phe, the amino acid corresponding to or at position 410 is other than Gly, the amino acid corresponding to or at position 412 is other than Phe, the amino acid corresponding to or at position 414 is other than Glu, the amino acid corresponding to or at position 419 is other than Ile, the amino acid corresponding to or at position 421 is other than Glu, the amino acid corresponding to or at position 422 is other than Tyr.

In another preferred embodiment, the mutated HPPD comprises a variant of the sequence of SEQ ID NO: 50, or a homologue or functional equivalent thereof, in which:
the amino acid corresponding to or at position 367 is Val, and the amino acid corresponding to or at position 375 is Leu.

In another preferred embodiment, the mutated HPPD comprises a variant of the sequence of SEQ ID NO: 50, or a homologue or functional equivalent thereof, in which:
the amino acid corresponding to or at position 367 is Val, and the amino acid corresponding to or at position 375 is Leu, and the amino acid corresponding to or at position 39 is Leu.

In another preferred embodiment, the mutated HPPD comprises a variant of the sequence of SEQ ID NO: 50, or a homologue or functional equivalent thereof, in which:
the amino acid corresponding to or at position 367 is Val, and the amino acid corresponding to or at position 375 is Leu, and the amino acid corresponding to or at position 39 is Trp.

In another preferred embodiment, the mutated HPPD comprises a variant of the sequence of SEQ ID NO: 50, or a homologue or functional equivalent thereof, in which:
the amino acid corresponding to or at position 345 is Ala, Arg, Asn, Asp, Cys, Gin, Gly, His, Ile, Leu, Lys, Met, Phe, Pro, Ser, Thr, Trp, Tyr, or Val, particularly preferred Gln

In another preferred embodiment, the mutated HPPD comprises a variant of the sequence of SEQ ID NO: 50, or a homologue or functional equivalent thereof, in which:
the amino acid corresponding to or at position 345 is Gin, and the amino acid corresponding to or at position 341 is Ile.

In another preferred embodiment, the mutated HPPD comprises a variant of the sequence of SEQ ID NO: 50, or a homologue or functional equivalent thereof, in which:
the amino acid corresponding to or at position 345 is Gin, and the amino acid corresponding to or at position 326 is Glu.

In another preferred embodiment, the mutated HPPD comprises a variant of the sequence of SEQ ID NO: 50, or a homologue or functional equivalent thereof, in which:
the amino acid corresponding to or at position 345 is Gin, and the amino acid corresponding to or at position 326 is Asp.

In another preferred embodiment, the mutated HPPD comprises a variant of the sequence of SEQ ID NO: 50, or a homologue or functional equivalent thereof, in which:
the amino acid corresponding to or at position 345 is Gin, and the amino acid corresponding to or at position 326 is Gin.

In another preferred embodiment, the mutated HPPD comprises a variant of the sequence of SEQ ID NO: 50, or a homologue or functional equivalent thereof, in which:
the amino acid corresponding to or at position 318 is Arg, Asn, Asp, Cys, Gin, Glu, Gly, His, Ile, Leu, Lys, Met, Phe, Pro, Ser, Thr, Trp, Tyr, or Val, particularly preferred Pro.

In another preferred embodiment, the mutated HPPD comprises a variant of the sequence of SEQ ID NO: 50, or a homologue or functional equivalent thereof, in which:
the amino acid corresponding to or at position 319 is Arg, Asn, Asp, Cys, Gin, Glu, Gly, His, Ile, Leu, Lys, Met, Phe, Pro, Ser, Thr, Trp, Tyr, Val, particularly preferred Pro.

In another preferred embodiment, the mutated HPPD comprises a variant of the sequence of SEQ ID NO: 50, or a homologue or functional equivalent thereof, in which:
the amino acid corresponding to or at position 318 is Pro, and the amino acid corresponding to or at position 319 is Pro.

In another preferred embodiment, the mutated HPPD comprises a variant of the sequence of SEQ ID NO: 50, or a homologue or functional equivalent thereof, in which:
the amino acid corresponding to or at position 321 is Ala, Arg, Asn, Asp, Cys, Gin, Glu, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 350 is Ala, Arg, Asn, Asp, Cys, Gin, Glu, Gly, His, Ile, Lys, Met, Phe, Pro, Ser, Thr, Trp, Tyr, or Val, particularly preferred Met.

In another preferred embodiment, the mutated HPPD comprises a variant of the sequence of SEQ ID NO: 50, or a homologue or functional equivalent thereof, in which:
the amino acid corresponding to or at position 405 is Ala, Arg, Asn, Asp, Cys, Gin, Glu, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 251 is Ala, Arg, Asn, Asp, Cys, Gin, Glu, Gly, His, Ile, Leu, Lys, Met, Phe, Pro, Ser, Thr, Trp, or Tyr, particularly preferred Ala.

In another preferred embodiment, the mutated HPPD comprises a variant of the sequence of SEQ ID NO: 50, or a homologue or functional equivalent thereof, in which:
the amino acid corresponding to or at position 317 is Ala, Arg, Asn, Asp, Cys, Glu, Gly, His, Ile, Leu, Lys, Met, Phe, Pro, Ser, Thr, Trp, Tyr, or Val, particularly preferred His or Met.

In another preferred embodiment, the mutated HPPD comprises a variant of the sequence of SEQ ID NO: 50, or a homologue or functional equivalent thereof, in which:
the amino acid corresponding to or at position 379 is Ala, Arg, Asn, Asp, Cys, Gin, Gly, His, Ile, Leu, Lys, Met, Phe, Pro, Ser, Thr, Trp, Tyr, or Val, particularly preferred Gin.

In another preferred embodiment, the mutated HPPD comprises a variant of the sequence of SEQ ID NO: 50, or a homologue or functional equivalent thereof, in which:
the amino acid corresponding to or at position 350 is Met, and the amino acid corresponding to or at position 318 is Arg.

In another preferred embodiment, the mutated HPPD comprises a variant of the sequence of SEQ ID NO: 50, or a homologue or functional equivalent thereof, in which:
the amino acid corresponding to or at position 350 is Met, and the amino acid corresponding to or at position 318 is Gly.

In another preferred embodiment, the mutated HPPD comprises a variant of the sequence of SEQ ID NO: 50, or a homologue or functional equivalent thereof, in which:
the amino acid corresponding to or at position 350 is Met, and the amino acid corresponding to or at position 318 is Arg, and the amino acid corresponding to or at position 317 is Asn.

In another preferred embodiment, the mutated HPPD comprises a variant of the sequence of SEQ ID NO: 50, or a homologue or functional equivalent thereof, in which:
the amino acid corresponding to or at position 210 is Ala, Arg, Asn, Asp, Cys, Gin, Glu, Gly, His, Ile, Leu, Lys, Met, Phe, Pro, Ser, Thr, Trp, or Tyr.

In another preferred embodiment, the mutated HPPD comprises a variant of the sequence of SEQ ID NO: 50, or a homologue or functional equivalent thereof, in which:
the amino acid corresponding to or at position 317 is His, and the amino acid corresponding to or at position 318 is Gly, and the amino acid corresponding to or at position 345 is Gin.

In another preferred embodiment, the mutated HPPD comprises a variant of the sequence of SEQ ID NO: 50, or a homologue or functional equivalent thereof, in which:
the amino acid corresponding to or at position 317 is Met, and the amino acid corresponding to or at position 318 is Gly, and the amino acid corresponding to or at position 345 is Gin.

In another preferred embodiment, the mutated HPPD comprises a variant of the sequence of SEQ ID NO: 50, or a homologue or functional equivalent thereof, in which:
the amino acid corresponding to or at position 363 is Ala, Arg, Asn, Asp, Cys, Gin, Glu, Gly, His, Ile, Leu, Lys, Met, Pro, Ser, Thr, Trp, Tyr, or Val, particularly preferred Ile.

In another preferred embodiment, the mutated HPPD comprises a variant of the sequence of SEQ ID NO: 50, or a homologue or functional equivalent thereof, in which:
the amino acid corresponding to or at position 419 is Ala, Arg, Asn, Asp, Cys, Gin, Glu, Gly, His, Leu, Lys, Met, Phe, Pro, Ser, Thr, Trp, Tyr, or Val, particularly preferred Val.

In another preferred embodiment, the mutated HPPD comprises a variant of the sequence of SEQ ID NO: 50, or a homologue or functional equivalent thereof, in which:
the amino acid corresponding to or at position 249 is Ala, Arg, Asn, Asp, Cys, Gin, Glu, Gly, His, Ile, Leu, Lys, Met, Phe, Pro, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 247 is Ala, Arg, Asn, Asp, Cys, Gin, Glu, Gly, His, Ile, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 407 is Ala, Arg, Asn, Asp, Cys, Gin, Glu, Gly, His, Ile, Leu, Lys, Met, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 306 is Ala, Arg, Asn, Asp, Cys, Gin, Glu, Gly, Ile, Leu, Lys, Met, Phe, Pro, Ser, Thr, Trp, Tyr, or Val, particularly preferred Lys.

In another preferred embodiment, the mutated HPPD comprises a variant of the sequence of SEQ ID NO: 50, or a homologue or functional equivalent thereof, in which:
the amino acid corresponding to or at position 30 is Ala, Arg, Asn, Asp, Cys, Gin, Glu, Gly, His, Ile, Leu, Lys, Met, Phe, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 54 is Ala, Arg, Asn, Asp, Cys, Gin, Glu, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 57 is Ala, Arg, Asn, Asp, Cys, Gin, Glu, Gly, His, Ile, Leu, Lys, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 84 is Ala, Arg, Asn, Asp, Cys, Gin, Glu, Gly, His, Ile, Leu, Lys, Met, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 212 is Ala, Arg, Asp, Cys, Gin, Glu, Gly, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 223 is Ala, Arg, Asn, Asp, Cys, Gin, Glu, Gly, His, Ile, Leu, Lys, Met, Phe, Pro, Ser, Thr, Trp, or Tyr.

In another preferred embodiment, the mutated HPPD comprises a variant of the sequence of SEQ ID NO: 50, or a homologue or functional equivalent thereof, in which:
the amino acid corresponding to or at position 243 is Ala, Arg, Asn, Asp, Cys, Gin, Glu, Gly, His, Ile, Leu, Lys, Met, Phe, Pro, Ser, Thr, Trp, or Tyr.

In another preferred embodiment, the mutated HPPD comprises a variant of the sequence of SEQ ID NO: 50, or a homologue or functional equivalent thereof, in which:
the amino acid corresponding to or at position 264 is Ala, Arg, Asp, Cys, Gin, Glu, Gly, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 291 is Ala, Arg, Asn, Asp, Cys, Gin, Glu, Gly, His, Ile, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 327 is Ala, Asn, Asp, Cys, Gin, Glu, Gly, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 331 is Ala, Arg, Asn, Asp, Cys, Gin, Glu, Gly, His, Ile, Leu, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 342 is Arg, Asn, Asp, Cys, Gin, Glu, Gly, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 373 is Ala, Arg, Asn, Asp, Cys, Gin, Glu, Gly, His, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 374 is Ala, Arg, Asn, Asp, Cys, Gin, Glu, Gly, His, Ile, Leu, Lys, Met, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 410 is Ala, Arg, Asn, Asp, Cys, Gin, Glu, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 412 is Ala, Arg, Asn, Asp, Cys, Gin, Glu, Gly, His, Ile, Leu, Lys, Met, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 414 is Ala, Arg, Asn, Asp, Cys, Gin, Gly, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 421 is Ala, Arg, Asn, Asp, Cys, Gin, Gly, His, Ile, Leu, Lys, Met, Phe, Pro, Ser, Thr, Trp, Tyr, or Val.

In another preferred embodiment, the mutated HPPD comprises a variant of the sequence of SEQ ID NO: 50, or a homologue or functional equivalent thereof, in which:
the amino acid corresponding to or at position 422 is Ala, Arg, Asn, Asp, Cys, Gin, Glu, Gly, His, Ile, Leu, Lys, Met, Phe, Pro, Ser, Thr, Trp, or Val.

In another preferred embodiment, the mutated HPPD comprises a variant of the sequence of SEQ ID NO: 50, or a homologue or functional equivalent thereof, in which:
the amino acid corresponding to or at position 251 is Ala, and the amino acid corresponding to or at position 405 is Asp.

In another preferred embodiment, the mutated HPPD comprises a variant of the sequence of SEQ ID NO: 50, or a homologue or functional equivalent thereof, in which:
the amino acid corresponding to or at position 327 is Gly, and the amino acid corresponding to or at position 421 is Asp.

In another preferred embodiment, the mutated HPPD comprises a variant of the sequence of SEQ ID NO: 50, or a homologue or functional equivalent thereof, in which:
the amino acid corresponding to or at position 251 is Ala, and the amino acid corresponding to or at position 306 is Arg, and the amino acid corresponding to or at position 317 is Leu, and the amino acid corresponding to or at position 318 is Pro, and the amino acid corresponding to or at position 321 is Pro, and the amino acid corresponding to or at position 331 is Glu, and the amino acid corresponding to or at position 350 is Met.

In another preferred embodiment, the mutated HPPD comprises a variant of the sequence of SEQ ID NO: 50, or a homologue or functional equivalent thereof, in which:
the amino acid corresponding to or at position 407 is Ala, Arg, Asn, Asp, Cys, Gin, Glu, Gly, His, Ile, Leu, Lys, Met, Pro, Ser, Thr, Trp, Tyr, or Val.

Following mutagenesis of one of the sequences as shown herein, the encoded protein can be expressed recombinantly and the activity of the protein can be determined using, for example, assays described herein.

It will be within the knowledge of the skilled artisan to identify conserved regions and motifs shared between the homologues, orthologues and paralogues of of SEQ ID NO: 2, 5, 8, 11, 14, 17, 20, 22, 24, 26, 28, 30, 32, 34, 36, 38, 40, 42, 44, 46, 48, 50, 53, 55, 57, 58, 59, 60, 61, 62, 63, 64, 65, 66, 67, and respectively SEQ ID NO: 48 or 50. Having identified such conserved regions that may represent suitable binding motifs, amino acids corresponding to the amino acids listed in Table 49a and 49b, 49c, and 49d can be chosen to be substituted by any other amino acid by conserved amino acids, and more preferably by the amino acids of tables 49a and 49b, 49c, and 49d.

Numerous crop plants, for example Clearfield® oilseed rape, tolerant to imidazolinones, for example imazamox, have been generated with the aid of classic breeding methods (mutagenesis). Crop plants such as soybeans, cotton, corn, beet and oilseed rape, resistant to glyphosate or glufosinate, which are available under the tradenames RoundupReady® (glyphosate) and Liberty Link® (glufosinate) have been generated with the aid of genetic engineering methods.

Accordingly, the term "crop plants" also includes plants which, with the aid of genetic engineering, produce one or more toxins, for example those of the bacterial strain *Bacillus* ssp. Toxins which are produced by such genetically modified plants include, for example, insecticidal proteins of *Bacillus* spp., in particular *B. thuringiensis,* such as the endotoxins Cry1Ab, Cry1Ac, Cry1F, Cry1Fa2, Cry2Ab, Cry3A, Cry3Bb1, Cry9c, Cry34Ab1 or Cry35Ab1; or vegetative insecticidal proteins (VIPs), for example VIP1, VIP2, VIP3, or VIP3A; insecticidal proteins of nema-tode-colonizing bacteria, for example *Photorhabdus* spp. or *Xenorhabdusspp*.; toxins of animal organisms, for example wasp, spider or scorpion toxins; fungal toxins, for example from Strep-tomycetes; plant lectins, for example from peas or barley; agglutinins; proteinase inhibitors, for example trypsin inhibitors, serine protease inhibitors, patatin, cystatin or papain inhibitors, ribosome-inactivating proteins (RIPs), for example ricin, corn-RIP, abrin, luffin, saporin or bryodin; steroid-metabolizing enzymes, for example 3-hydroxysteroid oxidase, ecdysteroid-IDP glycosyl transferase, cholesterol oxidase, ecdysone inhibitors, or HMG-CoA reductase; ion channel blockers, for example inhibitors of sodium channels or calcium channels; juvenile hormone esterase; receptors of the diuretic hormone (helicokinin receptors); stilbene synthase, bibenzyl synthase, chitinases and glucanases. In the plants, these toxins may also be produced as pre-toxins, hybrid proteins or truncated or otherwise modified proteins. Hybrid proteins are characterized by a novel combination of different protein domains (see, for example, WO 2002/015701). Further examples of such toxins or genetically modified plants which produce these toxins are disclosed in EP-A 374 753, WO 93/007278, WO 95/34656, EP-A 427 529, EP-A 451 878, WO 03/018810 and WO 03/052073. The methods for producing these genetically modified plants are known to the person skilled in the art and disclosed, for example, in the publications mentioned above. Numerous of the toxins mentioned above bestow, upon the plants by which they are produced, tolerance to pests from all taxonomic classes of arthropods, in particular to beetles (Coeleropta), dipterans (Diptera) and butterflies (Lepidoptera) and to nematodes (Nematoda).

Genetically modified plants which produce one or more genes coding for insecticidal toxins are described, for example, in the publications mentioned above, and some of them are commercially available, such as, for example, YieldGard® (corn varieties producing the toxin Cry1Ab), YieldGard® Plus (corn varieties which produce the toxins Cry1Ab and Cry3Bb1), Star-link® (corn varieties which produce the toxin Cry9c), Herculex® RW (corn varieties which produce the toxins Cry34Ab1, Cry35Ab1 and the enzyme phosphinothricin-N-acetyltransferase [PAT]); NuCOTN® 33B (cotton varieties which produce the toxin Cry1Ac), Bollgard® I (cotton varieties which produce the toxin Cry1Ac), Bollgard® II (cotton varieties which produce the toxins Cry1Ac and Cry2Ab2); VIPCOT® (cotton varieties which produce a VIP toxin); NewLeaf® (potato varieties which produce the toxin Cry3A); Bt-Xtra®, NatureGard®, KnockOut®, BiteGard®, Protecta®, Bt11 (for example Agrisure® CB) and Bt176 from Syngenta Seeds SAS, France (corn varieties which produce the toxin Cry1Ab and the PAT enyzme), MIR604 from Syngenta Seeds SAS, France (corn varieties which produce a modified version of the toxin Cry3A, see WO 03/018810), MON 863 from Monsanto Europe S.A., Belgium (corn varieties which produce the toxin Cry3Bb1), IPC 531 from Monsanto Europe S.A., Belgium (cotton varieties which produce a modified version of the toxin Cry1Ac) and 1507 from Pioneer Overseas Corporation, Belgium (corn varieties which produce the toxin Cry1F and the PAT enzyme).

Accordingly, the term "crop plants" also includes plants which, with the aid of genetic engineering, produce one or more proteins which are more robust or have increased resistance to bacterial, viral or fungal pathogens, such as, for example, pathogenesis-related proteins (PR proteins, see EP-A 0 392 225), resistance proteins (for example potato varieties producing two resistance genes against *Phytophthora infestans* from the wild Mexican potato *Solanum bulbocastanum*) or T4 lysozyme (for example potato cultivars which, by producing this protein, are resistant to bacteria such as *Erwinia amylvora*)*.*

Accordingly, the term "crop plants" also includes plants whose productivity has been improved with the aid of genetic engineering methods, for example by enhancing the potential yield (for example biomass, grain yield, starch, oil or protein content), tolerance to drought, salt or other limiting environmental factors or resistance to pests and fungal, bacterial and viral pathogens.

The term "crop plants" also includes plants whose ingredients have been modified with the aid of genetic engineering methods in particular for improving human or animal diet, for example by oil plants producing health-promoting long-chain omega 3 fatty acids or monounsaturated omega 9 fatty acids (for example Nexera® oilseed rape).

The term "crop plants" also includes plants which have been modified with the aid of genetic engineering methods for improving the production of raw materials, for example by increasing the amylopectin content of potatoes (Amflora® potato).

Furthermore, it has been found that the compounds of formula I are also suitable for the defoliation and/or desiccation of plant parts, for which crop plants such as cotton, potato, oilseed rape, sunflower, soybean or field beans, in particular cotton, are suitable. In this regard, there have been found compositions for the desiccation and/or defoliation of plants, processes for preparing these compositions and methods for desiccating and/or defoliating plants using the compounds of formula I.

As desiccants, the compounds of formula I are particularly suitable for desiccating the above-ground parts of crop plants such as potato, oilseed rape, sunflower and soybean, but also cereals. This makes possible the fully mechanical harvesting of these important crop plants.

Also of economic interest is to facilitate harvesting, which is made possible by concentrating within a certain period of time the dehiscence, or reduction of adhesion to the tree, in citrus fruit, olives and other species and varieties of pomaceous fruit, stone fruit and nuts. The same mechanism, i.e. the promotion of the development of abscission tissue between fruit part or leaf part and shoot part of the plants is also essential for the readily controllable defoliation of useful plants, in particular cotton.

Moreover, a shortening of the time interval in which the individual cotton plants mature leads to an increased fiber quality after harvesting.

The compounds of formula I, or the herbicidal compositions comprising the compounds of formula I, can be used, for example, in the form of ready-to-spray aqueous solutions, powders, suspensions, also highly concentrated aqueous, oily or other suspensions or dispersions, emulsions, oil dispersions, pastes, dusts, materials for broadcasting, or granules, by means of spraying, atomizing, dusting, spreading, watering or treatment of the seed or mixing with the seed. The use forms depend on the intended purpose; in each case, they should ensure the finest possible distribution of the active ingredients according to the invention.

The herbicidal compositions comprise a herbicidally effective amount of at least one compound of the formula I or an agriculturally useful salt of I, and auxiliaries which are customary for the formulation of crop protection agents.

Examples of auxiliaries customary for the formulation of crop protection agents are inert auxiliaries, solid carriers, surfactants (such as dispersants, protective colloids, emulsifiers, wetting agents and tackifiers), organic and inorganic thickeners, bactericides, antifreeze agents, antifoams, if appropriate colorants and, for seed formulations, adhesives.

Examples of thickeners (i.e. compounds which impart to the formulation modified flow properties, i.e. high viscosity in the state of rest and low viscosity in motion) are polysaccharides, such as xanthan gum (Kelzan® from Kelco), Rhodopol® 23 (Rhone Poulenc) or Veegum® (from R.T. Vanderbilt), and also organic and inorganic sheet minerals, such as Attaclay® (from Engelhardt).

Examples of antifoams are silicone emulsions (such as, for example, Silikon® SRE, Wacker or Rhodorsil® from Rhodia), long-chain alcohols, fatty acids, salts of fatty acids, organofluorine compounds and mixtures thereof.

Bactericides can be added for stabilizing the aqueous herbicidal formulation. Examples of bactericides are bactericides based on diclorophen and benzyl alcohol hemiformal (Proxel® from ICI or Acticide® RS from Thor Chemie and Kathon® MK from Rohm & Haas), and also isothiazolinone derivates, such as alkylisothiazolinones and benzisothiazolinones (Acticide MBS from Thor Chemie).

Examples of antifreeze agents are ethylene glycol, propylene glycol, urea or glycerol.

Examples of colorants are both sparingly water-soluble pigments and water-soluble dyes. Examples which may be mentioned are the dyes known under the names Rhodamin B, C.I. Pigment Red 112 and C.I. Solvent Red 1, and also pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Examples of adhesives are polyvinylpyrrolidone, polyvinyl acetate, polyvinyl alcohol and tylose.

Suitable inert auxiliaries are, for example, the following:
mineral oil fractions of medium to high boiling point, such as kerosene and diesel oil, furthermore coal tar oils and oils of vegetable or animal origin, aliphatic, cyclic and aromatic hydrocarbons, for example paraffin, tetrahydronaphthalene, alkylated naphthalenes and their derivatives, alkylated benzenes and their derivatives, alcohols such as methanol, ethanol, propanol, butanol and cyclohexanol, ketones such as cyclohexanone or strongly polar solvents, for example amines such as N-methylpyrrolidone, and water.

Solid carriers are mineral earths such as silicas, silica gels, silicates, talc, kaolin, limestone, lime, chalk, bole, loess, clay, dolomite, diatomaceous earth, calcium sulfate, magnesium sulfate and magnesium oxide, ground synthetic materials, fertilizers such as ammonium sulfate, ammonium phosphate, ammonium nitrate and ureas, and products of vegetable origin, such as cereal meal, tree bark meal, wood meal and nutshell meal, cellulose powders, or other solid carriers.

Suitable surfactants (adjuvants, wetting agents, tackifiers, dispersants and also emulsifiers) are the alkali metal salts, alkaline earth metal salts and ammonium salts of aromatic sulfonic acids, for example lignosulfonic acids (e.g. Borrespers-types, Borregaard), phenolsulfonic acids, naphthalenesulfonic acids (Morwet types, Akzo Nobel) and dibutylnaphthalenesulfonic acid (Nekal types, BASF SE), and of fatty acids, alkyl- and alkylarylsulfonates, alkyl sulfates, lauryl ether sulfates and fatty alcohol sulfates, and salts of sulfated hexa-, hepta- and octadecanols, and also of fatty alcohol glycol ethers, condensates of sulfonated naphthalene and its derivatives with formaldehyde, condensates of naphthalene or of the naphthalenesulfonic acids with phenol and formaldehyde, polyoxyethylene octylphenol ether, ethoxylated isooctyl-, octyl- or nonylphenol, alkylphenyl or tributylphenyl polyglycol ether, alkylaryl polyether alcohols, isotridecyl alcohol, fatty alcohol/ethylene oxide condensates, ethoxylated castor oil, polyoxyethylene alkyl ethers or polyoxypropylene alkyl ethers, lauryl alcohol polyglycol ether acetate, sorbitol esters, lignosulfite waste liquors and proteins, denatured proteins, polysaccharides (e.g. methylcellulose), hydrophobically modified starches, polyvinyl alcohol (Mowiol types Clariant), polycarboxylates (BASF SE, Sokalan types), polyalkoxylates, polyvinylamine (BASF SE, Lupamine types), polyethyleneimine (BASF SE, Lupasol types), polyvinylpyrrolidone and copolymers thereof.

Powders, materials for broadcasting and dusts can be prepared by mixing or grinding the active ingredients together with a solid carrier.

Granules, for example coated granules, impregnated granules and homogeneous granules, can be prepared by binding the active ingredients to solid carriers.

Aqueous use forms can be prepared from emulsion concentrates, suspensions, pastes, wettable powders or water-dispersible granules by adding water. To prepare emulsions, pastes or oil dispersions, the compounds of formula I or la, either as such or dissolved in an oil or solvent, can be homogenized in water by means of a wetting agent, tackifier, dispersant or emulsifier. Alternatively, it is also possible to prepare concentrates comprising active substance, wetting agent, tackifier, dispersant or emulsifier and, if desired, solvent or oil, which are suitable for dilution with water.

The concentrations of the compounds of formula I in the ready-to-use preparations can be varied within wide ranges. In general, the formulations comprise from 0.001 to 98% by weight, preferably 0.01 to 95% by weight of at least one active compound. The active compounds are employed in a purity of from 90% to 100%, preferably 95% to 100% (according to NMR spectrum).

The formulations or ready-to-use preparations may also comprise acids, bases or buffer systems, suitable examples being phosphoric acid or sulfuric acid, or urea or ammonia.

The compounds of formula I of the invention can for example be formulated as follows:

### 1. Products for dilution with water

### A. Water-soluble concentrates

10 parts by weight of active compound are dissolved in 90 parts by weight of water or a water-soluble solvent. As an alternative, wetters or other adjuvants are added. The active compound dissolves upon dilution with water. This gives a formulation with an active compound content of 10% by weight.

### B. Dispersible concentrates

20 parts by weight of active compound are dissolved in 70 parts by weight of cyclohexanone with addition of 10 parts by weight of a dispersant, for example polyvinylpyrrolidone. Dilution with water gives a dispersion. The active compound content is 20% by weight.

### C. Emulsifiable concentrates

15 parts by weight of active compound are dissolved in 75 parts by weight of an organic solvent (e.g. alkylaromatics) with addition of calcium dodecylbenzenesulfonate and castor oil ethoxylate (in each case 5 parts by weight). Dilution with water gives an emulsion. The formulation has an active compound content of 15% by weight.

### D. Emulsions

25 parts by weight of active compound are dissolved in 35 parts by weight of an organic solvent (e.g. alkylaromatics) with addition of calcium dodecylbenzenesulfonate and castor oil ethoxylate (in each case 5 parts by weight). This mixture is introduced into 30 parts by weight of water by means of an emulsifier (e.g. Ultraturrax) and made into a homogeneous emulsion. Dilution with water gives an emulsion. The formulation has an active compound content of 25% by weight.

### E. Suspensions

In an agitated ball mill, 20 parts by weight of active compound are comminuted with addition of 10 parts by weight of dispersants and wetters and 70 parts by weight of water or an organic solvent to give a fine active compound suspension. Dilution with water gives a stable suspension of the active compound. The active compound content in the formulation is 20% by weight.

### F. Water-dispersible granules and water-soluble granules

50 parts by weight of active compound are ground finely with addition of 50 parts by weight of dispersants and wetters and made into water-dispersible or water-soluble granules by means of technical appliances (for example extrusion, spray tower, fluidized bed). Dilution with water gives a stable dispersion or solution of the active compound. The formulation has an active compound content of 50% by weight.

### G. Water-dispersible powders and water-soluble powders

75 parts by weight of active compound are ground in a rotor-stator mill with addition of 25 parts by weight of dispersants, wetters and silica gel. Dilution with water gives a stable dispersion or solution of the active compound. The active compound content of the formulation is 75% by weight.

### H. Gel formulations

In a ball mill, 20 parts by weight of active compound, 10 parts by weight of dispersant, 1 part by weight of gelling agent and 70 parts by weight of water or of an organic solvent are ground to give a fine suspension. Dilution with water gives a stable suspension with active compound content of 20% by weight.

### 2. Products to be applied undiluted

### I . Dusts

5 parts by weight of active compound are ground finely and mixed intimately with 95 parts by weight of finely divided kaolin. This gives a dusting powder with an active compound content of 5% by weight.

### J. Granules (GR, FG, GG, MG)

0.5 parts by weight of active compound are ground finely and associated with 99.5 parts by weight of carriers. Current methods here are extrusion, spray-drying or the fluidized bed. This gives granules to be applied undiluted with an active compound content of 0.5% by weight.

### K. ULV solutions (UL)

10 parts by weight of active compound are dissolved in 90 parts by weight of an organic solvent, for example xylene. This gives a product to be applied undiluted with an active compound content of 10% by weight.

The compounds of formula I or the herbicidal compositions comprising them can be applied pre- or post-emergence, or together with the seed of a crop plant. It is also possible to apply the herbicidal compositions or active compounds by applying seed, pretreated with the herbicidal compositions or active compounds, of a crop plant. If the active compounds are less well tolerated by certain crop plants, application techniques may be used in which the herbicidal compositions are sprayed, with the aid of the spraying equipment, in such a way that as far as possible they do not come into contact with the leaves of the sensitive crop plants, while the active compounds reach the leaves of undesirable plants growing underneath, or the bare soil surface (post-directed, lay-by).

In a further embodiment, the compounds of formula I or the herbicidal compositions can be applied by treating seed.

The treatment of seed comprises essentially all procedures familiar to the person skilled in the art (seed dressing, seed coating, seed dusting, seed soaking, seed film coating, seed multilayer coating, seed encrusting, seed dripping and seed pelleting) based on the compounds of formula I according to the invention or the compositions prepared therefrom. Here, the herbicidal compositions can be applied diluted or undiluted.

The term seed comprises seed of all types, such as, for example, corns, seeds, fruits, tubers, cuttings and similar forms. Here, preferably, the term seed describes corns and seeds.

The seed used can be seed of the useful plants mentioned above, but also the seed of transgenic plants or plants obtained by customary breeding methods.

The rates of application of active compound are from 0.001 to 3.0, preferably 0.01 to 1.0, kg/ha of active substance (a.s.), depending on the control target, the season, the target plants and the growth stage. To treat the seed, the compounds of formula I are generally employed in amounts of from 0.001 to 10 kg per 100 kg of seed.

It may also be advantageous to use the compounds of formula I in combination with safeners, also termed herbicide safeners. Safeners are chemical compounds which prevent or reduce damage to useful plants without substantially affecting the herbicidal action of the compounds of formula I on unwanted plants. They can be used both before sowing (for example in the treatment of seed, or on cuttings or seedlings) and before or after the emergence of the useful plant. The safeners and the compounds of formula I can be used simultaneously or in succession.

Suitable safeners are, for example, (quinolin-8-oxy)acetic acids, 1-phenyl-5-haloalkyl-1*H-*1,2,4-triazole-3-carboxylic acids, 1-phenyl-4,5-dihydro-5-alkyl-1*H*-pyrazole-3,5-dicarboxylic acids, 4,5-dihydro-5,5-diaryl-3-isoxazolecarboxylic acids, dichloroacetamides, alpha-oximinophenylacetonitriles, acetophenone oximes, 4,6-dihalo-2-phenylpyrimidines, N-[[4-(aminocarbonyl)phenyl]sulfonyl]-2-benzamides, 1,8-naphthalic anhydride, 2-halo-4-(haloalkyl)-5-thiazolecarboxylic acids, phosphorothiolates and O-phenyl N-alkylcarbamates and their agriculturally useful salts and, provided that they have an acid function, their agriculturally useful derivatives, such as amides, esters and thioesters.

To broaden the activity spectrum and to obtain synergistic effects, the compounds of the formula I can be mixed and jointly applied with numerous representatives of other compounds having herbicidal activity (herbicides B) or growth-regulating activitiy, optionally in combination with safeners. Suitable mixing partners are, for example, 1,2,4-thiadiazoles, 1,3,4-thiadiazoles, amides, aminophosphoric acid and its derivatives, aminotriazoles, anilides, aryloxy/heteroaryl-oxyalkanoic acids and their derivatives, benzoic acid and its derivatives, benzothiadiazinones, 2-(hetaroyl/aroyl)-1,3-cyclohexanediones, heteroaryl aryl ketones, benzylisoxazolidinones, meta-CF₃-phenyl derivatives, carbamates, quinoline carboxylic acid and its derivatives, chloroacetanilides, cyclohexenone oxime ether derivates, diazines, dichloropropionic acid and its derivatives, dihydrobenzofurans, dihydrofuran-3-ones, dinitroanilines, dinitrophenols, diphenyl ethers, dipyridyls, halocarboxylic acids and their derivatives, ureas, 3-phenyluracils, imidazoles, imidazolinones, N-phenyl-3,4,5,6-tetrahydrophthalimides, oxadiazoles, oxiranes, phenols, aryloxy- and heteroaryloxyphenoxypropionic esters, phenylacetic acid and its derivatives, 2-phenylpropionic acid and its derivatives, pyrazoles, phenylpyrazoles, pyridazines, pyridine-carboxylic acid and its derivatives, pyrimidyl ethers, sulfonamides, sulfonylureas, triazines, triazinones, triazolinones, triazolecarboxamides, uracils and also phenylpyrazolines and isoxazolines and their derivatives.

Moreover, it may be useful to apply the compounds of formula I alone or in combination with other herbicides B or else also mixed with further crop protection agents, jointly, for example with compositions for controlling pests or phytopathogenic fungi or bacteria. Also of interest is the miscibility with mineral salt solutions which are employed for alleviating nutritional and trace element deficiencies. Other additives such as nonphytotoxic oils and oil concentrates may also be added.

Examples of herbicides B which can be used in combination with the benzamide compounds of formula I according to the present invention are:
b1) from the group of the lipid biosynthesis inhibitors:
   alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl-[1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1 '-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5), benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate and vernolate;
b2) from the group of the ALS inhibitors:
   amidosulfuron, azimsulfuron, bensulfuron, bensulfuron-methyl, bispyribac, bispyribac-sodium, chlorimuron, chlorimuron-ethyl, chlorsulfuron, cinosulfuron, cloransulam, cloransulam-methyl, cyclosulfamuron, diclosulam, ethametsulfuron, ethametsulfuron-methyl, ethoxysulfuron, flazasulfuron, florasulam, flucarbazone, flucarbazone-sodium, flucetosulfuron, flumetsulam, flupyrsulfuron, flupyrsulfuron-methyl-sodium, foramsulfuron, halosulfuron, halosulfuron-methyl, imazamethabenz, imazamethabenz-methyl, imazamox, imazapic, imazapyr, imazaquin, imazethapyr, imazosulfuron, iodosulfuron, iodosulfuron-methyl-sodium, mesosulfuron, metosulam, metsulfuron, metsulfuron-methyl, nicosulfuron, orthosulfamuron, oxasulfuron, penoxsulam, primisulfuron, primisulfuron-methyl, propoxycarbazone, propoxycarbazone-sodium, prosulfuron, pyrazosulfuron, pyrazosulfuron-ethyl, pyribenzoxim, pyrimisulfan, pyriftalid, pyriminobac, pyriminobac-methyl, pyrithiobac, pyrithiobac-sodium, pyroxsulam, rimsulfuron, sulfometuron, sulfometuron-methyl, sulfosulfuron, thiencarbazone, thiencarbazone-methyl, thifensulfuron, thifensulfuron-methyl, triasulfuron, tribenuron, tribenuron-methyl, trifloxysulfuron, triflusulfuron, triflusulfuron-methyl and tritosulfuron;
b3) from the group of the photosynthesis inhibitors:
   ametryn, amicarbazone, atrazine, bentazone, bentazone-sodium, bromacil, bromofenoxim, bromoxynil and its salts and esters, chlorobromuron, chloridazone, chlorotoluron, chloroxuron, cyanazine, desmedipham, desmetryn, dimefuron, dimethametryn, diquat, diquatdibromide, diuron, fluometuron, hexazinone, ioxynil and its salts and esters, isoproturon, isouron, karbutilate, lenacil, linuron, metamitron, methabenzthiazuron, metobenzuron, metoxuron, metribuzin, monolinuron, neburon, paraquat, paraquat-dichloride, paraquat-dimetilsulfate, pentanochlor, phenmedipham, phenmedipham-ethyl, prometon, prometryn, propanil, propazine, pyridafol, pyridate, siduron, simazine, simetryn, tebuthiuron, terbacil, terbumeton, terbuthylazine, terbutryn, thidiazuron, trietazine, 1-(6-tert-butylpyrimidin-4-yl)-2-hydroxy-4-methoxy-3-methyl-2H-pyrrol-5-one (CAS 1654744-66-7), 1-(5-tert-butylisoxazol-3-yl)-2-hydroxy-4-methoxy-3-methyl-2H-pyrrol-5-one (CAS 1637455-12-9), 1-(5-tert-butylisoxazol-3-yl)-4-chloro-2-hydroxy-3-methyl-2H-pyrrol-5-one (CAS 1637453-94-1), 1-(5-tert-butyl-1-methyl-pyrazol-3-yl)-4-chloro-2-hydroxy-3-methyl-2H-pyrrol-5-one (CAS 1654057-29-0), 1-(5-tert-butyl-1-methyl-pyrazol-3-yl)-3-chloro-2-hydroxy-4-methyl-2H-pyrrol-5-one (CAS 1654747-80-4), 4-hydroxy-1-methoxy-5-methyl-3-[4-(trifluoromethyl)-2-pyridyl]imidazolidin-2-one; (CAS 2023785-78-4), 4-hydroxy-1,5-dimethyl-3-[4-(trifluoromethyl)-2-pyridyl]imidazolidin-2-one (CAS 2023785-79-5), 5-ethoxy-4-hydroxy-1-methyl-3-[4-(trifluoromethyl)-2-pyridyl]imidazolidin-2-one (CAS 1701416-69-4), 4-hydroxy-1-methyl-3-[4-(trifluoromethyl)-2-pyridyl]imidazolidin-2-one (CAS 1708087-22-2), 4-hydroxy-1,5-dimethyl-3-[1-methyl-5-(trifluoromethyl)pyrazol-3-yl]imidazolidin-2-one (CAS 2023785-80-8) and 1-(5-tert-butylisoxazol-3-yl)-4-ethoxy-5-hydroxy-3-methyl-imidazolidin-2-one (CAS 1844836-64-1);
b4) from the group of the protoporphyrinogen-IX oxidase inhibitors:
   acifluorfen, acifluorfen-sodium, azafenidin, bencarbazone, benzfendizone, bifenox, butafenacil, carfentrazone, carfentrazone-ethyl, chlomethoxyfen, cinidon-ethyl, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, 2-chloro-5-[3,6-dihydro-3-methyl-2,6-dioxo-4-(trifluoromethyl)-1(2*H*)-pyrimidinyl]-4-fluoro-N-[(isopropyl)methylsulfamoyl]benzamide (H-1; CAS 372137-35-4), ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (H-2; CAS 353292-31-6), N-ethyl-3-(2,6-dichloro-4-trifluoro-methylphenoxy)-5-methyl-1*H*-pyrazole-1-carboxamide (H-3; CAS 452098-92-9), N-tetrahydrofurfuryl-3-(2,6-dichloro-4-trifluoromethylphenoxy)-5-methyl-1*H*-pyrazole-1-carboxamide (H-4; CAS 915396-43-9), N-ethyl-3-(2-chloro-6-fluoro-4-trifluoromethylphenoxy)-5-methyl-1*H*-pyrazole-1-carboxamide (H-5; CAS 452099-05-7), N-tetrahydrofurfuryl-3-(2-chloro-6-fluoro-4-trifluoromethylphenoxy)-5-methyl-1*H*-pyrazole-1-carboxamide (H-6; CAS 45100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7), 1,5-dimethyl-6-thioxo-3-(2,2,7-trifluoro-3-oxo-4-(prop-2-ynyl)-3,4-dihydro-2H-benzo[b][1,4]oxazin-6-yl)-1,3,5-triazinane-2,4-dione (trifludimoxazin), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0), 1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0), methyl (*E*)-4-[2-chloro-5-[4-chloro-5-(difluoromethoxy)-1*H*-methyl-pyrazol-3-yl]-4-fluoro-phenoxy]-3-methoxy-but-2-enoate (CAS 948893-00-3), and 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4);
b5) from the group of the bleacher herbicides:
   aclonifen, amitrol, beflubutamid, benzobicyclon, benzofenap, clomazone, diflufenican, fenquinotrione, flumeturon, fluridone, flurochloridone, flurtamone, isoxaflutole, mesotrione, norflurazon, oxotrione (CAS 1486617-21-3), picolinafen, pyrasulfotole, pyrazolynate, pyrazoxyfen, sulcotrione, tefuryltrione, tembotrione, tolpyralate, topramezone, 4-hydroxy-3-[[2-[(2-methoxyethoxy)methyl]-6-(trifluoromethyl)-3-pyridyl]carbonyl]bicyclo[3.2.1]oct-3-en-2-one (H-7; CAS 352010-68-5, bicyclopyrone), 4-(3-trifluoromethylphenoxy)-2-(4-trifluoromethylphenyl)-pyrimidine (H-8; CAS 180608-33-7) -chloro-3-methylsulfanyl-N-(1-methyltetrazol-5-yl)-4-(trifluoromethyl)benzamide (CAS 1361139-71-0), 2-(2,4-dichlorophenyl)methyl-4,4-dimethyl-3-isoxazolidone (CAS 81777-95-9) and 2-(2,5-dichlorophenyl)methyl-4,4-dimethyl-3-isoxazolidinone (CAS 81778-66-7);
b6) from the group of the EPSP synthase inhibitors:
   glyphosate, glyphosate-isopropylammonium and glyphosate-trimesium (sulfosate);
b7) from the group of the glutamine synthase inhibitors:
   bilanaphos (bialaphos), bilanaphos-sodium, glufosinate and glufosinate-ammonium;
b8) from the group of the DHP synthase inhibitors:
   asulam;
b9) from the group of the mitose inhibitors:
   amiprophos, amiprophos-methyl, benfluralin, butamiphos, butralin, carbetamide, chlorpropham, chlorthal, chlorthal-dimethyl, dinitramine, dithiopyr, ethalfluralin, fluchloralin, oryzalin, pendimethalin, prodiamine, propham, propyzamide, tebutam, thiazopyr and trifluralin;
b10) from the group of the VLCFA inhibitors:
   acetochlor, alachlor, anilofos, butachlor, cafenstrole, dimethachlor, dimethanamid, dimethenamid-P, diphenamid, fentrazamide, flufenacet, mefenacet, metazachlor, metolachlor, metolachlor-S, naproanilide, napropamide, pethoxamid, piperophos, pretilachlor, propachlor, propisochlor, pyroxasulfone (KIH-485) and thenylchlor;

Compounds of the formula 2: 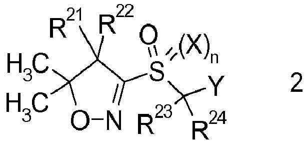 in which the variables have the following meanings:
Y is phenyl or 5- or 6-membered heteroaryl as defined at the outset, which radicals may be substituted by one to three groups R^{aa}; R²¹, R²², R²³, R²⁴ are H, halogen or C₁-C₄-alkyl; X is O or NH; N is 0 or 1.

Compounds of the formula 2 have in particular the following meanings: 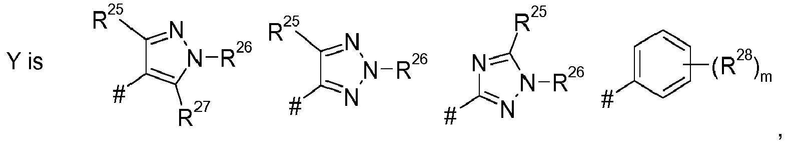
where # denotes the bond to the skeleton of the molecule; and
R²¹, R²², R²³, R²⁴ are H, Cl, F or CH₃; R²⁵ is halogen, C₁-C₄-alkyl or C₁-C₄-haloalkyl; R²⁶ is C₁-C₄-alkyl; R²⁷ is halogen, C₁-C₄-alkoxy or C₁-C₄-haloalkoxy; R²⁸ is H, halogen, C₁-C₄-alkyl, C₁-C₄-haloalkyl or C₁-C₄-haloalkoxy; M is 0, 1, 2 or 3; X is oxygen; N is 0 or 1.

Preferred compounds of the formula 2 have the following meanings: 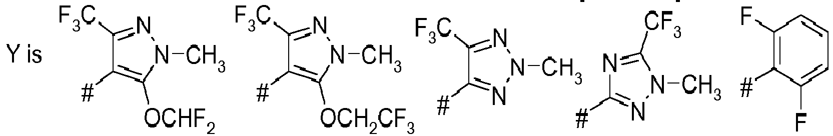 R²¹ is H; R²², R²³ are F; R²⁴ is H or F; X is oxygen; N is 0 or 1.

Particularly preferred compounds of the formula 2 are:
3-[5-(2,2-difluoroethoxy)-1-methyl-3-trifluoromethyl-1H-pyrazol-4-ylmethanesulfonyl]-4-fluoro-5,5-dimethyl-4,5-dihydroisoxazole (2-1); 3-{[5-(2,2-difluoroethoxy)-1-methyl-3-trifluoromethyl-1H-pyrazol-4-yl]fluoromethanesulfonyl}-5,5-dimethyl-4,5-dihydroisoxazole (2-2); 4-(4-fluoro-5,5-dimethyl-4,5-dihydroisoxazole-3-sulfonylmethyl)-2-methyl-5-trifluoromethyl-2H-[1,2,3]triazole (2-3); 4-[(5,5-dimethyl-4,5-dihydroisoxazole-3-sulfonyl)fluoromethyl]-2-methyl-5-trifluoromethyl-2H-[1,2,3]triazole (2-4); 4-(5,5-dimethyl-4,5-dihydroisoxazole-3-sulfonylmethyl)-2-methyl-5-trifluoromethyl-2H-[1,2,3]triazole (2-5); 3-{[5-(2,2-difluoroethoxy)-1-methyl-3-trifluoromethyl-1H-pyrazol-4-yl]difluoromethanesulfonyl}-5,5-dimethyl-4,5-dihydroisoxazole (2-6); 4-[(5,5-dimethyl-4,5-dihydroisoxazole-3-sulfonyl)difluoromethyl]-2-methyl-5-trifluoromethyl-2H-[1,2,3]triazole (2-7); 3-{[5-(2,2-difluoroethoxy)-1-methyl-3-trifluoromethyl-1H-pyrazol-4-yl]difluoromethanesulfonyl}-4-fluoro-5,5-dimethyl-4,5-dihydroisoxazole (2-8); 4-[difluoro-(4-fluoro-5,5-dimethyl-4,5-dihydroisoxazole-3-sulfonyl)methyl]-2-methyl-5-trifluoromethyl-2H-[1,2,3]triazole (2-9);
   b11) from the group of the cellulose biosynthesis inhibitors:
      chlorthiamid, dichlobenil, flupoxam and isoxaben;
   b12) from the group of the decoupler herbicides:
      dinoseb, dinoterb and DNOC and its salts;
   b13) from the group of the auxin herbicides:
      2,4-D and its salts and esters, 2,4-DB and its salts and esters, aminopyralid and its salts such as aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, fluroxypyr, fluroxypyr-butometyl, fluroxypyr-meptyl, halauxifen and its salts and esters, MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, 5,6-dichloro-2-cyclopropyl-4-pyrimidinecarboxylic acid (H-9; CAS 858956-08-8) and its salts and esters, florpyrauxifen, florpyrauxifen-benzyl (CAS 1390661-72-9) and 4-amino-3-chloro-5-fluoro-6-(7-fluoro-1H-indol-6-yl)picolinic acid (CAS 1629965-65-6);
   b14) from the group of the auxin transport inhibitors: diflufenzopyr, diflufenzopyr-sodium, naptalam and naptalam-sodium;
   b15) from the group of the other herbicides: bromobutide, chlorflurenol, chlorflurenolmethyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, maleic hydrazide, mefluidide, metam, methyl azide, methyl bromide, methyl-dymron, methyl iodide, MSMA, oleic acid, oxaziclomefone, pelargonic acid, pyributicarb, quinoclamine, triaziflam, tridiphane and 6-chloro-3-(2-cyclopropyl-6-methylphenoxy)-4-pyridazinol (H-10; CAS 499223-49-3) and its salts and esters.

Examples of preferred safeners C are benoxacor, cloquintocet, cyometrinil, cyprosulfamide, dichlormid, dicyclonone, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (H-11; MON4660, CAS 71526-07-3) and 2,2,5-trimethyl-3-(dichloroacetyl)-1,3-oxazolidine (H-12; R-29148, CAS 52836-31-4).

The active compounds of groups b1) to b15) and the safeners C are known herbicides and safeners, see, for example, The Compendium of Pesticide Common Names (http://www.alanwood.net/pesticides/); B. Hock, C. Fedtke, R. R. Schmidt, Herbizide [Herbicides], Georg Thieme Verlag, Stuttgart, 1995. Further herbicidally active compounds are known from WO 96/26202, WO 97/41116, WO 97/41117, WO 97/41118, WO 01/83459 and WO 2008/074991 and from W. Kramer et al. (ed.) "Modern Crop Protection Compounds", Vol. 1, Wiley VCH, 2007 and the literature quoted therein.

The invention also relates to combinations comprising at least one benzamide compound of the formula I and at least one further active compound, in particular a compound having herbicide activity (herbicide B) preferably selected from the active compounds of groups b1 to b15, and/or a safener C.

The invention also relates to compositions in the form of a crop protection composition formulated as a 1-component composition comprising an active compound combination comprising at least one benzamide compound of the formula I and at least one further active compound, in particular a compound having herbicide activity (herbicide B) preferably selected from the active compounds of groups b1 to b15, and at least one solid or liquid carrier and/or one or more surfactants and, if desired, one or more further auxiliaries customary for crop protection compositions.

The invention also relates to compositions in the form of a crop protection composition formulated as a 1-component composition comprising an active compound combination comprising at least one benzamide compound of the formula I and at least one safener C and at least one solid or liquid carrier and/or one or more surfactants and, if desired, one or more further auxiliaries customary for crop protection compositions.

The invention also relates to compositions in the form of a crop protection composition formulated as a 1-component composition comprising an active compound combination comprising at least one benzamide compound of the formula I and at least one further active compound, in particular a compound having herbicide activity (herbicide B) which is preferably selected from the active compounds of groups b1 to b15, a safener C and at least one solid or liquid carrier and/or one or more surfactants and, if desired, one or more further auxiliaries customary for crop protection compositions.

The invention also relates to compositions in the form of a crop protection composition formulated as a 2-component composition comprising a first component comprising at least one compound of the formula I, a solid or liquid carrier and/or one or more surfactants and a second component comprising at least one further active compound, in particular a compound having herbicide activity (herbicide B) which is preferably selected from the active compounds of groups b1 to b15, a solid or liquid carrier and/or one or more surfactants, where additionally both components may also comprise further auxiliaries customary for crop protection compositions.

The invention also relates to compositions in the form of a crop protection composition formulated as a 2-component composition comprising a first component comprising at least one compound of the formula I, a solid or liquid carrier and/or one or more surfactants and a second component comprising at least one further active compound, in particular a compound having herbicide activity (herbicide B) which is preferably selected from the active compounds of groups b1 to b15, a solid or liquid carrier and/or one or more surfactants, where additionally both components may also comprise further auxiliaries customary for crop protection compositions, where the first component or the second component further comprises a safener C.

In binary compositions comprising at least one compound of the formula I as component A and at least one herbicide B, the weight ratio of the active compounds A:B is generally in the range of from 1:1000 to 1000:1, preferably in the range of from 1:500 to 500:1, in particular in the range of from 1:250 to 250:1 and particularly preferably in the range of from 1:75 to 75:1.

In binary compositions comprising at least one compound of the formula I as component A and at least one safener C, the weight ratio of the active compounds A:C is generally in the range of from 1:1000 to 1000:1, preferably in the range of from 1:500 to 500:1, in particular in the range of from 1:250 to 250:1 and particularly preferably in the range of from 1:75 to 75:1.

In ternary compositions comprising both at least one compound of the formula I as component A, at least one herbicide B and at least one safener C, the relative parts by weight of the components A:B are generally in the range of from 1:1000 to 1000:1, preferably in the range of from 1:500 to 500:1, in particular in the range of from 1:250 to 250:1 and particularly preferably in the range of from 1:75 to 75:1; the weight ratio of the components A:C is generally in the range of from 1:1000 to 1000:1, preferably in the range of from 1:500 to 500:1, in particular in the range of from 1:250 to 250:1 and particularly preferably in the range of from 1:75 to 75:1; and the weight ratio of the components B:C is generally in the range of from 1:1000 to 1000:1, preferably in the range of from 1:500 to 500:1, in particular in the range of from 1:250 to 250:1 and particularly preferably in the range of from 1:75 to 75:1. Preferably, the weight ratio of the components A + B to the component C is in the range of from 1:500 to 500:1, in particular in the range of from 1:250 to 250:1 and particularly preferably in the range of from 1:75 to 75:1.

Examples of particularly preferred compositions according to the invention comprising in each case one individualized compound of the formula I and one mixing partner or a mixing partner combination are given in Table B below.

A further aspect of the invention relates to the combinations B-1 to B-1406 listed in Table B below, where in each case one row of Table B corresponds to a herbicidal composition comprising one of the compounds of formula I individualized in the above description (component 1) and the further active compound from groups b1) to b15) and/or safener C stated in each case in the row in question (component 2). The active compounds in the combinations described are in each case preferably present in synergistically effective amounts.

Amongst these compositions B-1 to B-1406 a particular group of embodimemts relates to combinations B-1.1 to B-1406.1, where the compound of formulal (I) is 4-bromo-6-fluoro-2-methyl-N-(1-methyltetrazol-5-yl)-3-[[methyl(2,2,2-trifluoroethyl)carbamoyl]amino]benzamide and where the further active compound from groups b1) to b15) and/or safener C stated in each case in the row in question.

Amongst these compositions B-1 to B-1406 another particular group of embodimemts relates to combinations B-1.2 to B-1406.2, where the compound of formulal (I) is 4-bromo-6-fluoro-2-methyl-N-(1-methyltetrazol-5-yl)-3-[[ethyl(2,2,2-trifluoroethyl)carbamoyl]-amino]benzamide and where the further active compound from groups b1) to b15) and/or safener C stated in each case in the row in question.

Amongst these compositions B-1 to B-1406 a further particular group of embodimemts relates to combinations B-1.3 to B-1406.3, where the compound of formulal (I) is 2,4-dichloro-6-fluoro-N-(1-methyltetrazol-5-yl)-3-[[methyl(2,2,2-trifluoroethyl)carbamoyl]amino]benzamide and where the further active compound from groups b1) to b15) and/or safener C stated in each case in the row in question.

**Table B:**

| | | |
|---|---|---|
| | Herbicide(s) B | Safener C |
| B-1 | clodinafop-propargyl | -- |
| B-2 | cycloxydim | -- |
| B-3 | cyhalofop-butyl | -- |
| B-4 | fenoxaprop-P-ethyl | -- |
| B-5 | pinoxaden | -- |
| B-6 | profoxydim | -- |
| B-7 | tepraloxydim | -- |
| B-8 | tralkoxydim | -- |
| B-9 | esprocarb | -- |
| B-10 | prosulfocarb | -- |
| B-11 | thiobencarb | -- |
| B-12 | triallate | -- |
| B-13 | bensulfuron-methyl | -- |
| B-14 | bispyribac-sodium | -- |
| B-15 | cyclosulfamuron | -- |
| B-16 | flumetsulam | -- |
| B-17 | flupyrsulfuron-methyl-sodium | -- |
| B-18 | foramsulfuron | -- |
| B-19 | imazamox | -- |
| B-20 | imazapic | -- |
| B-21 | imazapyr | -- |
| B-22 | imazaquin | -- |
| B-23 | imazethapyr | -- |
| B-24 | imazosulfuron | -- |
| B-25 | iodosulfuron-methyl-sodium | -- |
| B-26 | mesosulfuron | -- |
| B-27 | nicosulfuron | -- |
| B-28 | penoxsulam | -- |
| B-29 | propoxycarbazone-sodium | -- |
| B-30 | pyrazosulfuron-ethyl | -- |
| B-31 | pyroxsulam | -- |
| B-32 | rimsulfuron | -- |
| B-33 | sulfosulfuron | -- |
| B-34 | thiencarbazone-methyl | -- |

| | Herbicide(s) B | Safener C |
|---|---|---|
| B-35 | tritosulfuron | -- |
| B-36 | 2,4-D and its salts and esters | -- |
| B-37 | aminopyralid and its salts and esters | -- |
| B-38 | clopyralid and its salts and esters | -- |
| B-39 | dicamba and its salts and esters | -- |
| B-40 | fluroxypyr-meptyl | -- |
| B-41 | quinclorac | -- |
| B-42 | quinmerac | -- |
| B-43 | H-9 | -- |
| B-44 | diflufenzopyr | -- |
| B-45 | diflufenzopyr-sodium | -- |
| B-46 | clomazone | -- |
| B-47 | diflufenican | -- |
| B-48 | fluorochloridone | -- |
| B-49 | isoxaflutol | -- |
| B-50 | mesotrione | -- |
| B-51 | picolinafen | -- |
| B-52 | sulcotrione | -- |
| B-53 | tefuryltrione | -- |
| B-54 | tembotrione | -- |
| B-55 | topramezone | -- |
| B-56 | H-7 | -- |
| B-57 | atrazine | -- |
| B-58 | diuron | -- |
| B-59 | fluometuron | -- |
| B-60 | hexazinone | -- |
| B-61 | isoproturon | -- |
| B-62 | metribuzin | -- |
| B-63 | propanil | -- |
| B-64 | terbuthylazine | -- |
| B-65 | paraquat dichloride | -- |
| B-66 | flumioxazin | -- |
| B-67 | oxyfluorfen | -- |
| B-68 | saflufenacil | -- |
| B-69 | sulfentrazone | -- |
| B-70 | H-1 | -- |
| B-71 | H-2 | -- |
| B-72 | glyphosate | -- |
| B-73 | glyphosate-isopropylammonium | -- |
| B-74 | glyphosate-trimesium (sulfosate) | -- |
| B-75 | glufosinate | -- |
| B-76 | glufosinate-ammonium | -- |
| B-77 | pendimethalin | -- |
| B-78 | trifluralin | -- |
| B-79 | acetochlor | -- |
| B-80 | cafenstrole | -- |
| B-81 | dimethenamid-P | -- |
| B-82 | fentrazamide | -- |
| B-83 | flufenacet | -- |
| B-84 | mefenacet | -- |
| B-85 | metazachlor | -- |
| B-86 | metolachlor-S | -- |
| B-87 | pyroxasulfone | -- |
| B-88 | isoxaben | -- |
| B-89 | dymron | -- |
| B-90 | indanofan | -- |
| B-91 | oxaziclomefone | -- |
| B-92 | triaziflam | -- |
| B-93 | chlorotoluron | -- |
| B-94 | pinoxaden | -- |
| B-95 | sethoxydim | -- |
| B-96 | clethodim | -- |
| B-97 | diclofop | -- |
| B-98 | quizalofop | -- |
| B-99 | thifensulfuron | -- |
| B-100 | tribenuron | -- |
| B-101 | metsulfuron | -- |
| B-102 | foramsulfuron | -- |
| B-103 | chlorimuron | -- |
| B-104 | chlorsulfuron | -- |
| B-105 | flucarbazone-sodium | -- |
| B-106 | propoxycarbazone-sodium | -- |
| B-107 | ethalfluralin | -- |
| B-108 | halauxifen | -- |
| B-109 | MCPA | -- |
| B-110 | bromoxynil | -- |
| B-111 | bentazone | -- |
| B-112 | carfentrazone | -- |
| B-113 | trifludimoxazin | -- |
| B-114 | bicyclopyrone | -- |
| B-115 | benzobicyclon | -- |
| B-116 | pyrasulfotole | -- |
| B-117 | diquat | -- |
| B-118 | cinmethylin | -- |
| B-119 | acetochlor | -- |
| B-120 | naptalam | -- |
| B-121 | atrazine + H-1 | -- |
| B-122 | atrazine + glyphosate | -- |
| B-123 | atrazine + mesotrione | -- |
| B-124 | atrazine + nicosulfuron | -- |
| B-125 | atrazine + tembotrione | -- |
| B-126 | atrazine + topramezone | -- |
| B-127 | clomazone + glyphosate | -- |
| B-128 | diflufenican + clodinafop-propargyl | -- |
| B-129 | diflufenican + fenoxaprop-P-ethyl | -- |
| B-130 | diflufenican + flupyrsulfuron-methyl-sodium | -- |
| B-131 | diflufenican + glyphosate | -- |
| B-132 | diflufenican + mesosulfuron-methyl | -- |
| B-133 | diflufenican + pinoxaden | -- |
| B-134 | diflufenican + pyroxsulam | -- |
| B-135 | flumetsulam + glyphosate | -- |
| B-136 | flumioxazin + glyphosate | -- |
| B-137 | imazapic + glyphosate | -- |
| B-138 | imazethapyr + glyphosate | -- |
| B-139 | isoxaflutol + H-1 | -- |
| B-140 | isoxaflutol + glyphosate | -- |
| B-141 | metazachlor + H-1 | -- |
| B-142 | metazachlor + glyphosate | -- |
| B-143 | metazachlor + mesotrione | -- |
| B-144 | metazachlor + nicosulfuron | -- |
| B-145 | metazachlor + terbuthylazine | -- |
| B-146 | metazachlor + topramezone | -- |
| B-147 | metribuzin + glyphosate | -- |
| B-148 | pendimethalin + H-1 | -- |
| B-149 | pendimethalin + clodinafop-propargyl | -- |
| B-150 | pendimethalin + fenoxaprop-P-ethyl | -- |
| B-151 | pendimethalin + flupyrsulfuron-methyl-sodium | -- |
| B-152 | pendimethalin + glyphosate | -- |
| B-153 | pendimethalin + mesosulfuron-methyl | -- |
| B-154 | pendimethalin + mesotrione | -- |
| B-155 | pendimethalin + nicosulfuron | -- |
| B-156 | pendimethalin + pinoxaden | -- |
| B-157 | pendimethalin + pyroxsulam | -- |
| B-158 | pendimethalin + tembotrione | -- |
| B-159 | pendimethalin + topramezone | -- |
| B-160 | pyroxasulfone + tembotrione | -- |
| B-161 | pyroxasulfone + topramezone | -- |
| B-162 | sulfentrazone + glyphosate | -- |
| B-163 | terbuthylazine + H-1 | -- |
| B-164 | terbuthylazine + foramsulfuron | -- |
| B-165 | terbuthylazine + glyphosate | -- |
| B-166 | terbuthylazine + mesotrione | -- |
| B-167 | terbuthylazine + nicosulfuron | -- |
| B-168 | terbuthylazine + tembotrione | -- |
| B-169 | terbuthylazine + topramezone | -- |
| B-170 | trifluralin + glyphosate | -- |
| B-171 | -- | benoxacor |
| B-172 | -- | cloquintocet |
| B-173 | -- | cyprosulfamide |
| B-174 | -- | dichlormid |
| B-175 | -- | fenchlorazole |
| B-176 | -- | fenclorim |
| B-177 | -- | isoxadifen |
| B-178 | -- | mefenpyr |
| B-179 | -- | H-11 |
| B-180 | -- | H-12 |
| B-181 | clodinafop-propargyl | benoxacor |
| B-182 | cycloxydim | benoxacor |
| B-183 | cyhalofop-butyl | benoxacor |
| B-184 | fenoxaprop-P-ethyl | benoxacor |
| B-185 | pinoxaden | benoxacor |
| B-186 | profoxydim | benoxacor |
| B-187 | tepraloxydim | benoxacor |
| B-188 | tralkoxydim | benoxacor |
| B-189 | esprocarb | benoxacor |
| B-190 | prosulfocarb | benoxacor |
| B-191 | thiobencarb | benoxacor |
| B-192 | triallate | benoxacor |
| B-193 | bensulfuron-methyl | benoxacor |
| B-194 | bispyribac-sodium | benoxacor |
| B-195 | cyclosulfamuron | benoxacor |
| B-196 | flumetsulam | benoxacor |
| B-197 | flupyrsulfuron-methyl-sodium | benoxacor |
| B-198 | foramsulfuron | benoxacor |
| B-199 | imazamox | benoxacor |
| B-200 | imazapic | benoxacor |
| B-201 | imazapyr | benoxacor |
| B-202 | imazaquin | benoxacor |
| B-203 | imazethapyr | benoxacor |
| B-204 | imazosulfuron | benoxacor |
| B-205 | iodosulfuron-methyl-sodium | benoxacor |
| B-206 | mesosulfuron | benoxacor |
| B-207 | nicosulfuron | benoxacor |
| B-208 | penoxsulam | benoxacor |
| B-209 | propoxycarbazone-sodium | benoxacor |
| B-210 | pyrazosulfuron-ethyl | benoxacor |
| B-211 | pyroxsulam | benoxacor |
| B-212 | rimsulfuron | benoxacor |
| B-213 | sulfosulfuron | benoxacor |
| B-214 | thiencarbazone-methyl | benoxacor |
| B-215 | tritosulfuron | benoxacor |
| B-216 | 2,4-D and its salts and esters | benoxacor |
| B-217 | aminopyralid and its salts and esters | benoxacor |
| B-218 | clopyralid and its salts and esters | benoxacor |
| B-219 | dicamba and its salts and esters | benoxacor |
| B-220 | fluroxypyr-meptyl | benoxacor |
| B-221 | quinclorac | benoxacor |
| B-222 | quinmerac | benoxacor |
| B-223 | H-9 | benoxacor |
| B-224 | diflufenzopyr | benoxacor |
| B-225 | diflufenzopyr-sodium | benoxacor |
| B-226 | clomazone | benoxacor |
| B-227 | diflufenican | benoxacor |
| B-228 | fluorochloridone | benoxacor |
| B-229 | isoxaflutol | benoxacor |
| B-230 | mesotrione | benoxacor |
| B-231 | picolinafen | benoxacor |
| B-232 | sulcotrione | benoxacor |
| B-233 | tefuryltrione | benoxacor |
| B-234 | tembotrione | benoxacor |
| B-235 | topramezone | benoxacor |
| B-236 | H-7 | benoxacor |
| B-237 | atrazine | benoxacor |
| B-238 | diuron | benoxacor |
| B-239 | fluometuron | benoxacor |
| B-240 | hexazinone | benoxacor |
| B-241 | isoproturon | benoxacor |
| B-242 | metribuzin | benoxacor |
| B-243 | propanil | benoxacor |
| B-244 | terbuthylazine | benoxacor |
| B-245 | paraquat dichloride | benoxacor |
| B-246 | flumioxazin | benoxacor |
| B-247 | oxyfluorfen | benoxacor |
| B-248 | saflufenacil | benoxacor |
| B-249 | sulfentrazone | benoxacor |
| B-250 | H-1 | benoxacor |
| B-251 | H-2 | benoxacor |
| B-252 | glyphosate | benoxacor |
| B-253 | glyphosate-isopropylammonium | benoxacor |
| B-254 | glyphosate-trimesium (sulfosate) | benoxacor |
| B-255 | glufosinate | benoxacor |
| B-256 | glufosinate-ammonium | benoxacor |
| B-257 | pendimethalin | benoxacor |
| B-258 | trifluralin | benoxacor |
| B-259 | acetochlor | benoxacor |
| B-260 | cafenstrole | benoxacor |
| B-261 | dimethenamid-P | benoxacor |
| B-262 | fentrazamide | benoxacor |
| B-263 | flufenacet | benoxacor |
| B-264 | mefenacet | benoxacor |
| B-265 | metazachlor | benoxacor |
| B-266 | metolachlor-S | benoxacor |
| B-267 | pyroxasulfone | benoxacor |
| B-268 | isoxaben | benoxacor |
| B-269 | dymron | benoxacor |
| B-270 | indanofan | benoxacor |
| B-271 | oxaziclomefone | benoxacor |
| B-272 | triaziflam | benoxacor |
| B-273 | atrazine + H-1 | benoxacor |
| B-274 | atrazine + glyphosate | benoxacor |
| B-275 | atrazine + mesotrione | benoxacor |
| B-276 | atrazine + nicosulfuron | benoxacor |
| B-277 | atrazine + tembotrione | benoxacor |
| B-278 | atrazine + topramezone | benoxacor |
| B-279 | clomazone + glyphosate | benoxacor |
| B-280 | diflufenican + clodinafop-propargyl | benoxacor |
| B-281 | diflufenican + fenoxaprop-P-ethyl | benoxacor |
| B-282 | diflufenican + flupyrsulfuron-methyl-sodium | benoxacor |
| B-283 | diflufenican + glyphosate | benoxacor |
| B-284 | diflufenican + mesosulfuron-methyl | benoxacor |
| B-285 | diflufenican + pinoxaden | benoxacor |
| B-286 | diflufenican + pyroxsulam | benoxacor |
| B-287 | flumetsulam + glyphosate | benoxacor |
| B-288 | flumioxazin + glyphosate | benoxacor |
| B-289 | imazapic + glyphosate | benoxacor |
| B-290 | imazethapyr + glyphosate | benoxacor |
| B-291 | isoxaflutol + H-1 | benoxacor |
| B-292 | isoxaflutol + glyphosate | benoxacor |
| B-293 | metazachlor + H-1 | benoxacor |
| B-294 | metazachlor + glyphosate | benoxacor |
| B-295 | metazachlor + mesotrione | benoxacor |
| B-296 | metazachlor + nicosulfuron | benoxacor |
| B-297 | metazachlor + terbuthylazine | benoxacor |
| B-298 | metazachlor + topramezone | benoxacor |
| B-299 | metribuzin + glyphosate | benoxacor |
| B-300 | pendimethalin + H-1 | benoxacor |
| B-301 | pendimethalin + clodinafop-propargyl | benoxacor |
| B-302 | pendimethalin + fenoxaprop-P-ethyl | benoxacor |
| B-303 | pendimethalin + flupyrsulfuron-methyl-sodium | benoxacor |
| B-304 | pendimethalin + glyphosate | benoxacor |
| B-305 | pendimethalin + mesosulfuron-methyl | benoxacor |
| B-306 | pendimethalin + mesotrione | benoxacor |
| B-307 | pendimethalin + nicosulfuron | benoxacor |
| B-308 | pendimethalin + pinoxaden | benoxacor |
| B-309 | pendimethalin + pyroxsulam | benoxacor |
| B-310 | pendimethalin + tembotrione | benoxacor |
| B-311 | pendimethalin + topramezone | benoxacor |
| B-312 | pyroxasulfone + tembotrione | benoxacor |
| B-313 | pyroxasulfone + topramezone | benoxacor |
| B-314 | sulfentrazone + glyphosate | benoxacor |
| B-315 | terbuthylazine + H-1 | benoxacor |
| B-316 | terbuthylazine + foramsulfuron | benoxacor |
| B-317 | terbuthylazine + glyphosate | benoxacor |
| B-318 | terbuthylazine + mesotrione | benoxacor |
| B-319 | terbuthylazine + nicosulfuron | benoxacor |
| B-320 | terbuthylazine + tembotrione | benoxacor |
| B-321 | terbuthylazine + topramezone | benoxacor |
| B-322 | trifluralin + glyphosate | benoxacor |
| B-323 | clodinafop-propargyl | cloquintocet |
| B-324 | cycloxydim | cloquintocet |
| B-325 | cyhalofop-butyl | cloquintocet |
| B-326 | fenoxaprop-P-ethyl | cloquintocet |
| B-327 | pinoxaden | cloquintocet |
| B-328 | profoxydim | cloquintocet |
| B-329 | tepraloxydim | cloquintocet |
| B-330 | tralkoxydim | cloquintocet |
| B-331 | esprocarb | cloquintocet |
| B-332 | prosulfocarb | cloquintocet |
| B-333 | thiobencarb | cloquintocet |
| B-334 | triallate | cloquintocet |
| B-335 | bensulfuron-methyl | cloquintocet |
| B-336 | bispyribac-sodium | cloquintocet |
| B-337 | cyclosulfamuron | cloquintocet |
| B-338 | flumetsulam | cloquintocet |
| B-339 | flupyrsulfuron-methyl-sodium | cloquintocet |
| B-340 | foramsulfuron | cloquintocet |
| B-341 | imazamox | cloquintocet |
| B-342 | imazapic | cloquintocet |
| B-343 | imazapyr | cloquintocet |
| B-344 | imazaquin | cloquintocet |
| B-345 | imazethapyr | cloquintocet |
| B-346 | imazosulfuron | cloquintocet |
| B-347 | iodosulfuron-methyl-sodium | cloquintocet |
| B-348 | mesosulfuron | cloquintocet |
| B-349 | nicosulfuron | cloquintocet |
| B-350 | penoxsulam | cloquintocet |
| B-351 | propoxycarbazone-sodium | cloquintocet |
| B-352 | pyrazosulfuron-ethyl | cloquintocet |
| B-353 | pyroxsulam | cloquintocet |
| B-354 | rimsulfuron | cloquintocet |
| B-355 | sulfosulfuron | cloquintocet |
| B-356 | thiencarbazone-methyl | cloquintocet |
| B-357 | tritosulfuron | cloquintocet |
| B-358 | 2,4-D and its salts and esters | cloquintocet |
| B-359 | aminopyralid and its salts and esters | cloquintocet |
| B-360 | clopyralid and its salts and esters | cloquintocet |
| B-361 | dicamba and its salts and esters | cloquintocet |
| B-362 | fluroxypyr-meptyl | cloquintocet |
| B-363 | quinclorac | cloquintocet |
| B-364 | quinmerac | cloquintocet |
| B-365 | H-9 | cloquintocet |
| B-366 | diflufenzopyr | cloquintocet |
| B-367 | diflufenzopyr-sodium | cloquintocet |
| B-368 | clomazone | cloquintocet |
| B-369 | diflufenican | cloquintocet |
| B-370 | fluorochloridone | cloquintocet |
| B-371 | isoxaflutol | cloquintocet |
| B-372 | mesotrione | cloquintocet |
| B-373 | picolinafen | cloquintocet |
| B-374 | sulcotrione | cloquintocet |
| B-375 | tefuryltrione | cloquintocet |
| B-376 | tembotrione | cloquintocet |
| B-377 | topramezone | cloquintocet |
| B-378 | H-7 | cloquintocet |
| B-379 | atrazine | cloquintocet |
| B-380 | diuron | cloquintocet |
| B-381 | fluometuron | cloquintocet |
| B-382 | hexazinone | cloquintocet |
| B-383 | isoproturon | cloquintocet |
| B-384 | metribuzin | cloquintocet |
| B-385 | propanil | cloquintocet |
| B-386 | terbuthylazine | cloquintocet |
| B-387 | paraquat dichloride | cloquintocet |
| B-388 | flumioxazin | cloquintocet |
| B-389 | oxyfluorfen | cloquintocet |
| B-390 | saflufenacil | cloquintocet |
| B-391 | sulfentrazone | cloquintocet |
| B-392 | H-1 | cloquintocet |
| B-393 | H-2 | cloquintocet |
| B-394 | glyphosate | cloquintocet |
| B-395 | glyphosate-isopropylammonium | cloquintocet |
| B-396 | glyphosate-trimesium (sulfosate) | cloquintocet |
| B-397 | glufosinate | cloquintocet |
| B-398 | glufosinate-ammonium | cloquintocet |
| B-399 | pendimethalin | cloquintocet |
| B-400 | trifluralin | cloquintocet |
| B-401 | acetochlor | cloquintocet |
| B-402 | cafenstrole | cloquintocet |
| B-403 | dimethenamid-P | cloquintocet |
| B-404 | fentrazamide | cloquintocet |
| B-405 | flufenacet | cloquintocet |
| B-406 | mefenacet | cloquintocet |
| B-407 | metazachlor | cloquintocet |
| B-408 | metolachlor-S | cloquintocet |
| B-409 | pyroxasulfone | cloquintocet |
| B-410 | isoxaben | cloquintocet |
| B-411 | dymron | cloquintocet |
| B-412 | indanofan | cloquintocet |
| B-413 | oxaziclomefone | cloquintocet |
| B-414 | triaziflam | cloquintocet |
| B-415 | atrazine + H-1 | cloquintocet |
| B-416 | atrazine + glyphosate | cloquintocet |
| B-417 | atrazine + mesotrione | cloquintocet |
| B-418 | atrazine + nicosulfuron | cloquintocet |
| B-419 | atrazine + tembotrione | cloquintocet |
| B-420 | atrazine + topramezone | cloquintocet |
| B-421 | clomazone + glyphosate | cloquintocet |
| B-422 | diflufenican + clodinafop-propargyl | cloquintocet |
| B-423 | diflufenican + fenoxaprop-p-ethyl | cloquintocet |
| B-424 | diflufenican + flupyrsulfuron-methyl-sodium | cloquintocet |
| B-425 | diflufenican + glyphosate | cloquintocet |
| B-426 | diflufenican + mesosulfuron-methyl | cloquintocet |
| B-427 | diflufenican + pinoxaden | cloquintocet |
| B-428 | diflufenican + pyroxsulam | cloquintocet |
| B-429 | flumetsulam + glyphosate | cloquintocet |
| B-430 | flumioxazin + glyphosate | cloquintocet |
| B-431 | imazapic + glyphosate | cloquintocet |
| B-432 | imazethapyr + glyphosate | cloquintocet |
| B-433 | isoxaflutol + H-1 | cloquintocet |
| B-434 | isoxaflutol + glyphosate | cloquintocet |
| B-435 | metazachlor + H-1 | cloquintocet |
| B-436 | metazachlor + glyphosate | cloquintocet |
| B-437 | metazachlor + mesotrione | cloquintocet |
| B-438 | metazachlor + nicosulfuron | cloquintocet |
| B-439 | metazachlor + terbuthylazine | cloquintocet |
| B-440 | metazachlor + topramezone | cloquintocet |
| B-441 | metribuzin + glyphosate | cloquintocet |
| B-442 | pendimethalin + H-1 | cloquintocet |
| B-443 | pendimethalin + clodinafop-propargyl | cloquintocet |
| B-444 | pendimethalin + fenoxaprop-P-ethyl | cloquintocet |
| B-445 | pendimethalin + flupyrsulfuron-methyl-sodium | cloquintocet |
| B-446 | pendimethalin + glyphosate | cloquintocet |
| B-447 | pendimethalin + mesosulfuron-methyl | cloquintocet |
| B-448 | pendimethalin + mesotrione | cloquintocet |
| B-449 | pendimethalin + nicosulfuron | cloquintocet |
| B-450 | pendimethalin + pinoxaden | cloquintocet |
| B-451 | pendimethalin + pyroxsulam | cloquintocet |
| B-452 | pendimethalin + tembotrione | cloquintocet |
| B-453 | pendimethalin + topramezone | cloquintocet |
| B-454 | pyroxasulfone + tembotrione | cloquintocet |
| B-455 | pyroxasulfone + topramezone | cloquintocet |
| B-456 | sulfentrazone + glyphosate | cloquintocet |
| B-457 | terbuthylazine + H-1 | cloquintocet |
| B-458 | terbuthylazine + foramsulfuron | cloquintocet |
| B-459 | terbuthylazine + glyphosate | cloquintocet |
| B-460 | terbuthylazine + mesotrione | cloquintocet |
| B-461 | terbuthylazine + nicosulfuron | cloquintocet |
| B-462 | terbuthylazine + tembotrione | cloquintocet |
| B-463 | terbuthylazine + topramezone | cloquintocet |
| B-464 | trifluralin + glyphosate | cloquintocet |
| B-465 | clodinafop-propargyl | dichlormid |
| B-466 | cycloxydim | dichlormid |
| B-467 | cyhalofop-butyl | dichlormid |
| B-468 | fenoxaprop-P-ethyl | dichlormid |
| B-469 | pinoxaden | dichlormid |
| B-470 | profoxydim | dichlormid |
| B-471 | tepraloxydim | dichlormid |
| B-472 | tralkoxydim | dichlormid |
| B-473 | esprocarb | dichlormid |
| B-474 | prosulfocarb | dichlormid |
| B-475 | thiobencarb | dichlormid |
| B-476 | triallate | dichlormid |
| B-477 | bensulfuron-methyl | dichlormid |
| B-478 | bispyribac-sodium | dichlormid |
| B-479 | cyclosulfamuron | dichlormid |
| B-480 | flumetsulam | dichlormid |
| B-481 | flupyrsulfuron-methyl-sodium | dichlormid |
| B-482 | foramsulfuron | dichlormid |
| B-483 | imazamox | dichlormid |
| B-484 | imazapic | dichlormid |
| B-485 | imazapyr | dichlormid |
| B-486 | imazaquin | dichlormid |
| B-487 | imazethapyr | dichlormid |
| B-488 | imazosulfuron | dichlormid |
| B-489 | iodosulfuron-methyl-sodium | dichlormid |
| B-490 | mesosulfuron | dichlormid |
| B-491 | nicosulfuron | dichlormid |
| B-492 | penoxsulam | dichlormid |
| B-493 | propoxycarbazone-sodium | dichlormid |
| B-494 | pyrazosulfuron-ethyl | dichlormid |
| B-495 | pyroxsulam | dichlormid |
| B-496 | rimsulfuron | dichlormid |
| B-497 | sulfosulfuron | dichlormid |
| B-498 | thiencarbazone-methyl | dichlormid |
| B-499 | tritosulfuron | dichlormid |
| B-500 | 2,4-D and its salts and esters | dichlormid |
| B-501 | aminopyralid and its salts and esters | dichlormid |
| B-502 | clopyralid and its salts and esters | dichlormid |
| B-503 | dicamba and its salts and esters | dichlormid |
| B-504 | fluroxypyr-meptyl | dichlormid |
| B-505 | quinclorac | dichlormid |
| B-506 | quinmerac | dichlormid |
| B-507 | H-9 | dichlormid |
| B-508 | diflufenzopyr | dichlormid |
| B-509 | diflufenzopyr-sodium | dichlormid |
| B-510 | clomazone | dichlormid |
| B-511 | diflufenican | dichlormid |
| B-512 | fluorochloridone | dichlormid |
| B-513 | isoxaflutol | dichlormid |
| B-514 | mesotrione | dichlormid |
| B-515 | picolinafen | dichlormid |
| B-516 | sulcotrione | dichlormid |
| B-517 | tefuryltrione | dichlormid |
| B-518 | tembotrione | dichlormid |
| B-519 | topramezone | dichlormid |
| B-520 | H-7 | dichlormid |
| B-521 | atrazine | dichlormid |
| B-522 | diuron | dichlormid |
| B-523 | fluometuron | dichlormid |
| B-524 | hexazinone | dichlormid |
| B-525 | isoproturon | dichlormid |
| B-526 | metribuzin | dichlormid |
| B-527 | propanil | dichlormid |
| B-528 | terbuthylazine | dichlormid |
| B-529 | paraquat dichloride | dichlormid |
| B-530 | flumioxazin | dichlormid |
| B-531 | oxyfluorfen | dichlormid |
| B-532 | saflufenacil | dichlormid |
| B-533 | sulfentrazone | dichlormid |
| B-534 | H-1 | dichlormid |
| B-535 | H-2 | dichlormid |
| B-536 | glyphosate | dichlormid |
| B-537 | glyphosate-isopropylammonium | dichlormid |
| B-538 | glyphosate-trimesium (sulfosate) | dichlormid |
| B-539 | glufosinate | dichlormid |
| B-540 | glufosinate-ammonium | dichlormid |
| B-541 | pendimethalin | dichlormid |
| B-542 | trifluralin | dichlormid |
| B-543 | acetochlor | dichlormid |
| B-544 | cafenstrole | dichlormid |
| B-545 | dimethenamid-P | dichlormid |
| B-546 | fentrazamide | dichlormid |
| B-547 | flufenacet | dichlormid |
| B-548 | mefenacet | dichlormid |
| B-549 | metazachlor | dichlormid |
| B-550 | metolachlor-S | dichlormid |
| B-551 | pyroxasulfone | dichlormid |
| B-552 | isoxaben | dichlormid |
| B-553 | dymron | dichlormid |
| B-554 | indanofan | dichlormid |
| B-555 | oxaziclomefone | dichlormid |
| B-556 | triaziflam | dichlormid |
| B-557 | atrazine + H-1 | dichlormid |
| B-558 | atrazine + glyphosate | dichlormid |
| B-559 | atrazine + mesotrione | dichlormid |
| B-560 | atrazine + nicosulfuron | dichlormid |
| B-561 | atrazine + tembotrione | dichlormid |
| B-562 | atrazine + topramezone | dichlormid |
| B-563 | clomazone + glyphosate | dichlormid |
| B-564 | diflufenican + clodinafop-propargyl | dichlormid |
| B-565 | diflufenican + fenoxaprop-p-ethyl | dichlormid |
| B-566 | diflufenican + flupyrsulfuron-methyl-sodium | dichlormid |
| B-567 | diflufenican + glyphosate | dichlormid |
| B-568 | diflufenican + mesosulfuron-methyl | dichlormid |
| B-569 | diflufenican + pinoxaden | dichlormid |
| B-570 | diflufenican + pyroxsulam | dichlormid |
| B-571 | flumetsulam + glyphosate | dichlormid |
| B-572 | flumioxazin + glyphosate | dichlormid |
| B-573 | imazapic + glyphosate | dichlormid |
| B-574 | imazethapyr + glyphosate | dichlormid |
| B-575 | isoxaflutol + H-1 | dichlormid |
| B-576 | isoxaflutol + glyphosate | dichlormid |
| B-577 | metazachlor + H-1 | dichlormid |
| B-578 | metazachlor + glyphosate | dichlormid |
| B-579 | metazachlor + mesotrione | dichlormid |
| B-580 | metazachlor + nicosulfuron | dichlormid |
| B-581 | metazachlor + terbuthylazine | dichlormid |
| B-582 | metazachlor + topramezone | dichlormid |
| B-583 | metribuzin + glyphosate | dichlormid |
| B-584 | pendimethalin + H-1 | dichlormid |
| B-585 | pendimethalin + clodinafop-propargyl | dichlormid |
| B-586 | pendimethalin + fenoxaprop-P-ethyl | dichlormid |
| B-587 | pendimethalin + flupyrsulfuron-methyl-sodium | dichlormid |
| B-588 | pendimethalin + glyphosate | dichlormid |
| B-589 | pendimethalin + mesosulfuron-methyl | dichlormid |
| B-590 | pendimethalin + mesotrione | dichlormid |
| B-591 | pendimethalin + nicosulfuron | dichlormid |
| B-592 | pendimethalin + pinoxaden | dichlormid |
| B-593 | pendimethalin + pyroxsulam | dichlormid |
| B-594 | pendimethalin + tembotrione | dichlormid |
| B-595 | pendimethalin + topramezone | dichlormid |
| B-596 | pyroxasulfone + tembotrione | dichlormid |
| B-597 | pyroxasulfone + topramezone | dichlormid |
| B-598 | sulfentrazone + glyphosate | dichlormid |
| B-599 | terbuthylazine + H-1 | dichlormid |
| B-600 | terbuthylazine + foramsulfuron | dichlormid |
| B-601 | terbuthylazine + glyphosate | dichlormid |
| B-602 | terbuthylazine + mesotrione | dichlormid |
| B-603 | terbuthylazine + nicosulfuron | dichlormid |
| B-604 | terbuthylazine + tembotrione | dichlormid |
| B-605 | terbuthylazine + topramezone | dichlormid |
| B-606 | trifluralin + glyphosate | dichlormid |
| B-607 | clodinafop-propargyl | fenchlorazole |
| B-608 | cycloxydim | fenchlorazole |
| B-609 | cyhalofop-butyl | fenchlorazole |
| B-610 | fenoxaprop-P-ethyl | fenchlorazole |
| B-611 | pinoxaden | fenchlorazole |
| B-612 | profoxydim | fenchlorazole |
| B-613 | tepraloxydim | fenchlorazole |
| B-614 | tralkoxydim | fenchlorazole |
| B-615 | esprocarb | fenchlorazole |
| B-616 | prosulfocarb | fenchlorazole |
| B-617 | thiobencarb | fenchlorazole |
| B-618 | triallate | fenchlorazole |
| B-619 | bensulfuron-methyl | fenchlorazole |
| B-620 | bispyribac-sodium | fenchlorazole |
| B-621 | cyclosulfamuron | fenchlorazole |
| B-622 | flumetsulam | fenchlorazole |
| B-623 | flupyrsulfuron-methyl-sodium | fenchlorazole |
| B-624 | foramsulfuron | fenchlorazole |
| B-625 | imazamox | fenchlorazole |
| B-626 | imazapic | fenchlorazole |
| B-627 | imazapyr | fenchlorazole |
| B-628 | imazaquin | fenchlorazole |
| B-629 | imazethapyr | fenchlorazole |
| B-630 | imazosulfuron | fenchlorazole |
| B-631 | iodosulfuron-methyl-sodium | fenchlorazole |
| B-632 | mesosulfuron | fenchlorazole |
| B-633 | nicosulfuron | fenchlorazole |
| B-634 | penoxsulam | fenchlorazole |
| B-635 | propoxycarbazone-sodium | fenchlorazole |
| B-636 | pyrazosulfuron-ethyl | fenchlorazole |
| B-637 | pyroxsulam | fenchlorazole |
| B-638 | rimsulfuron | fenchlorazole |
| B-639 | sulfosulfuron | fenchlorazole |
| B-640 | thiencarbazone-methyl | fenchlorazole |
| B-641 | tritosulfuron | fenchlorazole |
| B-642 | 2,4-D and its salts and esters | fenchlorazole |
| B-643 | aminopyralid and its salts and esters | fenchlorazole |
| B-644 | clopyralid and its salts and esters | fenchlorazole |
| B-645 | dicamba and its salts and esters | fenchlorazole |
| B-646 | fluroxypyr-meptyl | fenchlorazole |
| B-647 | quinclorac | fenchlorazole |
| B-648 | quinmerac | fenchlorazole |
| B-649 | H-9 | fenchlorazole |
| B-650 | diflufenzopyr | fenchlorazole |
| B-651 | diflufenzopyr-sodium | fenchlorazole |
| B-652 | clomazone | fenchlorazole |
| B-653 | diflufenican | fenchlorazole |
| B-654 | fluorochloridone | fenchlorazole |
| B-655 | isoxaflutol | fenchlorazole |
| B-656 | mesotrione | fenchlorazole |
| B-657 | picolinafen | fenchlorazole |
| B-658 | sulcotrione | fenchlorazole |
| B-659 | tefuryltrione | fenchlorazole |
| B-660 | tembotrione | fenchlorazole |
| B-661 | topramezone | fenchlorazole |
| B-662 | H-7 | fenchlorazole |
| B-663 | atrazine | fenchlorazole |
| B-664 | diuron | fenchlorazole |
| B-665 | fluometuron | fenchlorazole |
| B-666 | hexazinone | fenchlorazole |
| B-667 | isoproturon | fenchlorazole |
| B-668 | metribuzin | fenchlorazole |
| B-669 | propanil | fenchlorazole |
| B-670 | terbuthylazine | fenchlorazole |
| B-671 | paraquat dichloride | fenchlorazole |
| B-672 | flumioxazin | fenchlorazole |
| B-673 | oxyfluorfen | fenchlorazole |
| B-674 | saflufenacil | fenchlorazole |
| B-675 | sulfentrazone | fenchlorazole |
| B-676 | H-1 | fenchlorazole |
| B-677 | H-2 | fenchlorazole |
| B-678 | glyphosate | fenchlorazole |
| B-679 | glyphosate-isopropylammonium | fenchlorazole |
| B-680 | glyphosate-trimesium (sulfosate) | fenchlorazole |
| B-681 | glufosinate | fenchlorazole |
| B-682 | glufosinate-ammonium | fenchlorazole |
| B-683 | pendimethalin | fenchlorazole |
| B-684 | trifluralin | fenchlorazole |
| B-685 | acetochlor | fenchlorazole |
| B-686 | cafenstrole | fenchlorazole |
| B-687 | dimethenamid-P | fenchlorazole |
| B-688 | fentrazamide | fenchlorazole |
| B-689 | flufenacet | fenchlorazole |
| B-690 | mefenacet | fenchlorazole |
| B-691 | metazachlor | fenchlorazole |
| B-692 | metolachlor-S | fenchlorazole |
| B-693 | pyroxasulfone | fenchlorazole |
| B-694 | isoxaben | fenchlorazole |
| B-695 | dymron | fenchlorazole |
| B-696 | indanofan | fenchlorazole |
| B-697 | oxaziclomefone | fenchlorazole |
| B-698 | triaziflam | fenchlorazole |
| B-699 | atrazine + H-1 | fenchlorazole |
| B-700 | atrazine + glyphosate | fenchlorazole |
| B-701 | atrazine + mesotrione | fenchlorazole |
| B-702 | atrazine + nicosulfuron | fenchlorazole |
| B-703 | atrazine + tembotrione | fenchlorazole |
| B-704 | atrazine + topramezone | fenchlorazole |
| B-705 | clomazone + glyphosate | fenchlorazole |
| B-706 | diflufenican + clodinafop-propargyl | fenchlorazole |
| B-707 | diflufenican + fenoxaprop-P-ethyl | fenchlorazole |
| B-708 | diflufenican + flupyrsulfuron-methyl-sodium | fenchlorazole |
| B-709 | diflufenican + glyphosate | fenchlorazole |
| B-710 | diflufenican + mesosulfuron-methyl | fenchlorazole |
| B-711 | diflufenican + pinoxaden | fenchlorazole |
| B-712 | diflufenican + pyroxsulam | fenchlorazole |
| B-713 | flumetsulam + glyphosate | fenchlorazole |
| B-714 | flumioxazin + glyphosate | fenchlorazole |
| B-715 | imazapic + glyphosate | fenchlorazole |
| B-716 | imazethapyr + glyphosate | fenchlorazole |
| B-717 | isoxaflutol + H-1 | fenchlorazole |
| B-718 | isoxaflutol + glyphosate | fenchlorazole |
| B-719 | metazachlor + H-1 | fenchlorazole |
| B-720 | metazachlor + glyphosate | fenchlorazole |
| B-721 | metazachlor + mesotrione | fenchlorazole |
| B-722 | metazachlor + nicosulfuron | fenchlorazole |
| B-723 | metazachlor + terbuthylazine | fenchlorazole |
| B-724 | metazachlor + topramezone | fenchlorazole |
| B-725 | metribuzin + glyphosate | fenchlorazole |
| B-726 | pendimethalin + H-1 | fenchlorazole |
| B-727 | pendimethalin + clodinafop-propargyl | fenchlorazole |
| B-728 | pendimethalin + fenoxaprop-P-ethyl | fenchlorazole |
| B-729 | pendimethalin + flupyrsulfuron-methyl-sodium | fenchlorazole |
| B-730 | pendimethalin + glyphosate | fenchlorazole |
| B-731 | pendimethalin + mesosulfuron-methyl | fenchlorazole |
| B-732 | pendimethalin + mesotrione | fenchlorazole |
| B-733 | pendimethalin + nicosulfuron | fenchlorazole |
| B-734 | pendimethalin + pinoxaden | fenchlorazole |
| B-735 | pendimethalin + pyroxsulam | fenchlorazole |
| B-736 | pendimethalin + tembotrione | fenchlorazole |
| B-737 | pendimethalin + topramezone | fenchlorazole |
| B-738 | pyroxasulfone + tembotrione | fenchlorazole |
| B-739 | pyroxasulfone + topramezone | fenchlorazole |
| B-740 | sulfentrazone + glyphosate | fenchlorazole |
| B-741 | terbuthylazine + H-1 | fenchlorazole |
| B-742 | terbuthylazine + foramsulfuron | fenchlorazole |
| B-743 | terbuthylazine + glyphosate | fenchlorazole |
| B-744 | terbuthylazine + mesotrione | fenchlorazole |
| B-745 | terbuthylazine + nicosulfuron | fenchlorazole |
| B-746 | terbuthylazine + tembotrione | fenchlorazole |
| B-747 | terbuthylazine + topramezone | fenchlorazole |
| B-748 | trifluralin + glyphosate | fenchlorazole |
| B-749 | clodinafop-propargyl | fenclorim |
| B-750 | cycloxydim | fenclorim |
| B-751 | cyhalofop-butyl | fenclorim |
| B-752 | fenoxaprop-P-ethyl | fenclorim |
| B-753 | pinoxaden | fenclorim |
| B-754 | profoxydim | fenclorim |
| B-755 | tepraloxydim | fenclorim |
| B-756 | tralkoxydim | fenclorim |
| B-757 | esprocarb | fenclorim |
| B-758 | prosulfocarb | fenclorim |
| B-759 | thiobencarb | fenclorim |
| B-760 | triallate | fenclorim |
| B-761 | bensulfuron-methyl | fenclorim |
| B-762 | bispyribac-sodium | fenclorim |
| B-763 | cyclosulfamuron | fenclorim |
| B-764 | flumetsulam | fenclorim |
| B-765 | flupyrsulfuron-methyl-sodium | fenclorim |
| B-766 | foramsulfuron | fenclorim |
| B-767 | imazamox | fenclorim |
| B-768 | imazapic | fenclorim |
| B-769 | imazapyr | fenclorim |
| B-770 | imazaquin | fenclorim |
| B-771 | imazethapyr | fenclorim |
| B-772 | imazosulfuron | fenclorim |
| B-773 | iodosulfuron-methyl-sodium | fenclorim |
| B-774 | mesosulfuron | fenclorim |
| B-775 | nicosulfuron | fenclorim |
| B-776 | penoxsulam | fenclorim |
| B-777 | propoxycarbazone-sodium | fenclorim |
| B-778 | pyrazosulfuron-ethyl | fenclorim |
| B-779 | pyroxsulam | fenclorim |
| B-780 | rimsulfuron | fenclorim |
| B-781 | sulfosulfuron | fenclorim |
| B-782 | thiencarbazone-methyl | fenclorim |
| B-783 | tritosulfuron | fenclorim |
| B-784 | 2,4-D and its salts and esters | fenclorim |
| B-785 | aminopyralid and its salts and esters | fenclorim |
| B-786 | clopyralid and its salts and esters | fenclorim |
| B-787 | dicamba and its salts and esters | fenclorim |
| B-788 | fluroxypyr-meptyl | fenclorim |
| B-789 | quinclorac | fenclorim |
| B-790 | quinmerac | fenclorim |
| B-791 | H-9 | fenclorim |
| B-792 | diflufenzopyr | fenclorim |
| B-793 | diflufenzopyr-sodium | fenclorim |
| B-794 | clomazone | fenclorim |
| B-795 | diflufenican | fenclorim |
| B-796 | fluorochloridone | fenclorim |
| B-797 | isoxaflutol | fenclorim |
| B-798 | mesotrione | fenclorim |
| B-799 | picolinafen | fenclorim |
| B-800 | sulcotrione | fenclorim |
| B-801 | tefuryltrione | fenclorim |
| B-802 | tembotrione | fenclorim |
| B-803 | topramezone | fenclorim |
| B-804 | H-7 | fenclorim |
| B-805 | atrazine | fenclorim |
| B-806 | diuron | fenclorim |
| B-807 | fluometuron | fenclorim |
| B-808 | hexazinone | fenclorim |
| B-809 | isoproturon | fenclorim |
| B-810 | metribuzin | fenclorim |
| B-811 | propanil | fenclorim |
| B-812 | terbuthylazine | fenclorim |
| B-813 | paraquat dichloride | fenclorim |
| B-814 | flumioxazin | fenclorim |
| B-815 | oxyfluorfen | fenclorim |
| B-816 | saflufenacil | fenclorim |
| B-817 | sulfentrazone | fenclorim |
| B-818 | H-1 | fenclorim |
| B-819 | H-2 | fenclorim |
| B-820 | glyphosate | fenclorim |
| B-821 | glyphosate-isopropylammonium | fenclorim |
| B-822 | glyphosate-trimesium (sulfosate) | fenclorim |
| B-823 | glufosinate | fenclorim |
| B-824 | glufosinate-ammonium | fenclorim |
| B-825 | pendimethalin | fenclorim |
| B-826 | trifluralin | fenclorim |
| B-827 | acetochlor | fenclorim |
| B-828 | cafenstrole | fenclorim |
| B-829 | dimethenamid-P | fenclorim |
| B-830 | fentrazamide | fenclorim |
| B-831 | flufenacet | fenclorim |
| B-832 | mefenacet | fenclorim |
| B-833 | metazachlor | fenclorim |
| B-834 | metolachlor-S | fenclorim |
| B-835 | pyroxasulfone | fenclorim |
| B-836 | isoxaben | fenclorim |
| B-837 | dymron | fenclorim |
| B-838 | indanofan | fenclorim |
| B-839 | oxaziclomefone | fenclorim |
| B-840 | triaziflam | fenclorim |
| B-841 | atrazine + H-1 | fenclorim |
| B-842 | atrazine + glyphosate | fenclorim |
| B-843 | atrazine + mesotrione | fenclorim |
| B-844 | atrazine + nicosulfuron | fenclorim |
| B-845 | atrazine + tembotrione | fenclorim |
| B-846 | atrazine + topramezone | fenclorim |
| B-847 | clomazone + glyphosate | fenclorim |
| B-848 | diflufenican + clodinafop-propargyl | fenclorim |
| B-849 | diflufenican + fenoxaprop-P-ethyl | fenclorim |
| B-850 | diflufenican + flupyrsulfuron-methyl-sodium | fenclorim |
| B-851 | diflufenican + glyphosate | fenclorim |
| B-852 | diflufenican + mesosulfuron-methyl | fenclorim |
| B-853 | diflufenican + pinoxaden | fenclorim |
| B-854 | diflufenican + pyroxsulam | fenclorim |
| B-855 | flumetsulam + glyphosate | fenclorim |
| B-856 | flumioxazin + glyphosate | fenclorim |
| B-857 | imazapic + glyphosate | fenclorim |
| B-858 | imazethapyr + glyphosate | fenclorim |
| B-859 | isoxaflutol + H-1 | fenclorim |
| B-860 | isoxaflutol + glyphosate | fenclorim |
| B-861 | metazachlor + H-1 | fenclorim |
| B-862 | metazachlor + glyphosate | fenclorim |
| B-863 | metazachlor + mesotrione | fenclorim |
| B-864 | metazachlor + nicosulfuron | fenclorim |
| B-865 | metazachlor + terbuthylazine | fenclorim |
| B-866 | metazachlor + topramezone | fenclorim |
| B-867 | metribuzin + glyphosate | fenclorim |
| B-868 | pendimethalin + H-1 | fenclorim |
| B-869 | pendimethalin + clodinafop-propargyl | fenclorim |
| B-870 | pendimethalin + fenoxaprop-P-ethyl | fenclorim |
| B-871 | pendimethalin + flupyrsulfuron-methyl-sodium | fenclorim |
| B-872 | pendimethalin + glyphosate | fenclorim |
| B-873 | pendimethalin + mesosulfuron-methyl | fenclorim |
| B-874 | pendimethalin + mesotrione | fenclorim |
| B-875 | pendimethalin + nicosulfuron | fenclorim |
| B-876 | pendimethalin + pinoxaden | fenclorim |
| B-877 | pendimethalin + pyroxsulam | fenclorim |
| B-878 | pendimethalin + tembotrione | fenclorim |
| B-879 | pendimethalin + topramezone | fenclorim |
| B-880 | pyroxasulfone + tembotrione | fenclorim |
| B-881 | pyroxasulfone + topramezone | fenclorim |
| B-882 | sulfentrazone + glyphosate | fenclorim |
| B-883 | terbuthylazine + H-1 | fenclorim |
| B-884 | terbuthylazine + foramsulfuron | fenclorim |
| B-885 | terbuthylazine + glyphosate | fenclorim |
| B-886 | terbuthylazine + mesotrione | fenclorim |
| B-887 | terbuthylazine + nicosulfuron | fenclorim |
| B-888 | terbuthylazine + tembotrione | fenclorim |
| B-889 | terbuthylazine + topramezone | fenclorim |
| B-890 | trifluralin + glyphosate | fenclorim |
| B-891 | clodinafop-propargyl | isoxadifen |
| B-892 | cycloxydim | isoxadifen |
| B-893 | cyhalofop-butyl | isoxadifen |
| B-894 | fenoxaprop-P-ethyl | isoxadifen |
| B-895 | pinoxaden | isoxadifen |
| B-896 | profoxydim | isoxadifen |
| B-897 | tepraloxydim | isoxadifen |
| B-898 | tralkoxydim | isoxadifen |
| B-899 | esprocarb | isoxadifen |
| B-900 | prosulfocarb | isoxadifen |
| B-901 | thiobencarb | isoxadifen |
| B-902 | triallate | isoxadifen |
| B-903 | bensulfuron-methyl | isoxadifen |
| B-904 | bispyribac-sodium | isoxadifen |
| B-905 | cyclosulfamuron | isoxadifen |
| B-906 | flumetsulam | isoxadifen |
| B-907 | flupyrsulfuron-methyl-sodium | isoxadifen |
| B-908 | foramsulfuron | isoxadifen |
| B-909 | imazamox | isoxadifen |
| B-910 | imazapic | isoxadifen |
| B-911 | imazapyr | isoxadifen |
| B-912 | imazaquin | isoxadifen |
| B-913 | imazethapyr | isoxadifen |
| B-914 | imazosulfuron | isoxadifen |
| B-915 | iodosulfuron-methyl-sodium | isoxadifen |
| B-916 | mesosulfuron | isoxadifen |
| B-917 | nicosulfuron | isoxadifen |
| B-918 | penoxsulam | isoxadifen |
| B-919 | propoxycarbazone-sodium | isoxadifen |
| B-920 | pyrazosulfuron-ethyl | isoxadifen |
| B-921 | pyroxsulam | isoxadifen |
| B-922 | rimsulfuron | isoxadifen |
| B-923 | sulfosulfuron | isoxadifen |
| B-924 | thiencarbazone-methyl | isoxadifen |
| B-925 | tritosulfuron | isoxadifen |
| B-926 | 2,4-D and its salts and esters | isoxadifen |
| B-927 | aminopyralid and its salts and esters | isoxadifen |
| B-928 | clopyralid and its salts and esters | isoxadifen |
| B-929 | dicamba and its salts and esters | isoxadifen |
| B-930 | fluroxypyr-meptyl | isoxadifen |
| B-931 | quinclorac | isoxadifen |
| B-932 | quinmerac | isoxadifen |
| B-933 | H-9 | isoxadifen |
| B-934 | diflufenzopyr | isoxadifen |
| B-935 | diflufenzopyr-sodium | isoxadifen |
| B-936 | clomazone | isoxadifen |
| B-937 | diflufenican | isoxadifen |
| B-938 | fluorochloridone | isoxadifen |
| B-939 | isoxaflutol | isoxadifen |
| B-940 | mesotrione | isoxadifen |
| B-941 | picolinafen | isoxadifen |
| B-942 | sulcotrione | isoxadifen |
| B-943 | tefuryltrione | isoxadifen |
| B-944 | tembotrione | isoxadifen |
| B-945 | topramezone | isoxadifen |
| B-946 | H-7 | isoxadifen |
| B-947 | atrazine | isoxadifen |
| B-948 | diuron | isoxadifen |
| B-949 | fluometuron | isoxadifen |
| B-950 | hexazinone | isoxadifen |
| B-951 | isoproturon | isoxadifen |
| B-952 | metribuzin | isoxadifen |
| B-953 | propanil | isoxadifen |
| B-954 | terbuthylazine | isoxadifen |
| B-955 | paraquat dichloride | isoxadifen |
| B-956 | flumioxazin | isoxadifen |
| B-957 | oxyfluorfen | isoxadifen |
| B-958 | saflufenacil | isoxadifen |
| B-959 | sulfentrazone | isoxadifen |
| B-960 | H-1 | isoxadifen |
| B-961 | H-2 | isoxadifen |
| B-962 | glyphosate | isoxadifen |
| B-963 | glyphosate-isopropylammonium | isoxadifen |
| B-964 | glyphosate-trimesium (sulfosate) | isoxadifen |
| B-965 | glufosinate | isoxadifen |
| B-966 | glufosinate-ammonium | isoxadifen |
| B-967 | pendimethalin | isoxadifen |
| B-968 | trifluralin | isoxadifen |
| B-969 | acetochlor | isoxadifen |
| B-970 | cafenstrole | isoxadifen |
| B-971 | dimethenamid-P | isoxadifen |
| B-972 | fentrazamide | isoxadifen |
| B-973 | flufenacet | isoxadifen |
| B-974 | mefenacet | isoxadifen |
| B-975 | metazachlor | isoxadifen |
| B-976 | metolachlor-S | isoxadifen |
| B-977 | pyroxasulfone | isoxadifen |
| B-978 | isoxaben | isoxadifen |
| B-979 | dymron | isoxadifen |
| B-980 | indanofan | isoxadifen |
| B-981 | oxaziclomefone | isoxadifen |
| B-982 | triaziflam | isoxadifen |
| B-983 | atrazine + H-1 | isoxadifen |
| B-984 | atrazine + glyphosate | isoxadifen |
| B-985 | atrazine + mesotrione | isoxadifen |
| B-986 | atrazine + nicosulfuron | isoxadifen |
| B-987 | atrazine + tembotrione | isoxadifen |
| B-988 | atrazine + topramezone | isoxadifen |
| B-989 | clomazone + glyphosate | isoxadifen |
| B-990 | diflufenican + clodinafop-propargyl | isoxadifen |
| B-991 | diflufenican + fenoxaprop-P-ethyl | isoxadifen |
| B-992 | diflufenican + flupyrsulfuron-methyl-sodium | isoxadifen |
| B-993 | diflufenican + glyphosate | isoxadifen |
| B-994 | diflufenican + mesosulfuron-methyl | isoxadifen |
| B-995 | diflufenican + pinoxaden | isoxadifen |
| B-996 | diflufenican + pyroxsulam | isoxadifen |
| B-997 | flumetsulam + glyphosate | isoxadifen |
| B-998 | flumioxazin + glyphosate | isoxadifen |
| B-999 | imazapic + glyphosate | isoxadifen |
| B-1000 | imazethapyr + glyphosate | isoxadifen |
| B-1001 | isoxaflutol + H-1 | isoxadifen |
| B-1002 | isoxaflutol + glyphosate | isoxadifen |
| B-1003 | metazachlor + H-1 | isoxadifen |
| B-1004 | metazachlor + glyphosate | isoxadifen |
| B-1005 | metazachlor + mesotrione | isoxadifen |
| B-1006 | metazachlor + nicosulfuron | isoxadifen |
| B-1007 | metazachlor + terbuthylazine | isoxadifen |
| B-1008 | metazachlor + topramezone | isoxadifen |
| B-1009 | metribuzin + glyphosate | isoxadifen |
| B-1010 | pendimethalin + H-1 | isoxadifen |
| B-1011 | pendimethalin + clodinafop-propargyl | isoxadifen |
| B-1012 | pendimethalin + fenoxaprop-P-ethyl | isoxadifen |
| B-1013 | pendimethalin + flupyrsulfuron-methyl-sodium | isoxadifen |
| B-1014 | pendimethalin + glyphosate | isoxadifen |
| B-1015 | pendimethalin + mesosulfuron-methyl | isoxadifen |
| B-1016 | pendimethalin + mesotrione | isoxadifen |
| B-1017 | pendimethalin + nicosulfuron | isoxadifen |
| B-1018 | pendimethalin + pinoxaden | isoxadifen |
| B-1019 | pendimethalin + pyroxsulam | isoxadifen |
| B-1020 | pendimethalin + tembotrione | isoxadifen |
| B-1021 | pendimethalin + topramezone | isoxadifen |
| B-1022 | pyroxasulfone + tembotrione | isoxadifen |
| B-1023 | pyroxasulfone + topramezone | isoxadifen |
| B-1024 | sulfentrazone + glyphosate | isoxadifen |
| B-1025 | terbuthylazine + H-1 | isoxadifen |
| B-1026 | terbuthylazine + foramsulfuron | isoxadifen |
| B-1027 | terbuthylazine + glyphosate | isoxadifen |
| B-1028 | terbuthylazine + mesotrione | isoxadifen |
| B-1029 | terbuthylazine + nicosulfuron | isoxadifen |
| B-1030 | terbuthylazine + tembotrione | isoxadifen |
| B-1031 | terbuthylazine + topramezone | isoxadifen |
| B-1032 | trifluralin + glyphosate | isoxadifen |
| B-1033 | clodinafop-propargyl | mefenpyr |
| B-1034 | cycloxydim | mefenpyr |
| B-1035 | cyhalofop-butyl | mefenpyr |
| B-1036 | fenoxaprop-P-ethyl | mefenpyr |
| B-1037 | pinoxaden | mefenpyr |
| B-1038 | profoxydim | mefenpyr |
| B-1039 | tepraloxydim | mefenpyr |
| B-1040 | tralkoxydim | mefenpyr |
| B-1041 | esprocarb | mefenpyr |
| B-1042 | prosulfocarb | mefenpyr |
| B-1043 | thiobencarb | mefenpyr |
| B-1044 | triallate | mefenpyr |
| B-1045 | bensulfuron-methyl | mefenpyr |
| B-1046 | bispyribac-sodium | mefenpyr |
| B-1047 | cyclosulfamuron | mefenpyr |
| B-1048 | flumetsulam | mefenpyr |
| B-1049 | flupyrsulfuron-methyl-sodium | mefenpyr |
| B-1050 | foramsulfuron | mefenpyr |
| B-1051 | imazamox | mefenpyr |
| B-1052 | imazapic | mefenpyr |
| B-1053 | imazapyr | mefenpyr |
| B-1054 | imazaquin | mefenpyr |
| B-1055 | imazethapyr | mefenpyr |
| B-1056 | imazosulfuron | mefenpyr |
| B-1057 | iodosulfuron-methyl-sodium | mefenpyr |
| B-1058 | mesosulfuron | mefenpyr |
| B-1059 | nicosulfuron | mefenpyr |
| B-1060 | penoxsulam | mefenpyr |
| B-1061 | propoxycarbazone-sodium | mefenpyr |
| B-1062 | pyrazosulfuron-ethyl | mefenpyr |
| B-1063 | pyroxsulam | mefenpyr |
| B-1064 | rimsulfuron | mefenpyr |
| B-1065 | sulfosulfuron | mefenpyr |
| B-1066 | thiencarbazone-methyl | mefenpyr |
| B-1067 | tritosulfuron | mefenpyr |
| B-1068 | 2,4-D and its salts and esters | mefenpyr |
| B-1069 | aminopyralid and its salts and esters | mefenpyr |
| B-1070 | clopyralid and its salts and esters | mefenpyr |
| B-1071 | dicamba and its salts and esters | mefenpyr |
| B-1072 | fluroxypyr-meptyl | mefenpyr |
| B-1073 | quinclorac | mefenpyr |
| B-1074 | quinmerac | mefenpyr |
| B-1075 | H-9 | mefenpyr |
| B-1076 | diflufenzopyr | mefenpyr |
| B-1077 | diflufenzopyr-sodium | mefenpyr |
| B-1078 | clomazone | mefenpyr |
| B-1079 | diflufenican | mefenpyr |
| B-1080 | fluorochloridone | mefenpyr |
| B-1081 | isoxaflutol | mefenpyr |
| B-1082 | mesotrione | mefenpyr |
| B-1083 | picolinafen | mefenpyr |
| B-1084 | sulcotrione | mefenpyr |
| B-1085 | tefuryltrione | mefenpyr |
| B-1086 | tembotrione | mefenpyr |
| B-1087 | topramezone | mefenpyr |
| B-1088 | H-7 | mefenpyr |
| B-1089 | atrazine | mefenpyr |
| B-1090 | diuron | mefenpyr |
| B-1091 | fluometuron | mefenpyr |
| B-1092 | hexazinone | mefenpyr |
| B-1093 | isoproturon | mefenpyr |
| B-1094 | metribuzin | mefenpyr |
| B-1095 | propanil | mefenpyr |
| B-1096 | terbuthylazine | mefenpyr |
| B-1097 | paraquat dichloride | mefenpyr |
| B-1098 | flumioxazin | mefenpyr |
| B-1099 | oxyfluorfen | mefenpyr |
| B-1100 | saflufenacil | mefenpyr |
| B-1101 | sulfentrazone | mefenpyr |
| B-1102 | H-1 | mefenpyr |
| B-1103 | H-2 | mefenpyr |
| B-1104 | glyphosate | mefenpyr |
| B-1105 | glyphosate-isopropylammonium | mefenpyr |
| B-1106 | glyphosate-trimesium (sulfosate) | mefenpyr |
| B-1107 | glufosinate | mefenpyr |
| B-1108 | glufosinate-ammonium | mefenpyr |
| B-1109 | pendimethalin | mefenpyr |
| B-1110 | trifluralin | mefenpyr |
| B-1111 | acetochlor | mefenpyr |
| B-1112 | cafenstrole | mefenpyr |
| B-1113 | dimethenamid-P | mefenpyr |
| B-1114 | fentrazamide | mefenpyr |
| B-1115 | flufenacet | mefenpyr |
| B-1116 | mefenacet | mefenpyr |
| B-1117 | metazachlor | mefenpyr |
| B-1118 | metolachlor-S | mefenpyr |
| B-1119 | pyroxasulfone | mefenpyr |
| B-1120 | isoxaben | mefenpyr |
| B-1121 | dymron | mefenpyr |
| B-1122 | indanofan | mefenpyr |
| B-1123 | oxaziclomefone | mefenpyr |
| B-1124 | triaziflam | mefenpyr |
| B-1125 | atrazine + H-1 | mefenpyr |
| B-1126 | atrazine + glyphosate | mefenpyr |
| B-1127 | atrazine + mesotrione | mefenpyr |
| B-1128 | atrazine + nicosulfuron | mefenpyr |
| B-1129 | atrazine + tembotrione | mefenpyr |
| B-1130 | atrazine + topramezone | mefenpyr |
| B-1131 | clomazone + glyphosate | mefenpyr |
| B-1132 | diflufenican + clodinafop-propargyl | mefenpyr |
| B-1133 | diflufenican + fenoxaprop-P-ethyl | mefenpyr |
| B-1134 | diflufenican + flupyrsulfuron-methyl-sodium | mefenpyr |
| B-1135 | diflufenican + glyphosate | mefenpyr |
| B-1136 | diflufenican + mesosulfuron-methyl | mefenpyr |
| B-1137 | diflufenican + pinoxaden | mefenpyr |
| B-1138 | diflufenican + pyroxsulam | mefenpyr |
| B-1139 | flumetsulam + glyphosate | mefenpyr |
| B-1140 | flumioxazin + glyphosate | mefenpyr |
| B-1141 | imazapic + glyphosate | mefenpyr |
| B-1142 | imazethapyr + glyphosate | mefenpyr |
| B-1143 | isoxaflutol + H-1 | mefenpyr |
| B-1144 | isoxaflutol + glyphosate | mefenpyr |
| B-1145 | metazachlor + H-1 | mefenpyr |
| B-1146 | metazachlor + glyphosate | mefenpyr |
| B-1147 | metazachlor + mesotrione | mefenpyr |
| B-1148 | metazachlor + nicosulfuron | mefenpyr |
| B-1149 | metazachlor + terbuthylazine | mefenpyr |
| B-1150 | metazachlor + topramezone | mefenpyr |
| B-1151 | metribuzin + glyphosate | mefenpyr |
| B-1152 | pendimethalin + H-1 | mefenpyr |
| B-1153 | pendimethalin + clodinafop-propargyl | mefenpyr |
| B-1154 | pendimethalin + fenoxaprop-P-ethyl | mefenpyr |
| B-1155 | pendimethalin + flupyrsulfuron-methyl-sodium | mefenpyr |
| B-1156 | pendimethalin + glyphosate | mefenpyr |
| B-1157 | pendimethalin + mesosulfuron-methyl | mefenpyr |
| B-1158 | pendimethalin + mesotrione | mefenpyr |
| B-1159 | pendimethalin + nicosulfuron | mefenpyr |
| B-1160 | pendimethalin + pinoxaden | mefenpyr |
| B-1161 | pendimethalin + pyroxsulam | mefenpyr |
| B-1162 | pendimethalin + tembotrione | mefenpyr |
| B-1163 | pendimethalin + topramezone | mefenpyr |
| B-1164 | pyroxasulfone + tembotrione | mefenpyr |
| B-1165 | pyroxasulfone + topramezone | mefenpyr |
| B-1166 | sulfentrazone + glyphosate | mefenpyr |
| B-1167 | terbuthylazine + H-1 | mefenpyr |
| B-1168 | terbuthylazine + foramsulfuron | mefenpyr |
| B-1169 | terbuthylazine + glyphosate | mefenpyr |
| B-1170 | terbuthylazine + mesotrione | mefenpyr |
| B-1171 | terbuthylazine + nicosulfuron | mefenpyr |
| B-1172 | terbuthylazine + tembotrione | mefenpyr |
| B-1173 | terbuthylazine + topramezone | mefenpyr |
| B-1174 | trifluralin + glyphosate | mefenpyr |
| B-1175 | clodinafop-propargyl | H-12 |
| B-1176 | cycloxydim | H-12 |
| B-1177 | cyhalofop-butyl | H-12 |
| B-1178 | fenoxaprop-P-ethyl | H-12 |
| B-1179 | pinoxaden | H-12 |
| B-1180 | profoxydim | H-12 |
| B-1181 | tepraloxydim | H-12 |
| B-1182 | tralkoxydim | H-12 |
| B-1183 | esprocarb | H-12 |
| B-1184 | prosulfocarb | H-12 |
| B-1185 | thiobencarb | H-12 |
| B-1186 | triallate | H-12 |
| B-1187 | bensulfuron-methyl | H-12 |
| B-1188 | bispyribac-sodium | H-12 |
| B-1189 | cyclosulfamuron | H-12 |
| B-1190 | flumetsulam | H-12 |
| B-1191 | flupyrsulfuron-methyl-sodium | H-12 |
| B-1192 | foramsulfuron | H-12 |
| B-1193 | imazamox | H-12 |
| B-1194 | imazapic | H-12 |
| B-1195 | imazapyr | H-12 |
| B-1196 | imazaquin | H-12 |
| B-1197 | imazethapyr | H-12 |
| B-1198 | imazosulfuron | H-12 |
| B-1199 | iodosulfuron-methyl-sodium | H-12 |
| B-1200 | mesosulfuron | H-12 |
| B-1201 | nicosulfuron | H-12 |
| B-1202 | penoxsulam | H-12 |
| B-1203 | propoxycarbazone-sodium | H-12 |
| B-1204 | pyrazosulfuron-ethyl | H-12 |
| B-1205 | pyroxsulam | H-12 |
| B-1206 | rimsulfuron | H-12 |
| B-1207 | sulfosulfuron | H-12 |
| B-1208 | thiencarbazone-methyl | H-12 |
| B-1209 | tritosulfuron | H-12 |
| B-1210 | 2,4-D and its salts and esters | H-12 |
| B-1211 | aminopyralid and its salts and esters | H-12 |
| B-1212 | clopyralid and its salts and esters | H-12 |
| B-1213 | dicamba and its salts and esters | H-12 |
| B-1214 | fluroxypyr-meptyl | H-12 |
| B-1215 | quinclorac | H-12 |
| B-1216 | quinmerac | H-12 |
| B-1217 | B-9 | H-12 |
| B-1218 | diflufenzopyr | H-12 |
| B-1219 | diflufenzopyr-sodium | H-12 |
| B-1220 | clomazone | H-12 |
| B-1221 | diflufenican | H-12 |
| B-1222 | fluorochloridone | H-12 |
| B-1223 | isoxaflutol | H-12 |
| B-1224 | mesotrione | H-12 |
| B-1225 | picolinafen | H-12 |
| B-1226 | sulcotrione | H-12 |
| B-1227 | tefuryltrione | H-12 |
| B-1228 | tembotrione | H-12 |
| B-1229 | topramezone | H-12 |
| B-1230 | H-7 | H-12 |
| B-1231 | atrazine | H-12 |
| B-1232 | diuron | H-12 |
| B-1233 | fluometuron | H-12 |
| B-1234 | hexazinone | H-12 |
| B-1235 | isoproturon | H-12 |
| B-1236 | metribuzin | H-12 |
| B-1237 | propanil | H-12 |
| B-1238 | terbuthylazine | H-12 |
| B-1239 | paraquat dichloride | H-12 |
| B-1240 | flumioxazin | H-12 |
| B-1241 | oxyfluorfen | H-12 |
| B-1242 | saflufenacil | H-12 |
| B-1243 | sulfentrazone | H-12 |
| B-1244 | H-1 | H-12 |
| B-1245 | H-2 | H-12 |
| B-1246 | glyphosate | H-12 |
| B-1247 | glyphosate-isopropylammonium | H-12 |
| B-1248 | glyphosate-trimesium (sulfosate) | H-12 |
| B-1249 | glufosinate | H-12 |
| B-1250 | glufosinate-ammonium | H-12 |
| B-1251 | pendimethalin | H-12 |
| B-1252 | trifluralin | H-12 |
| B-1253 | acetochlor | H-12 |
| B-1254 | cafenstrole | H-12 |
| B-1255 | dimethenamid-P | H-12 |
| B-1256 | fentrazamide | H-12 |
| B-1257 | flufenacet | H-12 |
| B-1258 | mefenacet | H-12 |
| B-1259 | metazachlor | H-12 |
| B-1260 | metolachlor-S | H-12 |
| B-1261 | pyroxasulfone | H-12 |
| B-1262 | isoxaben | H-12 |
| B-1263 | dymron | H-12 |
| B-1264 | indanofan | H-12 |
| B-1265 | oxaziclomefone | H-12 |
| B-1266 | triaziflam | H-12 |
| B-1267 | atrazine + H-1 | H-12 |
| B-1268 | atrazine + glyphosate | H-12 |
| B-1269 | atrazine + mesotrione | H-12 |
| B-1270 | atrazine + nicosulfuron | H-12 |
| B-1271 | atrazine + tembotrione | H-12 |
| B-1272 | atrazine + topramezone | H-12 |
| B-1273 | clomazone + glyphosate | H-12 |
| B-1274 | diflufenican + clodinafop-propargyl | H-12 |
| B-1275 | diflufenican + fenoxaprop-P-ethyl | H-12 |
| B-1276 | diflufenican + flupyrsulfuron-methyl-sodium | H-12 |
| B-1277 | diflufenican + glyphosate | H-12 |
| B-1278 | diflufenican + mesosulfuron-methyl | H-12 |
| B-1279 | diflufenican + pinoxaden | H-12 |
| B-1280 | diflufenican + pyroxsulam | H-12 |
| B-1281 | flumetsulam + glyphosate | H-12 |
| B-1282 | flumioxazin + glyphosate | H-12 |
| B-1283 | imazapic + glyphosate | H-12 |
| B-1284 | imazethapyr + glyphosate | H-12 |
| B-1285 | isoxaflutol + H-1 | H-12 |
| B-1286 | isoxaflutol + glyphosate | H-12 |
| B-1287 | metazachlor + H-1 | H-12 |
| B-1288 | metazachlor + glyphosate | H-12 |
| B-1289 | metazachlor + mesotrione | H-12 |
| B-1290 | metazachlor + nicosulfuron | H-12 |
| B-1291 | metazachlor + terbuthylazine | H-12 |
| B-1292 | metazachlor + topramezone | H-12 |
| B-1293 | metribuzin + glyphosate | H-12 |
| B-1294 | pendimethalin + H-1 | H-12 |
| B-1295 | pendimethalin + clodinafop-propargyl | H-12 |
| B-1296 | pendimethalin + fenoxaprop-P-ethyl | H-12 |
| B-1297 | pendimethalin + flupyrsulfuron-methyl-sodium | H-12 |
| B-1298 | pendimethalin + glyphosate | H-12 |
| B-1299 | pendimethalin + mesosulfuron-methyl | H-12 |
| B-1300 | pendimethalin + mesotrione | H-12 |
| B-1301 | pendimethalin + nicosulfuron | H-12 |
| B-1302 | pendimethalin + pinoxaden | H-12 |
| B-1303 | pendimethalin + pyroxsulam | H-12 |
| B-1304 | pendimethalin + tembotrione | H-12 |
| B-1305 | pendimethalin + topramezone | H-12 |
| B-1306 | pyroxasulfone + tembotrione | H-12 |
| B-1307 | pyroxasulfone + topramezone | H-12 |
| B-1308 | sulfentrazone + glyphosate | H-12 |
| B-1309 | terbuthylazine + H-1 | H-12 |
| B-1310 | terbuthylazine + foramsulfuron | H-12 |
| B-1311 | terbuthylazine + glyphosate | H-12 |
| B-1312 | terbuthylazine + mesotrione | H-12 |
| B-1313 | terbuthylazine + nicosulfuron | H-12 |
| B-1314 | terbuthylazine + tembotrione | H-12 |
| B-1315 | terbuthylazine + topramezone | H-12 |
| B-1316 | trifluralin + glyphosate | H-12 |
| B-1317 | 2-1 | - |
| B-1318 | 2-2 | - |
| B-1319 | 2-3 | - |
| B-1320 | 2-4 | - |
| B-1321 | 2-5 | - |
| B-1322 | 2-6 | - |
| B-1323 | 2-7 | - |
| B-1324 | 2-8 | - |
| B-1325 | 2-9 | - |
| B-1326 | 2-1 | benoxacor |
| B-1327 | 2-2 | benoxacor |
| B-1328 | 2-3 | benoxacor |
| B-1329 | 2-4 | benoxacor |
| B-1330 | 2-5 | benoxacor |
| B-1331 | 2-6 | benoxacor |
| B-1332 | 2-7 | benoxacor |
| B-1333 | 2-8 | benoxacor |
| B-1334 | 2-9 | benoxacor |
| B-1335 | 2-1 | cloquintocet |
| B-1336 | 2-2 | cloquintocet |
| B-1337 | 2-3 | cloquintocet |
| B-1338 | 2-4 | cloquintocet |
| B-1339 | 2-5 | cloquintocet |
| B-1340 | 2-6 | cloquintocet |
| B-1341 | 2-7 | cloquintocet |
| B-1342 | 2-8 | cloquintocet |
| B-1343 | 2-9 | cloquintocet |
| B-1344 | 2-1 | cyprosulfamide |
| B-1345 | 2-2 | cyprosulfamide |
| B-1346 | 2-3 | cyprosulfamide |
| B-1347 | 2-4 | cyprosulfamide |
| B-1348 | 2-5 | cyprosulfamide |
| B-1349 | 2-6 | cyprosulfamide |
| B-1350 | 2-7 | cyprosulfamide |
| B-1351 | 2-8 | cyprosulfamide |
| B-1352 | 2-9 | cyprosulfamide |
| B-1353 | 2-1 | dichlormid |
| B-1354 | 2-2 | dichlormid |
| B-1355 | 2-3 | dichlormid |
| B-1356 | 2-4 | dichlormid |
| B-1357 | 2-5 | dichlormid |
| B-1358 | 2-6 | dichlormid |
| B-1359 | 2-7 | dichlormid |
| B-1360 | 2-8 | dichlormid |
| B-1361 | 2-9 | dichlormid |
| B-1362 | 2-1 | fenchlorazole |
| B-1363 | 2-2 | fenchlorazole |
| B-1364 | 2-3 | fenchlorazole |
| B-1365 | 2-4 | fenchlorazole |
| B-1366 | 2-5 | fenchlorazole |
| B-1367 | 2-6 | fenchlorazole |
| B-1368 | 2-7 | fenchlorazole |
| B-1369 | 2-8 | fenchlorazole |
| B-1370 | 2-9 | fenchlorazole |
| B-1371 | 2-1 | isoxadifen |
| B-1372 | 2-2 | isoxadifen |
| B-1373 | 2-3 | isoxadifen |
| B-1374 | 2-4 | isoxadifen |
| B-1375 | 2-5 | isoxadifen |
| B-1376 | 2-6 | isoxadifen |
| B-1377 | 2-7 | isoxadifen |
| B-1378 | 2-8 | isoxadifen |
| B-1379 | 2-9 | isoxadifen |
| B-1380 | 2-1 | mefenpyr |
| B-1381 | 2-2 | mefenpyr |
| B-1382 | 2-3 | mefenpyr |
| B-1383 | 2-4 | mefenpyr |
| B-1384 | 2-5 | mefenpyr |
| B-1385 | 2-6 | mefenpyr |
| B-1386 | 2-7 | mefenpyr |
| B-1387 | 2-8 | mefenpyr |
| B-1388 | 2-9 | mefenpyr |
| B-1389 | 2-1 | H-11 |
| B-1390 | 2-2 | H-11 |
| B-1391 | 2-3 | H-11 |
| B-1392 | 2-4 | H-11 |
| B-1393 | 2-5 | H-11 |
| B-1394 | 2-6 | H-11 |
| B-1395 | 2-7 | H-11 |
| B-1396 | 2-8 | H-11 |
| B-1397 | 2-9 | H-11 |
| B-1398 | 2-1 | H-12 |
| B-1399 | 2-2 | H-12 |
| B-1400 | 2-3 | H-12 |
| B-1401 | 2-4 | H-12 |
| B-1402 | 2-5 | H-12 |
| B-1403 | 2-6 | H-12 |
| B-1404 | 2-7 | H-12 |
| B-1405 | 2-8 | H-12 |
| B-1406 | 2-9 | H-12 |

The compounds of formula I and the compositions according to the invention may also have a plant-strengthening action. Accordingly, they are suitable for mobilizing the defense system of the plants against attack by unwanted microorganisms, such as harmful fungi, but also viruses and bacteria. Plant-strengthening (resistance-inducing) substances are to be understood as meaning, in the present context, those substances which are capable of stimulating the defense system of treated plants in such a way that, when subsequently inoculated by unwanted microorganisms, the treated plants display a substantial degree of resistance to these microorganisms.

The compounds of formula I can be employed for protecting plants against attack by unwanted microorganisms within a certain period of time after the treatment. The period of time within which their protection is effected generally extends from 1 to 28 days, preferably from 1 to 14 days, after the treatment of the plants with the compounds of formula I, or, after treatment of the seed, for up to 9 months after sowing.

The compounds of formula I and the compositions according to the invention are also suitable for increasing the harvest yield.

Moreover, they have reduced toxicity and are tolerated well by the crop plants.

### Examples

The preparation of the compounds of formula I is illustrated by examples; however, the subject matter of the present invention is not limited to the examples given. With appropriate modification of the starting materials, the procedures given in the examples below were used to obtain further compounds I. The compounds obtained in this manner are listed in table C, together with physical data. The products shown below were characterized by determination of the melting point, NMR spectroscopy or the masses ([m/z]) determined by HPLC-MS spectrometry.
HPLC-MS: high performance liquid chromatography coupled with mass spectrometry;
MeOD: Tetradeuteromethanol
MS: Mass spectroscopy
THF: tetrahydrofuran
TFA: trifluoroacetic acid
s: singlet
d: doublet
t: triplet
m: multiplet
HPLC column: RP-18 column (Chromolith Speed ROD from Merck KgaA, Germany), 50*4.6 mm; mobile phase: acetonitrile + 0.1% TFA/water + 0.1% TFA, using a gradient from 5:95 to 100:0 over 5 minutes at 40 °C, flow rate 1.8 mL/min.
MS: quadrupole electrospray ionization, 80 V (positive mode).
HPLC column: Luna-C18(2) 5 µm column (Phenomenex), 2.0*50 mm; mobile phase: acetonitrile + 0.0625% TFA/water + 0.0675% TFA, using a gradient from 10:90 to 80:20 over 4.0 minutes at 40 °C, flow rate 0.8 mL/min.
MS: quadrupole electrospray ionization, 70 V (positive mode).

### Example 1: 4-bromo-3-(diethylcarbamothioylamino)-6-fluoro-2-methyl-N-(1-methyltetrazol-5-yl)benzamide (compound formula (I.A' .VII), where, R¹, R^{2a}, R^{2b} and R³ are as defined in line 722 of table A

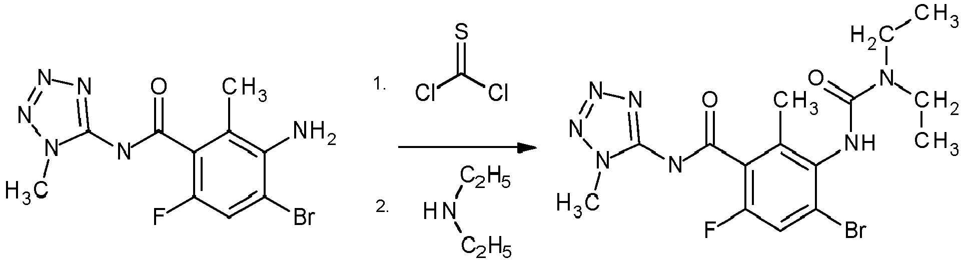

### Step 1: 4-bromo-6-fluoro-3-isothiocyanato-2-methyl-N-(1-methyltetrazol-5-yl)benzamide

A solution of 3-amino-4-bromo-6-fluoro-2-methyl-N-(1-methyltetrazol-5-yl)benzamide (8.0 g, 24 mmol) in 500 ml THF was cooled to 0 °C and thiophosgene (3.2 g, 28 mmol) was added. The mixture was heated to reflux for 8 hours and stirred for additional 48 h at 22°C. The obtained crude solution of the isothiocyanate was used without further purification in step 2.

### Step 2: 4-bromo-3-(diethylcarbamothioylamino)-6-fluoro-2-methyl-N-(1-methyltetrazol-5-yl)benzamide

An aliquot of the solution of 4-bromo-6-fluoro-3-isothiocyanato-2-methyl-N-(1-methyltetrazol-5-yl)benzamide in THF (containing ca. 400 mg of the isothiocyanate, 1.08 mmol) was treated with triethylamine (218 mg) and a solution of diethylamine (79 mg) in THF (5 ml). The mixture was stirred for 4 h at 22°C. The solvent was evaporated in vacuo and the residue was dissolved in ethyl acetate. The thus obtained solution was washed with 2N aqueous hydrochloric acid and brine and dried over magnesium sulfate. The solvent was evaporated in vacuo and the remainder was purified by column chromatography to yield 164 mg of the product as a solid.
¹H NMR (400 MHz, MeOD), δ 7.50 (d, 1 H), 4.05 (s, 3 H), 3.7-3.9 (m, 4 H), 2.35 (s, 3 H), 1.3 (t, 6 H).

By analogy to the method described in the Example 1, the following compounds of formula (I.A.' VII) according to Table C (examples 2 to 12) and compounds of formula (I.B' .VII) according to Table F (examples 13 and 14) were prepared:

**Table C:**

| | | | | | | |
|---|---|---|---|---|---|---|
| 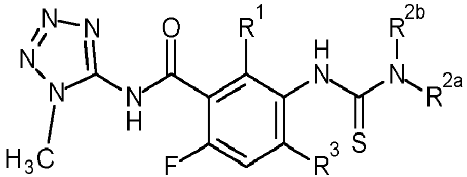 | | | | I.A' .VII | | |

| cpd. no | in table A | R¹ | R3 | R^{2a} | R^{2b} | MS (m/z) |
|---|---|---|---|---|---|---|
| 1 | 722 | CH₃ | CH₃CH₂ | CH₃CH₂ | Br | 444.0 |
| 2 | 644 | CH₃ | CH₃CH₂ | CH₃ | Br | 432.0 |
| 3 | 692 | CH₃ | C₆H₅ | CH₃ | Br | 478.0 |
| 4 | 1088 | CH₃ | NR^{2a}R^{2b} = | 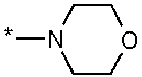 | Br | 457.9 |
| 5 | 932 | CH₃ | cyclopropyl | cyclopropyl | Br | 470.0 |
| 6 | 656 | CH₃ | (CH₃)₂CH | CH₃ | Br | 446.0 |
| 7 | 650 | CH₃ | CH₃CH₂CH₂ | CH₃ | Br | 446.0 |
| 8 | 746 | CH₃ | cyclopropyl | CH₃CH₂ | Br | 456.0 |
| 9 | 632 | CH₃ | 2,6-(CH₃)₂-C₆H₃ | H | Br | 493.9 |
| 10 | 578 | CH₃ | (CH₃)₃C | H | Br | 445.9 |
| 11 | 608 | CH₃ | C₆H₅ | H | Br | 447.8 |
| 12 | 584 | CH₃ | cyclopropyl | H | Br | 427.8 |

**Table D:**

| | | | | | | |
|---|---|---|---|---|---|---|
| 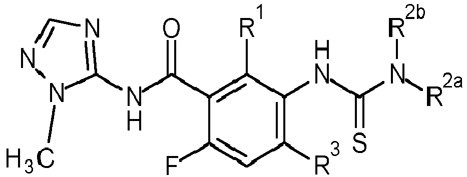 | | | | I.B' .VII | | |

| cpd. no | in table A | R¹ | R3 | R^{2a} | R^{2b} | MS (m/z) |
|---|---|---|---|---|---|---|
| 1 | 722 | CH₃ | CH₃CH₂ | CH₃CH₂ | Br | 442.9 |
| 2 | 644 | CH₃ | CH₃CH₂ | CH₃ | Br | 430.8 |

### Use examples

The herbicidal activity of the compounds of formula I was demonstrated by the following greenhouse experiments:
The culture containers used were plastic flowerpots containing loamy sand with approximately 3.0% of humus as the substrate. The seeds of the test plants were sown separately for each species.

For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the plants had rooted. This cover caused uniform germination of the test plants, unless this had been impaired by the active ingredients.

For the post-emergence treatment, the test plants were first grown to a height of 3 to 15 cm, depending on the plant habit, and only then treated with the active ingredients which had been suspended or emulsified in water by means of finely distributing nozzles. For this purpose, the test plants were either sown directly and grown in the same containers, or they were first grown separately as seedlings and transplanted into the test containers a few days prior to treatment.

Depending on the species, the plants were kept at 10 - 25°C or 20 - 25°C, respectively. The test period extended over 2 to 4 weeks. During this time, the plants were tended, and their response to the individual treatments was evaluated.

Evaluation was carried out using a scale from 0 to 100. 100 means no emergence of the plants, or complete destruction of at least the aerial moieties, and 0 means no damage, or normal course of growth. A good herbicidal activity is given at values of at least 70 and a very good herbicidal activity is given at values of at least 85.

### Test series 1:

At an application rate of 250 g/ha the following compounds were tested in post-emergence tests against:
ALOMY (Alopecurus myosuroiedes)
ECHCG (Echinocloa crus-galli)
SETFA (Setaria faberi)
In test series 1, compounds of examples 1, 2, 4, 5, 6, 7 and 8 showed > 85 % control of ALOMY.
In test series 1, compounds of examples 1 to 10 showed > 85 % control of ECHCG.
In test series 1, compounds of examples 1, 2, 4, 5, 7, 8, 9 and 10 showed > 85 % control of SETFA.

## Claims

1. A compound of formula I, 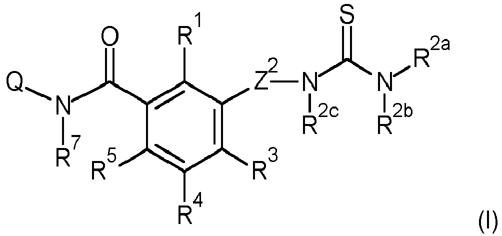 wherein
Q is selected from the group consisting of radicals of the formulae Q¹, Q², Q³ and Q⁴; 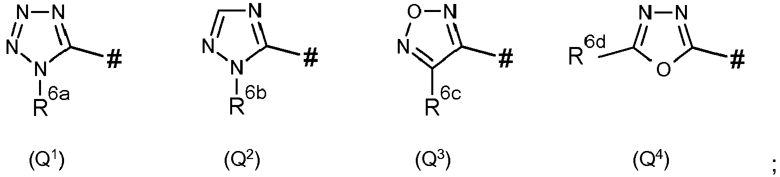
where # in formulae Q¹, Q², Q³ and Q⁴ indicates the point of attachment to the nitrogen atom;
R¹ is selected from the group consisting of cyano, halogen, nitro, C₁-C₈-alkyl, C₂-C₈-alkenyl, C₂-C₈-alkynyl, C₁-C₈-haloalkyl, C₁-C₄-alkoxy-C₁-C₄-alkyl, C₁-C₄-haloalkoxy-C₁-C₄-alkyl, C₁-C₈-alkoxy, C₁-C₄-alkoxy-C₁-C₄-alkoxy-Z¹-, C₁-C₆-haloalkoxy, R^{1b}-S(O)ₖ-Z¹-;
R^{2a} is selected from the group consisting of hydrogen, C₁-C₆-alkyl, C₁-C₆-alkoxy, C₂-C₆-alkenyl, C₂-C₆-alkynyl, C₁-C₆-alkylamino, di-C₁-C₆-alkylamino, C₃-C₁₀-cycloalkyl and C₃-C₁₀-cycloalkyl-C₁-C₄-alkyl, where the alkyl, alkoxy, alkenyl, alkynyl and cycloalkyl parts of the eight aforementioned radicals are unsubstituted, partially or completely halogenated, or substituted by 1 or 2 radicals R^{2f} and where the cycloalkyl parts may also be fused to a benzene ring, which is unsubstituted or substituted by 1, 2, 3 or 4 radicals R²¹, which are identical or different,
R^{2a} may also be selected from phenyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups R²¹, which are identical or different;
R^{2b} is selected from the group consisting of hydrogen, C₁-C₆-alkyl, C₂-C₆-alkenyl, C₂-C₆-alkynyl, C₃-C₇-cycloalkyl, C₃-C₇-cycloalkyl-C₁-C₄-alkyl, where the alkyl, alkenyl, alkynyl and cycloalkyl parts of the five aforementioned radicals are unsubstituted, partially or completely halogenated, or substituted by 1 or 2 radicals R^{2f};
R^{2b} may also be selected from phenyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups R²¹, which are identical or different;
or
R^{2a}, R^{2b} together with the nitrogen atom, to which they are bound may form a 4-, 5-, 6-, 7-or 8-membered, saturated or partially unsaturated heterocyclic radical, which may carry as a ring member a further heteroatom selected from O, S and N and where the heterocyclic radical is unsubstituted or carries 1, 2, 3 or 4 groups R^{2d} or carries 1 group R^{2e} and 0, 1, 2 or 3 groups R^{2d},
R^{2c} is selected from the group consisting of hydrogen, C₁-C₆-alkyl, C₂-C₆-alkenyl, C₂-C₆-alkynyl, C₁-C₆-alkylamino, di-C₁-C₆-alkylamino, C₃-C₁₀-cycloalkyl and C₃-C₁₀-cycloalkyl-C₁-C₄-alkyl, where the alkyl, alkenyl, alkynyl and cycloalkyl parts of the seven aforementioned radicals are unsubstituted, partially or completely halogenated, or substituted by 1 or 2 radicals R^{2f} and where the cycloalkyl parts may also be fused to a benzene ring, which is unsubstituted or substituted by 1, 2, 3 or 4 radicals R²¹, which are identical or different,
R^{2c} may also be selected from phenyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups R²¹, which are identical or different;
R^{2d} are identical or different and selected from the group consisting of halogen, CN, C₁-C₄-alkyl, C₁-C₄-haloalkyl, C₁-C₄-alkoxy and C₁-C₄-haloalkoxy, or two groups R^{2d}, which are bound to adjacent carbon atoms may together form a fused benzene ring, which is unsubstituted or substituted by 1, 2, 3 or 4 groups R²¹, which are identical or different;
R^{2e} is selected from the group consisting of =O, OH, R^{4b}-S(O)ₖ-Z⁴-, R^{4c}-C(O)-Z⁴-, R^{4d}O-C(O)-Z⁴-, R^{4e}R^{4f}N-C(O)-Z⁴-, R^{4g}R^{4h}N-Z⁴- and R^{4e}R^{4f}NS(O)₂-Z⁴-;
R^{2f} is selected from the group consisting of OH, CN, NH₂, SH, SCN, nitro, tri(C₁-C₄-alkyl)silyl, C₁-C₄-alkoxy, C₁-C₄-haloalkoxy, R^{3e}R^{3f}N-, R^{3c}-C(O)-, HO-C(O)-R^{3d}O-C(O)-, R^{3d}S-C(O)-, R^{3e}R^{3f}N-C(O)-, R^{3e}R^{3f}N-NR^{3h}-C(O)-, R^{3c}-C(S)-, R^{3d}O-C(S)-, R^{3d}S-C(S)-, R^{3e}R^{3f}N-C(S), R^{3c}-S(O)ₙ-, R^{3c}-S(O)₂-O-, R^{3d}O-S(O)₂-, R^{3e}R^{3f}NS(O)₂-, R^{3c}-C(O)-NR^{3g}-, R^{3d}O-C(O)-NR^{3g}-, R^{3e}R^{3f}N-C(O)-NR^{3g}-, R^{3e}R^{3f}N-NR^{3h}-C(O)-NR^{3g}-, R^{3d}O-S(O)₂-NR^{3g}-, R^{3e}R^{3f}NS(O)₂-NR^{3g}-, R^{3c}-C(O)-NR^{3g}-C(O)-, R^{3d}O-C(O)-NR^{3g}-C(O)-, R^{3e}R^{3f}N-C(O)-NR^{3g}-C(O)-, R^{3d}O-S(O)₂-NR^{3g}-C(O)-, R^{3e}R^{3f}NS(O)₂-NR^{3g}-C(O)-, R^{3c}-C(O)-NR^{3g}-S(O)₂-, R^{3d}O-C(O)-NR^{3g}-S(O)₂-, R^{3e}R^{3f}N-C(O)-NR^{3g}-S(O)₂-, (OH)₂P(O)-, (C₁-C₄-alkoxy)₂P(O)-, R^{3d}O-N=CH-, phenyl-X and heterocyclyl-X, where heterocyclyl in the last two mentioned radicals is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups R¹¹, which are identical or different;
R³ is selected from the group consisting of hydrogen, cyano, thiocyanato, halogen, nitro, hydroxy-Z³-, C₁-C₆-alkyl, C₂-C₈-alkenyl, C₂-C₈-alkynyl, C₃-C₁₀-cycloalkyl-Z³-, C₃-C₆-cycloalkenyl-Z³-, C₃-C₁₀-cycloalkoxy-Z³-, C₃-C₁₀-cycloalkyl-C₁-C₂-alkoxy, where the cyclic groups of the four aforementioned radicals are unsubstituted or partially or completely halogenated, C₁-C₄-cyanoalkyl, C₁-C₈-haloalkyl, C₂-C₈-haloalkenyl, C₃-C₈-haloalkynyl, C₁-C₈-alkoxy-Z³-, C₁-C₈-haloalkoxy-Z³-, C₁-C₄-alkoxy-C₁-C₄-alkoxy-Z³-, C₁-C₄-haloalkoxy-C₁-C₄-alkoxy-Z³-, C₂-C₈-alkenyloxy-Z³-, C₂-C₈-alkynyloxy-Z³-, C₂-C₈-haloalkenyloxy-Z³-, C₃-C₈-haloalkynyloxy-Z³-, R^{3b}-S(O)ₖ-Z³-, R^{3c}-C(O)-Z³-, R^{3d}O-C(O)-Z³-, R^{3d}O-N=CH-Z³-, R^{3e}R^{3f}N-C(O)-Z³-, R^{3g}R^{3h}N-Z³-, R²²C(O)O-Z³-, R²⁵OC(O)O-Z³-, (R²²)₂NC(O)O-Z³-, R²⁵S(O)₂O-Z³-, R²²OS(O)₂-Z³-, (R²²)₂NS(O)₂-Z³-, R²⁵OC(O)N(R²²)-Z³-, (R²²)₂NC(O)N(R²²)-Z³-, (R²²)₂NS(O)₂N(R²²)-Z³-, (OH)₂P(O)-Z³-, (C₁-C₄-alkoxy)₂P(O)-Z³-, phenyl-Z^{3a}-, heterocyclyl-Z^{3a}-, where heterocyclyl is a 3-, 4-, 5- or 6-membered monocyclic or 8-, 9- or 10-membered bicyclic saturated, partially unsaturated or aromatic heterocycle, which contains 1, 2, 3 or 4 heteroatoms as ring members, which are selected from the group consisting of O, N and S, where cyclic groups in phenyl-Z^{3a}-and heterocyclyl-Z^{3a}- are unsubstituted or substituted by 1, 2, 3 or 4 groups R²¹, which are identical or different;
R⁴ is selected from the group consisting of hydrogen, halogen, cyano-Z¹, C₁-C₈-alkyl, nitro, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₂-C₈-alkenyl, C₂-C₈-alkynyl, C₁-C₈-haloalkyl, C₁-C₃-alkylamino, C₁-C₃-dialkylamino, C₁-C₃-alkylamino-S(O)ₖ, C₁-C₃-alkylcarbonyl, C₁-C₈-alkoxy, C₁-C₄-alkoxy-C₁-C₄-alkyl, C₁-C₄-alkoxy-C₁-C₄-alkoxy-Z¹-, C₁-C₄-alkylthio-C₁-C₄-alkylthio-Z¹-, C₂-C₆-alkenyloxy, C₂-C₆-alkynyloxy, C₁-C₆-haloalkoxy, C₁-C₄-haloalkoxy-C₁-C₄-alkyl, C₁-C₄-haloalkoxy-C₁-C₄-alkoxy-Z¹-, R^{1b}-S(O)ₖ-Z¹-, phenoxy-Z¹- and heterocyclyloxy-Z¹-, where heterocyclyloxy is an oxygen bound 5- or 6- membered monocyclic or 8-, 9- or 10-membered bicyclic saturated, partially unsaturated or aromatic heterocycle, which contains 1, 2, 3 or 4 heteroatoms as ring members, which are selected from the group consisting of O, N and S, where the cyclic groups in phenoxy and heterocyclyloxy are unsubstituted or substituted by 1, 2, 3 or 4 groups R¹¹, which are identical or different;
R⁵ is hydrogen, halogen, cyano-Z¹-, nitro, C₁-C₈-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₂-C₈-alkenyl, C₂-C₈-alkynyl, C₁-C₈-haloalkyl, C₁-C₃-alkylamino, C₁-C₃-dialkylamino, C₁-C₃-alkylamino-S(O)ₖ, C₁-C₃-alkylcarbonyl, C₁-C₈-alkoxy, C₁-C₄-alkoxy-C₁-C₄-alkyl, C₁-C₄-alkoxy-C₁-C₄-alkoxy-Z¹-, C₁-C₄-alkylthio-C₁-C₄-alkyl, C₁-C₄-alkylthio-C₁-C₄-alkylthio-Z¹-, C₂-C₆-alkenyloxy, C₂-C₆-alkynyloxy, C₁-C₆-haloalkoxy, C₁-C₄-haloalkoxy-C₁-C₄-alkyl, C₁-C₄-haloalkoxy-C₁-C₄-alkoxy-Z¹-, R^{1b}-S(O)ₖ-Z¹-, phenoxy-Z¹- and heterocyclyloxy-Z¹-, where heterocyclyloxy is an 5- or 6-membered monocyclic or 8-, 9- or 10-membered bicyclic saturated, partially unsaturated or aromatic heterocycle which is bound via an oxygen atom and which contains 1, 2, 3 or 4 heteroatoms as ring members, which are selected from the group consisting of O, N and S, where the cyclic groups in phenoxy and heterocyclyloxy are unsubstituted or substituted by 1, 2, 3 or 4 groups R¹¹, which are identical or different;
R6a, R^{6b}, R^{6c}, R^{6d} are, independently of each other, selected from the group consisting of C₁-C₆-alkyl, C₃-C₇-cycloalkyl, C₃-C₇-cycloalkyl-C₁-C₄-alkyl, where the C₃-C₇-cycloalkyl groups of the two aforementioned radicals are unsubstituted or partially or completely halogenated, C₁-C₆-haloalkyl, C₂-C₆-alkenyl, C₂-C₆-alkynyl, C₂-C₆-haloalkenyl, C₃-C₆-haloalkynyl, C₁-C₄-alkoxy-C₁-C₄-alkyl, C₁-C₄-haloalkoxy-C₁-C₄-alkyl, R^{b}-S(O)ₙ-C₁-C₃-alkyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
R⁷ is selected from the group consisting of hydyrogen, CN, NH₂, C₁-C₆-alkyl, C₃-C₇-cycloalkyl, C₃-C₇-cycloalkyl-C₁-C₄-alkyl, where the C₃-C₇-cycloalkyl groups of the two aforementioned radicals are unsubstituted or partially or completely halogenated, C₁-C₆-haloalkyl, C₂-C₆-alkenyl, C₂-C₆-alkynyl, C₂-C₆-haloalkenyl, C₃-C₆-haloalkynyl, C₁-C₄-alkoxy-C₁-C₄-alkyl, C₁-C₄-haloalkoxy-C₁-C₄-alkyl, C₁-C₄-cyanoalkyl, R^{3b}-S(O)ₖ-Z^{3b}-, R^{3c}-C(O)-Z^{3b}-, R^{3d}O-C(O)-Z^{3b}-, R^{3d}O-N=CH-Z^{3b}-, R^{3e}R^{3f}N-C(O)-Z^{3b}-, R^{3g}R^{3h}N-Z^{3b}-, R²²C(O)O-Z^{3b}-, R²⁵OC(O)O-Z^{3b}-, (R²²)₂NC(O)O-Z^{3b}-, R²⁵S(O)₂O-Z^{3b}-, R²²OS(O)₂-Z^{3b}-, (R²²)₂NS(O)₂-Z^{3b}-, R²⁵OC(O)N(R²²)-Z^{3b}-, (R²²)₂NC(O)N(R²²)-Z^{3b}-, (R²²)₂NS(O)₂N(R²²)-Z^{3b}-, (OH)₂P(O)-Z^{3b}-, (C₁-C₄-alkoxy)₂P(O)-Z^{3b}-, phenyl-Z^{3a}-, heterocyclyl-Z^{3a}-, where heterocyclyl is a 3-, 4-, 5- or 6-membered monocyclic or 8-, 9-or 10-membered bicyclic saturated, partially unsaturated or aromatic heterocycle, which contains 1, 2, 3 or 4 heteroatoms as ring members, which are selected from the group consisting of O, N and S, where cyclic groups in phenyl-Z^{3a}- and heterocyclyl-Z^{3a}- are unsubstituted or substituted by 1, 2, 3 or 4 groups R²¹, which are identical or different;
R¹¹, R²¹ independently of each other are selected from the group consisting of cyano, OH, halogen, nitro, C₁-C₆-alkyl, C₃-C₇-cycloalkyl, C₃-C₇-halocycloalkyl, C₁-C₆-haloalkyl, C₂-C₆-alkenyl, C₂-C₆-haloalkenyl, C₂-C₆-alkynyl, C₃-C₆-haloalkynyl, C₁-C₆-alkoxy, C₁-C₄-alkoxy-C₁-C₄-alkyl, C₁-C₄-alkylthio-C₁-C₄-alkyl, C₁-C₄-haloalkoxy-C₁-C₄-alkyl, C₁-C₄-alkoxy-C₁-C₄-alkoxy, C₃-C₇-cycloalkoxy and C₁-C₆-haloalkoxy, or two radicals R¹¹ or R²¹, resepctively, which are bound to the same carbon atom, together may form a group =O or two radicals R¹¹ or R²¹, resepctively, which are bound to adjacent carbon atoms, together may form a group selected from C₃-C₅-alkylene, O-C₂-C₄-alkylene and O-(C₁-C₃-alkylene)-O-;
Z¹, Z³ independently of each other are selected from the group consisting of a covalent bond and C₁-C₄-alkanediyl, which is unsubstituted, partly or completely fluorinated;
Z² is a covalent bond or a bivalent radical selected from C₁-C₄-alkanediyl and C₁-C₄-alkanediyl-O-C₁-C₄-alkanediyl,
where in the last two mentioned radicals the C₁-C₄-alkanediyl groups are linear and where the C₁-C₄-alkanediyl is unsubstituted, or partly or completely fluorinated or substituted by 1, 2, 3 or 4 groups R^{z},
Z^{3a} is selected from the group consisting of a covalent bond, C₁-C₄-alkanediyl, O-C₁-C₄-alkanediyl, C₁-C₄-alkanediyl-O and C₁-C₄-alkanediyl-O-C₁-C₄-alkanediyl;
Z^{3b} is selected from the group consisting of C₁-C₄-alkanediyl, C₁-C₄-alkanediyl-O and C₁-C₄-alkanediyl-O-C₁-C₄-alkanediyl;
R^{z} are identical or different and selected from the group consisting of C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₁-C₆-alkoxy, C₂-C₆-alkenyl, C₁-C₄-alkyl-C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkylS(O)ₙ-C₁-C₄-alkyl, C₁-C₄-alkylamino-C₁-C₄-alkyl, C₁-C₄-dialkylamino-C₁-C₄-alkyl, C₁-C₆-cyanoalkyl, phenyl, benzyl, heterocyclyl and heterocyclylmethyl, where heterocyclyl in the last two mentioned radicals is a 5- or 6-membered monocyclic saturated, partially unsaturated or aromatic heterocycle, which contains 1, 2, 3 or 4 heteroatoms as ring members, which are selected from the group consisting of O, N and S, where phenyl, benzyl, heterocyclyl and heterocyclylmethyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
R^{b}, R^{1b}, R^{3b}, R^{4b} independently of each other are selected from the group consisting of C₁-C₆-alkyl, C₃-C₇-cycloalkyl, C₁-C₆-haloalkyl, C₂-C₆-alkenyl, C₂-C₆-haloalkenyl, C₂-C₆-alkynyl, C₃-C₆-haloalkynyl, phenyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
R^{3c}, R^{4c} independently of each other are selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₁-C₆-alkoxy, C₂-C₆-alkenyl, C₁-C₄-alkyl-C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkyl-S(O)ₙ-C₁-C₄-alkyl, C₁-C₄-alkylamino-C₁-C₄-alkyl, C₁-C₄-dialkylamino-C₁-C₄-alkyl, C₁-C₆-cyanoalkyl, phenyl, benzyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, benzyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
R^{3d}, R^{4d} independently of each other are selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₁-C₆-alkoxy, C₂-C₆-alkenyl, C₁-C₄-alkyl-C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkyl-S(O)ₙ-C₁-C₄-alkyl, C₁-C₄-alkylamino-C₁-C₄-alkyl, C₁-C₄-dialkylamino-C₁-C₄-alkyl, C₁-C₆-cyanoalkyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
R^{3e}, R^{3f}, R^{4e}, R^{4f} independently of each other are selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy; or
R^{3e} and R^{3f} or R^{4e} and R^{4f} together with the nitrogen atom, to which they are bound may form a 4-, 5-, 6- or 7-membered, saturated or unsaturated heterocyclic radical, which may carry as a ring member a further heteroatom selected from O, S and N and which is unsubstituted or carries 1, 2, 3 or 4 groups, which are identical or different and selected from the group consisting of =O, halogen, C₁-C₄-alkyl, C₁-C₄-haloalkyl, C₁-C₄-alkoxy and C₁-C₄-haloalkoxy;
R^{3g}, R^{4g} independently of each other are selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkylsulfonyl, C₁-C₄-alkylcarbonyl, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy;
R^{3h}, R^{4h} independently of each other are selected from the group consisting of hydrogen, C₁-C₆-alkyl, C₃-C₇-cycloalkyl, C₃-C₇-cycloalkyl-C₁-C₄-alkyl, where the C₃-C₇-cycloalkyl groups in the two aforementioned radicals are unsubstituted or partially or completely halogenated, C₁-C₆-haloalkyl, C₂-C₆-alkenyl, C₂-C₆-haloalkenyl, C₂-C₆-alkynyl, C₃-C₆-haloalkynyl, C₁-C₄-alkoxy-C₁-C₄-alkyl, C₁-C₄-alkylsulfonyl, C₁-C₄-alkylcarbonyl, a radical C(O)R^{k}, phenyl and benzyl, where phenyl and benzyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy; or
R^{3g} and R^{3h} or R^{4g} and R^{4h} together with the nitrogen atom, to which they are bound may form a 4-, 5-, 6- or 7-membered, saturated or partially unsaturated heterocyclic radical, which may carry as a ring member a further heteroatom selected from O, S and N and which is unsubstituted or carries 1, 2, 3 or 4 groups, which are identical or different and selected from the group consisting of =O, halogen, C₁-C₄-alkyl, C₁-C₄-haloalkyl, C₁-C₄-alkoxy and C₁-C₄-haloalkoxy;
R²² is selected from the group consisting of hydrogen, C₁-C₆-alkyl, C₁-C₆-haloalkyl, C₂-C₆-alkenyl, C₂-C₆-haloalkenyl, C₂-C₆-alkynyl, C₃-C₆-haloalkynyl, C₃-C₆-cycloalkyl, C₃-C₆-cycloalkenyl, C₃-C₆-halocycloalkyl, C₃-C₆-cycloalkyl-C₁-C₆-alkyl, C₁-C₆-alkoxy-C₁-C₆-alkyl, C₃-C₆-cycloalkyl-C₁-C₆-alkoxy-C₁-C₆-alkyl, phenyl-Z¹, phenyl-O-C₁-C₆-alkyl, phenyl-N(R²³)-C₁-C₆-alkyl, phenyl-S(O)ₙ-C₁-C₆-alkyl, heterocyclyl-Z¹, heterocyclyl-N(R²³)-C₁-C₆-alkyl, heterocyclyl-O-C₁-C₆-alkyl, heterocyclyl-S(O)ₙ-C₁-C₆-alkyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, heterocyclyl are unsubstituted or substituted by 1, 2, 3 or 4 groups , which are identical or different and selected from the group consisting of cyano, halogen, nitro, thiocyanato, C₁-C₆-alkyl, C₁-C₆-haloalkyl, C₃-C₆-cycloalkyl, C(O)OR²³, C(O)N(R²³)₂, OR²³, N(R²³)₂, S(O)ₙR²⁴, S(O)₂OR²³, S(O)₂N(R²³)₂, and R²³O-C₁-C₆-alkyl, and where heterocyclyl bears 0, 1 or 2 oxo groups;
R²³ is selected from the group consisting of hydrogen, C₁-C₆-alkyl, C₁-C₆-haloalkyl, C₂-C₆-alkenyl, C₂-C₆-alkynyl, C₃-C₆-cycloalkyl, C₃-C₆-cycloalkyl-C₁-C₆-alkyl, and phenyl;
R²⁴ is selected from the group consisting of C₁-C₆-alkyl, C₁-C₆-haloalkyl, C₂-C₆-alkenyl, C₂-C₆-alkynyl, C₃-C₆-cycloalkyl, C₃-C₆-cycloalkyl-C₁-C₆-alkyl, and phenyl;
R²⁵ is selected from the group consisting of C₁-C₆-alkyl, C₁-C₆-haloalkyl, C₂-C₆-alkenyl, C₂-C₆-haloalkenyl, C₂-C₆-alkynyl, C₃-C₆-haloalkynyl, C₃-C₆-cycloalkyl, C₃-C₆-cycloalkenyl, C₃-C₆-halocycloalkyl, C₃-C₆-cycloalkyl-C₁-C₆-alkyl, C₁-C₆-alkoxy-C₁-C₆-alkyl, C₃-C₆-cycloalkyl-C₁-C₆-alkoxy-C₁-C₆-alkyl, phenyl-Z¹, phenyl-O-C₁-C₆-alkyl, phenyl-N(R²³)-C₁-C₆-alkyl, phenyl-S(O)ₙ-C₁-C₆-alkyl, heterocyclyl-Z¹, heterocyclyl-N(R²³)-C₁-C₆-alkyl, heterocyclyl-O-C₁-C₆-alkyl, heterocyclyl-S(O)ₙ-C₁-C₆-alkyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, heterocyclyl are unsubstituted or substituted by 1, 2, 3 or 4 groups , which are identical or different and selected from the group consisting of cyano, halogen, nitro, thiocyanato, C₁-C₆-alkyl, C₁-C₆-haloalkyl, C₃-C₆-cycloalkyl, C(O)OR²³, C(O)N(R²³)₂, OR²³, N(R²³)₂, S(O)ₙR²⁴, S(O)₂OR²³, S(O)₂N(R²³)₂, and R²³O-C₁-C₆-alkyl, and where heterocyclyl bears 0, 1 or 2 oxo groups;
X is a covalent bond, O, S(O)ₙ or NR, where R is hydrogen or C₁-C₄-alkyl;
k is 0, 1 or 2;
n is 0, 1 or 2;
p is 0, 1 or 2;
R^{k} has the meanings of R^{3c};
an N-oxide or an agriculturally suitable salt thereof.

2. The compound of claim 1, where Q is Q¹.

3. The compound of claim 1, where Q is Q².

4. The compound of any one of the preceding claims, where R¹ is selected from the group consisting of halogen, nitro, cyano C₁-C₄-alkyl, C₁-C₄-haloalkyl, C₁-C₄-alkoxy-C₁-C₄-alkoxy-Z¹- and R^{1b}-S(O)ₖ-Z¹-.

5. The compound of any one of the preceding claims, where R^{2a} is selected from the group consisting of hydrogen, C₁-C₆-alkyl, C₁-C₆-alkoxy, C₃-C₇-cycloalkyl, C₃-C₇-cycloalkyl-C₁-C₄-alkyl, C₁-C₆-haloalkyl, phenyl, benzyl, and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, benzyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy.

6. The compound of any one of the preceding claims, where R^{2b} is selected from the group consisting of hydrogen, C₁-C₆-alkyl, C₃-C₇-cycloalkyl, C₃-C₇-cycloalkyl-C₁-C₄-alkyl, C₁-C₆-haloalkyl, phenyl, benzyl and heterocyclyl, where heterocyclyl is a 5- or 6-membered monocyclic saturated, partially unsaturated or aromatic heterocycle, which contains 1, 2, 3 or 4 heteroatoms as ring members, which are selected from the group consisting of O, N and S, where phenyl, benzyl and heterocyclyl are unsubstituted or substituted by 1, 2, 3 or 4 groups, which are identical or different and selected from the group consisting of halogen, C₁-C₄-alkyl, C₁-C₄-haloalkyl, C₁-C₄-alkoxy and C₁-C₄-haloalkoxy.

7. The compound of any one of the claims 1 to 4, where R^{2a} and R^{2b} together with the nitrogen atom, to which they are bound form a 4-, 5-, 6- or 7-membered, saturated or unsaturated heterocyclic radical, which may carry as a ring member a further heteroatom selected from O, S and N and which is unsubstituted or may carry 1, 2, 3 or 4 groups, which are identical or different and selected from the group consisting of =O, OH, halogen, C₁-C₄-alkyl, C₁-C₄-haloalkyl, C₁-C₄-alkoxy and C₁-C₄-haloalkoxy.

8. The compound of any one of the preceding claims, where R^{2c} is hydrogen or C₁-C₄-alkyl.

9. The compound of any one of the preceding claims, having one or more of the following features i) to v):
i) R³ is selected from the group consisting of halogen, nitro, C₁-C₆-alkyl, C₁-C₈-haloalkyl, C₁-C₈-haloalkoxy-Z³, and R^{3b}-S(O)ₖ-Z³;
ii) R⁴ is hydrogen or halogen;
iii) R⁵ is hydrogen or halogen;
iv) R^{6a}, R^{6b}, R^{6c}, R^{6d} are, independently of each other selected from the group consisting of C₁-C₆-alkyl, C₁-C₄-alkoxy-C₁-C₄-alkyl, and phenyl;
v) R⁷ is hydrogen.

10. The compound of any one of the preceding claims, where Z² is covalent bond.

11. The compound of any one of the preceding claims, which is selected from the compounds of formulae I.A' and I.B' 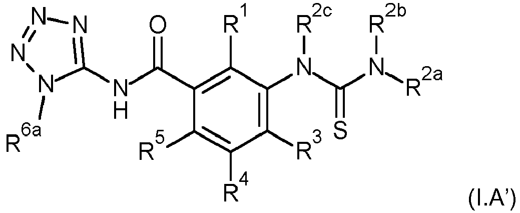 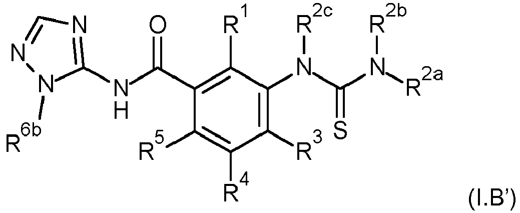 the N-oxides and the agriculturally suitable salts thereof.

12. The compound of claim 11, where
R¹ is halogen or C₁-C₂-alkyl;
R^{2a} is selected from the group consisting of C₁-C₄-alkyl, C₁-C₄-alkoxy, C₃-C₆-cycloalkyl, C₃-C₆-cycloalkyl-methyl, C₁-C₄-fluoroalkyl, phenyl and benzyl, where phenyl and benzyl are unsubstituted or substituted by 1, 2, 3 or 4 radicals selected from the group consisting of halogen, methyl and methoxy;
R^{2b} is selected from the group consisting of hydrogen, C₁-C₄-alkyl, C₃-C₆-cycloalkyl, C₃-C₆-cycloalkyl-methyl and C₁-C₄-fluoroalkyl; or
R^{2a}, R^{2b} together with the nitrogen atom, to which they are bound form a 5- or 6-membered, saturated heterocyclic radical, which may carry as a ring member a further heteroatom selected from O, S and N and where the heterocyclic radical is selected from the group consisting of 4-morpholinyl, 4-thiomorpholinyl, 1-piperidinyl and 1-pyrrolidinyl, where 4-morpholinyl, 4-thiomorpholinyl, 1-piperidinyl and 1-pyrrolidinyl are unsubstituted or carry 1, 2, 3 or 4 groups R^{2d} which are selected from the group consisting of C₁-C₄-alkyl and C₁-C₄-fluoroalkyl;
R^{2c} is selected from the group consisting of hydrogen and C₁-C₄-alkyl;
R³ is selected from the group consisting of halogen, C₁-C₂-alkyl, C₁-C₂-fluoroalkyl, S-C₁-C₂-alkyl, S(O)-C₁-C₂-alkyl, S(O)₂-C₁-C₂-alkyl, S-C₁-C₂-fluoroalkyl, S(O)-C₁-C₂-fluoroalkyl and S(O)₂-C₁-C₂-fluoroalkyl;
R⁴ is hydrogen,
R⁵ is fluorine;
R^{6a}, R^{6b}, independently of each other are selected from the group consisting of C₁-C₄-alkyl and C₁-C₄-alkoxy-C₁-C₄-alkyl.

13. A composition comprising at least one compound as claimed in any one of claims 1 to 12, an N-oxide or an agriculturally suitable salt thereof, and at least one auxiliary, which is customary for formulating crop protection compounds.

14. The use of a compound as claimed in any one of claims 1 to 12 or of the composition of claim 13 for controlling unwanted vegetation.

15. A method for controlling unwanted vegetation which comprises allowing a herbicidally effective amount of at least one compound as claimed in any one of claims 1 to 12 or of the compositions of claim 13 to act on plants, their seed and/or their habitat.


SEQUENCE LISTING

<110> BASF SE

<120> Benzamide compounds and their use as herbicides

<130> 171053EP01

<160> 69

<170> BiSSAP 1.3

<210> 1
<211> 1305
<212> DNA
<213> Hordeum vulgare

<400> 1
atgccgccca cccccaccac ccccgcggct accggcgccg ccgccgcggt gacgccggag 60

cacgcgcgac cgcaccgaat ggtccgcttc aacccgcgca gcgaccgctt ccacacgctc 120

tccttccacc acgtcgagtt ctggtgcgcg gacgccgcct ccgccgccgg ccgcttcgcg 180

ttcgcgctcg gcgcgccgct cgccgccagg tccgacctct ccacggggaa ctccgcgcac 240

gcctcccagc tgctccgctc gggctccctc gccttcctct tcaccgcgcc ctacgccaac 300

ggctgcgacg ccgccaccgc ctccctgccc tccttctccg ccgacgccgc gcgccggttc 360

tccgccgacc acgggatcgc ggtgcgctcc gtagcgctgc gcgtcgcaga cgccgccgag 420

gccttccgcg ccagcgtcga cgggggcgcg cgcccggcct tcgcccccgt ggacctcggc 480

cgcggcttcg gcttcgcgga ggtcgagctc tacggcgacg tcgtgctccg cttcgtcagc 540

cacccggacg gcacggacgt gcccttcttg ccggggttcg agggcgtgac caacccggac 600

gccgtggact acggcctgac gcggttcgac cacgtcgtcg gcaacgtccc ggagcttgcc 660

cccgccgcag cctacatcgc cgggttcacg gggttccacg agttcgccga gttcacggcg 720

gaggacgtgg gcacgaccga gagcgggctc aactcggtgg tgctcgccaa caactcggag 780

ggcgtgctgc tgccgctcaa cgagccggtg cacggcacca agcgccggag ccagatacag 840

acgttcctgg aacaccacgg cggcccgggc gtgcagcaca tcgcggtggc cagcagtgac 900

gtgctcagga cgctcaggaa gatgcgtgcg cgctccgcca tgggcggctt cgacttcctg 960

ccacccccgc tgccgaagta ctacgaaggc gtgcgacgcc ttgccgggga tgtcctctcg 1020

gaggcgcaga tcaaggaatg ccaggagctg ggtgtgctcg tcgataggga cgaccaaggg 1080

gtgttgctcc aaatcttcac caagccagta ggggacaggc cgaccttgtt cctggagatg 1140

atccagagga tcgggtgcat ggagaaggac gagagagggg aagagtacca gaagggtggc 1200

tgcggcgggt tcggcaaagg caacttctcc gagctgttca agtccattga agattacgag 1260

aagtcccttg aagccaagca atctgctgca gttcagggat catag 1305

<210> 2
<211> 434
<212> PRT
<213> Hordeum vulgare

<400> 2
Met Pro Pro Thr Pro Thr Thr Pro Ala Ala Thr Gly Ala Ala Ala Ala
1               5                   10                  15
Val Thr Pro Glu His Ala Arg Pro His Arg Met Val Arg Phe Asn Pro
            20                  25                  30
Arg Ser Asp Arg Phe His Thr Leu Ser Phe His His Val Glu Phe Trp
        35                  40                  45
Cys Ala Asp Ala Ala Ser Ala Ala Gly Arg Phe Ala Phe Ala Leu Gly
    50                  55                  60
Ala Pro Leu Ala Ala Arg Ser Asp Leu Ser Thr Gly Asn Ser Ala His
65                  70                  75                  80
Ala Ser Gln Leu Leu Arg Ser Gly Ser Leu Ala Phe Leu Phe Thr Ala
                85                  90                  95
Pro Tyr Ala Asn Gly Cys Asp Ala Ala Thr Ala Ser Leu Pro Ser Phe
            100                 105                 110
Ser Ala Asp Ala Ala Arg Arg Phe Ser Ala Asp His Gly Ile Ala Val
        115                 120                 125
Arg Ser Val Ala Leu Arg Val Ala Asp Ala Ala Glu Ala Phe Arg Ala
    130                 135                 140
Ser Val Asp Gly Gly Ala Arg Pro Ala Phe Ala Pro Val Asp Leu Gly
145                 150                 155                 160
Arg Gly Phe Gly Phe Ala Glu Val Glu Leu Tyr Gly Asp Val Val Leu
                165                 170                 175
Arg Phe Val Ser His Pro Asp Gly Thr Asp Val Pro Phe Leu Pro Gly
            180                 185                 190
Phe Glu Gly Val Thr Asn Pro Asp Ala Val Asp Tyr Gly Leu Thr Arg
        195                 200                 205
Phe Asp His Val Val Gly Asn Val Pro Glu Leu Ala Pro Ala Ala Ala
    210                 215                 220
Tyr Ile Ala Gly Phe Thr Gly Phe His Glu Phe Ala Glu Phe Thr Ala
225                 230                 235                 240
Glu Asp Val Gly Thr Thr Glu Ser Gly Leu Asn Ser Val Val Leu Ala
                245                 250                 255
Asn Asn Ser Glu Gly Val Leu Leu Pro Leu Asn Glu Pro Val His Gly
            260                 265                 270
Thr Lys Arg Arg Ser Gln Ile Gln Thr Phe Leu Glu His His Gly Gly
        275                 280                 285
Pro Gly Val Gln His Ile Ala Val Ala Ser Ser Asp Val Leu Arg Thr
    290                 295                 300
Leu Arg Lys Met Arg Ala Arg Ser Ala Met Gly Gly Phe Asp Phe Leu
305                 310                 315                 320
Pro Pro Pro Leu Pro Lys Tyr Tyr Glu Gly Val Arg Arg Leu Ala Gly
                325                 330                 335
Asp Val Leu Ser Glu Ala Gln Ile Lys Glu Cys Gln Glu Leu Gly Val
            340                 345                 350
Leu Val Asp Arg Asp Asp Gln Gly Val Leu Leu Gln Ile Phe Thr Lys
        355                 360                 365
Pro Val Gly Asp Arg Pro Thr Leu Phe Leu Glu Met Ile Gln Arg Ile
    370                 375                 380
Gly Cys Met Glu Lys Asp Glu Arg Gly Glu Glu Tyr Gln Lys Gly Gly
385                 390                 395                 400
Cys Gly Gly Phe Gly Lys Gly Asn Phe Ser Glu Leu Phe Lys Ser Ile
                405                 410                 415
Glu Asp Tyr Glu Lys Ser Leu Glu Ala Lys Gln Ser Ala Ala Val Gln
            420                 425                 430
Gly Ser

<210> 3
<211> 1470
<212> DNA
<213> Fragilariopsis cylindrus

<400> 3
atgggtttct cttcagtatc gttatttccg ctatcgtggt cggcgtcggc agtaacatca 60

acctatctcg agacgtccac tagtacacaa tcgaatgaag ctgctactac cacttcgaca 120

acggcgtggg aaccaaaatt atcatcatta gaagaaagaa tagaaacgga aacgattgag 180

tcgatcggtt tccatcatat agaattttat tgtggagatg ctcgtagtat ggcaaatcaa 240

ttcgctgtct cattgggtat gtccgtcacg ggtatcaccg gccaatccac ggggaatgat 300

caatgcattt cctatggatt acaaagtgga gagcagtttc gactattatt aactgctccc 360

tattcacgag cgagagccac tactcgcgat gacgacgacg acgacgacga caacagtcct 420

gatttggatg ccgacgctcc gatgccactc cctaattata atgtagaaga tgctcatact 480

ttcttccaaa atcatggctt agcagctcga gcggttggca tagaagtcat ggatgccaaa 540

aaagctttcg aggtatccgt ggccaatggc gcaattccag tactggaacc aacctttctt 600

cccaacggat gctacatctc agaagttgaa ttgtacggtg acgttgtgtt gagatacgtg 660

agtttcatca catcgaatga aaatcatact tataatgatg atgcatcaca accatttcta 720

cctcatttag caccaataat tgatcaaagt aggaaagagg atgatgataa taatgatgat 780

ggttttgggt tatataaaat cgaccatgcc gttgggaatg ttcccaattt acaagaggta 840

tactcacata tccaaaaatt tacaggattt catgaatttg ctgaatttac atcagaagat 900

gttggaactg tagactctgg attaaattct gttgttttag ccagtgacag tgaagcgatt 960

ctgttaccta taaatgaacc aactaatgga cgacgaaaat cacaaattca aacgtatcta 1020

gaacagaacg agggccctgg tctacaacat ttagcagtca aaacgaaaga tatattttca 1080

accgtccgaa agatgcgaag aagtcaacaa ggtatgtcgg gatttgaatt gatgaaacga 1140

ccgagtgagg aatattacaa agaacttcct gatcgacttg gtgatcaatt gacacccacg 1200

cagtatcaag aattagagga acttggtatc cttgcggatt ccgatgagga aggaattttg 1260

atgcaaattt ttaccaagcc cgtcggtgat cgacctacat tcttttttga actaattcaa 1320

cgaatcggtt gcgtcattga gcatgacgat gacgacaggc aggagttatc agttgatctt 1380

gaacgaccag gatgtggtgg ttttggtaag ggtaatttcc gagaactttt cagatcaatt 1440

gaagagcacg agaaaacttt aaaggtatag 1470

<210> 4
<211> 1467
<212> DNA
<213> Fragilariopsis cylindrus

<400> 4

atgggtttta gcagcgttag cctgtttccg ctgagctggt cagcaagcgc agttaccagc 60

acctatctgg aaaccagcac cagcacccag agcaatgaag cagcaaccac caccagtacc 120

accgcatggg aaccgaaact gagcagcctg gaagaacgta ttgaaaccga aaccattgaa 180

agcattggct ttcatcacat tgaattttat tgcggtgatg cacgtagcat ggcaaatcag 240

tttgcagtta gcctgggtat gagcgttacc ggtattaccg gtcagagcac cggtaatgat 300

cagtgtatta gctatggtct gcagagcggt gaacagtttc gtctgctgct gaccgcaccg 360

tatagccgtg cacgtgcaac cacccgtgat gatgatgatg acgatgacga taatagtccg 420

gatctggatg cagatgcacc gatgccgctg ccgaattata atgttgaaga tgcccatacc 480

ttttttcaga atcatggtct ggcagcacgt gcagttggta ttgaagttat ggatgccaaa 540

aaagcctttg aagttagcgt tgcaaatggt gcaattccgg ttctggaacc gacctttctg 600

ccgaatggtt gttatatttc tgaagtggaa ctgtatggtg atgttgttct gcgttatgtg 660

agctttatta ccagcaatga aaaccacacc tacaatgatg atgccagcca gccgtttctg 720

ccgcatctgg caccgattat tgatcagagc cgtaaagaag atgatgataa taatgatgat 780

ggctttggcc tgtataaaat tgatcatgcc gttggtaatg tgccgaatct gcaagaagtt 840

tatagccata ttcagaaatt taccggcttt catgaatttg ccgaatttac cagcgaagat 900

gttggcaccg ttgatagcgg tctgaatagc gttgttctgg caagcgatag cgaagcaatt 960

ctgctgccga ttaatgaacc gaccaatggt cgtcgtaaaa gccagattca gacatatctg 1020

gaacagaatg aaggtccggg tctgcagcat ctggccgtta aaaccaaaga tatttttagc 1080

accgtgcgta aaatgcgtcg tagccagcag ggtatgagcg gttttgaact gatgaaacgt 1140

ccgagcgaag aatattataa agaactgccg gatcgtctgg gtgatcagct gaccccgacc 1200

cagtatcaag aattagaaga actgggtatt ctggcagata gtgatgaaga aggtattctg 1260

atgcagattt ttaccaaacc ggttggtgat cgtccgacct ttttttttga actgattcag 1320

cgtattggct gcgtgattga acatgatgat gatgatcgtc aagaactgag cgttgatctg 1380

gaacgtccgg gttgtggtgg ttttggtaaa ggtaattttc gtgaactgtt tcgcagcatt 1440

gaagaacatg aaaaaaccct gaaagtg 1467

<210> 5
<211> 489
<212> PRT
<213> Fragilariopsis cylindrus

<400> 5
Met Gly Phe Ser Ser Val Ser Leu Phe Pro Leu Ser Trp Ser Ala Ser
1               5                   10                  15
Ala Val Thr Ser Thr Tyr Leu Glu Thr Ser Thr Ser Thr Gln Ser Asn
            20                  25                  30
Glu Ala Ala Thr Thr Thr Ser Thr Thr Ala Trp Glu Pro Lys Leu Ser
        35                  40                  45

Ser Leu Glu Glu Arg Ile Glu Thr Glu Thr Ile Glu Ser Ile Gly Phe
    50                  55                  60
His His Ile Glu Phe Tyr Cys Gly Asp Ala Arg Ser Met Ala Asn Gln
65                  70                  75                  80
Phe Ala Val Ser Leu Gly Met Ser Val Thr Gly Ile Thr Gly Gln Ser
                85                  90                  95
Thr Gly Asn Asp Gln Cys Ile Ser Tyr Gly Leu Gln Ser Gly Glu Gln
            100                 105                 110
Phe Arg Leu Leu Leu Thr Ala Pro Tyr Ser Arg Ala Arg Ala Thr Thr
        115                 120                 125
Arg Asp Asp Asp Asp Asp Asp Asp Asp Asn Ser Pro Asp Leu Asp Ala
    130                 135                 140
Asp Ala Pro Met Pro Leu Pro Asn Tyr Asn Val Glu Asp Ala His Thr
145                 150                 155                 160
Phe Phe Gln Asn His Gly Leu Ala Ala Arg Ala Val Gly Ile Glu Val
                165                 170                 175
Met Asp Ala Lys Lys Ala Phe Glu Val Ser Val Ala Asn Gly Ala Ile
            180                 185                 190
Pro Val Leu Glu Pro Thr Phe Leu Pro Asn Gly Cys Tyr Ile Ser Glu
        195                 200                 205
Val Glu Leu Tyr Gly Asp Val Val Leu Arg Tyr Val Ser Phe Ile Thr
    210                 215                 220
Ser Asn Glu Asn His Thr Tyr Asn Asp Asp Ala Ser Gln Pro Phe Leu
225                 230                 235                 240
Pro His Leu Ala Pro Ile Ile Asp Gln Ser Arg Lys Glu Asp Asp Asp
                245                 250                 255
Asn Asn Asp Asp Gly Phe Gly Leu Tyr Lys Ile Asp His Ala Val Gly
            260                 265                 270
Asn Val Pro Asn Leu Gln Glu Val Tyr Ser His Ile Gln Lys Phe Thr
        275                 280                 285
Gly Phe His Glu Phe Ala Glu Phe Thr Ser Glu Asp Val Gly Thr Val
    290                 295                 300
Asp Ser Gly Leu Asn Ser Val Val Leu Ala Ser Asp Ser Glu Ala Ile
305                 310                 315                 320
Leu Leu Pro Ile Asn Glu Pro Thr Asn Gly Arg Arg Lys Ser Gln Ile
                325                 330                 335
Gln Thr Tyr Leu Glu Gln Asn Glu Gly Pro Gly Leu Gln His Leu Ala
            340                 345                 350
Val Lys Thr Lys Asp Ile Phe Ser Thr Val Arg Lys Met Arg Arg Ser
        355                 360                 365
Gln Gln Gly Met Ser Gly Phe Glu Leu Met Lys Arg Pro Ser Glu Glu
    370                 375                 380
Tyr Tyr Lys Glu Leu Pro Asp Arg Leu Gly Asp Gln Leu Thr Pro Thr
385                 390                 395                 400
Gln Tyr Gln Glu Leu Glu Glu Leu Gly Ile Leu Ala Asp Ser Asp Glu
                405                 410                 415
Glu Gly Ile Leu Met Gln Ile Phe Thr Lys Pro Val Gly Asp Arg Pro
            420                 425                 430
Thr Phe Phe Phe Glu Leu Ile Gln Arg Ile Gly Cys Val Ile Glu His
        435                 440                 445
Asp Asp Asp Asp Arg Gln Glu Leu Ser Val Asp Leu Glu Arg Pro Gly
    450                 455                 460
Cys Gly Gly Phe Gly Lys Gly Asn Phe Arg Glu Leu Phe Arg Ser Ile
465                 470                 475                 480
Glu Glu His Glu Lys Thr Leu Lys Val
                485

<210> 6
<211> 1341
<212> DNA
<213> Chlorella sp.

<400> 6

atggtggtcg aggctgcggc cgcctccaac ggcaatggcg caggggagga ggtgttcagc 60

aagaagctcg tgggatatga cggtttccag cgccacaacc cacgctccga ccgcttcccc 120

atgcacaagt tccaccacgt cgagttctgg tgcggcgatg ccaccaccac cagctgcagg 180

caagcgcgcc ccccagaagc acccatgttt gggtatgggc tgggcctgac tctggtggcc 240

aagagcgacc agtccacggg caaccaccac tacgcgtcgt acgtcatgca gtcgggcgat 300

cttgtgatgg cctttaccgc gccctacagc acccagacag acaagagcgg cagcagcccg 360

cccgcagcgt acgaccagga cgccgcctac gccttcctca agaagcacgg catggcggtg 420

cgcgcctttg gaatcctggt ggacgatgcc gcggaggcgt accgcatagc cactgcccac 480

ggcggggtgg gtgtggcgcc acccaccacc cgcacggacg cagccagcgg caccagcctg 540

acgtggagcg aggtgcagct gtacggcgac tgcgtgctgc gctttgtcag cggcgactac 600

gagggcgcct tcatccccgg ctaccagccc gtggaagacg cgccccaagt ctcctacggc 660

ctgcagcgcc tggaccatgc ggtgggcaac gtgccagagc tgatccctca agtggagtac 720

atggctcgca gcctgggctg gcacgagttt gctgagttca ctgccgagga tgtgggcact 780

gtggactcgg gcctcaactc catggtcatg gccaacaaca acgagatgat tctgctgccg 840

gtcaacgagc ccacccacgg caccaagcgc aagagccaga tccagacctt cctggagcag 900

aatgaggggc ccgggctgca gcacatggcc ctgaaaacag acgacatcgt agccaccatg 960

cgacagctcc gggccaggtc tgcgtttggc ggcttcgact tcatgcccag gccttcgcct 1020

gactactacc gcaagctgcc tgcccgcatc ggcagcctgc tgacggcgca gcagtacaag 1080

gacgttgagg agctggggct gcttgtggac aaggatgacc agggcgtgct gctccagatc 1140

ttcaccaagc cgctgggcga ccgacccacc gtgtttttcg aaatcatcca gcgcctgtgc 1200

gccctggagc cgcaggcgcc caagagccag cgcggcgcgg tgccttccga ggtcggcggc 1260

tgcggcggct ttggcaaggg caacttcagt gagctgttca agagcatcga ggtgtacgag 1320

acggatctgg gcatcaacta a 1341

<210> 7
<211> 1341
<212> DNA
<213> Chlorella sp

<400> 7
atggttgttg aagcagcagc agcaagcaat ggtaatggtg ccggtgaaga agtgtttagc 60

aaaaaactgg tgggctatga tggttttcag cgtcataatc cgcgtagcga tcgttttccg 120

atgcataaat ttcatcatgt ggaattttgg tgtggtgatg caaccaccac cagttgtcgt 180

caggcacgtc ctccggaagc accgatgttt ggttatggtc tgggtctgac cctggttgca 240

aaaagcgatc agagcaccgg taatcatcat tatgcaagct atgttatgca gagcggtgat 300

ctggttatgg catttaccgc accgtatagc acccagaccg ataaaagcgg tagcagtccg 360

cctgcagcat acgatcagga tgcagcatac gcctttctga aaaaacatgg tatggcagtt 420

cgtgcatttg gtattctggt tgatgatgca gcagaagcat atcgtattgc aaccgcacat 480

ggtggtgttg gtgttgcacc tccgaccacc cgtaccgatg cagcaagcgg caccagcctg 540

acctggtctg aagttcagct gtatggtgat tgtgttctgc gttttgttag cggtgattat 600

gaaggtgcat ttattccggg ttatcagccg gttgaagatg caccgcaggt tagctatggt 660

ctgcagcgtc tggatcatgc agttggtaat gttccggaac tgattccgca ggttgaatat 720

atggcacgta gcctgggttg gcatgaattt gcagaattta ccgcagaaga tgttggcacc 780

gttgatagcg gtctgaatag catggttatg gccaataaca atgaaatgat tctgctgccg 840

gtgaatgaac cgacccacgg caccaaacgt aaaagccaga ttcagacctt tctggaacag 900

aatgaaggtc cgggtctgca gcacatggca ctgaaaaccg atgatattgt tgcaaccatg 960

cgtcagctgc gtgcacgtag cgcatttggt ggttttgatt ttatgcctcg tccgagtccg 1020

gattattatc gtaaactgcc tgcacgtatt ggtagcctgc tgaccgcaca gcagtataaa 1080

gatgttgaag aactgggtct gctggttgat aaagatgatc agggtgttct gctgcagatt 1140

tttaccaaac cgctgggtga tcgtccgacc gttttttttg aaattattca gcgtctgtgt 1200

gcactggaac cgcaggcacc gaaaagccag cgtggtgcag ttccgagcga agttggtggt 1260

tgtggtggtt ttggtaaagg taattttagc gaactgttta aaagcattga agtgtatgaa 1320

accgatctgg gcatcaatta a 1341

<210> 8
<211> 446
<212> PRT
<213> Chlorella sp

<400> 8
Met Val Val Glu Ala Ala Ala Ala Ser Asn Gly Asn Gly Ala Gly Glu
1               5                   10                  15
Glu Val Phe Ser Lys Lys Leu Val Gly Tyr Asp Gly Phe Gln Arg His
            20                  25                  30
Asn Pro Arg Ser Asp Arg Phe Pro Met His Lys Phe His His Val Glu
        35                  40                  45
Phe Trp Cys Gly Asp Ala Thr Thr Thr Ser Cys Arg Gln Ala Arg Pro
    50                  55                  60
Pro Glu Ala Pro Met Phe Gly Tyr Gly Leu Gly Leu Thr Leu Val Ala
65                  70                  75                  80
Lys Ser Asp Gln Ser Thr Gly Asn His His Tyr Ala Ser Tyr Val Met
                85                  90                  95
Gln Ser Gly Asp Leu Val Met Ala Phe Thr Ala Pro Tyr Ser Thr Gln
            100                 105                 110
Thr Asp Lys Ser Gly Ser Ser Pro Pro Ala Ala Tyr Asp Gln Asp Ala
        115                 120                 125
Ala Tyr Ala Phe Leu Lys Lys His Gly Met Ala Val Arg Ala Phe Gly
    130                 135                 140
Ile Leu Val Asp Asp Ala Ala Glu Ala Tyr Arg Ile Ala Thr Ala His
145                 150                 155                 160

Gly Gly Val Gly Val Ala Pro Pro Thr Thr Arg Thr Asp Ala Ala Ser
            165                 170                 175
Gly Thr Ser Leu Thr Trp Ser Glu Val Gln Leu Tyr Gly Asp Cys Val
            180                 185                 190
Leu Arg Phe Val Ser Gly Asp Tyr Glu Gly Ala Phe Ile Pro Gly Tyr
        195                 200                 205
Gln Pro Val Glu Asp Ala Pro Gln Val Ser Tyr Gly Leu Gln Arg Leu
    210                 215                 220
Asp His Ala Val Gly Asn Val Pro Glu Leu Ile Pro Gln Val Glu Tyr
225                 230                 235                 240
Met Ala Arg Ser Leu Gly Trp His Glu Phe Ala Glu Phe Thr Ala Glu
            245                 250                 255
Asp Val Gly Thr Val Asp Ser Gly Leu Asn Ser Met Val Met Ala Asn
            260                 265                 270
Asn Asn Glu Met Ile Leu Leu Pro Val Asn Glu Pro Thr His Gly Thr
        275                 280                 285
Lys Arg Lys Ser Gln Ile Gln Thr Phe Leu Glu Gln Asn Glu Gly Pro
    290                 295                 300
Gly Leu Gln His Met Ala Leu Lys Thr Asp Asp Ile Val Ala Thr Met
305                 310                 315                 320
Arg Gln Leu Arg Ala Arg Ser Ala Phe Gly Gly Phe Asp Phe Met Pro
            325                 330                 335
Arg Pro Ser Pro Asp Tyr Tyr Arg Lys Leu Pro Ala Arg Ile Gly Ser
            340                 345                 350
Leu Leu Thr Ala Gln Gln Tyr Lys Asp Val Glu Glu Leu Gly Leu Leu
        355                 360                 365
Val Asp Lys Asp Asp Gln Gly Val Leu Leu Gln Ile Phe Thr Lys Pro
    370                 375                 380
Leu Gly Asp Arg Pro Thr Val Phe Phe Glu Ile Ile Gln Arg Leu Cys
385                 390                 395                 400
Ala Leu Glu Pro Gln Ala Pro Lys Ser Gln Arg Gly Ala Val Pro Ser
            405                 410                 415
Glu Val Gly Gly Cys Gly Gly Phe Gly Lys Gly Asn Phe Ser Glu Leu
            420                 425                 430
Phe Lys Ser Ile Glu Val Tyr Glu Thr Asp Leu Gly Ile Asn
        435                 440                 445

<210> 9
<211> 1257
<212> DNA
<213> Thalassiosira

<400> 9
tttcatcata ttgaattctt cgccagtgat gcgcttacga cagccaagcg gtttgagcta      60

gcgttgggat tgccaattac gtgttggagt tcattggcta cggggaacga tgtttgtgtt     120

acctacggat tggaggggat gcaaactcga aagattgaaa ccgacaacgc aaacaagaac     180

ggaggggcag gatccaatgt acaaatgact gcacctcttc ctcttccggg gtatgatatt     240

gacaaagctc atgagtttta ctcgaagcac ggtttggcag tacgagctgt gggagtggaa     300

gtgaaggatg caactgtagc ttatgcaaat gctgttgaga atggtgctac aggagtattg     360

gagcctacga ttgttgaaaa ctttaacagc gatggagatt cgcagaagtg tcatatggct     420

gaggtggaat tgtatggtga tgtggtgttg agattagtca gttttcatgg agattgtagt     480

gccgaacaat ctacattcct tcctcacttg tcgccgtatc catccaacag caacaagaac     540

aaaccaactt acggacttgc tcgtctagat cacacggtgg gcaatgttcc caacctcctc     600

gcgacgcaac gatacattca aacattcacc aactaccatc ccttcgcaga gttcactccg 660

gaagatgtgg gaacagtcga ctctggtctt aatagtgtag tacttgcatc agacaacgag 720

aatgttttgc tgcctctcaa tgaacctacc gaaggtaaac gaaagagtca gattcaaaca 780

tatctagagc agaacgaggg accgggactg cagcatattg ccatcaagac gaatgatatc 840

tttgatacca ttgcaaagat gagacacgca gaagagaact ttggaggttt cgagttgatg 900

aaacgtccat cggatgagta ttacaaagag ttgccttcga gattgggtga taagttgact 960

gtcgagcaat acaaacagct ggaagagttg ggaatactgg cagatgcaga tgacgaaggt 1020

atattgcttc aaatattcac aaagccattg ggagacaggc ccaccttgtt ccttgagatt 1080

attcagcgaa ttgggtgtgt gttaccggat gacgatgaag caactgatga aggggaagct 1140

aagaatgcac acaatagaat agtcagagaa cgtcccggat gtggtggatt tggtcagggc 1200

aacttccgtg aactcttcaa agcaattgaa gaacatgaga agacactcaa ggtctaa 1257

<210> 10
<211> 1257
<212> DNA
<213> Thalassiosira

<400> 10
tttcatcata tcgaattttt tgcctcagat gcactgacca ccgcaaaacg ctttgaactg 60

gcactgggtc tgccgattac ctgttggagc agcctggcaa ccggtaatga tgtttgtgtt 120

acctatggtc tggaaggtat gcagacccgt aaaattgaaa ccgataacgc caataaaaac 180

ggtggtgcag gtagcaatgt tcagatgacc gcaccgctgc cgctgcctgg ttatgatatt 240

gataaagccc atgaattcta tagcaaacat ggtctggcag ttcgtgcagt tggtgttgaa 300

gttaaagatg caaccgttgc ctatgcaaat gcagttgaaa atggtgcaac cggtgttctg 360

gaaccgacca ttgttgaaaa ctttaatagt gatggcgata gccagaaatg tcacatggcc 420

gaagttgaac tgtatggtga tgttgttctg cgtctggtta gctttcatgg tgattgtagc 480

gcagaacaga gcacctttct gccgcatctg agcccgtatc cgagcaatag caataaaaac 540

aaaccgacct atggcctggc acgtctggat cataccgttg gtaatgttcc gaatctgctg 600

gcaacccagc gttatattca gacctttacc aactatcatc cgtttgcaga atttacaccg 660

gaagatgttg gcaccgttga tagcggtctg aatagcgttg ttctggcaag cgataatgaa 720

aatgttctgc tgccgctgaa tgaaccgacc gaaggtaaac gtaaaagcca gattcagacc 780

tatctggaac agaatgaagg tccgggtctg cagcatattg caattaaaac caatgatatt 840

tttgatacca ttgccaaaat gcgccatgcc gaagaaaatt ttggtggttt tgaactgatg 900

aaacgtccgt ccgatgaata ctataaagaa ctgccgagcc gtctgggtga taaactgacc 960

gttgaacagt ataaacagct ggaagaactg ggtattctgg cagatgcaga tgatgaaggt 1020

attctgctgc agatttttac caaaccgctg ggtgatcgtc cgaccctgtt tctggaaatt 1080

attcagcgta ttggttgtgt tctgccggat gatgatgaag caaccgatga aggtgaagca 1140

aaaaatgccc ataatcgtat tgttcgtgaa cgtccgggtt gtggtggttt tggtcagggt 1200

aattttcgcg aactgtttaa agccattgaa gaacatgaaa aaaccctgaa agtttaa 1257

<210> 11
<211> 418
<212> PRT
<213> Thalassiosira pseudonana

<400> 11
Phe His His Ile Glu Phe Phe Ala Ser Asp Ala Leu Thr Thr Ala Lys
1               5                   10                  15
Arg Phe Glu Leu Ala Leu Gly Leu Pro Ile Thr Cys Trp Ser Ser Leu
            20                  25                  30
Ala Thr Gly Asn Asp Val Cys Val Thr Tyr Gly Leu Glu Gly Met Gln
        35                  40                  45
Thr Arg Lys Ile Glu Thr Asp Asn Ala Asn Lys Asn Gly Gly Ala Gly
    50                  55                  60
Ser Asn Val Gln Met Thr Ala Pro Leu Pro Leu Pro Gly Tyr Asp Ile
65                  70                  75                  80
Asp Lys Ala His Glu Phe Tyr Ser Lys His Gly Leu Ala Val Arg Ala
                85                  90                  95
Val Gly Val Glu Val Lys Asp Ala Thr Val Ala Tyr Ala Asn Ala Val
            100                 105                 110
Glu Asn Gly Ala Thr Gly Val Leu Glu Pro Thr Ile Val Glu Asn Phe
        115                 120                 125
Asn Ser Asp Gly Asp Ser Gln Lys Cys His Met Ala Glu Val Glu Leu
    130                 135                 140
Tyr Gly Asp Val Val Leu Arg Leu Val Ser Phe His Gly Asp Cys Ser
145                 150                 155                 160
Ala Glu Gln Ser Thr Phe Leu Pro His Leu Ser Pro Tyr Pro Ser Asn
                165                 170                 175
Ser Asn Lys Asn Lys Pro Thr Tyr Gly Leu Ala Arg Leu Asp His Thr
            180                 185                 190
Val Gly Asn Val Pro Asn Leu Leu Ala Thr Gln Arg Tyr Ile Gln Thr
        195                 200                 205
Phe Thr Asn Tyr His Pro Phe Ala Glu Phe Thr Pro Glu Asp Val Gly
    210                 215                 220
Thr Val Asp Ser Gly Leu Asn Ser Val Val Leu Ala Ser Asp Asn Glu
225                 230                 235                 240
Asn Val Leu Leu Pro Leu Asn Glu Pro Thr Glu Gly Lys Arg Lys Ser
                245                 250                 255
Gln Ile Gln Thr Tyr Leu Glu Gln Asn Glu Gly Pro Gly Leu Gln His
            260                 265                 270
Ile Ala Ile Lys Thr Asn Asp Ile Phe Asp Thr Ile Ala Lys Met Arg
        275                 280                 285
His Ala Glu Glu Asn Phe Gly Gly Phe Glu Leu Met Lys Arg Pro Ser
    290                 295                 300
Asp Glu Tyr Tyr Lys Glu Leu Pro Ser Arg Leu Gly Asp Lys Leu Thr
305                 310                 315                 320
Val Glu Gln Tyr Lys Gln Leu Glu Glu Leu Gly Ile Leu Ala Asp Ala
                325                 330                 335
Asp Asp Glu Gly Ile Leu Leu Gln Ile Phe Thr Lys Pro Leu Gly Asp
            340                 345                 350
Arg Pro Thr Leu Phe Leu Glu Ile Ile Gln Arg Ile Gly Cys Val Leu
        355                 360                 365
Pro Asp Asp Asp Glu Ala Thr Asp Glu Gly Glu Ala Lys Asn Ala His
    370                 375                 380

Asn Arg Ile Val Arg Glu Arg Pro Gly Cys Gly Gly Phe Gly Gln Gly
385                 390                 395                 400
Asn Phe Arg Glu Leu Phe Lys Ala Ile Glu Glu His Glu Lys Thr Leu
                405                 410                 415
Lys Val

<210> 12
<211> 1092
<212> DNA
<213> Cyanothece

<400> 12
atggaaatcg atcatattca tttctacgtt gaagatgcag cacatcaacg agattggttt      60

attgataaaa tggggtttca atccatcagc aacagtatcc atgatgacac ttatagcgaa     120

gtagtaggga atcagtctgt ttactttatc ttatcttctc ccctcaacga tgctagtcca     180

gtttcttatt acttgaaatc tcatcctccg ggggttgctg atgttgcttt tcgtgttgac     240

aatcttaatt ttttattaga caaagtatcc cgttttaagg tcgaaattat taatcaatct     300

agtctaacag cttttcctct aaataaacca gtgaaattcg cgaaacttaa aggatggggt     360

tctgtcaatc ataccttaat tgatcaggca agtcctagga cttttattag ctcaaaaatg     420

attgctaaaa gcgatattat tgggattgat catgttgttt taaatgttcc tcaaggtgaa     480

ctccccttag ccataaattg gtacaaaaat gtatttgatt ttataagtca tcaacagttc     540

aacatccaaa cagaacattc ggggttatct agtgaagcct tagttgatag ttcaggaaaa     600

gtacaattta atattaatca accaagttct actaattctc agattcagga atttttagac     660

cataataacg gttcaggcat tcaacatatt ggtttaaaat caagtaatat tttacaaagt     720

gttgcacaaa tgcgtcaaag gggattaccc tttttatccg ttcctaattc ctattaccaa     780

aacctaaaag aattgattag aaaatcgaca atttcttgtt taagccaaca ggaactagaa     840

caaattgaaa ctgaacaaat tctagtttgt tggccagaag ataacccgac ttcaatcctg     900

atgcaaattt tcactcaacc catttttaag cagccgactt tcttttttga attaattcaa     960

agacgcaacc aagcacaggg atttggccaa ggtaattttc aagcgttatt tgaagccata    1020

gaatcagaac aaatcaagag aaatagggta tcctcacgag tcactttaca ggctgtaaca    1080

ccccaatctt ga                                                        1092

<210> 13
<211> 1089
<212> DNA
<213> Cyanothece

<400> 13
atggaaattg atcatattca tttttatgtg gaagatgcag cccatcagcg tgattggttt      60

attgataaaa tgggctttca gagcattagc aatagcattc atgatgatac ctatagcgaa     120

gttgtgggta atcagagcgt gtatttcatt ctgagcagtc cgctgaatga tgcaagtccg     180

gttagctatt atctgaaaag ccatcctccg ggtgttgcag atgttgcatt tcgtgttgat 240

aatctgaatt ttctgctgga taaagtgagc cgctttaaag tggaaatcat taatcagagc 300

agcctgaccg catttccgct gaataaaccg gttaaatttg ccaaactgaa aggttggggt 360

agcgttaatc ataccctgat tgatcaggca agtccgcgta cctttattag cagcaaaatg 420

attgccaaaa gcgatattat tggcattgat catgtggttc tgaatgttcc gcagggtgaa 480

ctgccgctgg caattaattg gtacaaaaat gtgtttgatt ttattagcca tcagcagttt 540

aatattcaga ccgaacatag cggtctgagc agcgaagcac tggttgatag cagcggtaaa 600

gttcagttta atattaatca gccgagcagc accaatagcc agattcaaga atttctggat 660

cataataatg gcagcggcat tcagcatatt ggtctgaaaa gcagcaatat tctgcagagc 720

gttgcacaga tgcgtcagcg tggtctgccg tttctgagcg ttccgaatag ctattatcag 780

aatctgaaag aactgattcg caaaagcacc attagctgtc tgagccagca agaactggaa 840

caaattgaaa ccgaacaaat tctggtttgt tggcctgaag ataatccgac cagcattctg 900

atgcagattt ttacccagcc gatttttaaa cagccgacct ttttttttga actgattcag 960

cgtcgtaatc aggcacaggg ttttggtcag ggtaattttc aggcactgtt tgaagcaatt 1020

gaaagtgaac aaattaaacg taatcgtgtt agcagccgtg ttaccctgca ggcagttaca 1080

ccgcagagc 1089

<210> 14
<211> 363
<212> PRT
<213> Cyanothece

<400> 14
Met Glu Ile Asp His Ile His Phe Tyr Val Glu Asp Ala Ala His Gln
1               5                   10                  15
Arg Asp Trp Phe Ile Asp Lys Met Gly Phe Gln Ser Ile Ser Asn Ser
            20                  25                  30
Ile His Asp Asp Thr Tyr Ser Glu Val Val Gly Asn Gln Ser Val Tyr
        35                  40                  45
Phe Ile Leu Ser Ser Pro Leu Asn Asp Ala Ser Pro Val Ser Tyr Tyr
    50                  55                  60
Leu Lys Ser His Pro Pro Gly Val Ala Asp Val Ala Phe Arg Val Asp
65                  70                  75                  80
Asn Leu Asn Phe Leu Leu Asp Lys Val Ser Arg Phe Lys Val Glu Ile
                85                  90                  95
Ile Asn Gln Ser Ser Leu Thr Ala Phe Pro Leu Asn Lys Pro Val Lys
            100                 105                 110
Phe Ala Lys Leu Lys Gly Trp Gly Ser Val Asn His Thr Leu Ile Asp
        115                 120                 125
Gln Ala Ser Pro Arg Thr Phe Ile Ser Ser Lys Met Ile Ala Lys Ser
    130                 135                 140
Asp Ile Ile Gly Ile Asp His Val Val Leu Asn Val Pro Gln Gly Glu
145                 150                 155                 160
Leu Pro Leu Ala Ile Asn Trp Tyr Lys Asn Val Phe Asp Phe Ile Ser
                165                 170                 175
His Gln Gln Phe Asn Ile Gln Thr Glu His Ser Gly Leu Ser Ser Glu

180                 185                 190
Ala Leu Val Asp Ser Ser Gly Lys Val Gln Phe Asn Ile Asn Gln Pro
        195                 200                 205
Ser Ser Thr Asn Ser Gln Ile Gln Glu Phe Leu Asp His Asn Asn Gly
    210                 215                 220
Ser Gly Ile Gln His Ile Gly Leu Lys Ser Ser Asn Ile Leu Gln Ser
225                 230                 235                 240
Val Ala Gln Met Arg Gln Arg Gly Leu Pro Phe Leu Ser Val Pro Asn
                245                 250                 255
Ser Tyr Tyr Gln Asn Leu Lys Glu Leu Ile Arg Lys Ser Thr Ile Ser
            260                 265                 270
Cys Leu Ser Gln Gln Glu Leu Glu Gln Ile Glu Thr Glu Gln Ile Leu
        275                 280                 285
Val Cys Trp Pro Glu Asp Asn Pro Thr Ser Ile Leu Met Gln Ile Phe
    290                 295                 300
Thr Gln Pro Ile Phe Lys Gln Pro Thr Phe Phe Phe Glu Leu Ile Gln
305                 310                 315                 320
Arg Arg Asn Gln Ala Gln Gly Phe Gly Gln Gly Asn Phe Gln Ala Leu
                325                 330                 335
Phe Glu Ala Ile Glu Ser Glu Gln Ile Lys Arg Asn Arg Val Ser Ser
            340                 345                 350
Arg Val Thr Leu Gln Ala Val Thr Pro Gln Ser
        355                 360

<210> 15
<211> 1068
<212> DNA
<213> Acaryochloris marina

<400> 15
atggattttg atcatattca tttttatgtt catgattcca agcaatgcca gcgttggttt      60

actaacgttt taggatttca atatcttggg agcaatacta cggctgatcg gcagattgaa     120

gttgtctctt ctggggcgat tgtctgtata ttttccagcc ctctaaaccg aaccagccca     180

gttgcccagt atctacaaca acaccctcct ggtgtcgttg atttggcttt cttggtcccg     240

gacgttcagg ctacgcttac ctgcgctgtc cagtcaggag caaccctttt acaacctttg     300

accgaagaaa aaaacgacca aggaacgtta acttggggaa aagtacgagg gtggggagcg     360

ttagaacata ccttggtaga gcgaagaggg caaacctcca ctctgccatc cagcattttt     420

cccatctcaa ttcatgggca tgatgcccat cagagtctat ttacccagat tgatcatggg     480

gttttgaatg tgggtaagca tcagctgcaa gctgctgtga gttggtatca gcgcatattt     540

ggatttaaaa ctcaccgata ttttgatatt caaacgcgtc gttcaggtct gcgcagtgaa     600

gtgttgaccc atccccaagg ccaaatcaag tttccgatca atgaacccac ctcagcgaat     660

tcccaaatcc aagaatttct agaggtcaat cggggggcag gtattcaaca tatcgcattg     720

ggaacttcta atattgttga aacggttact cagcttaagc atcgagggct atccatccta     780

gatattccac ccagctacta tcaacgctta cgacaccagt ttgagcaagt ctattcccac     840

ctcgattggc atgccctgga aacacaacat attctggctg attttgagga agatgctgga     900

gccggaattc tattgcaaac cttcacaaag cctatctttc cacaacccac tttttctttt     960

gaaattattg agcgccaacg gcaggcccaa gggttcggac aacgaaactt tttggccctt    1020

tttcaagcca tggagcggga acaacagaaa cggggagtat tgctatag 1068

<210> 16
<211> 1065
<212> DNA
<213> Acaryochloris marina

<400> 16
atggattttg atcacatcca cttttatgtg catgacagca aacagtgtca gcgttggttt 60

accaatgttc tgggttttca gtatctgggt agcaatacca ccgcagatcg tcagattgaa 120

gttgttagca gcggtgcaat tgtttgcatt tttagcagtc cgctgaatcg taccagtccg 180

gttgcacagt atctgcagca gcatccgcct ggtgttgttg atctggcatt tctggttccg 240

gatgttcagg caaccctgac ctgtgcagtt cagagcggtg ccaccctgct gcagccgctg 300

accgaagaaa aaaatgatca gggtacactg acctggggta aagttcgtgg ttggggtgca 360

ctggaacaca ccctggttga acgtcgtggt cagaccagca ccctgccgag cagcattttt 420

ccgattagca ttcatggtca tgatgcacat cagagcctgt ttacccagat tgatcatggt 480

gttctgaatg ttggtaaaca tcagctgcag gcagcagtta gctggtatca gcgtattttt 540

ggctttaaaa cccaccgcta ttttgatatt cagacccgtc gtagcggtct gcgtagcgaa 600

gttctgaccc atccgcaggg tcagatcaaa tttccgatca atgaaccgac cagcgcaaat 660

agccagattc aagaatttct ggaagttaat cgtggtgccg gtattcagca tattgcactg 720

ggcaccagca atattgttga aaccgttacc cagctgaaac atcgtggtct gagcattctg 780

gatattccgc ctagctatta tcagcgtctg cgtcatcagt ttgaacaggt ttatagccat 840

ctggattggc atgccctgga aacccagcat attctggcag attttgaaga agatgcaggc 900

gcaggtattc tgctgcaaac ctttaccaaa ccgatttttc cgcagccgac cttttttttc 960

gaaattattg aacgtcagcg tcaggcacag ggttttggtc agcgcaattt tctggcactg 1020

tttcaggcaa tggaacgtga acagcagaaa cgtggtgtgc tgctg 1065

<210> 17
<211> 355
<212> PRT
<213> Acaryochloris marina

<400> 17
Met Asp Phe Asp His Ile His Phe Tyr Val His Asp Ser Lys Gln Cys
1               5                   10                  15
Gln Arg Trp Phe Thr Asn Val Leu Gly Phe Gln Tyr Leu Gly Ser Asn
            20                  25                  30
Thr Thr Ala Asp Arg Gln Ile Glu Val Val Ser Ser Gly Ala Ile Val
        35                  40                  45
Cys Ile Phe Ser Ser Pro Leu Asn Arg Thr Ser Pro Val Ala Gln Tyr
    50                  55                  60
Leu Gln Gln His Pro Pro Gly Val Val Asp Leu Ala Phe Leu Val Pro
65                  70                  75                  80

Asp Val Gln Ala Thr Leu Thr Cys Ala Val Gln Ser Gly Ala Thr Leu
                85                  90                  95
Leu Gln Pro Leu Thr Glu Glu Lys Asn Asp Gln Gly Thr Leu Thr Trp
            100                 105                 110
Gly Lys Val Arg Gly Trp Gly Ala Leu Glu His Thr Leu Val Glu Arg
        115                 120                 125
Arg Gly Gln Thr Ser Thr Leu Pro Ser Ser Ile Phe Pro Ile Ser Ile
    130                 135                 140
His Gly His Asp Ala His Gln Ser Leu Phe Thr Gln Ile Asp His Gly
145                 150                 155                 160
Val Leu Asn Val Gly Lys His Gln Leu Gln Ala Ala Val Ser Trp Tyr
                165                 170                 175
Gln Arg Ile Phe Gly Phe Lys Thr His Arg Tyr Phe Asp Ile Gln Thr
            180                 185                 190
Arg Arg Ser Gly Leu Arg Ser Glu Val Leu Thr His Pro Gln Gly Gln
        195                 200                 205
Ile Lys Phe Pro Ile Asn Glu Pro Thr Ser Ala Asn Ser Gln Ile Gln
    210                 215                 220
Glu Phe Leu Glu Val Asn Arg Gly Ala Gly Ile Gln His Ile Ala Leu
225                 230                 235                 240
Gly Thr Ser Asn Ile Val Glu Thr Val Thr Gln Leu Lys His Arg Gly
                245                 250                 255
Leu Ser Ile Leu Asp Ile Pro Pro Ser Tyr Tyr Gln Arg Leu Arg His
            260                 265                 270
Gln Phe Glu Gln Val Tyr Ser His Leu Asp Trp His Ala Leu Glu Thr
        275                 280                 285
Gln His Ile Leu Ala Asp Phe Glu Glu Asp Ala Gly Ala Gly Ile Leu
    290                 295                 300
Leu Gln Thr Phe Thr Lys Pro Ile Phe Pro Gln Pro Thr Phe Phe Phe
305                 310                 315                 320
Glu Ile Ile Glu Arg Gln Arg Gln Ala Gln Gly Phe Gly Gln Arg Asn
                325                 330                 335
Phe Leu Ala Leu Phe Gln Ala Met Glu Arg Glu Gln Gln Lys Arg Gly
            340                 345                 350
Val Leu Leu
        355

<210> 18
<211> 1020
<212> DNA
<213> Synechocystis sp. PCC 6803

<400> 18
atggaattcg actatcttca tttatacgtt gacgattatc agtcagctca tcgttgttat      60

caacgtcaat ggggtttcac ttgcgtaaat aaaattatta ctgaccaagg aattactggc     120

atctaccaac aggggcaaat acttctgcta atttcggcat cggaatctag tttgagtaga     180

tatgccgact atctccagaa acatcccccc ggcgtaggtg aagtcgcttg gcaggtggcc     240

aattggcaaa aaattcagca tcaattatca gaattacaga tagaaaccac accagttatt     300

catcctctga ctaaagcaga aggattaact tttttgctct ggggagatgt gcaccatagc     360

atttatcctg ttcgttctga gctaaatcag aataaaacat tgcatggtgt tggtttaacg     420

accatcgacc atgtggtgct aaacattgcc gccgatcaat ttacccaggc ttcccaatgg     480

tatcaacagg tgtttggctg gtcggtgcag cagagtttta ctgtcaatac gccccattct     540

ggtctgtata gcgaagccct ggccagtgcc aatgggaaag tccaatttaa cctcaattgt     600

cccaccaata acagttccca aattcaaact tttttagcca ataaccatgg ggctggtatt 660

caacatgtcg ctttttccac tacgagtatt acgcgaactg tggctcatct gcgggaaagg 720

ggcgtaaatt ttttaaaaat ccccactggc tattatcaac agcaaagaaa cagtagctat 780

tttaattatg caagtttgga ttgggatacc ttacagtgcc tagaaatttt gctggatgat 840

caagataata cgggggagcg attactgcta caaattttta gtcagccttg ctatggagta 900

ggcactctat tttgggaaat tattgaacgc cgccaccggg caaaaggatt tggtcaagga 960

aactttcaag ctctctatga agcggtggag actttagaaa aacagttaga agtgccataa 1020

<210> 19
<211> 1020
<212> DNA
<213> Synechocystis sp. PCC 6803

<400> 19
atggaattcg attacctgca tctgtatgtt gatgactatc agagcgctca ccgttgttac 60

cagcgtcagt ggggctttac ttgtgtgaac aaaatcatca ccgaccaggg tatcactggt 120

atttaccagc agggtcagat cctgctgctg atcagcgctt ctgaatcttc cctctctcgc 180

tatgccgatt acctccagaa acatccgcca ggtgtaggtg aagtcgcctg gcaggtcgca 240

aactggcaga aaattcagca ccagctgtcc gaactgcaga ttgaaactac cccggtgatt 300

cacccactga ccaaagcaga aggcctgact ttcctgctgt ggggtgacgt tcaccactcc 360

atctacccag tacgtagcga gctgaaccag aacaaaaccc tgcatggcgt tggtctgacc 420

actatcgatc acgtggttct gaacatcgca gcggaccagt tcacccaggc gagccagtgg 480

tatcagcagg tattcggttg gtccgttcag cagtctttca cggttaacac cccgcattcc 540

ggtctgtact ctgaagctct ggcgtctgcg aacggcaaag ttcagttcaa cctgaactgc 600

ccgaccaaca acagctccca gattcagacc ttcctggcga acaaccacgg tgctggtatc 660

cagcacgttg cattctctac tacctctatc acccgtacgg tcgctcacct gcgtgaacgt 720

ggcgtgaact tcctgaaaat cccgaccggt tactatcagc agcagcgcaa cagctcctac 780

ttcaactacg cgtctctgga ttgggatacc ctgcagtgcc tggagattct gctggacgac 840

caggacaaca ctggcgaacg cctgctgctg cagatctttt ctcagccgtg ctatggcgtg 900

ggtacgctgt tttgggaaat tatcgagcgc cgtcaccgtg ctaaaggctt tggccagggc 960

aactttcagg cactgtacga ggcagtagaa accctggaaa aacagctcga agtgccataa 1020

<210> 20
<211> 339
<212> PRT
<213> Synechocystis sp. PCC 6803

<400> 20
Met Glu Phe Asp Tyr Leu His Leu Tyr Val Asp Asp Tyr Gln Ser Ala

1                   5                   10                  15
His Arg Cys Tyr Gln Arg Gln Trp Gly Phe Thr Cys Val Asn Lys Ile
            20                  25                  30
Ile Thr Asp Gln Gly Ile Thr Gly Ile Tyr Gln Gln Gly Gln Ile Leu
        35                  40                  45
Leu Leu Ile Ser Ala Ser Glu Ser Ser Leu Ser Arg Tyr Ala Asp Tyr
    50                  55                  60
Leu Gln Lys His Pro Pro Gly Val Gly Glu Val Ala Trp Gln Val Ala
65                  70                  75                  80
Asn Trp Gln Lys Ile Gln His Gln Leu Ser Glu Leu Gln Ile Glu Thr
                85                  90                  95
Thr Pro Val Ile His Pro Leu Thr Lys Ala Glu Gly Leu Thr Phe Leu
            100                 105                 110
Leu Trp Gly Asp Val His His Ser Ile Tyr Pro Val Arg Ser Glu Leu
        115                 120                 125
Asn Gln Asn Lys Thr Leu His Gly Val Gly Leu Thr Thr Ile Asp His
    130                 135                 140
Val Val Leu Asn Ile Ala Ala Asp Gln Phe Thr Gln Ala Ser Gln Trp
145                 150                 155                 160
Tyr Gln Gln Val Phe Gly Trp Ser Val Gln Gln Ser Phe Thr Val Asn
                165                 170                 175
Thr Pro His Ser Gly Leu Tyr Ser Glu Ala Leu Ala Ser Ala Asn Gly
            180                 185                 190
Lys Val Gln Phe Asn Leu Asn Cys Pro Thr Asn Asn Ser Ser Gln Ile
        195                 200                 205
Gln Thr Phe Leu Ala Asn Asn His Gly Ala Gly Ile Gln His Val Ala
    210                 215                 220
Phe Ser Thr Thr Ser Ile Thr Arg Thr Val Ala His Leu Arg Glu Arg
225                 230                 235                 240
Gly Val Asn Phe Leu Lys Ile Pro Thr Gly Tyr Tyr Gln Gln Gln Arg
                245                 250                 255
Asn Ser Ser Tyr Phe Asn Tyr Ala Ser Leu Asp Trp Asp Thr Leu Gln
            260                 265                 270
Cys Leu Glu Ile Leu Leu Asp Asp Gln Asp Asn Thr Gly Glu Arg Leu
        275                 280                 285
Leu Leu Gln Ile Phe Ser Gln Pro Cys Tyr Gly Val Gly Thr Leu Phe
    290                 295                 300
Trp Glu Ile Ile Glu Arg Arg His Arg Ala Lys Gly Phe Gly Gln Gly
305                 310                 315                 320
Asn Phe Gln Ala Leu Tyr Glu Ala Val Glu Thr Leu Glu Lys Gln Leu
                325                 330                 335
Glu Val Pro

<210> 21
<211> 1326
<212> DNA
<213> Alopecurus myosuroides

<400> 21
atgcctccga ccaccgcaac cgcaaccggt gctgcagcag cagccgttac accggaacat      60

gcagcacgtc gttttccgcg tgttgttcgt gttaatccgc gtagcgatcg ttttccggtt     120

ctggcatttc atcatgttga attttggtgt gccgatgcag caagcgcagc aggtcgtttt     180

agctttgcac tgggtgcacc gctggcagca cgtagcgatc tgagcaccgg taatagcagc     240

catgcaagcc atctgctgcg tagtggtgca ctggcatttc tgtttaccgc accgtatgca     300

ccgcctccgc aggatgcagc agatgcagcc gctaccgcca gcattccgag ctttagcacc     360

gaagcagcac gtacctttag cagcgcacat ggtctggcag ttcgtagcgt tgcaattcgt     420

gttgcagatg ccgcagaagc atttcatacc agcgttgcgg gtggtgcacg tccggcattt 480

gcaccggcag atctgggtag cggttttggt ctggccgaag ttgaactgta tggtgatgtt 540

gttctgcgtt ttgttagtca tccggatggt gatgatgttc cgtttctgcc gggttttgaa 600

ggtgttagcc gtccgggtgc aatggattat ggtctgaccc gttttgatca tgttgttggt 660

aatgttccgg aaatggcacc ggttgcagca tatatgaaag gttttaccgg ctttcatgaa 720

tttgccgaat ttaccgcaga agatgttggc accgcagaaa gcggtctgaa tagcgttgtt 780

ctggcaaata atagcgaagc agttctgctg ccgctgaatg aaccggtgca tggcaccaaa 840

cgtcgtagcc agattcagac ctatctggat tatcatggtg gtccgggtgt tcagcatatt 900

gcactggcaa gcagtgatgt tctgcgtacc ctgcgtgaaa tgcgtgcacg tagcgcaatg 960

ggtggttttg aatttatggc accgccgcag gcaaaatatt atgaaggtgt tcgtcgtctg 1020

gctggtgatg ttctgagcga agcacagatt aaagaatgtc aggaactggg cgttctggtt 1080

gatcgtgatg atcagggtgt tctgctgcag atttttacca aaccggttgg tgatcgtcgt 1140

ccgacctttt ttctggaaat gattcagcgt attggctgca tggaaaaaga tgaaattggc 1200

caggaatatc agaaaggcgg ctgtggtggt tttggtaaag gtaattttag cgaactgttt 1260

aaaagcattg aagattatga aaaaagcctg gaagccaaac agagcgcagt tgcacagcag 1320

agctaa 1326

<210> 22
<211> 441
<212> PRT
<213> Alopecurus myosuroides

<400> 22
Met Pro Pro Thr Thr Ala Thr Ala Thr Gly Ala Ala Ala Ala Ala Val
1               5                   10                  15
Thr Pro Glu His Ala Ala Arg Arg Phe Pro Arg Val Val Arg Val Asn
            20                  25                  30
Pro Arg Ser Asp Arg Phe Pro Val Leu Ala Phe His His Val Glu Phe
        35                  40                  45
Trp Cys Ala Asp Ala Ala Ser Ala Ala Gly Arg Phe Ser Phe Ala Leu
    50                  55                  60
Gly Ala Pro Leu Ala Ala Arg Ser Asp Leu Ser Thr Gly Asn Ser Ser
65                  70                  75                  80
His Ala Ser His Leu Leu Arg Ser Gly Ala Leu Ala Phe Leu Phe Thr
                85                  90                  95
Ala Pro Tyr Ala Pro Pro Pro Gln Asp Ala Ala Asp Ala Ala Ala Thr
            100                 105                 110
Ala Ser Ile Pro Ser Phe Ser Thr Glu Ala Ala Arg Thr Phe Ser Ser
        115                 120                 125
Ala His Gly Leu Ala Val Arg Ser Val Ala Ile Arg Val Ala Asp Ala
    130                 135                 140
Ala Glu Ala Phe His Thr Ser Val Ala Gly Gly Ala Arg Pro Ala Phe
145                 150                 155                 160
Ala Pro Ala Asp Leu Gly Ser Gly Phe Gly Leu Ala Glu Val Glu Leu
                165                 170                 175
Tyr Gly Asp Val Val Leu Arg Phe Val Ser His Pro Asp Gly Asp Asp

180                 185                 190
Val Pro Phe Leu Pro Gly Phe Glu Gly Val Ser Arg Pro Gly Ala Met
        195                 200                 205
Asp Tyr Gly Leu Thr Arg Phe Asp His Val Val Gly Asn Val Pro Glu
    210                 215                 220
Met Ala Pro Val Ala Ala Tyr Met Lys Gly Phe Thr Gly Phe His Glu
225                 230                 235                 240
Phe Ala Glu Phe Thr Ala Glu Asp Val Gly Thr Ala Glu Ser Gly Leu
                245                 250                 255
Asn Ser Val Val Leu Ala Asn Asn Ser Glu Ala Val Leu Leu Pro Leu
            260                 265                 270
Asn Glu Pro Val His Gly Thr Lys Arg Arg Ser Gln Ile Gln Thr Tyr
        275                 280                 285
Leu Asp Tyr His Gly Gly Pro Gly Val Gln His Ile Ala Leu Ala Ser
    290                 295                 300
Ser Asp Val Leu Arg Thr Leu Arg Glu Met Arg Ala Arg Ser Ala Met
305                 310                 315                 320
Gly Gly Phe Glu Phe Met Ala Pro Pro Gln Ala Lys Tyr Tyr Glu Gly
                325                 330                 335
Val Arg Arg Leu Ala Gly Asp Val Leu Ser Glu Ala Gln Ile Lys Glu
            340                 345                 350
Cys Gln Glu Leu Gly Val Leu Val Asp Arg Asp Asp Gln Gly Val Leu
        355                 360                 365
Leu Gln Ile Phe Thr Lys Pro Val Gly Asp Arg Arg Pro Thr Phe Phe
    370                 375                 380
Leu Glu Met Ile Gln Arg Ile Gly Cys Met Glu Lys Asp Glu Ile Gly
385                 390                 395                 400
Gln Glu Tyr Gln Lys Gly Gly Cys Gly Gly Phe Gly Lys Gly Asn Phe
                405                 410                 415
Ser Glu Leu Phe Lys Ser Ile Glu Asp Tyr Glu Lys Ser Leu Glu Ala
            420                 425                 430
Lys Gln Ser Ala Val Ala Gln Gln Ser
        435                 440

<210> 23
<211> 1323
<212> DNA
<213> Alopecurus myosuroides

<400> 23
atgccaccaa ctactgctac tgctacaggt gctgctgctg cagctgttac tccagaacat      60

gctgctagaa ggttcccaag agttgttaga gttaacccaa ggtctgatag gttcccagtt     120

cttgctttcc atcatgttga gttttggtgt gctgatgctg cttctgctgc tggaagattt     180

tcttttgctc ttggtgctcc acttgctgct agatctgatt tgtctactgg aaactcttct     240

cacgcttctc accttttgag atctggtgct cttgctttcc ttttcactgc tccttatgct     300

ccaccaccac aagatgctgc agatgcagca gctactgctt ctattccatc tttttcaact     360

gaggctgcta ggactttctc ttctgctcat ggattggctg ttagatctgt ggctattaga     420

gttgcagatg ctgcagaggc tttccatact tctgttgctg gtggtgctag accagctttt     480

gctccagctg atcttggatc tggatttgga cttgctgagg ttgagcttta cggtgatgtt     540

gttcttagat tcgtgtctca cccagatggt gatgatgttc catttcttcc aggattcgag     600

ggtgttagta gaccaggtgc tatggattat ggactcacta ggttcgatca cgttgtggga     660

aatgttccag aaatggctcc agttgctgct tacatgaagg gattcactgg atttcatgag     720

ttcgctgagt tcactgctga ggatgttgga actgctgagt ctggacttaa ctctgttgtg 780

cttgctaaca actctgaggc tgttcttttg ccacttaatg agccagttca cggcactaag 840

agaagatctc agattcagac ttacctcgat taccatggtg gaccaggtgt tcaacatatt 900

gctcttgctt catctgatgt gcttaggact cttagagaga tgagagctag atctgctatg 960

ggaggatttg agtttatggc tccaccacaa gctaagtatt acgaaggtgt tagaaggctt 1020

gctggtgatg ttctttctga ggctcaaatc aaagagtgcc aagagcttgg agttcttgtg 1080

gatagagatg atcagggtgt gcttctccag attttcacta agccagttgg agataggcca 1140

acattcttct tggagatgat tcagaggatc ggctgcatgg aaaaggatga gattggacaa 1200

gagtaccaaa agggcggatg tggtggattt ggaaagggaa atttctccga gcttttcaag 1260

tccatcgagg attacgagaa gtctcttgag gctaagcaat ctgctgttgc tcaacagtct 1320

tga 1323

<210> 24
<211> 440
<212> PRT
<213> Alopecurus myosuroides

<400> 24
Met Pro Pro Thr Thr Ala Thr Ala Thr Gly Ala Ala Ala Ala Ala Val
1               5                   10                  15
Thr Pro Glu His Ala Ala Arg Arg Phe Pro Arg Val Val Arg Val Asn
            20                  25                  30
Pro Arg Ser Asp Arg Phe Pro Val Leu Ala Phe His His Val Glu Phe
        35                  40                  45
Trp Cys Ala Asp Ala Ala Ser Ala Ala Gly Arg Phe Ser Phe Ala Leu
    50                  55                  60
Gly Ala Pro Leu Ala Ala Arg Ser Asp Leu Ser Thr Gly Asn Ser Ser
65                  70                  75                  80
His Ala Ser His Leu Leu Arg Ser Gly Ala Leu Ala Phe Leu Phe Thr
                85                  90                  95
Ala Pro Tyr Ala Pro Pro Pro Gln Asp Ala Ala Asp Ala Ala Ala Thr
            100                 105                 110
Ala Ser Ile Pro Ser Phe Ser Thr Glu Ala Ala Arg Thr Phe Ser Ser
        115                 120                 125
Ala His Gly Leu Ala Val Arg Ser Val Ala Ile Arg Val Ala Asp Ala
    130                 135                 140
Ala Glu Ala Phe His Thr Ser Val Ala Gly Gly Ala Arg Pro Ala Phe
145                 150                 155                 160
Ala Pro Ala Asp Leu Gly Ser Gly Phe Gly Leu Ala Glu Val Glu Leu
                165                 170                 175
Tyr Gly Asp Val Val Leu Arg Phe Val Ser His Pro Asp Gly Asp Asp
            180                 185                 190
Val Pro Phe Leu Pro Gly Phe Glu Gly Val Ser Arg Pro Gly Ala Met
        195                 200                 205
Asp Tyr Gly Leu Thr Arg Phe Asp His Val Val Gly Asn Val Pro Glu
    210                 215                 220
Met Ala Pro Val Ala Ala Tyr Met Lys Gly Phe Thr Gly Phe His Glu
225                 230                 235                 240
Phe Ala Glu Phe Thr Ala Glu Asp Val Gly Thr Ala Glu Ser Gly Leu
                245                 250                 255
Asn Ser Val Val Leu Ala Asn Asn Ser Glu Ala Val Leu Leu Pro Leu

260                 265                 270
Asn Glu Pro Val His Gly Thr Lys Arg Arg Ser Gln Ile Gln Thr Tyr
        275                 280                 285
Leu Asp Tyr His Gly Gly Pro Gly Val Gln His Ile Ala Leu Ala Ser
    290                 295                 300
Ser Asp Val Leu Arg Thr Leu Arg Glu Met Arg Ala Arg Ser Ala Met
305                 310                 315                 320
Gly Gly Phe Glu Phe Met Ala Pro Pro Gln Ala Lys Tyr Tyr Glu Gly
                325                 330                 335
Val Arg Arg Leu Ala Gly Asp Val Leu Ser Glu Ala Gln Ile Lys Glu
            340                 345                 350
Cys Gln Glu Leu Gly Val Leu Val Asp Arg Asp Asp Gln Gly Val Leu
        355                 360                 365
Leu Gln Ile Phe Thr Lys Pro Val Gly Asp Arg Pro Thr Phe Phe Leu
    370                 375                 380
Glu Met Ile Gln Arg Ile Gly Cys Met Glu Lys Asp Glu Ile Gly Gln
385                 390                 395                 400
Glu Tyr Gln Lys Gly Gly Cys Gly Gly Phe Gly Lys Gly Asn Phe Ser
                405                 410                 415
Glu Leu Phe Lys Ser Ile Glu Asp Tyr Glu Lys Ser Leu Glu Ala Lys
            420                 425                 430
Gln Ser Ala Val Ala Gln Gln Ser
        435                 440

<210> 25
<211> 1323
<212> DNA
<213> Sorghum halapense

<400> 25
atgcctccga ccccgaccac cgcagcagca acaggtgccg cagttgcagc agcaagcgca      60

gaacaggcag catttcgtct ggttggtcat cgtaattttg ttcgtgttaa tccgcgtagc     120

gatcgttttc ataccctggc atttcatcat gttgaactgt ggtgtgccga tgcagccagc     180

gcagcaggtc gttttagctt tggtctgggt gcaccgctgg cagcacgtag cgatctgagc     240

accggtaata ccgcacatgc aagcctgctg ctgcgttcag gtgcactggc atttctgttt     300

accgcaccgt atgcccatgg tgctgatgca gcaaccgcaa gcctgccgag ctttagcgca     360

gcagaagcac gtcgttttgc agcagatcat ggtctggcag ttcgtgccgt tgcactgcgt     420

gttgcagatg ccgaagatgc atttcgtgca agcgttgcag ccggtgcacg tccggcattt     480

gaaccggttg aactgggtct gggttttcgt ctggccgaag ttgaactgta tggtgatgtt     540

gttctgcgtt atgttagcta tccggatgat gcagatgcaa gctttctgcc gggttttgtt     600

ggtgttagca gtccgggtgc ggcagattat ggcctgcgtc gttttgatca tattgtgggt     660

aatgttccgg aactggcacc ggcagcggca tattttgcag gttttaccgg ctttcatgaa     720

tttgcagaat ttaccgcaga agatgttggc accaccgaaa gcggtctgaa tagcatggtt     780

ctggcaaata atgccgaaaa tgttctgctg ccgctgaatg aaccggtgca tggcaccaaa     840

cgtcgtagcc agattcagac ctttctggat catcatggtg gtccgggtgt tcagcacatg     900

gcactggcaa gtgatgatgt gctgcgtacc ctgcgtgaaa tgcaggcatg tagtgcaatg     960

ggtggttttg aatttatggc accgccggca ccggaatatt atgatggtgt tcgtcgtcgt    1020

gccggtgatg ttctgaccga agcacagatt aaagaatgtc aggaactggg cgttctggtt 1080

gatcgtgatg atcagggtgt tctgctgcag atttttacca aaccggttgg tgatcgcccg 1140

accttttttc tggaaattat tcagcgtatt ggttgcatgg aaaaagatga aaaaggccag 1200

gaatatcaga aaggcggttg tggtggtttt ggtaaaggta attttagcca gctgtttaaa 1260

agcattgaag attatgaaaa aagcctggaa gcaaaacagg cagctgcagc acagggtccg 1320

taa 1323

<210> 26
<211> 440
<212> PRT
<213> Sorghum halapense

<400> 26
Met Pro Pro Thr Pro Thr Thr Ala Ala Ala Thr Gly Ala Ala Val Ala
1               5                   10                  15
Ala Ala Ser Ala Glu Gln Ala Ala Phe Arg Leu Val Gly His Arg Asn
            20                  25                  30
Phe Val Arg Val Asn Pro Arg Ser Asp Arg Phe His Thr Leu Ala Phe
        35                  40                  45
His His Val Glu Leu Trp Cys Ala Asp Ala Ala Ser Ala Ala Gly Arg
    50                  55                  60
Phe Ser Phe Gly Leu Gly Ala Pro Leu Ala Ala Arg Ser Asp Leu Ser
65                  70                  75                  80
Thr Gly Asn Thr Ala His Ala Ser Leu Leu Leu Arg Ser Gly Ala Leu
                85                  90                  95
Ala Phe Leu Phe Thr Ala Pro Tyr Ala His Gly Ala Asp Ala Ala Thr
            100                 105                 110
Ala Ser Leu Pro Ser Phe Ser Ala Ala Glu Ala Arg Arg Phe Ala Ala
        115                 120                 125
Asp His Gly Leu Ala Val Arg Ala Val Ala Leu Arg Val Ala Asp Ala
    130                 135                 140
Glu Asp Ala Phe Arg Ala Ser Val Ala Ala Gly Ala Arg Pro Ala Phe
145                 150                 155                 160
Glu Pro Val Glu Leu Gly Leu Gly Phe Arg Leu Ala Glu Val Glu Leu
                165                 170                 175
Tyr Gly Asp Val Val Leu Arg Tyr Val Ser Tyr Pro Asp Asp Ala Asp
            180                 185                 190
Ala Ser Phe Leu Pro Gly Phe Val Gly Val Ser Ser Pro Gly Ala Ala
        195                 200                 205
Asp Tyr Gly Leu Arg Arg Phe Asp His Ile Val Gly Asn Val Pro Glu
    210                 215                 220
Leu Ala Pro Ala Ala Ala Tyr Phe Ala Gly Phe Thr Gly Phe His Glu
225                 230                 235                 240
Phe Ala Glu Phe Thr Ala Glu Asp Val Gly Thr Thr Glu Ser Gly Leu
                245                 250                 255
Asn Ser Met Val Leu Ala Asn Asn Ala Glu Asn Val Leu Leu Pro Leu
            260                 265                 270
Asn Glu Pro Val His Gly Thr Lys Arg Arg Ser Gln Ile Gln Thr Phe
        275                 280                 285
Leu Asp His His Gly Gly Pro Gly Val Gln His Met Ala Leu Ala Ser
    290                 295                 300
Asp Asp Val Leu Arg Thr Leu Arg Glu Met Gln Ala Cys Ser Ala Met
305                 310                 315                 320
Gly Gly Phe Glu Phe Met Ala Pro Pro Ala Pro Glu Tyr Tyr Asp Gly
                325                 330                 335
Val Arg Arg Arg Ala Gly Asp Val Leu Thr Glu Ala Gln Ile Lys Glu

340                 345                 350
Cys Gln Glu Leu Gly Val Leu Val Asp Arg Asp Asp Gln Gly Val Leu
        355                 360                 365
Leu Gln Ile Phe Thr Lys Pro Val Gly Asp Arg Pro Thr Phe Phe Leu
    370                 375                 380
Glu Ile Ile Gln Arg Ile Gly Cys Met Glu Lys Asp Glu Lys Gly Gln
385                 390                 395                 400
Glu Tyr Gln Lys Gly Gly Cys Gly Gly Phe Gly Lys Gly Asn Phe Ser
                405                 410                 415
Gln Leu Phe Lys Ser Ile Glu Asp Tyr Glu Lys Ser Leu Glu Ala Lys
            420                 425                 430
Gln Ala Ala Ala Ala Gln Gly Pro
        435                 440

<210> 27
<211> 1323
<212> DNA
<213> Sorghum halapense

<400> 27
atgcctccga ccccgaccac cgcagcagca acaggtgccg cagttgcagc agcaagcgca      60

gaacaggcag catttcgtct ggttggtcat cgtaattttg ttcgtgttaa tccgcgtagc     120

gatcgttttc ataccctggc atttcatcat gttgaactgt ggtgtgccga tgcagccagc     180

gcagcaggtc gttttagctt tggtctgggt gcaccgctgg cagcacgtag cgatctgagc     240

accggtaata ccgcacatgc aagcctgctg ctgcgttcag gtgcactggc atttctgttt     300

accgcaccgt atgcccatgg tgctgatgca gcaaccgcaa gcctgccgag ctttagcgca     360

gcagaagcac gtcgttttgc agcagatcat ggtctggcag ttcgtgccgt tgcactgcgt     420

gttgcagatg ccgaagatgc atttcgtgca agcgttgcag ccggtgcacg tccggcattt     480

gaaccggttg aactgggtct gggttttcgt ctggccgaag ttgaactgta tggtgatgtt     540

gttctgcgtt atgttagcta tccggatgat gcagatgcaa gctttctgcc gggttttgtt     600

ggtgttacca gtccgggtgc ggcagattat ggcctgaaac gttttgatca tattgtgggt     660

aatgttccgg aactggcacc ggcagcggca tattttgcag gttttaccgg ctttcatgaa     720

tttgcagaat ttaccgcaga agatgttggc accaccgaaa gcggtctgaa tagcatggtt     780

ctggcaaata atgccgaaaa tgttctgctg ccgctgaatg aaccggtgca tggcaccaaa     840

cgtcgtagcc agattcagac ctttctggat catcatggtg gtccgggtgt tcagcacatg     900

gcactggcaa gtgatgatgt gctgcgtacc ctgcgtgaaa tgcaggcacg tagtgcaatg     960

ggtggttttg aatttatggc accgccggca ccggaatatt atgatggtgt tcgtcgtcgt    1020

gccggtgatg ttctgaccga agcacagatt aaagaatgtc aggaactggg cgttctggtt    1080

gatcgtgatg atcagggtgt tctgctgcag atttttacca aaccggttgg tgatcgcccg    1140

accttttttc tggaaattat tcagcgtatt ggttgcatgg aaaaagatga aaaaggccag    1200

gaatatcaga aaggcggttg tggtggtttt ggtaaaggta attttagcca gctgtttaaa    1260

agcattgaag attatgaaaa aagcctggaa gcaaaacagg cagctgcagc acagggtccg    1320

taa                                                                 1323

<210> 28
<211> 453
<212> PRT
<213> Sorghum halapense

<400> 28
Met Pro Pro Thr Pro Thr Thr Ala Ala Ala Thr Gly Ala Ala Val Ala
1               5                   10                  15
Ala Ala Ser Ala Glu Gln Ala Ala Phe Arg Leu Val Gly His Arg Asn
            20                  25                  30
Phe Val Arg Val Asn Pro Arg Ser Asp Arg Phe His Thr Leu Ala Phe
        35                  40                  45
His His Val Glu Leu Trp Cys Ala Asp Ala Ala Ser Ala Ala Gly Arg
    50                  55                  60
Phe Ser Phe Gly Leu Gly Ala Pro Leu Ala Ala Arg Ser Asp Leu Ser
65                  70                  75                  80
Thr Gly Asn Thr Ala His Ala Ser Leu Leu Leu Arg Ser Gly Ala Leu
                85                  90                  95
Ala Phe Leu Phe Thr Ala Pro Tyr Ala His Gly Ala Asp Ala Ala Thr
            100                 105                 110
Ala Ser Leu Pro Ser Phe Ser Ala Ala Glu Ala Arg Arg Phe Ala Ala
        115                 120                 125
Asp His Gly Leu Ala Val Arg Ala Val Ala Leu Arg Val Ala Asp Ala
    130                 135                 140
Glu Asp Ala Phe Arg Ala Ser Val Ala Ala Gly Ala Arg Pro Ala Phe
145                 150                 155                 160
Glu Pro Val Glu Leu Gly Leu Gly Phe Arg Leu Ala Glu Val Glu Leu
                165                 170                 175
Tyr Gly Asp Val Val Leu Arg Tyr Val Ser Tyr Pro Thr Thr Arg Thr
            180                 185                 190
Arg Pro Ser Cys Arg Gly Ser Trp Ala Asp Asp Ala Asp Ala Ser Phe
        195                 200                 205
Leu Pro Gly Phe Val Gly Val Thr Ser Pro Gly Ala Ala Asp Tyr Gly
    210                 215                 220
Leu Lys Arg Phe Asp His Ile Val Gly Asn Val Pro Glu Leu Ala Pro
225                 230                 235                 240
Ala Ala Ala Tyr Phe Ala Gly Phe Thr Gly Phe His Glu Phe Ala Glu
                245                 250                 255
Phe Thr Ala Glu Asp Val Gly Thr Thr Glu Ser Gly Leu Asn Ser Met
            260                 265                 270
Val Leu Ala Asn Asn Ala Glu Asn Val Leu Leu Pro Leu Asn Glu Pro
        275                 280                 285
Val His Gly Thr Lys Arg Arg Ser Gln Ile Gln Thr Phe Leu Asp His
    290                 295                 300
His Gly Gly Pro Gly Val Gln His Met Ala Leu Ala Ser Asp Asp Val
305                 310                 315                 320
Leu Arg Thr Leu Arg Glu Met Gln Ala Arg Ser Ala Met Gly Gly Phe
                325                 330                 335
Glu Phe Met Ala Pro Pro Ala Pro Glu Tyr Tyr Asp Gly Val Arg Arg
            340                 345                 350
Arg Ala Gly Asp Val Leu Thr Glu Ala Gln Ile Lys Glu Cys Gln Glu
        355                 360                 365
Leu Gly Val Leu Val Asp Arg Asp Asp Gln Gly Val Leu Leu Gln Ile
    370                 375                 380
Phe Thr Lys Pro Val Gly Asp Arg Pro Thr Phe Phe Leu Glu Ile Ile
385                 390                 395                 400
Gln Arg Ile Gly Cys Met Glu Lys Asp Glu Lys Gly Gln Glu Tyr Gln
                405                 410                 415
Lys Gly Gly Cys Gly Gly Phe Gly Lys Gly Asn Phe Ser Gln Leu Phe

420                 425                 430
Lys Ser Ile Glu Asp Tyr Glu Lys Ser Leu Glu Ala Lys Gln Ala Ala
        435                 440                 445
Ala Ala Gln Gly Pro
    450

<210> 29
<211> 1311
<212> DNA
<213> Poa annua

<400> 29
atgcctccga ccaccgcaac cgccaccgca gcagcaaccg ttacaccgga acatgcagca      60

cgtcgttttc cgcgtgttgt tcgtgttaat ccgcgtagcg atcgttttcc ggttctgagc     120

tttcatcatg ttgaattttg gtgtgccgat gcagcaagcg cagcaggtcg ttttagcttt     180

gcactgggtg caccgctggc agcacgtagc gatctgagca ccggtaatag cgcacatgca     240

agcctgctgc tgcgttcagg tgcactggca tttctgttta ccgcaccgta tgcaccgcag     300

ccgcaggatg cagataccgc aagcattccg agctttagcg cagatgcagc acgtgcattt     360

agcgcagcac atggtctggc agttcgtagc gttgcagttc gtgttgcaga tgccgcagat     420

gcatttcgtg caagcattgc agccggtgca cgtccggcat ttgcaccggc agatctgggt     480

cgtggttttg gtctggccga agttgaactg tatggtgatg ttgttctgcg ttttgttagc     540

catccggatg cagatgatgc accgccgttt ctgccgggtt ttgaagcagt tagccgtcgt     600

ccgggtgccg ttgattatgg tctgacccgt tttgatcatg ttgttggtaa tgttccggaa     660

atgggtccgg tgattgatta tattaaaggc tttatgggct ttcatgaatt tgccgaattt     720

accgcagaag atgttggcac caccgaaagc ggtctgaata gcgttgttct ggcaaataat     780

agcgaagcag ttctgctgcc gctgaatgaa ccggtgcatg gcaccaaacg tcgtagccag     840

attcagacct atctggaata tcatggtggt ccgggtgttc agcatattgc actggcaagc     900

agtgatgttc tgcgtaccct gcgtgaaatg caggcacgtt cagcaatggg tggttttgaa     960

tttatggcac cgccgcagcc gaaatattat gaaggtgttc gtcgtattgc cggtgatgtt    1020

ctgagcgaag cacagattaa agaatgtcag gaactgggcg ttctggttga tcgtgatgat    1080

cagggtgttc tgctgcagat ttttaccaaa ccggttggtg atcgtccgac cttttttctg    1140

gaaatgattc agcgtattgg ctgcatggaa aaagatgaac gtggtcagga atatcagaaa    1200

ggcggttgtg gcggttttgg taaaggtaat tttagcgaac tgtttaaaag cattgaagat    1260

tatgaaaaaa gcctggaagc caaacagagc gcagttgcac agcagagcta a            1311


<210> 30
<211> 436
<212> PRT
<213> Poa annua

<400> 30

Met Pro Pro Thr Thr Ala Thr Ala Thr Ala Ala Ala Thr Val Thr Pro
1               5                   10                  15
Glu His Ala Ala Arg Arg Phe Pro Arg Val Val Arg Val Asn Pro Arg
            20                  25                  30
Ser Asp Arg Phe Pro Val Leu Ser Phe His His Val Glu Phe Trp Cys
        35                  40                  45
Ala Asp Ala Ala Ser Ala Ala Gly Arg Phe Ser Phe Ala Leu Gly Ala
    50                  55                  60
Pro Leu Ala Ala Arg Ser Asp Leu Ser Thr Gly Asn Ser Ala His Ala
65                  70                  75                  80
Ser Leu Leu Leu Arg Ser Gly Ala Leu Ala Phe Leu Phe Thr Ala Pro
                85                  90                  95
Tyr Ala Pro Gln Pro Gln Asp Ala Asp Thr Ala Ser Ile Pro Ser Phe
            100                 105                 110
Ser Ala Asp Ala Ala Arg Ala Phe Ser Ala Ala His Gly Leu Ala Val
        115                 120                 125
Arg Ser Val Ala Val Arg Val Ala Asp Ala Ala Asp Ala Phe Arg Ala
    130                 135                 140
Ser Ile Ala Ala Gly Ala Arg Pro Ala Phe Ala Pro Ala Asp Leu Gly
145                 150                 155                 160
Arg Gly Phe Gly Leu Ala Glu Val Glu Leu Tyr Gly Asp Val Val Leu
                165                 170                 175
Arg Phe Val Ser His Pro Asp Ala Asp Asp Ala Pro Pro Phe Leu Pro
            180                 185                 190
Gly Phe Glu Ala Val Ser Arg Arg Pro Gly Ala Val Asp Tyr Gly Leu
        195                 200                 205
Thr Arg Phe Asp His Val Val Gly Asn Val Pro Glu Met Gly Pro Val
    210                 215                 220
Ile Asp Tyr Ile Lys Gly Phe Met Gly Phe His Glu Phe Ala Glu Phe
225                 230                 235                 240
Thr Ala Glu Asp Val Gly Thr Thr Glu Ser Gly Leu Asn Ser Val Val
                245                 250                 255
Leu Ala Asn Asn Ser Glu Ala Val Leu Leu Pro Leu Asn Glu Pro Val
            260                 265                 270
His Gly Thr Lys Arg Arg Ser Gln Ile Gln Thr Tyr Leu Glu Tyr His
        275                 280                 285
Gly Gly Pro Gly Val Gln His Ile Ala Leu Ala Ser Ser Asp Val Leu
    290                 295                 300
Arg Thr Leu Arg Glu Met Gln Ala Arg Ser Ala Met Gly Gly Phe Glu
305                 310                 315                 320
Phe Met Ala Pro Pro Gln Pro Lys Tyr Tyr Glu Gly Val Arg Arg Ile
                325                 330                 335
Ala Gly Asp Val Leu Ser Glu Ala Gln Ile Lys Glu Cys Gln Glu Leu
            340                 345                 350
Gly Val Leu Val Asp Arg Asp Asp Gln Gly Val Leu Leu Gln Ile Phe
        355                 360                 365
Thr Lys Pro Val Gly Asp Arg Pro Thr Phe Phe Leu Glu Met Ile Gln
    370                 375                 380
Arg Ile Gly Cys Met Glu Lys Asp Glu Arg Gly Gln Glu Tyr Gln Lys
385                 390                 395                 400
Gly Gly Cys Gly Gly Phe Gly Lys Gly Asn Phe Ser Glu Leu Phe Lys
                405                 410                 415
Ser Ile Glu Asp Tyr Glu Lys Ser Leu Glu Ala Lys Gln Ser Ala Val
            420                 425                 430
Ala Gln Gln Ser
        435

<210> 31
<211> 1305
<212> DNA
<213> Poa annua

<400> 31

atgccaccaa ctactgctac tgctacagct gctgctactg ttactccaga acatgctgct 60

agaaggttcc caagagttgt tagagttaac ccaaggtctg ataggttccc agttctttct 120

ttccaccacg ttgaattttg gtgtgctgat gctgcttctg ctgctggaag attttctttt 180

gctcttggtg ctccacttgc tgctagatct gatttgtcta ctggaaattc tgctcacgct 240

tctttgcttt tgaggtctgg tgctcttgct ttccttttta ctgctcctta tgctccacaa 300

ccacaggatg ctgatactgc atcaattcca tctttctcag ctgatgctgc aagggctttt 360

tctgctgctc atggattggc tgttagatct gttgctgtta gagttgctga tgcagctgat 420

gctttcagag cttctattgc tgcaggtgct agaccagctt ttgctccagc tgatcttgga 480

agaggatttg gacttgctga ggttgagctt tacggtgatg ttgttcttag attcgtgtct 540

cacccagatg ctgatgatgc tccatttctt ccaggatttg aggctgtttc tagaccaggt 600

gctgttgatt atggactcac taggttcgat cacgttgtgg gaaatgttcc agaaatggga 660

ccagtgatcg attacatcaa gggattcatg ggattccatg agttcgctga gtttactgct 720

gaggatgttg gaactactga gtctggactt aactctgttg tgcttgctaa caactctgag 780

gctgttcttt tgccacttaa tgagccagtt cacggcacta agagaagatc tcagattcag 840

acttaccttg agtaccatgg tggaccaggt gttcaacata ttgctcttgc ttcatctgat 900

gtgcttagga ctcttagaga gatgcaagct agatctgcta tgggaggatt tgagtttatg 960

gctccaccac aacctaagta ttacgagggt gttagaagga ttgctggtga tgttctttcc 1020

gaggctcaaa tcaaagagtg tcaagagctt ggagtgcttg tggatagaga tgatcagggt 1080

gtgcttctcc agattttcac taagccagtt ggagataggc caacattctt cttggagatg 1140

attcagagga tcggctgcat ggaaaaggat gagagaggtc aagagtatca aaagggcgga 1200

tgtggtggat ttggaaaggg aaatttctcc gagcttttca agtccatcga ggattacgag 1260

aagtctcttg aggctaagca atctgctgtt gctcaacagt cttga 1305

<210> 32
<211> 434
<212> PRT
<213> Poa annua

<400> 32
Met Pro Pro Thr Thr Ala Thr Ala Thr Ala Ala Ala Thr Val Thr Pro
1               5                   10                  15
Glu His Ala Ala Arg Arg Phe Pro Arg Val Val Arg Val Asn Pro Arg
            20                  25                  30
Ser Asp Arg Phe Pro Val Leu Ser Phe His His Val Glu Phe Trp Cys
        35                  40                  45
Ala Asp Ala Ala Ser Ala Ala Gly Arg Phe Ser Phe Ala Leu Gly Ala
    50                  55                  60
Pro Leu Ala Ala Arg Ser Asp Leu Ser Thr Gly Asn Ser Ala His Ala
65                  70                  75                  80
Ser Leu Leu Leu Arg Ser Gly Ala Leu Ala Phe Leu Phe Thr Ala Pro
                85                  90                  95

Tyr Ala Pro Gln Pro Gln Asp Ala Asp Thr Ala Ser Ile Pro Ser Phe
            100                 105                 110
Ser Ala Asp Ala Ala Arg Ala Phe Ser Ala Ala His Gly Leu Ala Val
        115                 120                 125
Arg Ser Val Ala Val Arg Val Ala Asp Ala Ala Asp Ala Phe Arg Ala
    130                 135                 140
Ser Ile Ala Ala Gly Ala Arg Pro Ala Phe Ala Pro Ala Asp Leu Gly
145                 150                 155                 160
Arg Gly Phe Gly Leu Ala Glu Val Glu Leu Tyr Gly Asp Val Val Leu
                165                 170                 175
Arg Phe Val Ser His Pro Asp Ala Asp Asp Ala Pro Phe Leu Pro Gly
            180                 185                 190
Phe Glu Ala Val Ser Arg Pro Gly Ala Val Asp Tyr Gly Leu Thr Arg
        195                 200                 205
Phe Asp His Val Val Gly Asn Val Pro Glu Met Gly Pro Val Ile Asp
    210                 215                 220
Tyr Ile Lys Gly Phe Met Gly Phe His Glu Phe Ala Glu Phe Thr Ala
225                 230                 235                 240
Glu Asp Val Gly Thr Thr Glu Ser Gly Leu Asn Ser Val Val Leu Ala
                245                 250                 255
Asn Asn Ser Glu Ala Val Leu Leu Pro Leu Asn Glu Pro Val His Gly
            260                 265                 270
Thr Lys Arg Arg Ser Gln Ile Gln Thr Tyr Leu Glu Tyr His Gly Gly
        275                 280                 285
Pro Gly Val Gln His Ile Ala Leu Ala Ser Ser Asp Val Leu Arg Thr
    290                 295                 300
Leu Arg Glu Met Gln Ala Arg Ser Ala Met Gly Gly Phe Glu Phe Met
305                 310                 315                 320
Ala Pro Pro Gln Pro Lys Tyr Tyr Glu Gly Val Arg Arg Ile Ala Gly
                325                 330                 335
Asp Val Leu Ser Glu Ala Gln Ile Lys Glu Cys Gln Glu Leu Gly Val
            340                 345                 350
Leu Val Asp Arg Asp Asp Gln Gly Val Leu Leu Gln Ile Phe Thr Lys
        355                 360                 365
Pro Val Gly Asp Arg Pro Thr Phe Phe Leu Glu Met Ile Gln Arg Ile
    370                 375                 380
Gly Cys Met Glu Lys Asp Glu Arg Gly Gln Glu Tyr Gln Lys Gly Gly
385                 390                 395                 400
Cys Gly Gly Phe Gly Lys Gly Asn Phe Ser Glu Leu Phe Lys Ser Ile
                405                 410                 415
Glu Asp Tyr Glu Lys Ser Leu Glu Ala Lys Gln Ser Ala Val Ala Gln
            420                 425                 430
Gln Ser

<210> 33
<211> 1323
<212> DNA
<213> Lolium multiflorum

<400> 33
atgcctccga caccggcaac cgcaaccggt gctgcagcag cagcagttac accggaacat      60

gcagcacgta gctttccgcg tgttgttcgt gttaatccgc gtagcgatcg ttttccggtt     120

ctgagctttc atcatgttga actgtggtgt gccgatgcag caagcgcagc aggtcgtttt     180

agctttgcac tgggtgctcc gctggcagcc cgtagcgatc tgagcaccgg taatagcgca     240

catgcaagcc tgctgctgcg tagcggtgca ctggcatttc tgtttaccgc accgtatgca     300

ccgcctccgc aggaagcagc aaccgcagct gcaaccgcaa gcattccgag ctttagcgca     360

gatgcagccc gtacctttgc agcagcacat ggtctggcag ttcgtagcgt tggtgttcgt 420

gttgccgatg cagcggaagc atttcgtgtt agcgttgccg gtggtgcacg tccggcattt 480

gcaccggcag atctgggtca tggttttggt ctggccgaag ttgaactgta tggtgatgtt 540

gttctgcgtt ttgttagcta tccggatgaa accgatctgc cgtttctgcc gggttttgaa 600

cgtgttagca gtccgggtgc cgttgattat ggtctgaccc gttttgatca tgttgttggt 660

aatgttccgg aaatggcacc ggttattgat tatatgaaag gctttctggg ctttcatgaa 720

tttgcagaat ttaccgcaga agatgttggc accaccgaaa gcggtctgaa tagcgttgtt 780

ctggcaaata atagcgaaaa tgttctgctg ccgctgaatg aaccggtgca tggcaccaaa 840

cgtcgtagcc agattcagac ctatctggat tatcatggtg gtccgggtgt tcagcatatt 900

gcactggcaa gcaccgatgt tctgcgtacc ctgcgtgaaa tgcgtgcacg taccccgatg 960

ggtggttttg aatttatggc accgccgcag gcaaaatatt atgaaggtgt tcgtcgtatt 1020

gccggtgatg ttctgagcga agaacaaatt aaagaatgtc aggaactggg cgttctggtt 1080

gatcgtgatg atcagggtgt tctgctgcag atttttacca aaccggttgg tgatcgtccg 1140

accttttttc tggaaatgat tcagcgtatt ggctgcatgg aaaaagatga agttggtcag 1200

gaatatcaga aaggcggttg tggtggtttt ggtaaaggta attttagcga actgtttaaa 1260

agcattgaag attatgaaaa aaccctggaa gccaaacaga gcgttgttgc acagaaaagc 1320

taa 1323

<210> 34
<211> 440
<212> PRT
<213> Lolium multiflorum

<400> 34
Met Pro Pro Thr Pro Ala Thr Ala Thr Gly Ala Ala Ala Ala Ala Val
1               5                   10                  15
Thr Pro Glu His Ala Ala Arg Ser Phe Pro Arg Val Val Arg Val Asn
            20                  25                  30
Pro Arg Ser Asp Arg Phe Pro Val Leu Ser Phe His His Val Glu Leu
        35                  40                  45
Trp Cys Ala Asp Ala Ala Ser Ala Ala Gly Arg Phe Ser Phe Ala Leu
    50                  55                  60
Gly Ala Pro Leu Ala Ala Arg Ser Asp Leu Ser Thr Gly Asn Ser Ala
65                  70                  75                  80
His Ala Ser Leu Leu Leu Arg Ser Gly Ala Leu Ala Phe Leu Phe Thr
                85                  90                  95
Ala Pro Tyr Ala Pro Pro Pro Gln Glu Ala Ala Thr Ala Ala Ala Thr
            100                 105                 110
Ala Ser Ile Pro Ser Phe Ser Ala Asp Ala Ala Arg Thr Phe Ala Ala
        115                 120                 125
Ala His Gly Leu Ala Val Arg Ser Val Gly Val Arg Val Ala Asp Ala
    130                 135                 140
Ala Glu Ala Phe Arg Val Ser Val Ala Gly Gly Ala Arg Pro Ala Phe
145                 150                 155                 160
Ala Pro Ala Asp Leu Gly His Gly Phe Gly Leu Ala Glu Val Glu Leu
                165                 170                 175

Tyr Gly Asp Val Val Leu Arg Phe Val Ser Tyr Pro Asp Glu Thr Asp
            180                 185                 190
Leu Pro Phe Leu Pro Gly Phe Glu Arg Val Ser Ser Pro Gly Ala Val
        195                 200                 205
Asp Tyr Gly Leu Thr Arg Phe Asp His Val Val Gly Asn Val Pro Glu
    210                 215                 220
Met Ala Pro Val Ile Asp Tyr Met Lys Gly Phe Leu Gly Phe His Glu
225                 230                 235                 240
Phe Ala Glu Phe Thr Ala Glu Asp Val Gly Thr Thr Glu Ser Gly Leu
                245                 250                 255
Asn Ser Val Val Leu Ala Asn Asn Ser Glu Asn Val Leu Leu Pro Leu
            260                 265                 270
Asn Glu Pro Val His Gly Thr Lys Arg Arg Ser Gln Ile Gln Thr Tyr
        275                 280                 285
Leu Asp Tyr His Gly Gly Pro Gly Val Gln His Ile Ala Leu Ala Ser
    290                 295                 300
Thr Asp Val Leu Arg Thr Leu Arg Glu Met Arg Ala Arg Thr Pro Met
305                 310                 315                 320
Gly Gly Phe Glu Phe Met Ala Pro Pro Gln Ala Lys Tyr Tyr Glu Gly
                325                 330                 335
Val Arg Arg Ile Ala Gly Asp Val Leu Ser Glu Glu Gln Ile Lys Glu
            340                 345                 350
Cys Gln Glu Leu Gly Val Leu Val Asp Arg Asp Asp Gln Gly Val Leu
        355                 360                 365
Leu Gln Ile Phe Thr Lys Pro Val Gly Asp Arg Pro Thr Phe Phe Leu
    370                 375                 380
Glu Met Ile Gln Arg Ile Gly Cys Met Glu Lys Asp Glu Val Gly Gln
385                 390                 395                 400
Glu Tyr Gln Lys Gly Gly Cys Gly Gly Phe Gly Lys Gly Asn Phe Ser
                405                 410                 415
Glu Leu Phe Lys Ser Ile Glu Asp Tyr Glu Lys Thr Leu Glu Ala Lys
            420                 425                 430
Gln Ser Val Val Ala Gln Lys Ser
        435                 440

<210> 35
<211> 1053
<212> DNA
<213> Synechococcus sp

<400> 35
atgaacccgt ccattcgaat tgtccaaggg atccaccacc tgcacttcta cctttgggat      60

ctgccccgtt ggcgggaaca cttttgtcgg gtttggggct tccgggtggc aagcgacgcc     120

ggcaacaccc tggagctgga gcagggatcc ctgcgcttgc gcctgtctca gccggcacgg     180

gcgggggacg aggtggaccg ccatttgcag cggcatgggc cgggggtggt ggatgtggcc     240

ttggcggtgg gagagcagga gctaccggcc ttggcggagc tgttgcgggg ccgaggcgcc     300

caactggcgt ggatcccggc agcagcggcg ctctgcctcc acacccccta cgggatccgg     360

cattctctga tccctggccc cttggatgcc gcccctgccg aagcgggcct gttttcccac     420

tgggatcacg tggtgttgaa cgtggagcag ggatccctgc aggcggcagc cgactggtat     480

gggcgggtgc tgggctggcg gcggctgtac cgctacagca tcggcaccgc cacctccggc     540

ctggaaagcg tggtggtggg ggatccggaa gcggggatcc aatgggccat caacgagccc     600

acctgtgccg cttcccagat tcaggagttt ttgcatgccc atggcggccc gggcattcag     660

cacgcggcgc tgcacagctc agacattgtt gccagcctgc gccggttgcg gcagggggga 720

gtggactttt tgcaagtggc gccgcagtac tacaccagcc tggaaaggga gctggggttg 780

gcgctccgtt ctgcccttgg gcaggccatc tcctggcaag acctggtgga gcagcagatc 840

cttctggatg ctaccctgcc cgcttctgat ggccaggatc gcccccttct gctgcagacc 900

tttacccagc ccctctttgg tcggcccacc tttttctttg aagtcattca acggctaggc 960

ggggccacgg gctttggcga ggccaatttt caggctttgt tcgaggccct ggaacggcaa 1020

cagcgacagc gacaccaggc gctgacccct tag 1053

<210> 36
<211> 350
<212> PRT
<213> Synechococcus sp

<400> 36
Met Asn Pro Ser Ile Arg Ile Val Gln Gly Ile His His Leu His Phe
1               5                   10                  15
Tyr Leu Trp Asp Leu Pro Arg Trp Arg Glu His Phe Cys Arg Val Trp
            20                  25                  30
Gly Phe Arg Val Ala Ser Asp Ala Gly Asn Thr Leu Glu Leu Glu Gln
        35                  40                  45
Gly Ser Leu Arg Leu Arg Leu Ser Gln Pro Ala Arg Ala Gly Asp Glu
    50                  55                  60
Val Asp Arg His Leu Gln Arg His Gly Pro Gly Val Val Asp Val Ala
65                  70                  75                  80
Leu Ala Val Gly Glu Gln Glu Leu Pro Ala Leu Ala Glu Leu Leu Arg
                85                  90                  95
Gly Arg Gly Ala Gln Leu Ala Trp Ile Pro Ala Ala Ala Ala Leu Cys
            100                 105                 110
Leu His Thr Pro Tyr Gly Ile Arg His Ser Leu Ile Pro Gly Pro Leu
        115                 120                 125
Asp Ala Ala Pro Ala Glu Ala Gly Leu Phe Ser His Trp Asp His Val
    130                 135                 140
Val Leu Asn Val Glu Gln Gly Ser Leu Gln Ala Ala Ala Asp Trp Tyr
145                 150                 155                 160
Gly Arg Val Leu Gly Trp Arg Arg Leu Tyr Arg Tyr Ser Ile Gly Thr
                165                 170                 175
Ala Thr Ser Gly Leu Glu Ser Val Val Val Gly Asp Pro Glu Ala Gly
            180                 185                 190
Ile Gln Trp Ala Ile Asn Glu Pro Thr Cys Ala Ala Ser Gln Ile Gln
        195                 200                 205
Glu Phe Leu His Ala His Gly Gly Pro Gly Ile Gln His Ala Ala Leu
    210                 215                 220
His Ser Ser Asp Ile Val Ala Ser Leu Arg Arg Leu Arg Gln Gly Gly
225                 230                 235                 240
Val Asp Phe Leu Gln Val Ala Pro Gln Tyr Tyr Thr Ser Leu Glu Arg
                245                 250                 255
Glu Leu Gly Leu Ala Leu Arg Ser Ala Leu Gly Gln Ala Ile Ser Trp
            260                 265                 270
Gln Asp Leu Val Glu Gln Gln Ile Leu Leu Asp Ala Thr Leu Pro Ala
        275                 280                 285
Ser Asp Gly Gln Asp Arg Pro Leu Leu Leu Gln Thr Phe Thr Gln Pro
    290                 295                 300
Leu Phe Gly Arg Pro Thr Phe Phe Phe Glu Val Ile Gln Arg Leu Gly
305                 310                 315                 320
Gly Ala Thr Gly Phe Gly Glu Ala Asn Phe Gln Ala Leu Phe Glu Ala
                325                 330                 335

Leu Glu Arg Gln Gln Arg Gln Arg His Gln Ala Leu Thr Pro
            340                 345                 350

<210> 37
<211> 1149
<212> DNA
<213> Blepharisma japonicum

<400> 37
atgacttatt acgacaagca agaaacgcgt ccagatcttg gcgaattcta tggtttccat      60

cacgttcgtt tttacgtctc caactcagag caagccgctt cgttctacac atctcgcttt     120

gggttttctc cggttgccta tgaaggattg gaaacaggaa accaaaaatt ctgtaccaat     180

gtcgtccgaa gcaaccatgt agtcatcgct tttacctcag ctctcactcc tgaagacaat     240

gaagtgaacc gtcacgttgg caagcatagt gatggagttc aagacattgc ctttagtgta     300

agtgacgcaa gagggatgta tgagaaagcg atagctaaag gctgtaaaag cttccgtgag     360

ccacaggttt tacaagatca atttggatct gttataatag cgtctctcca gacttatgga     420

gacactgttc acacattagt ccaaaatgtc gactatacag gacccttttt gcctggcttc     480

agagcaatca caaaagatga tccattaaac tctgcctttc ctcaggtaaa ttatgacatt     540

attgatcatg ttgtaggaaa tcagcctggt ggcgatatga ctcctacagt agaatggtat     600

gagaaatatc tagaatttca tcgatattgg tctgctgatg agtctgtaat ccataccgat     660

tattcagcat taaggtctgt tgtggttgct gattgggatg aagtgatcaa aatgcctatt     720

aatgagcctg ctgatggact tagaaaaagt caaatccaag aatatgtcga atattatggt     780

ggagcaggcg tacaacatat tgccttaaaa gtcaatgata ttatttcagt aataagcacc     840

ttaagggcta gaggtgtgga attcttagaa gttcctccta aatattatga tagcttaaga     900

aaaagacttg cgcattctgc ggtacaaatt gaagaagact taaaaagaat tgaagacctt     960

catattttgg ttgactttga cgaccgtggg tatttacttc agattttcac aaaaccagta    1020

gaagacagac ctactctgtt ttatgaaatt attcaaagac ataataacaa tggattcgga    1080

attggaaatt ttaaagccct atttgaatca ttggaacaag agcaagaaag aagaggtaat    1140

ttgatctaa                                                            1149


<210> 38
<211> 350
<212> PRT
<213> Blephrisman japonicum

<400> 38
Met Asn Pro Ser Ile Arg Ile Val Gln Gly Ile His His Leu His Phe
1               5                   10                  15
Tyr Leu Trp Asp Leu Pro Arg Trp Arg Glu His Phe Cys Arg Val Trp
            20                  25                  30
Gly Phe Arg Val Ala Ser Asp Ala Gly Asn Thr Leu Glu Leu Glu Gln
        35                  40                  45
Gly Ser Leu Arg Leu Arg Leu Ser Gln Pro Ala Arg Ala Gly Asp Glu

50                  55                  60
Val Asp Arg His Leu Gln Arg His Gly Pro Gly Val Val Asp Val Ala
65                  70                  75                  80
Leu Ala Val Gly Glu Gln Glu Leu Pro Ala Leu Ala Glu Leu Leu Arg
                85                  90                  95
Gly Arg Gly Ala Gln Leu Ala Trp Ile Pro Ala Ala Ala Ala Leu Cys
            100                 105                 110
Leu His Thr Pro Tyr Gly Ile Arg His Ser Leu Ile Pro Gly Pro Leu
        115                 120                 125
Asp Ala Ala Pro Ala Glu Ala Gly Leu Phe Ser His Trp Asp His Val
    130                 135                 140
Val Leu Asn Val Glu Gln Gly Ser Leu Gln Ala Ala Ala Asp Trp Tyr
145                 150                 155                 160
Gly Arg Val Leu Gly Trp Arg Arg Leu Tyr Arg Tyr Ser Ile Gly Thr
                165                 170                 175
Ala Thr Ser Gly Leu Glu Ser Val Val Val Gly Asp Pro Glu Ala Gly
            180                 185                 190
Ile Gln Trp Ala Ile Asn Glu Pro Thr Cys Ala Ala Ser Gln Ile Gln
        195                 200                 205
Glu Phe Leu His Ala His Gly Gly Pro Gly Ile Gln His Ala Ala Leu
    210                 215                 220
His Ser Ser Asp Ile Val Ala Ser Leu Arg Arg Leu Arg Gln Gly Gly
225                 230                 235                 240
Val Asp Phe Leu Gln Val Ala Pro Gln Tyr Tyr Thr Ser Leu Glu Arg
                245                 250                 255
Glu Leu Gly Leu Ala Leu Arg Ser Ala Leu Gly Gln Ala Ile Ser Trp
            260                 265                 270
Gln Asp Leu Val Glu Gln Gln Ile Leu Leu Asp Ala Thr Leu Pro Ala
        275                 280                 285
Ser Asp Gly Gln Asp Arg Pro Leu Leu Leu Gln Thr Phe Thr Gln Pro
    290                 295                 300
Leu Phe Gly Arg Pro Thr Phe Phe Phe Glu Val Ile Gln Arg Leu Gly
305                 310                 315                 320
Gly Ala Thr Gly Phe Gly Glu Ala Asn Phe Gln Ala Leu Phe Glu Ala
                325                 330                 335
Leu Glu Arg Gln Gln Arg Gln Arg His Gln Ala Leu Thr Pro
            340                 345                 350

<210> 39
<211> 1107
<212> DNA
<213> Picrophilus torridus

<400> 39
atgtatggca aaaatttaat ctcagaacta agggaaaagg agatctttaa acgattacat      60

cacgtggaat tttacgttag cagtgccaaa acatggtcat atttcatgaa caggggtctt     120

ggatttaaaa cagtggcata tgccggtcca gaaaccggga taagggacaa gatatcctat     180

gttatgtccc agggcactgc aaggatatct tttacatcat caatgaatga tgatagctat     240

atatcgaatc atgttaaaaa acacggggat ggcgtaaagg atatagcact tgaggtcgat     300

gatctggacg aggcaaaaag cctgatagaa aagtatggaa caaaggtttc aaaaataaat     360

gaaataaagg atggaaatgg aaagataaga actgcagaga taaaaacgta cggtgaaacc     420

gttcatacat taatagaaac cggggattac aatggcgtat tcatgcccgg ttatgaggaa     480

tctgaaataa attcaaaaaa cactgggata aaaaagatcg atcatatagt tggaaatgtc     540

tatgagggcg agatggatag ctgggttaat ttttacatag aaaaacttgg ctttgagcat     600

ttaataacct ttgatgataa agatataaga actgattaca gcgcattaag atcaaaggtt 660

gtaaaataca atgacgatat cgtatttcca ataaatgagc ctgcaaaggg cttaagaaaa 720

tcacagatag aggaatatct tgactattac aggtctgagg gcgttcagca catagcactg 780

ttaactgatg atataataaa aactgtatcc atgatggagg aaaacggcat agaattttta 840

aaaacaccag gatcatacta tgaatcccta tcatcaagga taggctcaat agacgaggat 900

ttaaatgaaa tagagaaaca taacatactt gtggatcgtg atgagaacgg atacctatta 960

cagatcttca caaagcctgt tactgacagg ccaacgttct tctttgaggt catacagaga 1020

aagggtgcaa ggtcattcgg caacggtaac tttaaggcac tttttgaggc gatagaaagg 1080

gagcaggcaa agagaggaaa cctatga 1107

<210> 40
<211> 368
<212> PRT
<213> Picrophilus torridus

<400> 40
Met Tyr Gly Lys Asn Leu Ile Ser Glu Leu Arg Glu Lys Glu Ile Phe
1               5                   10                  15
Lys Arg Leu His His Val Glu Phe Tyr Val Ser Ser Ala Lys Thr Trp
            20                  25                  30
Ser Tyr Phe Met Asn Arg Gly Leu Gly Phe Lys Thr Val Ala Tyr Ala
        35                  40                  45
Gly Pro Glu Thr Gly Ile Arg Asp Lys Ile Ser Tyr Val Met Ser Gln
    50                  55                  60
Gly Thr Ala Arg Ile Ser Phe Thr Ser Ser Met Asn Asp Asp Ser Tyr
65                  70                  75                  80
Ile Ser Asn His Val Lys Lys His Gly Asp Gly Val Lys Asp Ile Ala
                85                  90                  95
Leu Glu Val Asp Asp Leu Asp Glu Ala Lys Ser Leu Ile Glu Lys Tyr
            100                 105                 110
Gly Thr Lys Val Ser Lys Ile Asn Glu Ile Lys Asp Gly Asn Gly Lys
        115                 120                 125
Ile Arg Thr Ala Glu Ile Lys Thr Tyr Gly Glu Thr Val His Thr Leu
    130                 135                 140
Ile Glu Thr Gly Asp Tyr Asn Gly Val Phe Met Pro Gly Tyr Glu Glu
145                 150                 155                 160
Ser Glu Ile Asn Ser Lys Asn Thr Gly Ile Lys Lys Ile Asp His Ile
                165                 170                 175
Val Gly Asn Val Tyr Glu Gly Glu Met Asp Ser Trp Val Asn Phe Tyr
            180                 185                 190
Ile Glu Lys Leu Gly Phe Glu His Leu Ile Thr Phe Asp Asp Lys Asp
        195                 200                 205
Ile Arg Thr Asp Tyr Ser Ala Leu Arg Ser Lys Val Val Lys Tyr Asn
    210                 215                 220
Asp Asp Ile Val Phe Pro Ile Asn Glu Pro Ala Lys Gly Leu Arg Lys
225                 230                 235                 240
Ser Gln Ile Glu Glu Tyr Leu Asp Tyr Tyr Arg Ser Glu Gly Val Gln
                245                 250                 255
His Ile Ala Leu Leu Thr Asp Asp Ile Ile Lys Thr Val Ser Met Met
            260                 265                 270
Glu Glu Asn Gly Ile Glu Phe Leu Lys Thr Pro Gly Ser Tyr Tyr Glu
        275                 280                 285
Ser Leu Ser Ser Arg Ile Gly Ser Ile Asp Glu Asp Leu Asn Glu Ile

290                 295                 300
Glu Lys His Asn Ile Leu Val Asp Arg Asp Glu Asn Gly Tyr Leu Leu
305                 310                 315                 320
Gln Ile Phe Thr Lys Pro Val Thr Asp Arg Pro Thr Phe Phe Phe Glu
                325                 330                 335
Val Ile Gln Arg Lys Gly Ala Arg Ser Phe Gly Asn Gly Asn Phe Lys
            340                 345                 350
Ala Leu Phe Glu Ala Ile Glu Arg Glu Gln Ala Lys Arg Gly Asn Leu
        355                 360                 365

<210> 41
<211> 1164
<212> DNA
<213> Kordia algicida

<400> 41
atggcagcag aaataaaaaa cttaaaagat ttacaaaata cagaatacgg actcaaaaaa      60

ttatttgacg aagcagaaga ctttcttcca cttttaggaa cagactacgt agaattatac     120

gtcgggaacg ccaaacaatc ggcacatttc tacaaaacgg cttttggttt tcaatcagaa     180

gcttacgcag gattggaaac aggattaacc gacagagttt catacgtatt aaaacaagat     240

aaaattcgct tggtcttaac aacaccatta ggaaaaggtg gcgaaatcaa tgagcatatc     300

gatttacacg gcgatggcgt aaaagtagta gcactttggg tagaagatgc tacaaaagcc     360

tttgaagaaa cgaccaaaag aggcgcaaaa ccgtacatgg aaccaacaaa agaagaagat     420

gaaaacggat atgtaattcg ctcaggaatc tatacgtacg gagaaacggt tcatgttttt     480

gtagaacgta aaaactataa cggagtcttt ttaccaggat atcaaagatg ggaatctcac     540

tacaatccgg agccagttgg cttaaaattc atcgatcaca tggtaggaaa tgtaggttgg     600

ggagaaatga aagaatggtg tgaattctac gcgaaagtaa tgggatttgc gcaaattatc     660

tcctttacag atgatgatat ttctaccgat tttactgcgt tgatgagtaa agtaatgagt     720

aatggaaatg gtagaatcaa atttccaatc aatgaacccg cagaaggaaa aaagaaatcg     780

caaattgaag aatatctaga cttttacaat ggttcaggag tacaacatat tgcggttgct     840

acagacaata ttattgatac ggtttcgcaa atgcgcgaac gtggagtaga attcttatac     900

gttccagata catattatga tgacttgtta gaacgtgttg gcgacatcga tgaagatgta     960

gaagaactca aaaaacacgg aatcttaatt gatcgtgatg aagaaggata cttattgcag    1020

ttatttacca aaaccattgt agacagacca acaatgttct ttgaagtcat tcagcgtaaa    1080

ggcgcacaat catttggagt aggaaacttt aaagctttat ttgaagcgat agaaagagaa    1140

caagctgctc gcggaacatt gtaa                                           1164

<210> 42
<211> 368
<212> PRT
<213> Kordia algicida

<400> 42
Met Tyr Gly Lys Asn Leu Ile Ser Glu Leu Arg Glu Lys Glu Ile Phe
1               5                   10                  15
Lys Arg Leu His His Val Glu Phe Tyr Val Ser Ser Ala Lys Thr Trp
            20                  25                  30
Ser Tyr Phe Met Asn Arg Gly Leu Gly Phe Lys Thr Val Ala Tyr Ala
        35                  40                  45
Gly Pro Glu Thr Gly Ile Arg Asp Lys Ile Ser Tyr Val Met Ser Gln
    50                  55                  60
Gly Thr Ala Arg Ile Ser Phe Thr Ser Ser Met Asn Asp Asp Ser Tyr
65                  70                  75                  80
Ile Ser Asn His Val Lys Lys His Gly Asp Gly Val Lys Asp Ile Ala
                85                  90                  95
Leu Glu Val Asp Asp Leu Asp Glu Ala Lys Ser Leu Ile Glu Lys Tyr
            100                 105                 110
Gly Thr Lys Val Ser Lys Ile Asn Glu Ile Lys Asp Gly Asn Gly Lys
        115                 120                 125
Ile Arg Thr Ala Glu Ile Lys Thr Tyr Gly Glu Thr Val His Thr Leu
    130                 135                 140
Ile Glu Thr Gly Asp Tyr Asn Gly Val Phe Met Pro Gly Tyr Glu Glu
145                 150                 155                 160
Ser Glu Ile Asn Ser Lys Asn Thr Gly Ile Lys Lys Ile Asp His Ile
                165                 170                 175
Val Gly Asn Val Tyr Glu Gly Glu Met Asp Ser Trp Val Asn Phe Tyr
            180                 185                 190
Ile Glu Lys Leu Gly Phe Glu His Leu Ile Thr Phe Asp Asp Lys Asp
        195                 200                 205
Ile Arg Thr Asp Tyr Ser Ala Leu Arg Ser Lys Val Val Lys Tyr Asn
    210                 215                 220
Asp Asp Ile Val Phe Pro Ile Asn Glu Pro Ala Lys Gly Leu Arg Lys
225                 230                 235                 240
Ser Gln Ile Glu Glu Tyr Leu Asp Tyr Tyr Arg Ser Glu Gly Val Gln
                245                 250                 255
His Ile Ala Leu Leu Thr Asp Asp Ile Ile Lys Thr Val Ser Met Met
            260                 265                 270
Glu Glu Asn Gly Ile Glu Phe Leu Lys Thr Pro Gly Ser Tyr Tyr Glu
        275                 280                 285
Ser Leu Ser Ser Arg Ile Gly Ser Ile Asp Glu Asp Leu Asn Glu Ile
    290                 295                 300
Glu Lys His Asn Ile Leu Val Asp Arg Asp Glu Asn Gly Tyr Leu Leu
305                 310                 315                 320
Gln Ile Phe Thr Lys Pro Val Thr Asp Arg Pro Thr Phe Phe Phe Glu
                325                 330                 335
Val Ile Gln Arg Lys Gly Ala Arg Ser Phe Gly Asn Gly Asn Phe Lys
            340                 345                 350
Ala Leu Phe Glu Ala Ile Glu Arg Glu Gln Ala Lys Arg Gly Asn Leu
        355                 360                 365

<210> 43
<211> 1206
<212> DNA
<213> Rhodococcus sp.

<400> 43
atgacgatcg agcagactct caccgacaag gaacgcctgg caggtctcga cctcggccag      60

ctcgagcagt tggtcgggct cgtcgagtac gacggcaccc gcgacccgtt cccggtcagc     120

ggctgggatg ccgtcgtctg ggtggtcggc aacgccaccc agaccgccca ctacttccag     180

tccgcgttcg ggatgaccct cgtcgcctac tccggaccca ccaccggcaa ccgcgaccac 240

cacagcttcg tcctcgaatc cggggccgtc cgcttcgtca tcaaaggcgc cgtgaacccg 300

gacagccccc tgatcgacca ccaccgcacc cacggcgacg gcgtcgtcga catcgccctc 360

gccgtccccg acgtcgacaa gtgcatcgcc cacgcccgcg cccagggcgc caccgtcctc 420

gacgaacccc acgacgtgac cgacgaccac ggcaccgtcc gcctcgccgc gatcgccacc 480

tacggcgaca cccgccacac cctcgtcgac cgcagccact acaccggccc ctacctgccc 540

ggctacaccg cccgcacctc cggccacacc aaacggacgg gggcacccaa gcgcctgttc 600

caggccctcg accacgtcgt cggcaacgtc gaactcggca agatggacca ctgggtcgac 660

ttctacaacc gggtcatggg ctttacgaac atggccgagt tcgtcggcga ggacatcgcc 720

accgactact ccgcgctgat gagcaaggtc gtctccaacg gcaaccaccg ggtcaagttc 780

cccctcaacg aacccgccct cgccaagaaa cgctcgcaga tcgacgaata cctcgacttc 840

taccgcggcc ccggcgccca gcacctggcc ctggccacca atgacatcct caccgccgtc 900

gaccagctga ccgccgaggg cgtcgagttc ctggccaccc ccgactccta ctacgaggac 960

cccgaactgc gggcccggat cggcaacgtc cgcgccccca tcgccgaact gcagaaacgc 1020

ggcatcctcg tcgaccgcga cgaagacggc tacctgctgc agatcttcac caaacccctc 1080

gtcgaccggc ccaccgtgtt cttcgaactc atcgaacgcc acggctccct cggcttcggc 1140

atcggcaact tcaaagccct cttcgaggcc atcgaacgcg aacaagccgc ccgcggaaac 1200

ttctga 1206

<210> 44
<211> 401
<212> PRT
<213> Rhodococcus sp.

<400> 44
Met Thr Ile Glu Gln Thr Leu Thr Asp Lys Glu Arg Leu Ala Gly Leu
1               5                   10                  15
Asp Leu Gly Gln Leu Glu Gln Leu Val Gly Leu Val Glu Tyr Asp Gly
            20                  25                  30
Thr Arg Asp Pro Phe Pro Val Ser Gly Trp Asp Ala Val Val Trp Val
        35                  40                  45
Val Gly Asn Ala Thr Gln Thr Ala His Tyr Phe Gln Ser Ala Phe Gly
    50                  55                  60
Met Thr Leu Val Ala Tyr Ser Gly Pro Thr Thr Gly Asn Arg Asp His
65                  70                  75                  80
His Ser Phe Val Leu Glu Ser Gly Ala Val Arg Phe Val Ile Lys Gly
                85                  90                  95
Ala Val Asn Pro Asp Ser Pro Leu Ile Asp His His Arg Thr His Gly
            100                 105                 110
Asp Gly Val Val Asp Ile Ala Leu Ala Val Pro Asp Val Asp Lys Cys
        115                 120                 125
Ile Ala His Ala Arg Ala Gln Gly Ala Thr Val Leu Asp Glu Pro His
    130                 135                 140
Asp Val Thr Asp Asp His Gly Thr Val Arg Leu Ala Ala Ile Ala Thr
145                 150                 155                 160

Tyr Gly Asp Thr Arg His Thr Leu Val Asp Arg Ser His Tyr Thr Gly
                165                 170                 175
Pro Tyr Leu Pro Gly Tyr Thr Ala Arg Thr Ser Gly His Thr Lys Arg
            180                 185                 190
Asp Gly Ala Pro Lys Arg Leu Phe Gln Ala Leu Asp His Val Val Gly
        195                 200                 205
Asn Val Glu Leu Gly Lys Met Asp His Trp Val Asp Phe Tyr Asn Arg
    210                 215                 220
Val Met Gly Phe Thr Asn Met Ala Glu Phe Val Gly Glu Asp Ile Ala
225                 230                 235                 240
Thr Asp Tyr Ser Ala Leu Met Ser Lys Val Val Ser Asn Gly Asn His
                245                 250                 255
Arg Val Lys Phe Pro Leu Asn Glu Pro Ala Leu Ala Lys Lys Arg Ser
            260                 265                 270
Gln Ile Asp Glu Tyr Leu Asp Phe Tyr Arg Gly Pro Gly Ala Gln His
        275                 280                 285
Leu Ala Leu Ala Thr Asn Asp Ile Leu Thr Ala Val Asp Gln Leu Thr
    290                 295                 300
Ala Glu Gly Val Glu Phe Leu Ala Thr Pro Asp Ser Tyr Tyr Glu Asp
305                 310                 315                 320
Pro Glu Leu Arg Ala Arg Ile Gly Asn Val Arg Ala Pro Ile Ala Glu
                325                 330                 335
Leu Gln Lys Arg Gly Ile Leu Val Asp Arg Asp Glu Asp Gly Tyr Leu
            340                 345                 350
Leu Gln Ile Phe Thr Lys Pro Leu Val Asp Arg Pro Thr Val Phe Phe
        355                 360                 365
Glu Leu Ile Glu Arg His Gly Ser Leu Gly Phe Gly Ile Gly Asn Phe
    370                 375                 380
Lys Ala Leu Phe Glu Ala Ile Glu Arg Glu Gln Ala Ala Arg Gly Asn
385                 390                 395                 400
Phe

<210> 45
<211> 1209
<212> DNA
<213> Rhodococcus sp

<400> 45
atgactaccg ccgacattcg cctgacgccc cgcgaggtgg ccgcacatct ggagaccgac      60

gagctccggc agttggtcgg gctcgtcgaa cacgacgacg cgtcggatcc gtttcccgtg     120

gtcgcgatgg atgccgtggt gttcgtgtgc ggcaacgcga cgcagagcac gcagtacttc     180

gtctccacgt ggggcatgac cctcgtcgcc tacgccgggc cggagaccgg tcagcgctcg     240

cacaagtcct tcgtcctcga gtcggggtcg gcacggttcg tgctgcacgg cgccgtcgat     300

ccgaagagcc cgctcgcgga ccatcaccgg gcgcacggcg acggcgtggt ggacctggcg     360

atggaagttc tcgacgtcga ccgctgcatc gcgcatgcac gctcgcaggg ggccaccatt     420

ctcgaggagc cgcgcgacgt cacggatcag ttcggcaccg tgcggctcgc ggcgatcgcc     480

acgtacggca gcacccggca caccatcgtc gaccgaagcc gatacgacgg cccctacctc     540

cccggattcg tcgcgcgctc cagcggtttc gcggcgcgac cgggtaaacc cccgcgattg     600

ttccaggcgc tcgaccacgc cgtcggcaac gtcgagatgg gccggatgga tcactgggtc     660

cggttctaca accgcgtcat gggcttcacg aacatggccg aattcgtcgg cgacgacatc     720

gccacggagt actcggcgct gatgtcgaag gtcgtggcga acggcaatca ccgggtgaag 780

ttcccgctca acgaacccgc ggtgggaaag aagaagtcgc agatcgacga atatctcgag 840

ttctacggtg agccgggctg ccagcatctg gccctcgcga ccggagacat cctcgcgacg 900

gtggacgcgt tgcgggccga gggtgtcgaa ttcctgaaca cacccgacgc gtactacgag 960

gacccacagc tgcgcgcccg gatcggcagg gtgcgggtgc cggtggagga actgcagaag 1020

cgcggaatcc tcgtcgaccg cgacgaggac ggatacctcc tgcagatctt caccaaaccg 1080

ctcggcgacc ggccgaccgt gttcttcgag gtgatcgaac ggcacggttc gctcgggttc 1140

ggggcgggta acttccaggc cctgttcgaa tccatcgagc gtgagcaggc ggcgcgcggc 1200

aatctgtga 1209

<210> 46
<211> 402
<212> PRT
<213> Rhodococcus sp

<400> 46
Met Thr Thr Ala Asp Ile Arg Leu Thr Pro Arg Glu Val Ala Ala His
1               5                   10                  15
Leu Glu Thr Asp Glu Leu Arg Gln Leu Val Gly Leu Val Glu His Asp
            20                  25                  30
Asp Ala Ser Asp Pro Phe Pro Val Val Ala Met Asp Ala Val Val Phe
        35                  40                  45
Val Cys Gly Asn Ala Thr Gln Ser Thr Gln Tyr Phe Val Ser Thr Trp
    50                  55                  60
Gly Met Thr Leu Val Ala Tyr Ala Gly Pro Glu Thr Gly Gln Arg Ser
65                  70                  75                  80
His Lys Ser Phe Val Leu Glu Ser Gly Ser Ala Arg Phe Val Leu His
                85                  90                  95
Gly Ala Val Asp Pro Lys Ser Pro Leu Ala Asp His His Arg Ala His
            100                 105                 110
Gly Asp Gly Val Val Asp Leu Ala Met Glu Val Leu Asp Val Asp Arg
        115                 120                 125
Cys Ile Ala His Ala Arg Ser Gln Gly Ala Thr Ile Leu Glu Glu Pro
    130                 135                 140
Arg Asp Val Thr Asp Gln Phe Gly Thr Val Arg Leu Ala Ala Ile Ala
145                 150                 155                 160
Thr Tyr Gly Ser Thr Arg His Thr Ile Val Asp Arg Ser Arg Tyr Asp
                165                 170                 175
Gly Pro Tyr Leu Pro Gly Phe Val Ala Arg Ser Ser Gly Phe Ala Ala
            180                 185                 190
Arg Pro Gly Lys Pro Pro Arg Leu Phe Gln Ala Leu Asp His Ala Val
        195                 200                 205
Gly Asn Val Glu Met Gly Arg Met Asp His Trp Val Arg Phe Tyr Asn
    210                 215                 220
Arg Val Met Gly Phe Thr Asn Met Ala Glu Phe Val Gly Asp Asp Ile
225                 230                 235                 240
Ala Thr Glu Tyr Ser Ala Leu Met Ser Lys Val Val Ala Asn Gly Asn
                245                 250                 255
His Arg Val Lys Phe Pro Leu Asn Glu Pro Ala Val Gly Lys Lys Lys
            260                 265                 270
Ser Gln Ile Asp Glu Tyr Leu Glu Phe Tyr Gly Glu Pro Gly Cys Gln
        275                 280                 285
His Leu Ala Leu Ala Thr Gly Asp Ile Leu Ala Thr Val Asp Ala Leu
    290                 295                 300

Arg Ala Glu Gly Val Glu Phe Leu Asn Thr Pro Asp Ala Tyr Tyr Glu
305                 310                 315                 320
Asp Pro Gln Leu Arg Ala Arg Ile Gly Arg Val Arg Val Pro Val Glu
                325                 330                 335
Glu Leu Gln Lys Arg Gly Ile Leu Val Asp Arg Asp Glu Asp Gly Tyr
            340                 345                 350
Leu Leu Gln Ile Phe Thr Lys Pro Leu Gly Asp Arg Pro Thr Val Phe
        355                 360                 365
Phe Glu Val Ile Glu Arg His Gly Ser Leu Gly Phe Gly Ala Gly Asn
    370                 375                 380
Phe Gln Ala Leu Phe Glu Ser Ile Glu Arg Glu Gln Ala Ala Arg Gly
385                 390                 395                 400
Asn Leu

<210> 47
<211> 1395
<212> DNA
<213> Scenedesmus obliquus

<400> 47
atgggcgcag gtggagctca gggcccgaag gttgagctgg ttggctacgc caatttcgtt      60

cgcaacaacc ctcgcagcga taagttccct gtgcacaagt ttcaccacat cgagttttgg     120

tgcgcggacg ccaccaacac cttcaagcgc ttccagcatg gcctgggcat gaccctagtg     180

gccaagtccg accacagcac aggcaacagc aagtactgca gctacgtctt gcaaagcaac     240

gacctcgtgt tcaccttcac agcaccctac tcgcgcaagt gcgcagcagc agcgccttct     300

agcagcgagc cgctgccaga ttatgaccag cagcaggcct ttgaattcat ctgcacccac     360

ggcctggcgg caagggctgt aggcctgcag gtgggcgatg cagcgcaggc gtacgaggtt     420

tctgtggcaa acggcgcgaa gggtgtgcgt ccacccacca agctggagga tggcggcggc     480

tgtgctgtgg tcagcgaggt gctgctgtat ggtgacgtgg tgctgcgcta catcagcggc     540

aagtgggagg gcccctacct gccgggctac acagccacgc ctgatgagcc gcagatctgc     600

tacggcctgc accgcctgga ccacgccgtg ggcaatgtgc ccaagctcat cgagcacttg     660

gagcacgtca ttggcttcac aggcttccac gagtttgccg agtttgtggc ggaggacgtg     720

ggcactgtgg acagcgggct gaacagcatg gtgctggcca gcaacaacga gatggtgctg     780

ctgcccatga acgagcccac ctttggcacc aagcgcaagt cgcagatcca gacgtacctg     840

gagcagaacg agggccccgg gctgcagcac ctggcgctga agacgcacga catcctgtcc     900

accatgcgcg agatgcacgc acgctcgcgc tgcggcggct tcgagttcca ggcggcaccc     960

gggcacgact actacaagcg cgtggcggag aaagtgggtg acgtgctgtc cccggaggag    1020

tgggcagccg ttgagcagct gggcatcctg gtggaccagg acgaccaggg cgtgctgctg    1080

cagatcttca ccaagccgct gggggacagg cccaccatct tcattgagat cattgagcgg    1140

cgcggctgcc tcaaggagag cgcggcacag gcaggcagtg cggcagcagc agcagcagag    1200

cctacagcag ctggcggtga tgcagatgca gatggagcag cagcagcagt ggctgacaag    1260

ttcaaagaca tcgtgcaggt gcgtgaggat ggcgtggttg tggagcaagc tgctggctgt 1320

ggaggcttcg gcaaaggcaa cttcagcgag ctgttcaaaa gcattgagga gtacgagcgc 1380

acgctgcagg tgtaa 1395

<210> 48
<211> 464
<212> PRT
<213> Scenedesmus obliquus

<400> 48
Met Gly Ala Gly Gly Ala Gln Gly Pro Lys Val Glu Leu Val Gly Tyr
1               5                   10                  15
Ala Asn Phe Val Arg Asn Asn Pro Arg Ser Asp Lys Phe Pro Val His
            20                  25                  30
Lys Phe His His Ile Glu Phe Trp Cys Ala Asp Ala Thr Asn Thr Phe
        35                  40                  45
Lys Arg Phe Gln His Gly Leu Gly Met Thr Leu Val Ala Lys Ser Asp
    50                  55                  60
His Ser Thr Gly Asn Ser Lys Tyr Cys Ser Tyr Val Leu Gln Ser Asn
65                  70                  75                  80
Asp Leu Val Phe Thr Phe Thr Ala Pro Tyr Ser Arg Lys Cys Ala Ala
                85                  90                  95
Ala Ala Pro Ser Ser Ser Glu Pro Leu Pro Asp Tyr Asp Gln Gln Gln
            100                 105                 110
Ala Phe Glu Phe Ile Cys Thr His Gly Leu Ala Ala Arg Ala Val Gly
        115                 120                 125
Leu Gln Val Gly Asp Ala Ala Gln Ala Tyr Glu Val Ser Val Ala Asn
    130                 135                 140
Gly Ala Lys Gly Val Arg Pro Pro Thr Lys Leu Glu Asp Gly Gly Gly
145                 150                 155                 160
Cys Ala Val Val Ser Glu Val Leu Leu Tyr Gly Asp Val Val Leu Arg
                165                 170                 175
Tyr Ile Ser Gly Lys Trp Glu Gly Pro Tyr Leu Pro Gly Tyr Thr Ala
            180                 185                 190
Thr Pro Asp Glu Pro Gln Ile Cys Tyr Gly Leu His Arg Leu Asp His
        195                 200                 205
Ala Val Gly Asn Val Pro Lys Leu Ile Glu His Leu Glu His Val Ile
    210                 215                 220
Gly Phe Thr Gly Phe His Glu Phe Ala Glu Phe Val Ala Glu Asp Val
225                 230                 235                 240
Gly Thr Val Asp Ser Gly Leu Asn Ser Met Val Leu Ala Ser Asn Asn
                245                 250                 255
Glu Met Val Leu Leu Pro Met Asn Glu Pro Thr Phe Gly Thr Lys Arg
            260                 265                 270
Lys Ser Gln Ile Gln Thr Tyr Leu Glu Gln Asn Glu Gly Pro Gly Leu
        275                 280                 285
Gln His Leu Ala Leu Lys Thr His Asp Ile Leu Ser Thr Met Arg Glu
    290                 295                 300
Met His Ala Arg Ser Arg Cys Gly Gly Phe Glu Phe Gln Ala Ala Pro
305                 310                 315                 320
Gly His Asp Tyr Tyr Lys Arg Val Ala Glu Lys Val Gly Asp Val Leu
                325                 330                 335
Ser Pro Glu Glu Trp Ala Ala Val Glu Gln Leu Gly Ile Leu Val Asp
            340                 345                 350
Gln Asp Asp Gln Gly Val Leu Leu Gln Ile Phe Thr Lys Pro Leu Gly
        355                 360                 365
Asp Arg Pro Thr Ile Phe Ile Glu Ile Ile Glu Arg Arg Gly Cys Leu
    370                 375                 380
Lys Glu Ser Ala Ala Gln Ala Gly Ser Ala Ala Ala Ala Ala Ala Glu
385                 390                 395                 400

Pro Thr Ala Ala Gly Gly Asp Ala Asp Ala Asp Gly Ala Ala Ala Ala
                405                 410                 415
Val Ala Asp Lys Phe Lys Asp Ile Val Gln Val Arg Glu Asp Gly Val
            420                 425                 430
Val Val Glu Gln Ala Ala Gly Cys Gly Gly Phe Gly Lys Gly Asn Phe
        435                 440                 445
Ser Glu Leu Phe Lys Ser Ile Glu Glu Tyr Glu Arg Thr Leu Gln Val
    450                 455                 460

<210> 49
<211> 1395
<212> DNA
<213> Scenedesmus obliquus

<400> 49
atgggcgcag gtggagctca gggcccgaag gttgagctgg ttggctacgc caatttcgtt      60

cgcaacaacc ctcgcagcga taagttccct gtgcacaagt ttcaccacat cgagttttgg     120

tgcgcggacg ccaccaacac cttcaagcgc ttccagcatg gcctgggcat gaccctagtg     180

gccaagtccg accacagcac aggcaacagc aagtactgca gctacgtctt gcaaagcaac     240

gacctcgtgt tcaccttcac agcaccctac tcgcgcaagt gcgcagcagc agcgccttct     300

agcagcgagc cgctgccaga ttatgaccag cagcaggcct ttgaattcat ctgcacccac     360

ggcctggcgg caagggctgt aggcctgcag gtgggcgatg cagcgcaggc gtacgaggtt     420

tctgtggcaa acggcgcgaa gggtgtgcgt ccacccacca agctggagga tggcggcggc     480

tgtgctgtgg tcagcgaggt gctgctgtat ggtgacgtgg tgctgcgcta catcagcggc     540

aagtgggagg gcccctacct gccgggctac acagccacgc ctgatgagcc gcagatctgc     600

tacggcctgc accgcctgga ccacgccgtg ggcaatgtgc ccaagctcat cgagcacttg     660

gagcacgtca ttggcttcac aggcttccac gagtttgccg agtttgtggc ggaggacgtg     720

ggcactgtgg acagcgggct gaacagcatg gtgctggcca gcaacaacga gatggtgctg     780

ctgcccatga acgagcccac ctttggcacc aagcgcaagt cgcagatcca gacgtacctg     840

gagcagaacg agggccccgg gctgcagcac ctggcgctga agacgcacga catcctgtcc     900

accatgcgcg agatgcacgc acgctcgcgc tgcggcggct tcgagttcca ggcggcaccc     960

gggcacgact actacaagcg cgtggcggag aaagtgggtg acgtgctgtc cccggaggag    1020

tgggcagccg ttgagcagct gggcatcctg gtggaccagg acgaccaggg cgtgctgctg    1080

cagatcttca ccaagccgct gggggacagg cccaccatct tcattgagat cattgagcgg    1140

cgcggctgcc tcaaggagag cgcggcacag gcaggcagtg cggcagcagc agcagcagag    1200

cctacagcag ctggcggtga tgcagatgca gatggagcag cagcagcagt ggctgacaag    1260

ttcaaagaca tcgtgcaggt gcgtgaggat ggcgtggttg tggagcaagc tgctggctgt    1320

ggaggcttcg gcaaaggcaa cttcagcgag ctgttcaaaa gcattgagga gtacgagcgc    1380

acgctgcagg tgtaa                                                    1395

<210> 50
<211> 430
<212> PRT
<213> Scenedesmus obliquus

<400> 50
Met Gly Ala Gly Gly Ala Gln Gly Pro Lys Val Glu Leu Val Gly Tyr
1               5                   10                  15
Ala Asn Phe Val Arg Asn Asn Pro Arg Ser Asp Lys Phe Pro Val His
            20                  25                  30
Lys Phe His His Ile Glu Phe Trp Cys Ala Asp Ala Thr Asn Thr Phe
        35                  40                  45
Lys Arg Phe Gln His Gly Leu Gly Met Thr Leu Val Ala Lys Ser Asp
    50                  55                  60
His Ser Thr Gly Asn Ser Lys Tyr Cys Ser Tyr Val Leu Gln Ser Asn
65                  70                  75                  80
Asp Leu Val Phe Thr Phe Thr Ala Pro Tyr Ser Arg Lys Cys Ala Ala
                85                  90                  95
Ala Ala Pro Ser Ser Ser Glu Pro Leu Pro Asp Tyr Asp Gln Gln Gln
            100                 105                 110
Ala Phe Glu Phe Ile Cys Thr His Gly Leu Ala Ala Arg Ala Val Gly
        115                 120                 125
Leu Gln Val Gly Asp Ala Ala Gln Ala Tyr Glu Val Ser Val Ala Asn
    130                 135                 140
Gly Ala Lys Gly Val Arg Pro Pro Thr Lys Leu Glu Asp Gly Gly Gly
145                 150                 155                 160
Cys Ala Val Val Ser Glu Val Leu Leu Tyr Gly Asp Val Val Leu Arg
                165                 170                 175
Tyr Ile Ser Gly Lys Trp Glu Gly Pro Tyr Leu Pro Gly Tyr Thr Ala
            180                 185                 190
Thr Pro Asp Glu Pro Gln Ile Cys Tyr Gly Leu His Arg Leu Asp His
        195                 200                 205
Ala Val Gly Asn Val Pro Lys Leu Ile Glu His Leu Glu His Val Ile
    210                 215                 220
Gly Phe Thr Gly Phe His Glu Phe Ala Glu Phe Val Ala Glu Asp Val
225                 230                 235                 240
Gly Thr Val Asp Ser Gly Leu Asn Ser Met Val Leu Ala Ser Asn Asn
                245                 250                 255
Glu Met Val Leu Leu Pro Met Asn Glu Pro Thr Phe Gly Thr Lys Arg
            260                 265                 270
Lys Ser Gln Ile Gln Thr Tyr Leu Glu Gln Asn Glu Gly Pro Gly Leu
        275                 280                 285
Gln His Leu Ala Leu Lys Thr His Asp Ile Leu Ser Thr Met Arg Glu
    290                 295                 300
Met His Ala Arg Ser Arg Cys Gly Gly Phe Glu Phe Gln Ala Ala Pro
305                 310                 315                 320
Gly His Asp Tyr Tyr Lys Arg Val Ala Glu Lys Val Gly Asp Val Leu
                325                 330                 335
Ser Pro Glu Glu Trp Ala Ala Val Glu Gln Leu Gly Ile Leu Val Asp
            340                 345                 350
Gln Asp Asp Gln Gly Val Leu Leu Gln Ile Phe Thr Lys Pro Leu Gly
        355                 360                 365
Asp Arg Pro Thr Ile Phe Ile Glu Ile Ile Glu Arg Arg Gly Cys Leu
    370                 375                 380
Lys Glu Ser Ala Ala Gln Ala Gly Ser Ala Ala Ala Ala Val Glu Gln
385                 390                 395                 400
Ala Ala Gly Cys Gly Gly Phe Gly Lys Gly Asn Phe Ser Glu Leu Phe
                405                 410                 415
Lys Ser Ile Glu Glu Tyr Glu Arg Thr Leu Gln Val Leu Glu
            420                 425                 430

<210> 51
<211> 1305
<212> DNA
<213> Hordeum vulgare

<400> 51
atggcacctc caactccaac aacacctgcc gctactggtg cagccgcagc tgtaactcct 60

gaacatgcga ggccacatcg gatggttcga ttcaatccga gatctgatag attccatact 120

ctgagcttcc atcatgtgga attctggtgt gctgatgcag cttctgcagc tggacgtttc 180

gcttttgccc ttggagctcc tttagcagcg agatcagact tgagcacagg aaacagtgca 240

cacgcatctc aacttctgcg ttcaggaagc cttgcgttcc tgtttactgc accgtatgct 300

aacggatgcg acgcagcaac tgcctcactt ccttctttca gtgcagatgc agctagacga 360

ttctcagccg atcatggaat tgcagtcaga tctgtggctt tgcgagttgc tgatgctgcc 420

gaagctttca gggcatcagt tgatggaggt gctaggcctg cttttgctcc tgtggacttg 480

ggtagaggat ttggctttgc cgaggtcgaa ctctatggtg atgtggttct ccggtttgtc 540

tctcacccag atggaacaga tgttcctttc ttgccagggt ttgagggagt gacaaaccca 600

gatgcggtag attacggtct cacgagattc gaccatgtag tgggcaatgt accggaattg 660

gctcctgcgg ctgcttacat agctggcttt acgggatttc acgaattcgc ggaattcacc 720

gctgaggatg tcggaaccac agaatcaggg ctgaattccg tcgtccttgc caacaattcc 780

gaaggggtat tgctgcctct taacgagcct gtgcatggca cgaaaagacg tagccagata 840

cagaccttcc tagaacatca cggtggacca ggtgttcaac acattgctgt tgccagcagt 900

gatgtactca ggacgcttcg taagatgaga gctaggagtg cgatgggagg gtttgacttt 960

ctaccacctc cgctgccaaa atactatgag ggtgtgagga gactggctgg tgatgttttg 1020

tctgaagcgc agatcaagga gtgtcaggaa ttaggggtgc tcgttgacag agatgatcaa 1080

ggggtgcttc tccagatctt tactaagccg gttggtgata ggcctaccct ctttctagag 1140

atgattcaac gtatcgggtg tatggaaaag gacgagagag gtgaggagta tcaaaagggt 1200

ggatgcggcg gttttgggaa aggtaatttc tccgagctgt tcaagtcgat cgaagattac 1260

gagaaatccc ttgaggcgaa acaatctgca gctgttcaag gatcg 1305

<210> 52
<211> 1338
<212> DNA
<213> Arabidopsis

<400> 52
atgggccacc aaaacgccgc cgtttcagag aatcaaaacc atgatgacgg cgctgcgtcg 60

tcgccgggat tcaagctcgt cggattttcc aagttcgtaa gaaagaatcc aaagtctgat 120

aaattcaagg ttaagcgctt ccatcacatc gagttctggt gcggcgacgc aaccaacgtc 180

gctcgtcgct tctcctgggg tctggggatg agattctccg ccaaatccga tctttccacc 240

ggaaacatgg ttcacgcctc ttacctactc acctccggtg acctccgatt ccttttcact 300

gctccttact ctccgtctct ctccgccgga gagattaaac cgacaaccac agcttctatc 360

ccaagtttcg atcacggctc ttgtcgttcc ttcttctctt cacatggtct cggtgttaga 420

gccgttgcga ttgaagtaga agacgcagag tcagctttct ccatcagtgt agctaatggc 480

gctattcctt cgtcgcctcc tatcgtcctc aatgaagcag ttacgatcgc tgaggttaaa 540

ctatacggcg atgttgttct ccgatatgtt agttacaaag cagaagatac cgaaaaatcc 600

gaattcttgc cagggttcga gcgtgtagag gatgcgtcgt cgttcccatt ggattatggt 660

atccggcggc ttgaccacgc cgtgggaaac gttcctgagc ttggtccggc tttaacttat 720

gtagcggggt tcactggttt tcaccaattc gcagagttca cagcagacga cgttggaacc 780

gccgagagcg gtttaaattc agcggtcctg gctagcaatg atgaaatggt tcttctaccg 840

attaacgagc cagtgcacgg aacaaagagg aagagtcaga ttcagacgta tttggaacat 900

aacgaaggcg cagggctaca acatctggct ctgatgagtg aagacatatt caggaccctg 960

agagagatga ggaagaggag cagtattgga ggattcgact tcatgccttc tcctccgcct 1020

acttactacc agaatctcaa gaaacgggtc ggcgacgtgc tcagcgatga tcagatcaag 1080

gagtgtgagg aattagggat tcttgtagac agagatgatc aagggacgtt gcttcaaatc 1140

ttcacaaaac cactaggtga caggccgacg atatttatag agataatcca gagagtagga 1200

tgcatgatga aagatgagga agggaaggct taccagagtg gaggatgtgg tggttttggc 1260

aaaggcaatt tctctgagct cttcaagtcc attgaagaat acgaaaagac tcttgaagcc 1320

aaacagttag tgggatga 1338

<210> 53
<211> 445
<212> PRT
<213> Arabidopsis

<400> 53
Met Gly His Gln Asn Ala Ala Val Ser Glu Asn Gln Asn His Asp Asp
1               5                   10                  15
Gly Ala Ala Ser Ser Pro Gly Phe Lys Leu Val Gly Phe Ser Lys Phe
            20                  25                  30
Val Arg Lys Asn Pro Lys Ser Asp Lys Phe Lys Val Lys Arg Phe His
        35                  40                  45
His Ile Glu Phe Trp Cys Gly Asp Ala Thr Asn Val Ala Arg Arg Phe
    50                  55                  60
Ser Trp Gly Leu Gly Met Arg Phe Ser Ala Lys Ser Asp Leu Ser Thr
65                  70                  75                  80
Gly Asn Met Val His Ala Ser Tyr Leu Leu Thr Ser Gly Asp Leu Arg
                85                  90                  95
Phe Leu Phe Thr Ala Pro Tyr Ser Pro Ser Leu Ser Ala Gly Glu Ile
            100                 105                 110
Lys Pro Thr Thr Thr Ala Ser Ile Pro Ser Phe Asp His Gly Ser Cys

115                 120                 125
Arg Ser Phe Phe Ser Ser His Gly Leu Gly Val Arg Ala Val Ala Ile
    130                 135                 140
Glu Val Glu Asp Ala Glu Ser Ala Phe Ser Ile Ser Val Ala Asn Gly
145                 150                 155                 160
Ala Ile Pro Ser Ser Pro Pro Ile Val Leu Asn Glu Ala Val Thr Ile
                165                 170                 175
Ala Glu Val Lys Leu Tyr Gly Asp Val Val Leu Arg Tyr Val Ser Tyr
            180                 185                 190
Lys Ala Glu Asp Thr Glu Lys Ser Glu Phe Leu Pro Gly Phe Glu Arg
        195                 200                 205
Val Glu Asp Ala Ser Ser Phe Pro Leu Asp Tyr Gly Ile Arg Arg Leu
    210                 215                 220
Asp His Ala Val Gly Asn Val Pro Glu Leu Gly Pro Ala Leu Thr Tyr
225                 230                 235                 240
Val Ala Gly Phe Thr Gly Phe His Gln Phe Ala Glu Phe Thr Ala Asp
                245                 250                 255
Asp Val Gly Thr Ala Glu Ser Gly Leu Asn Ser Ala Val Leu Ala Ser
            260                 265                 270
Asn Asp Glu Met Val Leu Leu Pro Ile Asn Glu Pro Val His Gly Thr
        275                 280                 285
Lys Arg Lys Ser Gln Ile Gln Thr Tyr Leu Glu His Asn Glu Gly Ala
    290                 295                 300
Gly Leu Gln His Leu Ala Leu Met Ser Glu Asp Ile Phe Arg Thr Leu
305                 310                 315                 320
Arg Glu Met Arg Lys Arg Ser Ser Ile Gly Gly Phe Asp Phe Met Pro
                325                 330                 335
Ser Pro Pro Pro Thr Tyr Tyr Gln Asn Leu Lys Lys Arg Val Gly Asp
            340                 345                 350
Val Leu Ser Asp Asp Gln Ile Lys Glu Cys Glu Glu Leu Gly Ile Leu
        355                 360                 365
Val Asp Arg Asp Asp Gln Gly Thr Leu Leu Gln Ile Phe Thr Lys Pro
    370                 375                 380
Leu Gly Asp Arg Pro Thr Ile Phe Ile Glu Ile Ile Gln Arg Val Gly
385                 390                 395                 400
Cys Met Met Lys Asp Glu Glu Gly Lys Ala Tyr Gln Ser Gly Gly Cys
                405                 410                 415
Gly Gly Phe Gly Lys Gly Asn Phe Ser Glu Leu Phe Lys Ser Ile Glu
            420                 425                 430
Glu Tyr Glu Lys Thr Leu Glu Ala Lys Gln Leu Val Gly
        435                 440                 445

<210> 54
<211> 1299
<212> DNA
<213> Chlamydomonas

<400> 54
atgggcgctg gtggcgcttc taccacggta gcgaatggcg ggatcaagtt ggtagggcac      60

aagaattttg tgcgctataa tccacaatcc gaccggtttg ctattaagag gttccatagc     120

ttcgagttct ggtgcgcgga tgcgaccaac acatacaagc ggttctctta tggcctgggc     180

atgccgctgg tcgccaagtc cgaccagtcc accaacaacc agctctttgc ctcctacgtg     240

ctgcgctcca acgacctggt cttcaccttc accgcgccct acagccgcaa gtgcgcctcg     300

gtcagcgagg gcgttccgct gcgtcactac aacatcgacc atgcgtatga gttcatcaac     360

tcgcacgggc tggcggtgcg ggcagtaggc ctgctggtgg atgacgccaa gacggcgtac     420

gaggtgtctg tggcgcacgg ggccaagggc gtgctgccgc cggtggagtt gcgggatgag     480

gcgagcggca ccagccaggt catctcggag gtcattgttt acggggacgt cgttttccgc 540

tacgtgtcgg gctccttcga gggccctttc atggccggct acacgccagt cacagactcg 600

ccggtcgcgt cgattgggtt acagcgcgtg gaccacgcgg tgggcaacac acacgacctg 660

atcaaggccg tggagtacat caccgggttc tgtggcttcc acgagttctc agagtttgtt 720

gcggaggacg tgggcactgt ggacagcggc ctgaacagca tggtgcttgc caacaacgag 780

gagaccatat tgatgcctgt gaacgagccc accttcggca cgccgcgcaa gagccaaatc 840

cagacctacc tggagcagaa cgagggcccg gggctgcagc acctggcgct gctcagcaac 900

gacatcttca ccaccctgcg ggagatgcgc gcgcgcagcg agctgggtgg cttcgagttc 960

atgccgcggg caaatgcgaa gtactacaaa gacatgtacg cccgcatcgg cgactcgctc 1020

acgccgcagc agtacaggga ggtggaggag ctgggcatcc tggtggacaa ggacgaccag 1080

ggcgtgctgc tgcagatctt caccaagccg ctgggcgacc ggcccacggt gtttattgag 1140

atcatccagc gtgtgggctg catgcgggag gtgaaggagc ctgctacggg cgctgtggtg 1200

gggacggagc aggcggctgg ctgcggcggc ttcgggaaag gcaacttcgg cgccctcttc 1260

aagtccattg aggactatga gcgcacccta aatgtgtag 1299

<210> 55
<211> 432
<212> PRT
<213> Chamydomonas

<400> 55
Met Gly Ala Gly Gly Ala Ser Thr Thr Val Ala Asn Gly Gly Ile Lys
1               5                   10                  15
Leu Val Gly His Lys Asn Phe Val Arg Tyr Asn Pro Gln Ser Asp Arg
            20                  25                  30
Phe Ala Ile Lys Arg Phe His Ser Phe Glu Phe Trp Cys Ala Asp Ala
        35                  40                  45
Thr Asn Thr Tyr Lys Arg Phe Ser Tyr Gly Leu Gly Met Pro Leu Val
    50                  55                  60
Ala Lys Ser Asp Gln Ser Thr Asn Asn Gln Leu Phe Ala Ser Tyr Val
65                  70                  75                  80
Leu Arg Ser Asn Asp Leu Val Phe Thr Phe Thr Ala Pro Tyr Ser Arg
                85                  90                  95
Lys Cys Ala Ser Val Ser Glu Gly Val Pro Leu Arg His Tyr Asn Ile
            100                 105                 110
Asp His Ala Tyr Glu Phe Ile Asn Ser His Gly Leu Ala Val Arg Ala
        115                 120                 125
Val Gly Leu Leu Val Asp Asp Ala Lys Thr Ala Tyr Glu Val Ser Val
    130                 135                 140
Ala His Gly Ala Lys Gly Val Leu Pro Pro Val Glu Leu Arg Asp Glu
145                 150                 155                 160
Ala Ser Gly Thr Ser Gln Val Ile Ser Glu Val Ile Val Tyr Gly Asp
                165                 170                 175
Val Val Phe Arg Tyr Val Ser Gly Ser Phe Glu Gly Pro Phe Met Ala
            180                 185                 190
Gly Tyr Thr Pro Val Thr Asp Ser Pro Val Ala Ser Ile Gly Leu Gln
        195                 200                 205
Arg Val Asp His Ala Val Gly Asn Thr His Asp Leu Ile Lys Ala Val

210                 215                 220
Glu Tyr Ile Thr Gly Phe Cys Gly Phe His Glu Phe Ser Glu Phe Val
225                 230                 235                 240
Ala Glu Asp Val Gly Thr Val Asp Ser Gly Leu Asn Ser Met Val Leu
                245                 250                 255
Ala Asn Asn Glu Glu Thr Ile Leu Met Pro Val Asn Glu Pro Thr Phe
            260                 265                 270
Gly Thr Pro Arg Lys Ser Gln Ile Gln Thr Tyr Leu Glu Gln Asn Glu
        275                 280                 285
Gly Pro Gly Leu Gln His Leu Ala Leu Leu Ser Asn Asp Ile Phe Thr
    290                 295                 300
Thr Leu Arg Glu Met Arg Ala Arg Ser Glu Leu Gly Gly Phe Glu Phe
305                 310                 315                 320
Met Pro Arg Ala Asn Ala Lys Tyr Tyr Lys Asp Met Tyr Ala Arg Ile
                325                 330                 335
Gly Asp Ser Leu Thr Pro Gln Gln Tyr Arg Glu Val Glu Glu Leu Gly
            340                 345                 350
Ile Leu Val Asp Lys Asp Asp Gln Gly Val Leu Leu Gln Ile Phe Thr
        355                 360                 365
Lys Pro Leu Gly Asp Arg Pro Thr Val Phe Ile Glu Ile Ile Gln Arg
    370                 375                 380
Val Gly Cys Met Arg Glu Val Lys Glu Pro Ala Thr Gly Ala Val Val
385                 390                 395                 400
Gly Thr Glu Gln Ala Ala Gly Cys Gly Gly Phe Gly Lys Gly Asn Phe
                405                 410                 415
Gly Ala Leu Phe Lys Ser Ile Glu Asp Tyr Glu Arg Thr Leu Asn Val
            420                 425                 430

<210> 56
<211> 1299
<212> DNA
<213> Chlamydomonas

<400> 56
atgggagcgg gtggtgcagg caccggagat cgggaggggg gcattaagct cgtgggctac      60

aagaatttcg tgcgccagaa cccgctttca gacaaattca ccgtccacaa gtttcatcac     120

atcgatttct ggtgcggaga tgcaacaaac acatcgaagc ggttctccta cggcctgggc     180

atgccgctgg tcgccaagtc cgaccagtcc accaacaacc agctctttgc ctcctacgtg     240

ctgcgctcca acgacctggt cttcaccttc accgcgccct acagccgcaa gtgcgcctcg     300

gtcagcgagg gcgttccgct gcgtcactac aacatcgacc atgcgtatga gttcatcaac     360

tcgcacgggc tggcggtgcg ggcagtaggc ctgctggtgg atgacgccaa gacggcgtac     420

gaggtgtctg tggcgcacgg ggccaagggc gtgctgccgc cggtggagct gcgggatgag     480

gcgagcggca ccagccaggt catctcggag gtgctgctgt acggcgaggt cgtgctgcgc     540

tacgtgtcgg gctccttcca gggccccttc ctggccggct acacgcccgt cacagactcg     600

gccgtgacct ccttcggcct gcaacgtctg gaccacgcgg tgggcaacac ccatgacctg     660

atcaaggccg tggagtacat caccggcttc acaggtttcc acgagttctc agagtttgtt     720

gcggaggacg tgggcactgt ggacagcggc ctgaacagca tggtgctggc ctccaacaac     780

gaggcagtgc tgctgcctgt gaacgagccc acctttggca cgccgcgcaa gagccaaatc     840

cagacctacc tggagcagaa cgaggggccg gggctgcagc acctggcgct gctcagcaac 900

gacatcttca ccaccctgcg ggagatgcgc gcgcgcagcg agctgggtgg cttcgagttc 960

atgccacggg caaatgccaa gtactacaaa gacatgtacg cccgcatcgg cgactcgctc 1020

acgccgcagc agtacaggga ggtggaggag ctgggcatcc tggtggacaa ggacgaccag 1080

ggcgtgctgc tgcagatctt caccaagccg ctgggcgacc ggcccacggt gtttattgag 1140

atcatccagc gtgtgggctg catgcgggag gtgaaagagc ctgctacggg cgctgtggtg 1200

gggacggagc aggcggctgg ctgcggcggc ttcgggaaag gcaacttcgg tgccctcttc 1260

aagtccattg aggactatga gcgcacctta aatgtttaa 1299

<210> 57
<211> 432
<212> PRT
<213> Chlamydomonas

<400> 57
Met Gly Ala Gly Gly Ala Gly Thr Gly Asp Arg Glu Gly Gly Ile Lys
1               5                   10                  15
Leu Val Gly Tyr Lys Asn Phe Val Arg Gln Asn Pro Leu Ser Asp Lys
            20                  25                  30
Phe Thr Val His Lys Phe His His Ile Asp Phe Trp Cys Gly Asp Ala
        35                  40                  45
Thr Asn Thr Ser Lys Arg Phe Ser Tyr Gly Leu Gly Met Pro Leu Val
    50                  55                  60
Ala Lys Ser Asp Gln Ser Thr Asn Asn Gln Leu Phe Ala Ser Tyr Val
65                  70                  75                  80
Leu Arg Ser Asn Asp Leu Val Phe Thr Phe Thr Ala Pro Tyr Ser Arg
                85                  90                  95
Lys Cys Ala Ser Val Ser Glu Gly Val Pro Leu Arg His Tyr Asn Ile
            100                 105                 110
Asp His Ala Tyr Glu Phe Ile Asn Ser His Gly Leu Ala Val Arg Ala
        115                 120                 125
Val Gly Leu Leu Val Asp Asp Ala Lys Thr Ala Tyr Glu Val Ser Val
    130                 135                 140
Ala His Gly Ala Lys Gly Val Leu Pro Pro Val Glu Leu Arg Asp Glu
145                 150                 155                 160
Ala Ser Gly Thr Ser Gln Val Ile Ser Glu Val Leu Leu Tyr Gly Glu
                165                 170                 175
Val Val Leu Arg Tyr Val Ser Gly Ser Phe Gln Gly Pro Phe Leu Ala
            180                 185                 190
Gly Tyr Thr Pro Val Thr Asp Ser Ala Val Thr Ser Phe Gly Leu Gln
        195                 200                 205
Arg Leu Asp His Ala Val Gly Asn Thr His Asp Leu Ile Lys Ala Val
    210                 215                 220
Glu Tyr Ile Thr Gly Phe Thr Gly Phe His Glu Phe Ser Glu Phe Val
225                 230                 235                 240
Ala Glu Asp Val Gly Thr Val Asp Ser Gly Leu Asn Ser Met Val Leu
                245                 250                 255
Ala Ser Asn Asn Glu Ala Val Leu Leu Pro Val Asn Glu Pro Thr Phe
            260                 265                 270
Gly Thr Pro Arg Lys Ser Gln Ile Gln Thr Tyr Leu Glu Gln Asn Glu
        275                 280                 285
Gly Pro Gly Leu Gln His Leu Ala Leu Leu Ser Asn Asp Ile Phe Thr
    290                 295                 300
Thr Leu Arg Glu Met Arg Ala Arg Ser Glu Leu Gly Gly Phe Glu Phe

305                 310                 315                 320
Met Pro Arg Ala Asn Ala Lys Tyr Tyr Lys Asp Met Tyr Ala Arg Ile
                325                 330                 335
Gly Asp Ser Leu Thr Pro Gln Gln Tyr Arg Glu Val Glu Glu Leu Gly
            340                 345                 350
Ile Leu Val Asp Lys Asp Asp Gln Gly Val Leu Leu Gln Ile Phe Thr
        355                 360                 365
Lys Pro Leu Gly Asp Arg Pro Thr Val Phe Ile Glu Ile Ile Gln Arg
    370                 375                 380
Val Gly Cys Met Arg Glu Val Lys Glu Pro Ala Thr Gly Ala Val Val
385                 390                 395                 400
Gly Thr Glu Gln Ala Ala Gly Cys Gly Gly Phe Gly Lys Gly Asn Phe
                405                 410                 415
Gly Ala Leu Phe Lys Ser Ile Glu Asp Tyr Glu Arg Thr Leu Asn Val
            420                 425                 430

<210> 58
<211> 433
<212> PRT
<213> Physcomitrella

<400> 58
Met Gly Leu Asp Lys Ser Glu Ser Glu Gly Ser Val Val Gly Pro Leu
1               5                   10                  15
His Leu Val Gly Cys Glu Arg Phe Val Arg Asn Asn Pro Lys Thr Asp
            20                  25                  30
Arg Phe Gly Val Glu Arg Phe His His Val Glu Phe Trp Cys Gly Asp
        35                  40                  45
Ala Ser Asn Thr Trp Arg Arg Phe Ser Trp Gly Leu Gly Met His Leu
    50                  55                  60
Val Ala Lys Ser Asp Gln Thr Thr Gly Asn Gln Thr Tyr Cys Ser Tyr
65                  70                  75                  80
Ala Ile Gln Ser Asn Glu Leu Val Phe Ala Phe Thr Ala Pro Tyr Ser
                85                  90                  95
Ser Thr Ile Asp Gln Thr Asn Thr Lys Met Pro His Pro Gly Tyr Lys
            100                 105                 110
Ser Asp Glu Ala Arg Ser Phe Thr Asp Ser His Gly Leu Ala Val Arg
        115                 120                 125
Ala Val Gly Ile Leu Val Asp Asp Ala Asp Glu Ala Phe Arg Ile Ser
    130                 135                 140
Val Glu His Gly Ala Val Ser Val Leu Glu Pro His Val Leu Ser Asp
145                 150                 155                 160
Asp Ala Lys Gly Gly Lys Met Val Met Ala Glu Val Lys Leu Tyr Gly
                165                 170                 175
Asp Val Val Leu Arg Tyr Val Ser Glu Gln Gly Tyr Lys Gly Ser Met
            180                 185                 190
Leu Pro Asn Tyr Glu Glu Val Glu Ser Leu Pro Leu Ser Tyr Gly Leu
        195                 200                 205
Val Arg Leu Asp His Ala Val Gly Asn Val His Asn Leu Ala Glu Ala
    210                 215                 220
Val Asn Tyr Ile Ala Lys Phe Thr Gly Phe His Glu Phe Ala Glu Phe
225                 230                 235                 240
Thr Ala Gly Asp Val Gly Thr Thr Glu Ser Gly Leu Asn Ser Met Val
                245                 250                 255
Val Ala Ser Asn Asn Glu Met Val Leu Leu Pro Ile Asn Glu Pro Thr
            260                 265                 270
Phe Gly Thr Lys Arg Lys Ser Gln Ile Gln Thr Tyr Leu Glu His Asn
        275                 280                 285
Glu Gly Pro Gly Leu Gln His Leu Ala Leu Ile Cys Asp Asn Ile Phe
    290                 295                 300
Ser Thr Leu Arg Glu Met Arg Thr Arg Thr His Ile Gly Gly Phe Asp

305                 310                 315                 320
Phe Met Pro Lys Pro Pro Pro Thr Tyr Tyr Lys Asn Leu Ala Asn Arg
                325                 330                 335
Val Gly Asp Ile Leu Thr Ala Glu Gln Ile Lys Glu Cys Asp Glu Leu
            340                 345                 350
Gly Ile Leu Val Asp Lys Asp Asp Gln Gly Val Leu Leu Gln Ile Phe
        355                 360                 365
Thr Lys Pro Val Gly Asp Arg Pro Ser Ile Phe Val Glu Ile Ile Gln
    370                 375                 380
Arg Ile Gly Cys Met Asp Lys Asp Glu Ser Thr Gly Ala Thr Val Gln
385                 390                 395                 400
Lys Gly Gly Cys Gly Gly Phe Gly Lys Gly Asn Phe Ser Glu Leu Phe
                405                 410                 415
Lys Ser Ile Glu Glu Tyr Glu Lys Thr Leu Asp Gly Thr Leu Lys Val
            420                 425                 430
His

<210> 59
<211> 446
<212> PRT
<213> Oryza

<400> 59
Met Pro Pro Thr Pro Thr Pro Thr Ala Thr Thr Gly Ala Val Ser Ala
1               5                   10                  15
Ala Ala Ala Ala Gly Glu Asn Ala Gly Phe Arg Leu Val Gly His Arg
            20                  25                  30
Arg Phe Val Arg Ala Asn Pro Arg Ser Asp Arg Phe Gln Ala Leu Ala
        35                  40                  45
Phe His His Val Glu Leu Trp Cys Ala Asp Ala Ala Ser Ala Ala Gly
    50                  55                  60
Arg Phe Ala Phe Ala Leu Gly Ala Pro Leu Ala Ala Arg Ser Asp Leu
65                  70                  75                  80
Ser Thr Gly Asn Ser Ala His Ala Ser Leu Leu Leu Arg Ser Ala Ser
                85                  90                  95
Val Ala Phe Leu Phe Thr Ala Pro Tyr Gly Gly Asp His Gly Val Gly
            100                 105                 110
Ala Asp Ala Ala Thr Thr Ala Ser Ile Pro Ser Phe Ser Pro Gly Ala
        115                 120                 125
Ala Arg Arg Phe Ala Ala Asp His Gly Leu Ala Val His Ala Val Ala
    130                 135                 140
Leu Arg Val Ala Asp Ala Ala Asp Ala Phe Arg Ala Ser Val Ala Ala
145                 150                 155                 160
Gly Ala Arg Pro Ala Phe Gln Pro Ala Asp Leu Gly Gly Gly Phe Gly
                165                 170                 175
Leu Ala Glu Val Glu Leu Tyr Gly Asp Val Val Leu Arg Phe Val Ser
            180                 185                 190
His Pro Asp Gly Ala Asp Ala Pro Phe Leu Pro Gly Phe Glu Gly Val
        195                 200                 205
Ser Asn Pro Gly Ala Val Asp Tyr Gly Leu Arg Arg Phe Asp His Val
    210                 215                 220
Val Gly Asn Val Pro Glu Leu Ala Pro Val Ala Ala Tyr Ile Ser Gly
225                 230                 235                 240
Phe Thr Gly Phe His Glu Phe Ala Glu Phe Thr Ala Glu Asp Val Gly
                245                 250                 255
Thr Ala Glu Ser Gly Leu Asn Ser Val Val Leu Ala Asn Asn Ala Glu
            260                 265                 270
Thr Val Leu Leu Pro Leu Asn Glu Pro Val His Gly Thr Lys Arg Arg
        275                 280                 285
Ser Gln Ile Gln Thr Tyr Leu Asp His His Gly Gly Pro Gly Val Gln
    290                 295                 300
His Ile Ala Leu Ala Ser Asp Asp Val Leu Gly Thr Leu Arg Glu Met

305                 310                 315                 320
Arg Ala Arg Ser Ala Met Gly Gly Phe Glu Phe Leu Ala Pro Pro Pro
                325                 330                 335
Pro Asn Tyr Tyr Asp Gly Val Arg Arg Arg Ala Gly Asp Val Leu Ser
            340                 345                 350
Glu Glu Gln Ile Asn Glu Cys Gln Glu Leu Gly Val Leu Val Asp Arg
        355                 360                 365
Asp Asp Gln Gly Val Leu Leu Gln Ile Phe Thr Lys Pro Val Gly Asp
    370                 375                 380
Arg Pro Thr Phe Phe Leu Glu Met Ile Gln Arg Ile Gly Cys Met Glu
385                 390                 395                 400
Lys Asp Glu Ser Gly Gln Glu Tyr Gln Lys Gly Gly Cys Gly Gly Phe
                405                 410                 415
Gly Lys Gly Asn Phe Ser Glu Leu Phe Lys Ser Ile Glu Glu Tyr Glu
            420                 425                 430
Lys Ser Leu Glu Ala Lys Gln Ala Pro Thr Val Gln Gly Ser
        435                 440                 445

<210> 60
<211> 436
<212> PRT
<213> Triticum

<400> 60
Met Pro Pro Thr Pro Thr Thr Pro Ala Ala Thr Gly Ala Gly Ala Ala
1               5                   10                  15
Ala Ala Val Thr Pro Glu His Ala Arg Pro Arg Arg Met Val Arg Phe
            20                  25                  30
Asn Pro Arg Ser Asp Arg Phe His Thr Leu Ser Phe His His Val Glu
        35                  40                  45
Phe Trp Cys Ala Asp Ala Ala Ser Ala Ala Gly Arg Phe Ala Phe Ala
    50                  55                  60
Leu Gly Ala Pro Leu Ala Ala Arg Ser Asp Leu Ser Thr Gly Asn Ser
65                  70                  75                  80
Val His Ala Ser Gln Leu Leu Arg Ser Gly Asn Leu Ala Phe Leu Phe
                85                  90                  95
Thr Ala Pro Tyr Ala Asn Gly Cys Asp Ala Ala Thr Ala Ser Leu Pro
            100                 105                 110
Ser Phe Ser Ala Asp Ala Ala Arg Arg Phe Ser Ala Asp His Gly Leu
        115                 120                 125
Ala Val Arg Ser Ile Ala Leu Arg Val Ala Asp Ala Ala Glu Ala Phe
    130                 135                 140
Arg Ala Ser Val Asp Gly Gly Ala Arg Pro Ala Phe Ser Pro Val Asp
145                 150                 155                 160
Leu Gly Arg Gly Phe Gly Phe Ala Glu Val Glu Leu Tyr Gly Asp Val
                165                 170                 175
Val Leu Arg Phe Val Ser His Pro Asp Asp Thr Asp Val Pro Phe Leu
            180                 185                 190
Pro Gly Phe Glu Gly Val Ser Asn Pro Asp Ala Val Asp Tyr Gly Leu
        195                 200                 205
Thr Arg Phe Asp His Val Val Gly Asn Val Pro Glu Leu Ala Pro Ala
    210                 215                 220
Ala Ala Tyr Val Ala Gly Phe Ala Gly Phe His Glu Phe Ala Glu Phe
225                 230                 235                 240
Thr Thr Glu Asp Val Gly Thr Ala Glu Ser Gly Leu Asn Ser Met Val
                245                 250                 255
Leu Ala Asn Asn Ser Glu Gly Val Leu Leu Pro Leu Asn Glu Pro Val
            260                 265                 270
His Gly Thr Lys Arg Arg Ser Gln Ile Gln Thr Phe Leu Glu His His
        275                 280                 285
Gly Gly Ser Gly Val Gln His Ile Ala Val Ala Ser Ser Asp Val Leu
    290                 295                 300
Arg Thr Leu Arg Glu Met Arg Ala Arg Ser Ala Met Gly Gly Phe Asp

305                 310                 315                 320
Phe Leu Pro Pro Arg Cys Arg Lys Tyr Tyr Glu Gly Val Arg Arg Ile
                325                 330                 335
Ala Gly Asp Val Leu Ser Glu Ala Gln Ile Lys Glu Cys Gln Glu Leu
            340                 345                 350
Gly Val Leu Val Asp Arg Asp Asp Gln Gly Val Leu Leu Gln Ile Phe
        355                 360                 365
Thr Lys Pro Val Gly Asp Arg Pro Thr Leu Phe Leu Glu Met Ile Gln
    370                 375                 380
Arg Ile Gly Cys Met Glu Lys Asp Glu Arg Gly Glu Glu Tyr Gln Lys
385                 390                 395                 400
Gly Gly Cys Gly Gly Phe Gly Lys Gly Asn Phe Ser Glu Leu Phe Lys
                405                 410                 415
Ser Ile Glu Asp Tyr Glu Lys Ser Leu Glu Ala Lys Gln Ser Ala Ala
            420                 425                 430
Val Gln Gly Ser
        435

<210> 61
<211> 444
<212> PRT
<213> Zea

<400> 61
   Met Pro Pro Thr Pro Thr Ala Ala Ala Ala Gly Ala Ala Val Ala Ala
   1               5                   10                  15
   Ala Ser Ala Ala Glu Gln Ala Ala Phe Arg Leu Val Gly His Arg Asn
               20                  25                  30
   Phe Val Arg Phe Asn Pro Arg Ser Asp Arg Phe His Thr Leu Ala Phe
           35                  40                  45
   His His Val Glu Leu Trp Cys Ala Asp Ala Ala Ser Ala Ala Gly Arg
       50                  55                  60
   Phe Ser Phe Gly Leu Gly Ala Pro Leu Ala Ala Arg Ser Asp Leu Ser
   65                  70                  75                  80
   Thr Gly Asn Ser Ala His Ala Ser Leu Leu Leu Arg Ser Gly Ser Leu
                   85                  90                  95
   Ser Phe Leu Phe Thr Ala Pro Tyr Ala His Gly Ala Asp Ala Ala Thr
               100                 105                 110
   Ala Ala Leu Pro Ser Phe Ser Ala Ala Ala Ala Arg Arg Phe Ala Ala
           115                 120                 125
   Asp His Gly Leu Ala Val Arg Ala Val Ala Leu Arg Val Ala Asp Ala
       130                 135                 140
   Glu Glu Ala Phe Arg Thr Ser Val Ala Ala Gly Ala Arg Pro Ala Phe
   145                 150                 155                 160
   Gly Pro Val Asp Leu Gly Arg Gly Phe Arg Leu Ala Glu Val Glu Leu
                   165                 170                 175
   Tyr Gly Asp Val Val Leu Arg Tyr Val Ser Tyr Pro Asp Gly Ala Ala
               180                 185                 190
   Gly Glu Pro Phe Leu Pro Gly Phe Glu Gly Val Ala Ser Pro Gly Ala
           195                 200                 205
   Ala Asp Tyr Gly Leu Ser Arg Phe Asp His Ile Val Gly Asn Val Pro
       210                 215                 220
   Glu Leu Ala Pro Ala Ala Ala Tyr Phe Ala Gly Phe Thr Gly Phe His
   225                 230                 235                 240
   Glu Phe Ala Glu Phe Thr Thr Glu Asp Val Gly Thr Ala Glu Ser Gly
                   245                 250                 255
   Leu Asn Ser Met Val Leu Ala Asn Asn Ser Glu Asn Val Leu Leu Pro
               260                 265                 270
   Leu Asn Glu Pro Val His Gly Thr Lys Arg Arg Ser Gln Ile Gln Thr
           275                 280                 285
   Phe Leu Asp His His Gly Gly Pro Gly Val Gln His Met Ala Leu Ala
       290                 295                 300
   Ser Asp Asp Val Leu Arg Thr Leu Arg Glu Met Gln Ala Arg Ser Ala
   305                 310                 315                 320
   Met Gly Gly Phe Glu Phe Met Ala Pro Pro Thr Ser Asp Tyr Tyr Asp
                   325                 330                 335
   Gly Val Arg Arg Arg Ala Gly Asp Val Leu Thr Glu Ala Gln Ile Lys
               340                 345                 350
   Glu Cys Gln Glu Leu Gly Val Leu Val Asp Arg Asp Asp Gln Gly Val
           355                 360                 365
   Leu Leu Gln Ile Phe Pro Lys Pro Val Gly Asp Arg Pro Thr Leu Phe
       370                 375                 380
   Leu Glu Ile Ile Gln Arg Ile Gly Cys Met Glu Arg Asp Glu Lys Gly
   385                 390                 395                 400
   Gln Glu Tyr Gln Lys Gly Gly Cys Gly Gly Phe Gly Lys Gly Asn Phe
                   405                 410                 415
   Ser Gln Leu Phe Lys Ser Ile Glu Asp Tyr Glu Lys Ser Leu Glu Ala
               420                 425                 430
   Met Gln Ala Ala Ala Ala Ala Thr Ala Gln Gly Ser
           435                 440

<210> 62
<211> 443
<212> PRT
<213> Glycine

<220>
<221> VARIANT
<222> 393..393

<400> 62
Met Cys Asn Glu Ile Gln Ala Gln Ala Gln Ala Gln Ala Gln Pro Gly
1               5                   10                  15
Phe Lys Leu Val Gly Phe Lys Asn Phe Val Arg Thr Asn Pro Lys Ser
            20                  25                  30
Asp Arg Phe Gln Val Asn Arg Phe His His Ile Glu Phe Trp Cys Thr
        35                  40                  45
Asp Ala Thr Asn Ala Ser Arg Arg Phe Ser Trp Gly Leu Gly Met Pro
    50                  55                  60
Ile Val Ala Lys Ser Asp Leu Ser Thr Gly Asn Gln Ile His Ala Ser
65                  70                  75                  80
Tyr Leu Leu Arg Ser Gly Asp Leu Ser Phe Leu Phe Ser Ala Pro Tyr
                85                  90                  95
Ser Pro Ser Leu Ser Ala Gly Ser Ser Ala Ala Ser Ser Ala Ser Ile
            100                 105                 110
Pro Ser Phe Asp Ala Ala Thr Cys Leu Ala Phe Ala Ala Lys His Gly
        115                 120                 125
Phe Gly Val Arg Ala Ile Ala Leu Glu Val Ala Asp Ala Glu Ala Ala
    130                 135                 140
Phe Ser Ala Ser Val Ala Lys Gly Ala Glu Pro Ala Ser Pro Pro Val
145                 150                 155                 160
Leu Val Asp Asp Arg Thr Gly Phe Ala Glu Val Arg Leu Tyr Gly Asp
                165                 170                 175
Val Val Leu Arg Tyr Val Ser Tyr Lys Asp Ala Ala Pro Gln Ala Pro
            180                 185                 190
His Ala Asp Pro Ser Arg Trp Phe Leu Pro Gly Phe Glu Ala Ala Ala
        195                 200                 205
Ser Ser Ser Ser Phe Pro Glu Leu Asp Tyr Gly Ile Arg Arg Leu Asp
    210                 215                 220
His Ala Val Gly Asn Val Pro Glu Leu Ala Pro Ala Val Arg Tyr Leu
225                 230                 235                 240
Lys Gly Phe Ser Gly Phe His Glu Phe Ala Glu Phe Thr Ala Glu Asp
                245                 250                 255
Val Gly Thr Ser Glu Ser Gly Leu Asn Ser Val Val Leu Ala Asn Asn
            260                 265                 270
Ser Glu Thr Val Leu Leu Pro Leu Asn Glu Pro Val Tyr Gly Thr Lys

275                 280                 285
Arg Lys Ser Gln Ile Glu Thr Tyr Leu Glu His Asn Glu Gly Ala Gly
    290                 295                 300
Val Gln His Leu Ala Leu Val Thr His Asp Ile Phe Thr Thr Leu Arg
305                 310                 315                 320
Glu Met Arg Lys Arg Ser Phe Leu Gly Gly Phe Glu Phe Met Pro Ser
                325                 330                 335
Pro Pro Pro Thr Tyr Tyr Ala Asn Leu His Asn Arg Ala Ala Asp Val
            340                 345                 350
Leu Thr Val Asp Gln Ile Lys Gln Cys Glu Glu Leu Gly Ile Leu Val
        355                 360                 365
Asp Arg Asp Asp Gln Gly Thr Leu Leu Gln Ile Phe Thr Lys Pro Val
    370                 375                 380
Gly Asp Arg Pro Thr Ile Phe Ile Xaa Ile Ile Gln Arg Ile Gly Cys
385                 390                 395                 400
Met Val Glu Asp Glu Glu Gly Lys Val Tyr Gln Lys Gly Ala Cys Gly
                405                 410                 415
Gly Phe Gly Lys Gly Asn Phe Ser Glu Leu Phe Lys Ser Ile Glu Glu
            420                 425                 430
Tyr Glu Lys Thr Leu Glu Ala Lys Arg Thr Ala
        435                 440

<210> 63
<211> 445
<212> PRT
<213> Vitis

<400> 63
Met Gly Lys Gln Asn Thr Thr Thr Asn Asn Pro Ala Pro Gly Phe Lys
1               5                   10                  15
Leu Val Gly Phe Ser Asn Phe Leu Arg Thr Asn Pro Met Ser Asp Arg
            20                  25                  30
Phe Gly Val Lys Arg Phe His His Ile Glu Phe Trp Ser Thr Asp Ala
        35                  40                  45
Thr Asn Leu Ala Arg Arg Phe Ser Trp Gly Leu Gly Met Pro Ile Val
    50                  55                  60
Ala Lys Ser Asp Leu Ser Thr Gly Asn Val Ile His Ala Ser Tyr Leu
65                  70                  75                  80
Thr Arg Ser Gly Asp Leu Asn Phe Leu Phe Thr Ala Pro Tyr Ser Pro
                85                  90                  95
Ser Ile Ala Gly Asp Leu Glu Asn Ala Ala Ala Thr Ala Ser Ile Pro
            100                 105                 110
Ser Phe Asp His Ser Ala Cys His Ala Phe Ala Ala Ser His Gly Leu
        115                 120                 125
Gly Val Arg Ala Ile Ala Ile Glu Val Asp Asp Ala Glu Gly Ala Phe
    130                 135                 140
His Thr Ser Val Ala His Gly Ala Arg Pro Met Ser Pro Pro Val Thr
145                 150                 155                 160
Met Gly Gly Ser Val Val Ile Ser Glu Val His Leu Tyr Gly Asp Ala
                165                 170                 175
Val Leu Arg Tyr Val Ser Tyr Lys Asn Pro Asn Pro Asn Ala Thr Ser
            180                 185                 190
Asp Pro Ser Ser Trp Phe Leu Pro Gly Phe Glu Ala Val Asp Glu Gly
        195                 200                 205
Ser Ser Phe Pro Val Asp Phe Gly Leu Arg Arg Val Asp His Thr Val
    210                 215                 220
Gly Asn Val Pro Lys Leu Ala Pro Val Val Thr Tyr Leu Lys Gln Phe
225                 230                 235                 240
Thr Gly Phe His Glu Phe Ala Glu Phe Thr Ala Glu Asp Val Gly Thr
                245                 250                 255
Ser Glu Ser Gly Leu Asn Ser Val Val Leu Ala Ser Asn Asn Glu Met
            260                 265                 270
Val Leu Leu Pro Leu Asn Glu Pro Val Phe Gly Thr Lys Arg Lys Ser

275                 280                 285
Gln Ile Gln Thr Tyr Leu Glu His Asn Glu Gly Pro Gly Val Gln His
    290                 295                 300
Leu Ala Leu Met Ser Asp Asp Ile Phe Arg Thr Leu Arg Glu Met Arg
305                 310                 315                 320
Arg Arg Ser Gly Val Gly Gly Phe Asp Phe Met Pro Ser Pro Pro Pro
                325                 330                 335
Thr Tyr Tyr Arg Asn Val Lys Lys Arg Ala Gly Asp Val Leu Thr Asp
            340                 345                 350
Asp Gln Ile Lys Glu Cys Glu Glu Leu Gly Ile Leu Val Asp Lys Asp
        355                 360                 365
Asp Gln Gly Thr Leu Leu Gln Ile Phe Thr Lys Pro Leu Gly Asp Arg
    370                 375                 380
Pro Thr Ile Phe Ile Glu Ile Ile Gln Arg Leu Gly Cys Met Val Lys
385                 390                 395                 400
Asp Asp Glu Gly Lys Val Ser Gln Lys Gly Gly Cys Gly Gly Phe Gly
                405                 410                 415
Lys Gly Asn Phe Ser Glu Leu Phe Lys Ser Ile Glu Glu Tyr Glu Lys
            420                 425                 430
Thr Leu Gly Ala Lys Arg Ile Val Asp Pro Ala Pro Val
        435                 440                 445

<210> 64
<211> 358
<212> PRT
<213> Pseudomonas fluorescens

<400> 64
Met Ala Asp Gln Tyr Glu Asn Pro Met Gly Leu Met Gly Phe Glu Phe
1               5                   10                  15
Ile Glu Phe Ala Ser Pro Thr Pro Gly Thr Leu Glu Pro Ile Phe Glu
            20                  25                  30
Ile Met Gly Phe Thr Lys Val Ala Thr His Arg Ser Lys Asn Val His
        35                  40                  45
Leu Tyr Arg Gln Gly Glu Ile Asn Leu Ile Leu Asn Asn Gln Pro Asp
    50                  55                  60
Ser Leu Ala Ser Tyr Phe Ala Ala Glu His Gly Pro Ser Val Cys Gly
65                  70                  75                  80
Met Ala Phe Arg Val Lys Asp Ser Gln Gln Ala Tyr Asn Arg Ala Leu
                85                  90                  95
Glu Leu Gly Ala Gln Pro Ile His Ile Glu Thr Gly Pro Met Glu Leu
            100                 105                 110
Asn Leu Pro Ala Ile Lys Gly Ile Gly Gly Ala Pro Leu Tyr Leu Ile
        115                 120                 125
Asp Arg Phe Gly Glu Gly Ser Ser Ile Tyr Asp Ile Asp Phe Val Tyr
    130                 135                 140
Leu Glu Gly Val Asp Arg Asn Pro Val Gly Ala Gly Leu Lys Val Ile
145                 150                 155                 160
Asp His Leu Thr His Asn Val Tyr Arg Gly Arg Met Ala Tyr Trp Ala
                165                 170                 175
Asn Phe Tyr Glu Lys Leu Phe Asn Phe Arg Glu Ala Arg Tyr Phe Asp
            180                 185                 190
Ile Lys Gly Glu Tyr Thr Gly Leu Thr Ser Lys Ala Met Ser Ala Pro
        195                 200                 205
Asp Gly Met Ile Arg Ile Pro Leu Asn Glu Glu Ser Ser Lys Gly Ala
    210                 215                 220
Gly Gln Ile Glu Glu Phe Leu Met Gln Phe Asn Gly Glu Gly Ile Gln
225                 230                 235                 240
His Val Ala Phe Leu Thr Glu Asp Leu Val Lys Thr Trp Asp Ala Leu
                245                 250                 255
Lys Lys Ile Gly Met Arg Phe Met Thr Ala Pro Pro Asp Thr Tyr Tyr
            260                 265                 270
Glu Met Leu Glu Gly Arg Leu Pro Asn His Gly Glu Pro Val Asp Gln

275                 280                 285
Leu Gln Ala Arg Gly Ile Leu Leu Asp Gly Ser Ser Ile Glu Gly Asp
    290                 295                 300
Lys Arg Leu Leu Leu Gln Ile Phe Ser Glu Thr Leu Met Gly Pro Val
305                 310                 315                 320
Phe Phe Glu Phe Ile Gln Arg Lys Gly Asp Asp Gly Phe Gly Glu Gly
                325                 330                 335
Asn Phe Lys Ala Leu Phe Glu Ser Ile Glu Arg Asp Gln Val Arg Arg
            340                 345                 350
Gly Val Leu Thr Thr Asp
        355

<210> 65
<211> 358
<212> PRT
<213> Pseudomonas fluorescens

<400> 65
Met Ala Asp Leu Tyr Glu Asn Pro Met Gly Leu Met Gly Phe Glu Phe
1               5                   10                  15
Ile Glu Leu Ala Ser Pro Thr Pro Asn Thr Leu Glu Pro Ile Phe Glu
            20                  25                  30
Ile Met Gly Phe Thr Lys Val Ala Thr His Arg Ser Lys Asp Val His
        35                  40                  45
Leu Tyr Arg Gln Gly Ala Ile Asn Leu Ile Leu Asn Asn Glu Pro His
    50                  55                  60
Ser Val Ala Ser Tyr Phe Ala Ala Glu His Gly Pro Ser Val Cys Gly
65                  70                  75                  80
Met Ala Phe Arg Val Lys Asp Ser Gln Lys Ala Tyr Lys Arg Ala Leu
                85                  90                  95
Glu Leu Gly Ala Gln Pro Ile His Ile Glu Thr Gly Pro Met Glu Leu
            100                 105                 110
Asn Leu Pro Ala Ile Lys Gly Ile Gly Gly Ala Pro Leu Tyr Leu Ile
        115                 120                 125
Asp Arg Phe Gly Glu Gly Ser Ser Ile Tyr Asp Ile Asp Phe Val Phe
    130                 135                 140
Leu Glu Gly Val Asp Arg His Pro Val Gly Ala Gly Leu Lys Ile Ile
145                 150                 155                 160
Asp His Leu Thr His Asn Val Tyr Arg Gly Arg Met Ala Tyr Trp Ala
                165                 170                 175
Asn Phe Tyr Glu Lys Leu Phe Asn Phe Arg Glu Ile Arg Tyr Phe Asp
            180                 185                 190
Ile Lys Gly Glu Tyr Thr Gly Leu Thr Ser Lys Ala Met Thr Ala Pro
        195                 200                 205
Asp Gly Met Ile Arg Ile Pro Leu Asn Glu Glu Ser Ser Lys Gly Ala
    210                 215                 220
Gly Gln Ile Glu Glu Phe Leu Met Gln Phe Asn Gly Glu Gly Ile Gln
225                 230                 235                 240
His Val Ala Phe Leu Ser Asp Asp Leu Ile Lys Thr Trp Asp His Leu
                245                 250                 255
Lys Ser Ile Gly Met Arg Phe Met Thr Ala Pro Pro Asp Thr Tyr Tyr
            260                 265                 270
Glu Met Leu Glu Gly Arg Leu Pro Asn His Gly Glu Pro Val Gly Glu
        275                 280                 285
Leu Gln Ala Arg Gly Ile Leu Leu Asp Gly Ser Ser Glu Ser Gly Asp
    290                 295                 300
Lys Arg Leu Leu Leu Gln Ile Phe Ser Glu Thr Leu Met Gly Pro Val
305                 310                 315                 320
Phe Phe Glu Phe Ile Gln Arg Lys Gly Asp Asp Gly Phe Gly Glu Gly
                325                 330                 335
Asn Phe Lys Ala Leu Phe Glu Ser Ile Glu Arg Asp Gln Val Arg Arg
            340                 345                 350
Gly Val Leu Ser Thr Asp

        355

<210> 66
<211> 440
<212> PRT
<213> Avena sativa

<400> 66
Met Pro Pro Thr Pro Ala Thr Ala Thr Gly Ala Ala Ala Ala Ala Val
1               5                   10                  15
Thr Pro Glu His Ala Ala Arg Ser Phe Pro Arg Val Val Arg Val Asn
            20                  25                  30
Pro Arg Ser Asp Arg Phe Pro Val Leu Ser Phe His His Val Glu Leu
        35                  40                  45
Trp Cys Ala Asp Ala Ala Ser Ala Ala Gly Arg Phe Ser Phe Ala Leu
    50                  55                  60
Gly Ala Pro Leu Ala Ala Arg Ser Asp Leu Ser Thr Gly Asn Ser Ala
65                  70                  75                  80
His Ala Ser Leu Leu Leu Arg Ser Gly Ala Leu Ala Phe Leu Phe Thr
                85                  90                  95
Ala Pro Tyr Ala Pro Pro Pro Gln Glu Ala Ala Thr Ala Ala Ala Thr
            100                 105                 110
Ala Ser Ile Pro Ser Phe Ser Ala Asp Ala Ala Arg Thr Phe Ala Ala
        115                 120                 125
Ala His Gly Leu Ala Val Arg Ser Val Gly Val Arg Val Ala Asp Ala
    130                 135                 140
Ala Glu Ala Phe Arg Val Ser Val Ala Gly Gly Ala Arg Pro Ala Phe
145                 150                 155                 160
Ala Pro Ala Asp Leu Gly His Gly Phe Gly Leu Ala Glu Val Glu Leu
                165                 170                 175
Tyr Gly Asp Val Val Leu Arg Phe Val Ser Tyr Pro Asp Glu Thr Asp
            180                 185                 190
Leu Pro Phe Leu Pro Gly Phe Glu Arg Val Ser Ser Pro Gly Ala Val
        195                 200                 205
Asp Tyr Gly Leu Thr Arg Phe Asp His Val Val Gly Asn Val Pro Glu
    210                 215                 220
Met Ala Pro Val Ile Asp Tyr Met Lys Gly Phe Leu Gly Phe His Glu
225                 230                 235                 240
Phe Ala Glu Phe Thr Ala Glu Asp Val Gly Thr Thr Glu Ser Gly Leu
                245                 250                 255
Asn Ser Val Val Leu Ala Asn Asn Ser Glu Ala Val Leu Leu Pro Leu
            260                 265                 270
Asn Glu Pro Val His Gly Thr Lys Arg Arg Ser Gln Ile Gln Thr Tyr
        275                 280                 285
Leu Glu Tyr His Gly Gly Pro Gly Val Gln His Ile Ala Leu Ala Ser
    290                 295                 300
Asn Asp Val Leu Arg Thr Leu Arg Glu Met Arg Ala Arg Thr Pro Met
305                 310                 315                 320
Gly Gly Phe Glu Phe Met Ala Pro Pro Gln Ala Lys Tyr Tyr Glu Gly
                325                 330                 335
Val Arg Arg Ile Ala Gly Asp Val Leu Ser Glu Glu Gln Ile Lys Glu
            340                 345                 350
Cys Gln Glu Leu Gly Val Leu Val Asp Arg Asp Asp Gln Gly Val Leu
        355                 360                 365
Leu Gln Ile Phe Thr Lys Pro Val Gly Asp Arg Pro Thr Phe Phe Leu
    370                 375                 380
Glu Met Ile Gln Arg Ile Gly Cys Met Glu Lys Asp Glu Val Gly Gln
385                 390                 395                 400
Glu Tyr Gln Lys Gly Gly Cys Gly Gly Phe Gly Lys Gly Asn Phe Ser
                405                 410                 415
Glu Leu Phe Lys Ser Ile Glu Asp Tyr Glu Lys Ser Leu Glu Val Lys
            420                 425                 430
Gln Ser Val Val Ala Gln Lys Ser

435                 440

<210> 67
<211> 444
<212> PRT
<213> Zea mays

<400> 67
Met Gly Pro Thr Pro Thr Ala Ala Ala Ala Gly Ala Ala Val Ala Ala
1               5                   10                  15
Ala Ser Ala Ala Glu Gln Ala Ala Phe Arg Leu Val Gly His Arg Asn
            20                  25                  30
Phe Val Arg Phe Asn Pro Arg Ser Asp Arg Phe His Thr Leu Ala Phe
        35                  40                  45
His His Val Glu Leu Trp Cys Ala Asp Ala Ala Ser Ala Ala Gly Arg
    50                  55                  60
Phe Ser Phe Gly Leu Gly Ala Pro Leu Ala Ala Arg Ser Asp Leu Ser
65                  70                  75                  80
Thr Gly Asn Ser Ala His Ala Ser Leu Leu Leu Arg Ser Gly Ser Leu
                85                  90                  95
Ser Phe Leu Phe Thr Ala Pro Tyr Ala His Gly Ala Asp Ala Ala Thr
            100                 105                 110
Ala Ala Leu Pro Ser Phe Ser Ala Ala Ala Ala Arg Arg Phe Ala Ala
        115                 120                 125
Asp His Gly Leu Ala Val Arg Ala Val Ala Leu Arg Val Ala Asp Ala
    130                 135                 140
Glu Asp Ala Phe Arg Ala Ser Val Ala Ala Gly Ala Arg Pro Ala Phe
145                 150                 155                 160
Gly Pro Val Asp Leu Gly Arg Gly Phe Arg Leu Ala Glu Val Glu Leu
                165                 170                 175
Tyr Gly Asp Val Val Leu Arg Tyr Val Ser Tyr Pro Asp Gly Ala Ala
            180                 185                 190
Gly Glu Pro Phe Leu Pro Gly Phe Glu Gly Val Ala Ser Pro Gly Ala
        195                 200                 205
Ala Asp Tyr Gly Leu Ser Arg Phe Asp His Ile Val Gly Asn Val Pro
    210                 215                 220
Glu Leu Ala Pro Ala Ala Ala Tyr Phe Ala Gly Phe Thr Gly Phe His
225                 230                 235                 240
Glu Phe Ala Glu Phe Thr Thr Glu Asp Val Gly Thr Ala Glu Ser Gly
                245                 250                 255
Leu Asn Ser Met Val Leu Ala Asn Asn Ser Glu Asn Val Leu Leu Pro
            260                 265                 270
Leu Asn Glu Pro Val His Gly Thr Lys Arg Arg Ser Gln Ile Gln Thr
        275                 280                 285
Phe Leu Asp His His Gly Gly Pro Gly Val Gln His Met Ala Leu Ala
    290                 295                 300
Ser Asp Asp Val Leu Arg Thr Leu Arg Glu Met Gln Ala Arg Ser Ala
305                 310                 315                 320
Met Gly Gly Phe Glu Phe Met Ala Pro Pro Thr Ser Asp Tyr Tyr Asp
                325                 330                 335
Gly Val Arg Arg Arg Ala Gly Asp Val Leu Thr Glu Ala Gln Ile Lys
            340                 345                 350
Glu Cys Gln Glu Leu Gly Val Leu Val Asp Arg Asp Asp Gln Gly Val
        355                 360                 365
Leu Leu Gln Ile Phe Thr Lys Pro Val Gly Asp Arg Pro Thr Leu Phe
    370                 375                 380
Leu Glu Ile Ile Gln Arg Ile Gly Cys Met Glu Lys Asp Glu Lys Gly
385                 390                 395                 400
Gln Glu Tyr Gln Lys Gly Gly Cys Gly Gly Phe Gly Lys Gly Asn Phe
                405                 410                 415
Ser Gln Leu Phe Lys Ser Ile Glu Asp Tyr Glu Lys Ser Leu Glu Ala
            420                 425                 430
Lys Gln Ala Ala Ala Ala Ala Ala Ala Gln Gly Ser

435                    440

<210> 68
<211> 1332
<212> DNA
<213> Zea mays

<400> 68
atgggtccga ccccgaccgc aacagcagcc ggtgcagcag ttgcagcagc aagcgcagca      60

gaacaggcag catttcgtct ggttggtcat cgtaattttg ttcgttttaa tccgcgttcc     120

gatcgttttc ataccctggc atttcatcat gttgaactgt ggtgtgcaga tgcagcatca     180

gcagcaggtc gttttagctt tgcactgggt gcaccgctgg cagcacgtag cgatctgagc     240

accggtaata gcgcacatgc aagtctgctg ctgcgtagcg gtagcctgag ctttctgttt     300

accgcaccgt atgcacatgg tgccgatgca gccaccgcag cactgccgag ctttagcgca     360

gccgcagccc gtcgttttgc agcagatcat ggtctggcag ttcgtgcagt tgcactgcgt     420

gttgcagatg ccgaagatgc atttcgtgca agcgttgcag cgggtgcacg tccggcattt     480

ggtccggttg atctgggtcg tggttttcgc ctggccgaag tggaactgta tggtgatgtt     540

gttctgcgtt atgttagcta tccggatggt gccgcaggcg aaccgtttct gcctggtttt     600

gaaggtgttg caagtccggg tgcagccgat tatggcctga gccgttttga tcatattgtt     660

ggtaatgtgc cggaactggc accggcagca gcatattttg caggttttac cggttttcat     720

gaatttgccg aatttaccac cgaagatgtt ggcaccgcag aaagcggtct gaatagcatg     780

gcactggcaa ataatagcga aaatgtactg ctgccgctga atgaaccggt gcatggcacc     840

aaacgtcgta gccagattca gacctttctg gatcatcatg gtggtccggg tgttcagcat     900

attgcactgg catcagatga tgtgctgcgt accctgcgtg aaatgcaggc acgtagtgca     960

atgggtggct ttgaatttct gcctccgcct ccgagcgatt attatgatgg tgttcgtcgt    1020

gaagccggtg atgttctgac cgaagcacag attaatgaat gtcaagaact gggtgttatg    1080

gtggatcgtg atgatcaggg tgtcctgctg cagattttta ccaaaccggt tggtgatcgt    1140

ccgacctttt ttctggaaat tattcagcgt attggctgca tggaaaaaga tgaaaaaggc    1200

caagaatatc agaaaggtgg ttgtggtggt tttggcaaag ataactttag ccagctgttt    1260

aaaagcatcg aggattatga aaaaagcctg gaagcaaaac aagccgcagc agccgcaacc    1320

gcacagggta gc                                                        1332


<210> 69
<211> 1332
<212> DNA
<213> Zea mays

<400> 69
atgggtccga ccccgaccgc aacagcagcc ggtgcagcag ttgcagcagc aagcgcagca      60

gaacaggcag catttcgtct ggttggtcat cgtaattttg ttcgttttaa tccgcgtagc 120

gatcgttttc agaccctggc atttcatcat gttgaactgt ggtgtgcaga tgcagcatca 180

gcagcaggtc gttttagctt tgcactgggt gttccgctgg cagcacgtag tgatctgagc 240

accggtaata gcgcacatgc aagtctgctg ctgcgtagcg gtagcctgag cctgctgttt 300

accgcaccgt atgcacatgg tgccgatgca gccaccgcag cactgccgag ctttagcgca 360

gccgcagccc gtcgttttgc agcagatcat ggtctggcag ttcgtgcagt tgccctgcgt 420

gttgcagatg ccgaagatgc atttcgtgca agcgttgcag cgggtgcacg tccggcattt 480

ggtccggttg atctgggtcg tggttttcgc ctggccgaag tggaactgta tggtgatgtt 540

gttctgcgtt atgttagcta tccggatggt gccgcaggcg aaccgtttct gcctggtttt 600

gaaggtgttg caagtccggg tgcagccgat tatggcctga gccgttttga tcatattgtt 660

ggtaatgtgc cggaactggc accggcagca gcatatatgg caggttttac cggttttcat 720

gaatttgcag aatttaccac cgaagatgtt ggcaccaccg aaagcggtct gaatagcatg 780

gcactggcaa ataatagcga aaatgtactg ctgccgctga atgaaccggt gcatggcacc 840

aaacgtcgta gccagattca gacctttctg gatcatcatg gtggtccggg tgttcagcat 900

attgcactgg catcagatga tgtgctgcgt accctgcgtg aaatgcgtgc acgtagcgca 960

atgggtggct ttgaatttct gcctccgcct ctgagcgatt attatgatgg tgttcgtcgt 1020

tgtgccggtg atgttctgac cgaagcacag attaatgaat gtcaagaact gggtgtgatg 1080

gtggatcgtg atgatcaggg tgtcctgctg cagattttta ccaaaccggt tggtgatcgt 1140

ccgacctttt ttctggaaat tattcagcgt attggctgca tggaaaaaga tgaaaaaggc 1200

caagaatatc agaaaggtgg ttgtggtggt tttggcaaag gtaattttgg tcagctgttt 1260

aaaagcatcg aggactatga aaaaagcctg gaagcaaaac aagccgcagc agccgcaacc 1320

gcacagggta gc 1332